(19) Europäisches Patentamt
European Patent Office
Office européen des brevets

(11) EP 4 603 487 A1

(12) **EUROPEAN PATENT APPLICATION**
published in accordance with Art. 153(4) EPC

(43) Date of publication:
**20.08.2025 Bulletin 2025/34**

(21) Application number: **23896682.4**

(22) Date of filing: **24.11.2023**

(51) International Patent Classification (IPC):
***C07D 403/14*** *(2006.01)* ***C07D 239/24*** *(2006.01)*
***A61K 47/68*** *(2017.01)* ***A61K 35/00*** *(2006.01)*

(52) Cooperative Patent Classification (CPC):
**A61K 31/4745; A61K 35/00; A61K 39/395; A61K 45/00; A61K 47/54; A61K 47/65; A61K 47/68; A61P 35/00; C07D 239/24; C07D 403/14**

(86) International application number:
**PCT/CN2023/134038**

(87) International publication number:
**WO 2024/114531 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 CN 202211510297**
**08.12.2022 CN 202211573191**

(71) Applicant: **Sichuan Kelun-Biotech Biopharmaceutical Co., Ltd.**
**Chengdu, Sichuan 611138 (CN)**

(72) Inventors:
- **TIAN, Qiang**
  **Chengdu, Sichuan 611138 (CN)**
- **YUAN, Xiaoxi**
  **Chengdu, Sichuan 611138 (CN)**
- **ZHANG, Yitao**
  **Chengdu, Sichuan 611138 (CN)**
- **SONG, Hongmei**
  **Chengdu, Sichuan 611138 (CN)**
- **GE, Junyou**
  **Chengdu, Sichuan 611138 (CN)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

# (54) HETEROCYCLIC COMPOUND, AND PREPARATION METHOD THEREFOR AND USE THEREOF

(57) The present application relates to the field of medicine, and particularly relates to a drug linker compound. The drug may comprise a DNA topoisomerase inhibitor. A conjugate prepared from the drug linker compound, such as an antibody drug conjugate, has a better drug antibody coupling ratio, and has a good targeted killing effect on tumors such as lung cancer, melanoma, breast cancer, gastric cancer and colon cancer. In addition, further provided in the present application are a method for preparing the antibody drug-linker molecule, and the use thereof.

EP 4 603 487 A1

## Description

### TECHNICAL FIELD

The present invention relates to the field of compounds and, in particular, to a heterocyclic compound, which can be used to prepare conjugates, such as antibody-drug conjugates.

### BACKGROUND

In the field of anti-tumor drug research and development, ADCs have attracted global attention for their targeting. ADCs are a new type of anti-tumor drug formed by linking toxic drugs to antibodies and are usually composed of antibodies, toxic drugs and linkers. They take anti-tumor effects by relying on the targeting recognition of antibodies and the high activity of toxins. ADCs are more specific and effective than traditional small molecule drugs.

After ADCs enter the body, toxins are delivered to the tumor tissue through antibodies with antigen targeting. The ADCs bind to the antigen on the surface of tumor cells, and the cells engulf the ADCs which is then decomposed in lysosomes to release cytotoxins, destroy DNA or prevent cell division, and achieve the effect of killing cells.

The linkers ensure the stability of ADCs in the blood, and the toxins play a killing role after reaching the target. The toxins of ADCs mainly include microtubule inhibitors, DNA damaging agents and RNA polymerase inhibitors. The linkers also include various types of structures such as cleavable and non-cleavable types. The structures of toxic drugs and linkers are crucial to the efficacy and safety of ADCs. In view of this, the research and development of drug-linker compounds with excellent activity and safety is still a problem that needs to be solved in the art.

### SUMMARY

The present invention relates to a drug-linker compound, a preparation method therefor and use thereof, and the drug-linker compound can be used to form a conjugate with other molecules such as antibodies.

In one aspect, the present invention provides a drug-linker compound having a structure represented by formula G-M-[L-E-D]$_x$, wherein

G is a functional group or leaving group capable of reacting with a specific amino acid or glycosyl;

M, which is a joint linked to G, is

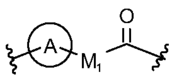,

ring A is a 5- to 6-membered aliphatic heterocycle or a 5- to 20-membered aromatic ring system, the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond and $C_{1-20}$ alkylene, $C_{2-20}$ alkenylene, $C_{2-20}$ alkynylene or amino; the $C_{1-20}$ alkylene, $C_{2-20}$ alkenylene, $C_{2-20}$ alkynylene or amino is optionally substituted by one or more suitable substituents;

L is a linker between the joint M and E, and L is selected from structures composed of one or more of the following: $C_{1-6}$ alkylene, -N(R')-, carbonyl, -O-, natural amino acids or non-natural amino acids and analogs thereof, short peptides consisting of amino acids,

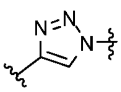, 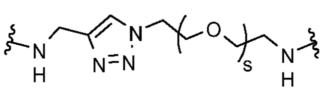, 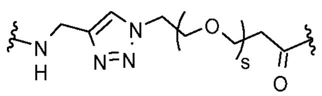, 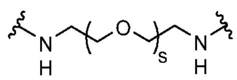,

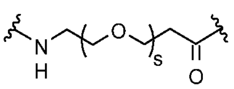, 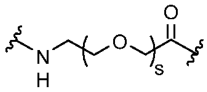, 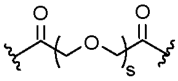, 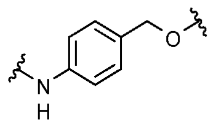, 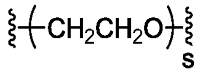 and

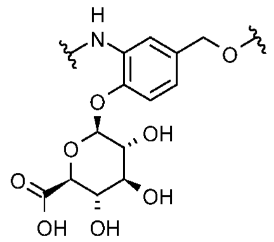;

wherein R' represents H, $C_{1-6}$ alkyl or a polyethylene glycol fragment containing 1-10 EO units; s is an integer from 1 to 20;

E is a structural fragment connecting L and D, wherein E is a single bond, $-NHCH_2-$or a structure selected from the group consisting of ;

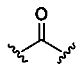, 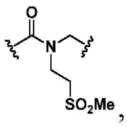, 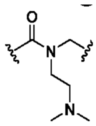 and 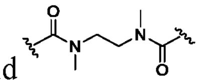

D is a cytotoxic drug fragment; and/or

x is selected from 1 to 10.

In some embodiments, the G is a functional group or leaving group capable of reacting with a specific amino acid or glycosyl in an antibody.

In some embodiments, the G is selected from the group consisting of halogen, halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, nitro, azido, cyano, alkenyl, alkynyl and an alkynyl-containing structural fragment, and the halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, alkenyl, alkynyl and an alkynyl-containing structural fragment are optionally substituted by one or more suitable substituents.

In some embodiments, the G is selected from the group consisting of halogen, halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, nitro, azido, cyano, alkenyl, alkynyl and an alkynyl-containing structural fragment.

In some embodiments, M is

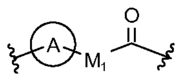,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, and the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, $C_{2-20}$ alkynylene or amino, and the $C_{1-20}$ alkylene, $C_{2-20}$ alkenylene, $C_{2-20}$ alkynylene or amino is optionally substituted by one or more suitable substituents.

In some embodiments, the M is

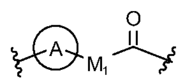,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, and the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, $C_{2-20}$ alkynylene or amino.

In some embodiments, M is

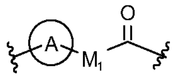,

wherein ring A is selected from the group consisting of

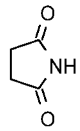, 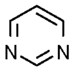, 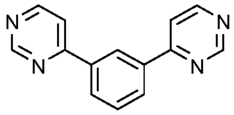, 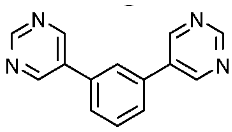, 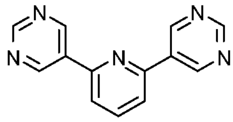,

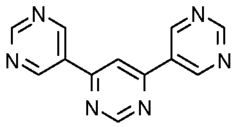 and 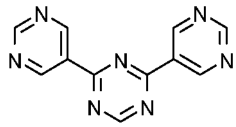;

$M_1$ is selected from the group consisting of a single bond, and $C_{1-6}$ alkylene, $C_{2-6}$ alkenylene, $C_{2-6}$ alkynylene or amino, and the $C_{1-6}$ alkylene, $C_{2-6}$ alkenylene, $C_{2-6}$ alkynylene or amino is optionally substituted by one or more suitable substituents.

In some embodiments, the M is selected from the group consisting of

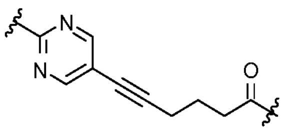 and 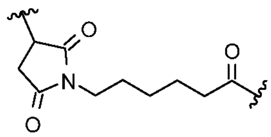;

In some embodiments, the M is

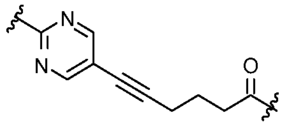.

In some embodiments, the M is selected from the group consisting of

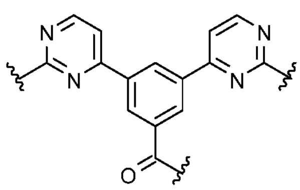, 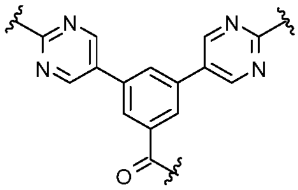, 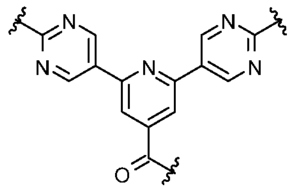,

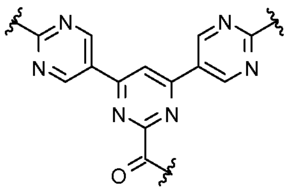, 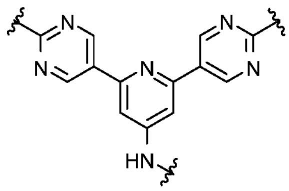 and 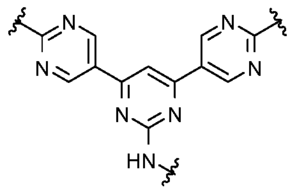

In some embodiments, the M is selected from the group consisting of

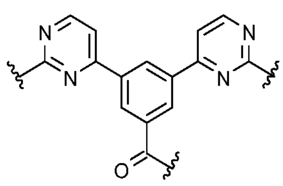, 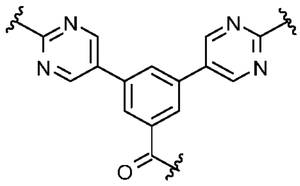 and 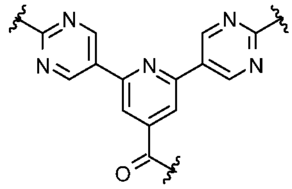

In some embodiments, the L is selected from structures composed of one or more of the following: $C_{16}$ alkylene, -N(R')-, carbonyl, -O-, Ala, Arg, Asn, Asp, Cit, Cys, Gln, Glu, Gly, His, Ile, Leu, Lys, Met, Phe, Pro, Ser, Thr, Trp, Tyr, Val, Lys($COCH_2CH_2(OCH_2CH_2)_rOCH_3$)), Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly),

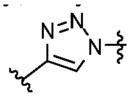, 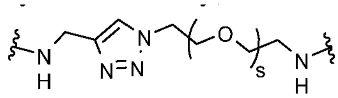,

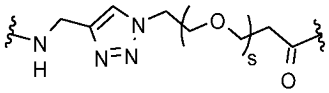, 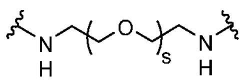, 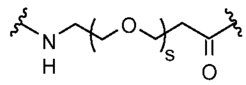, 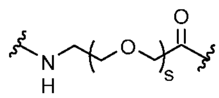,

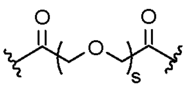, 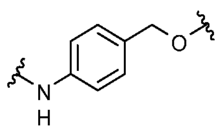, 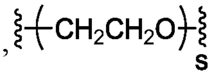 and 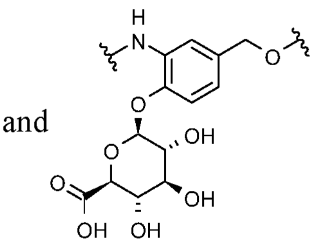;

wherein R' represents H, $C_{1-6}$ alkyl or a polyethylene glycol fragment containing 1-10 EO units; s is an integer from 1 to 20, such as 1 to 15, 1 to 10, 1 to 9, 1 to 8, 1 to 7, 1 to 6, 1 to 5, 2, 3, 4, 5, 6, 7, 8, 9, 10, 11, 12, 13, 14, 15, 16, 17, 18, 19 or 20.

In some embodiments, the L is selected from structures composed of one or more of the following: $C_{1-6}$ alkylene, carbonyl, -NH-, Ala-Ala, Ala-Lys, Ala-Pro, Gly-Glu, Gly-Gly, Phe-Lys,Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly,

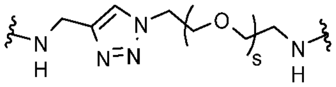, 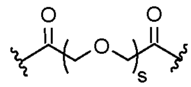,

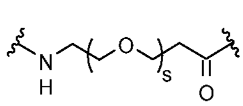, 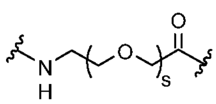 and 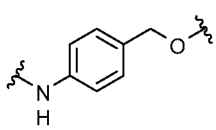;

wherein s is an integer from 1 to 20.

In some embodiments, the L is selected from structures composed of one or more of the following:

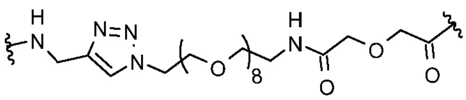, 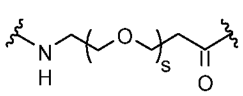, 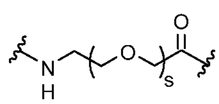,

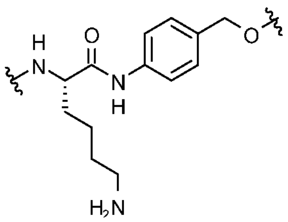, 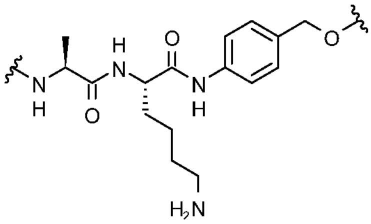, 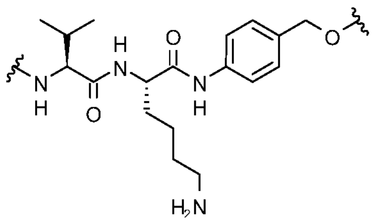,

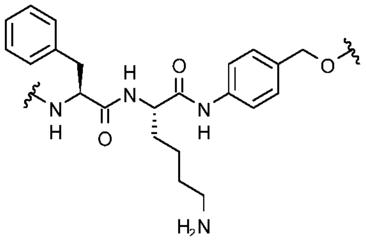, 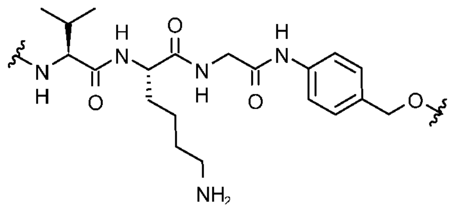,

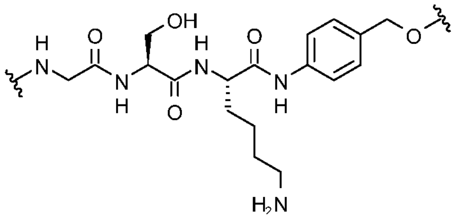, 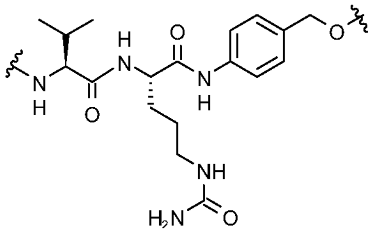,

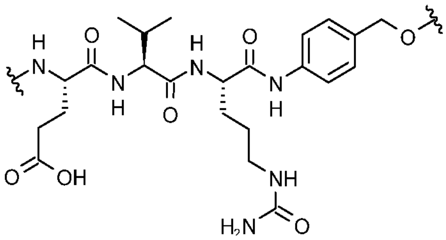, 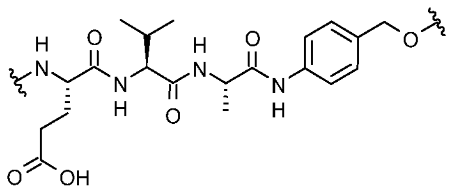,

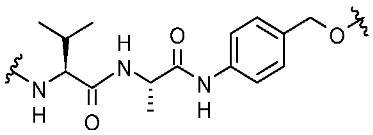, 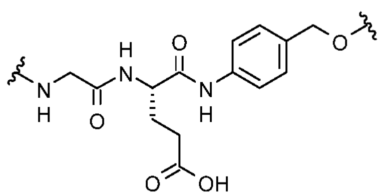, 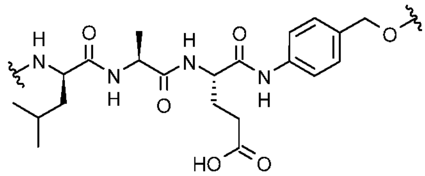,

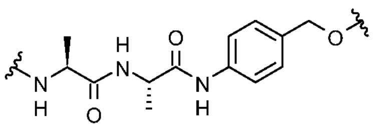, 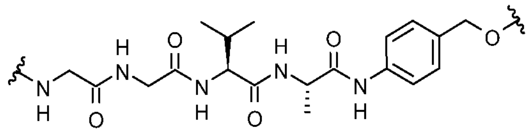,

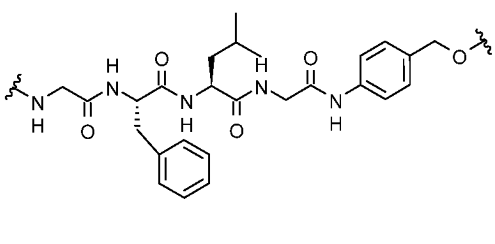, 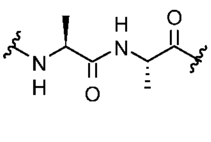, 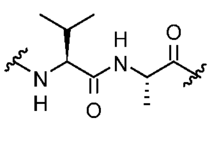, 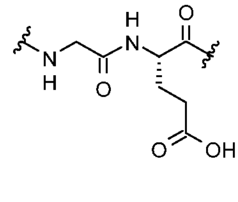,

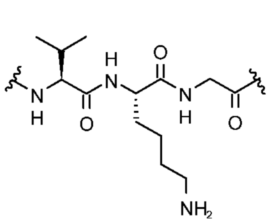, 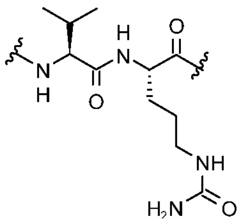, 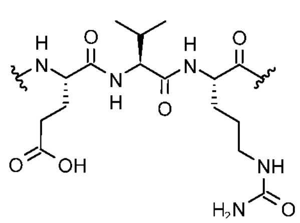, 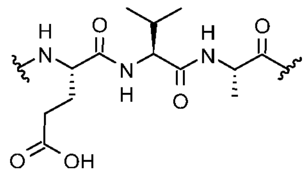,

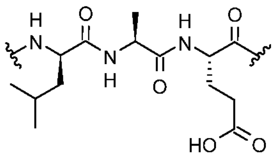, 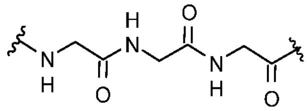, 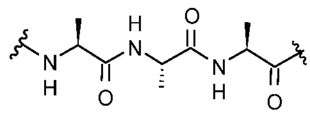,

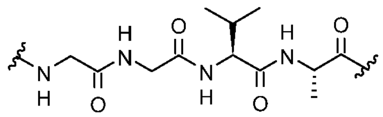, 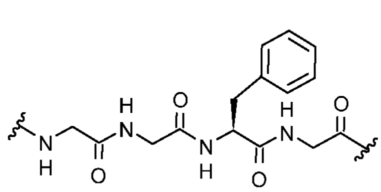 and 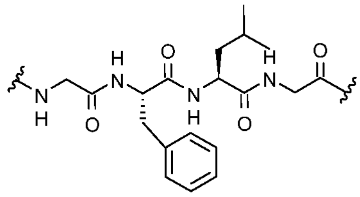.

In some embodiments, the L is a structure selected from the group consisting of:

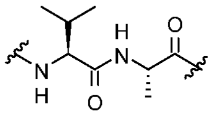, 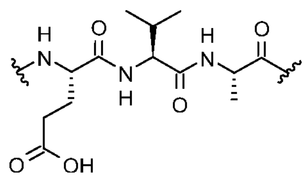, 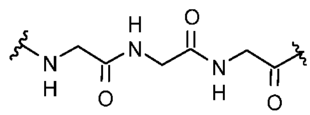,

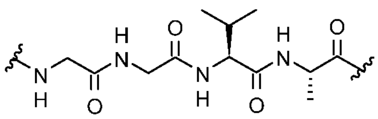, 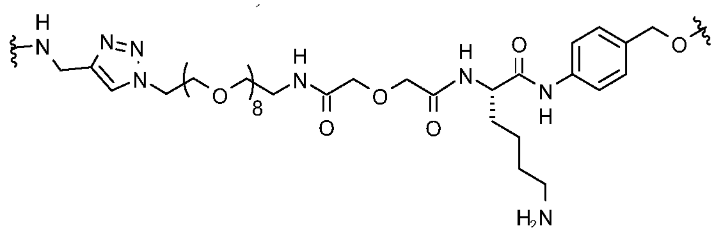,

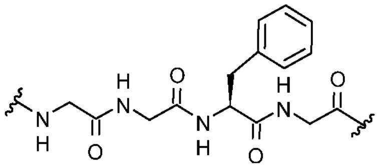, 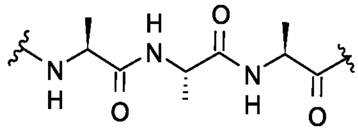,

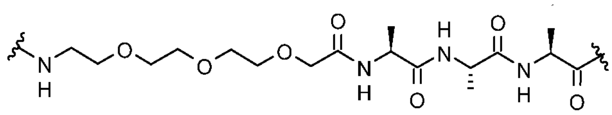

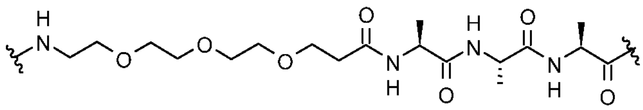,

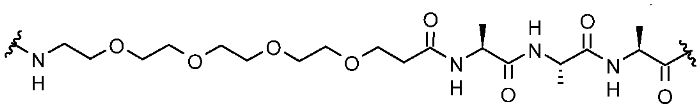

,

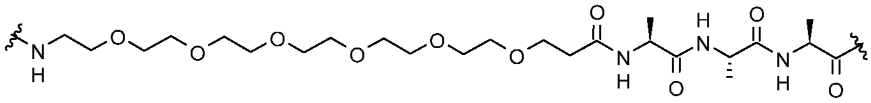

and

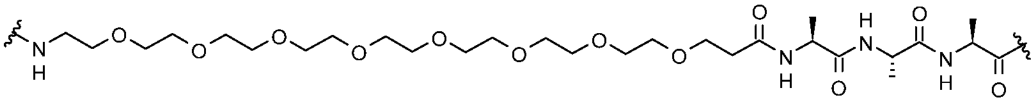

.

In some embodiments, the L is a structure selected from the group consisting of:

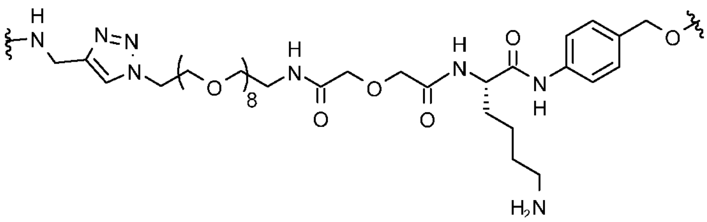

,

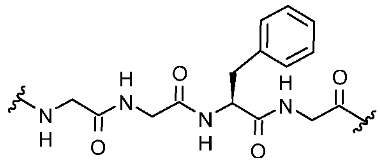

and

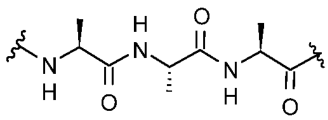

.

In some embodiments, the L is a structure selected from the group consisting of:

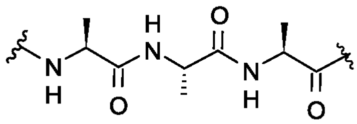

.

In some embodiments, the L is a structure selected from the group consisting of:

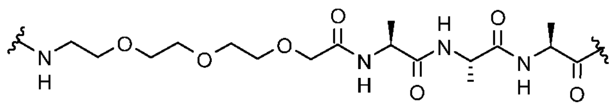

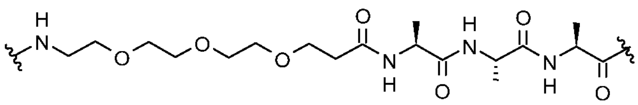

and

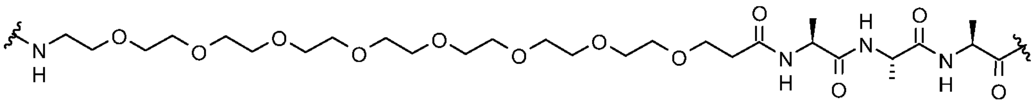

.

In some embodiments, the E is a single bond, $-NHCH_2-$,

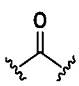

or

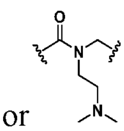

.

In some embodiments, the E is a single bond.

In some embodiments, the E is $-NHCH_2-$.

In some embodiments, the E is

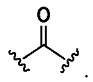

In some embodiments, the E is

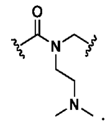

In some embodiments,

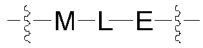

is a structure selected from the group consisting of:

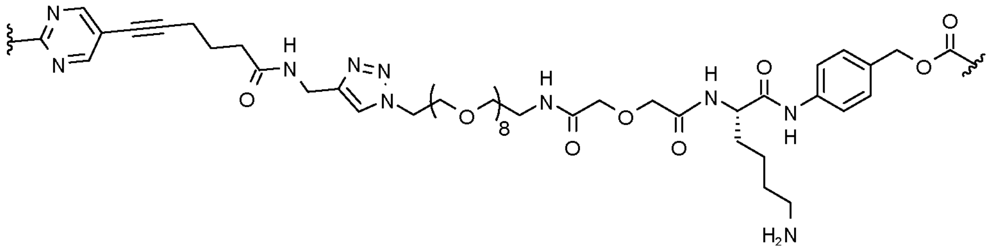

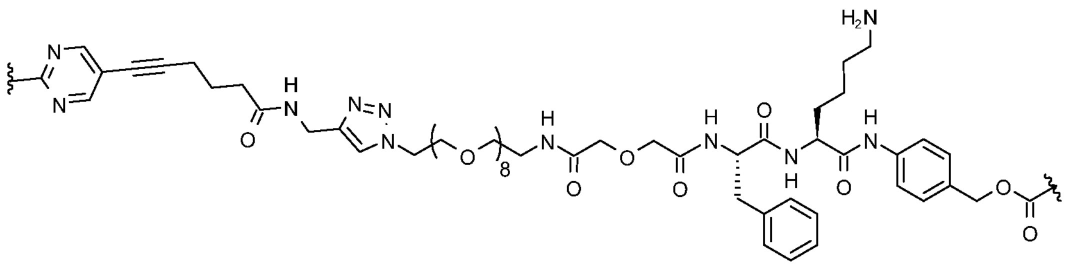

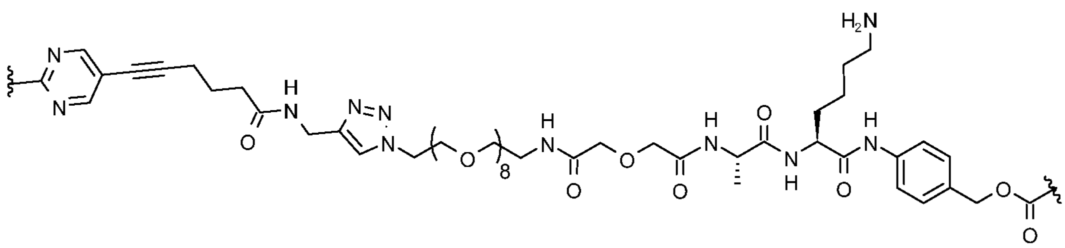

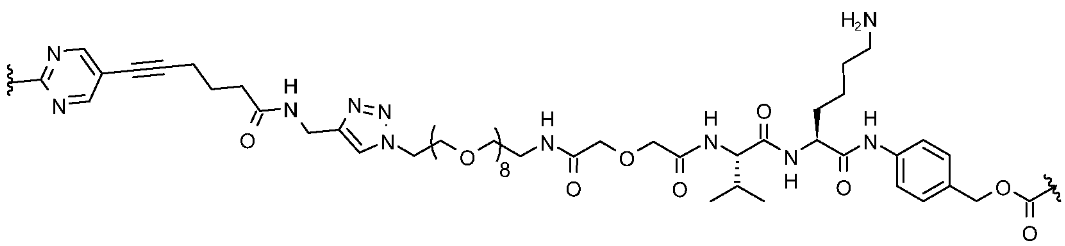

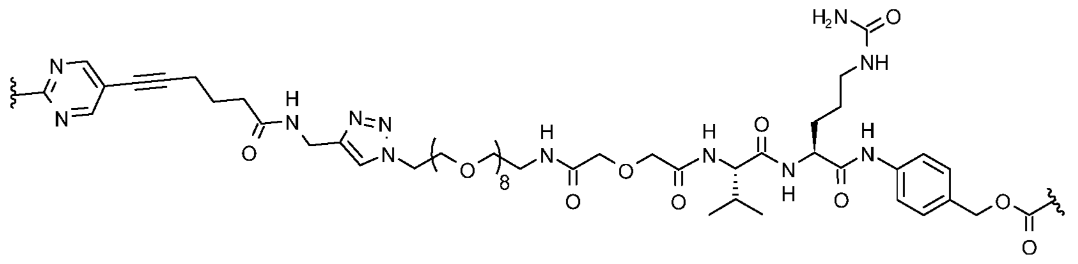

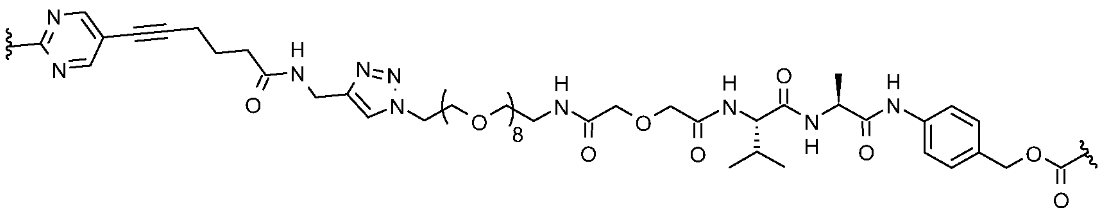

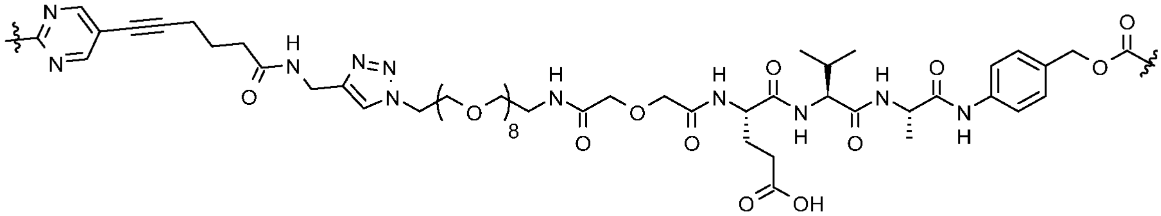

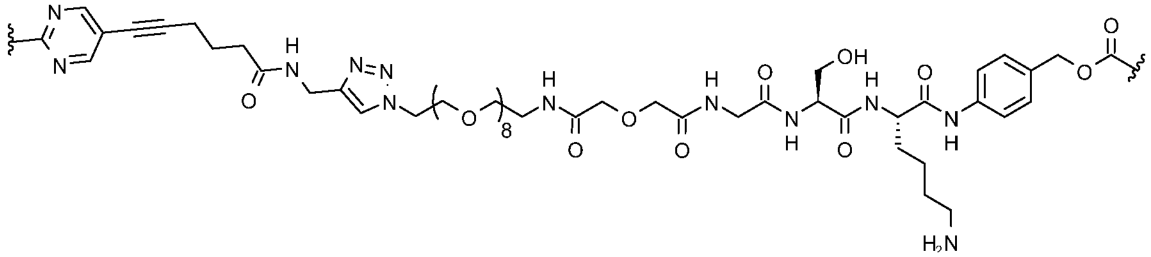

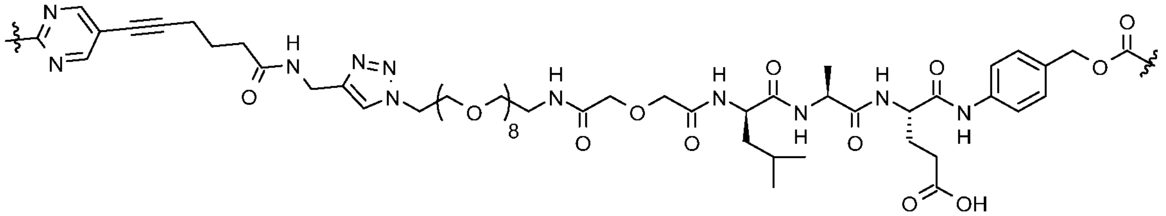

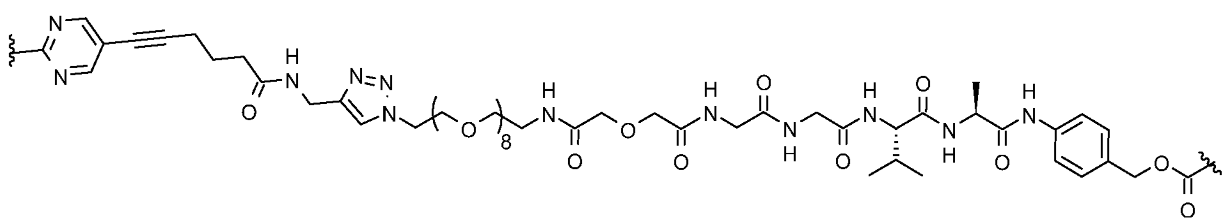

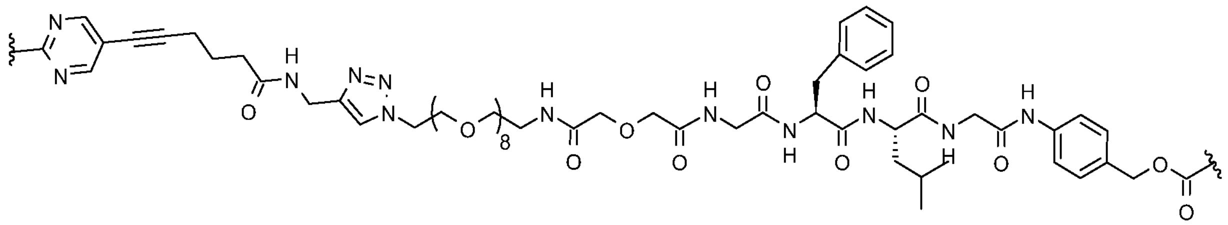

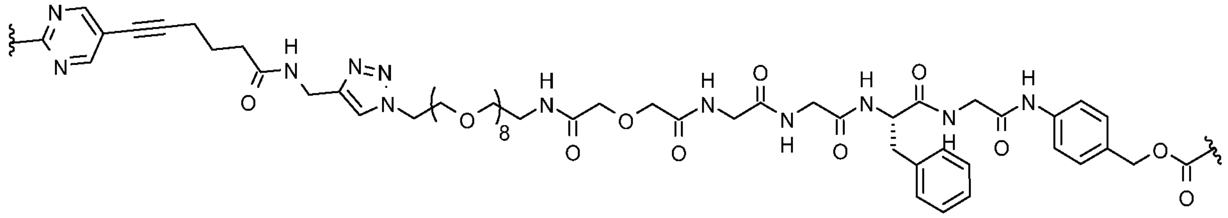

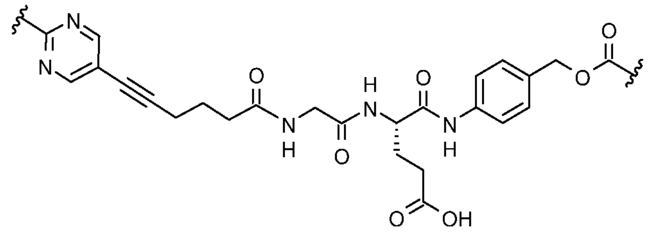

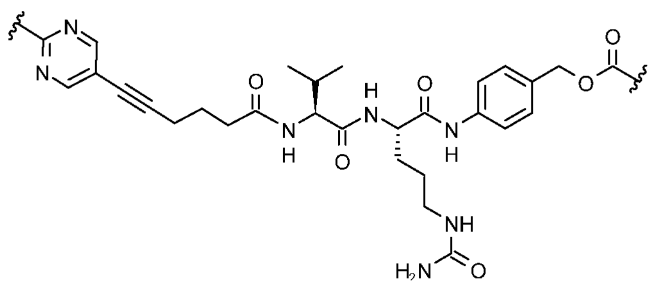

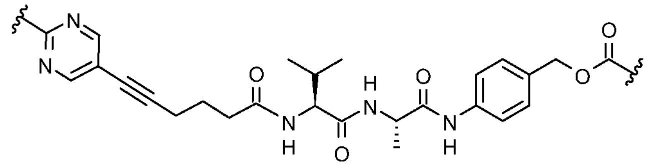

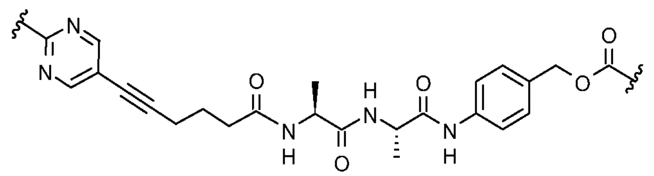

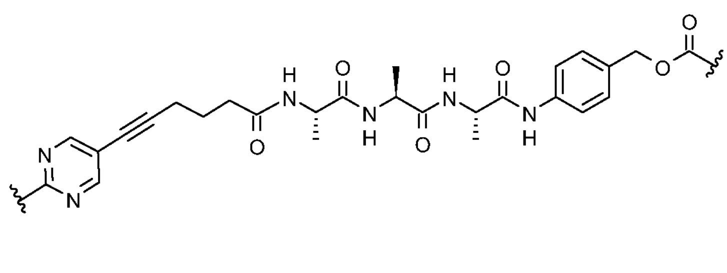

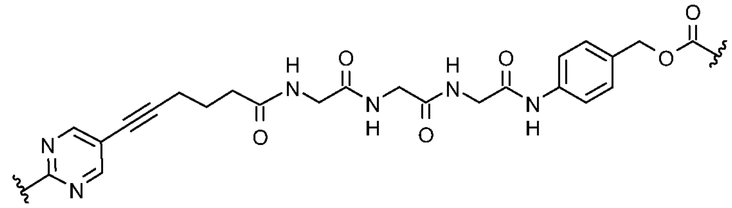

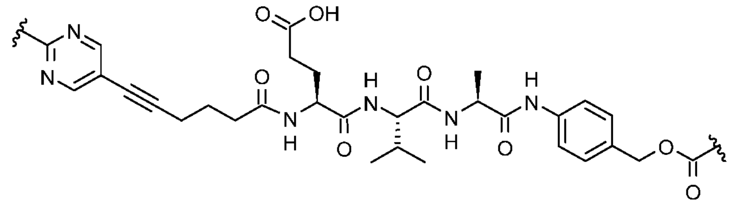

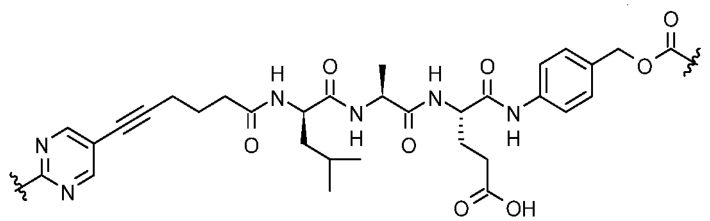

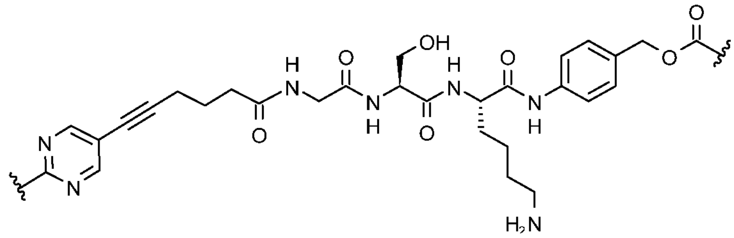

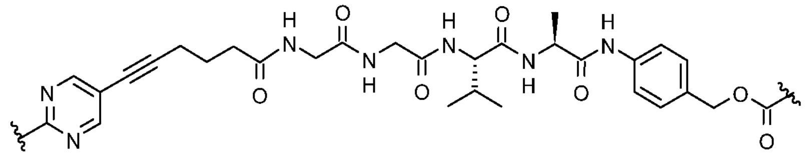

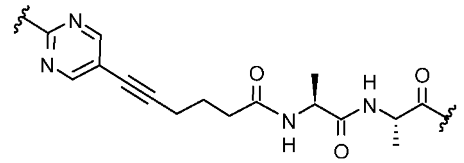

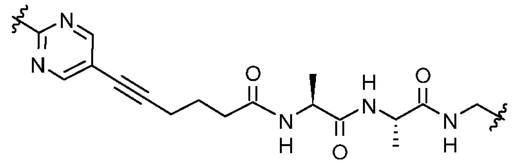

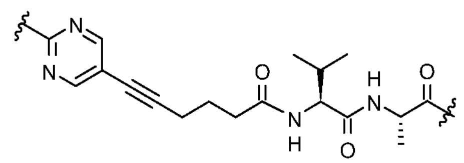

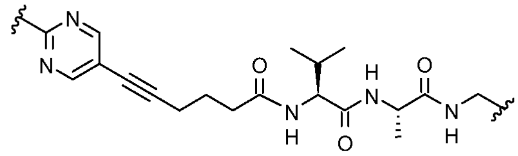

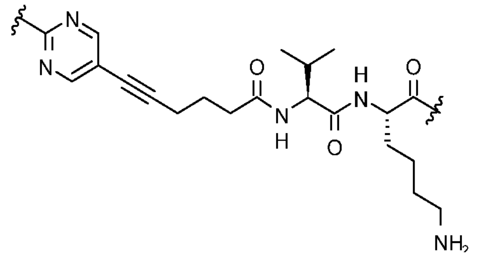

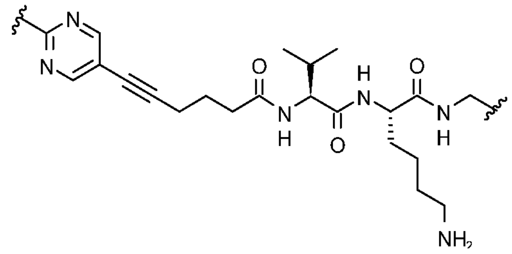

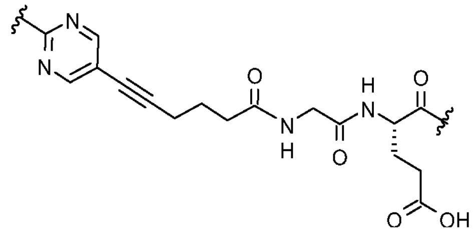

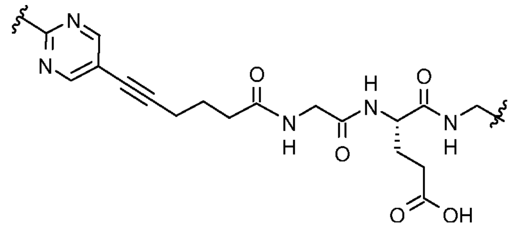

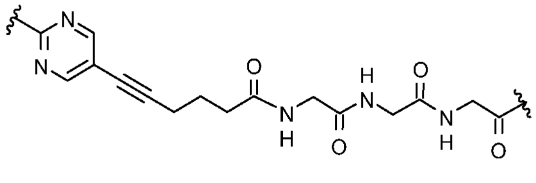

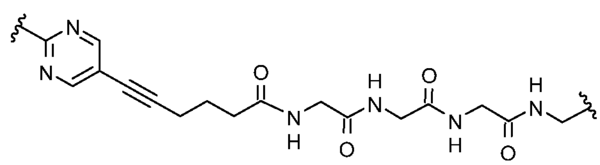

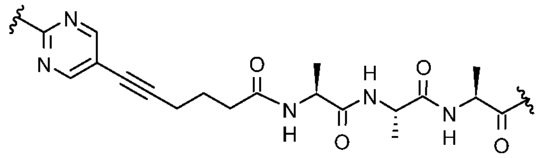

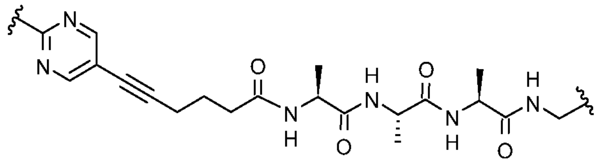

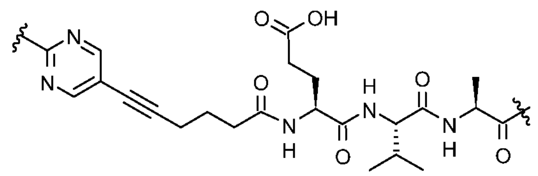

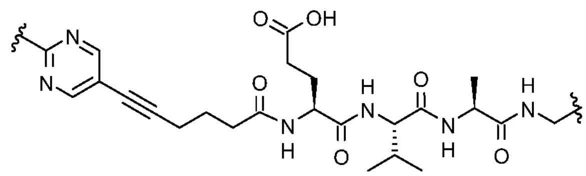

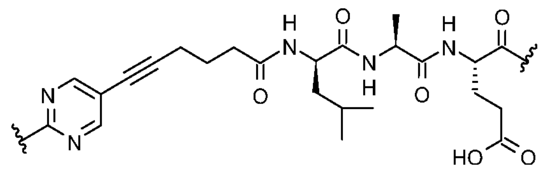

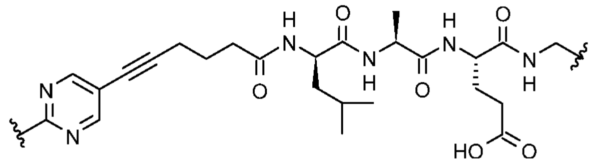

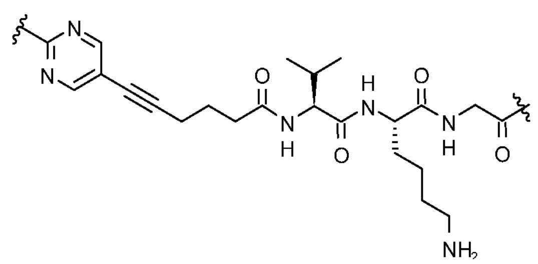

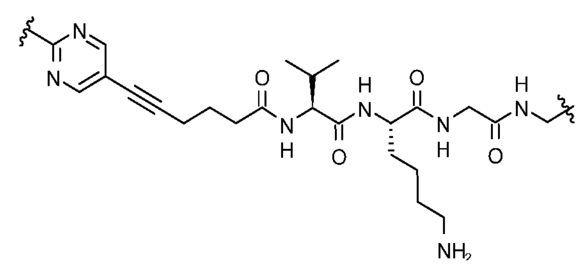

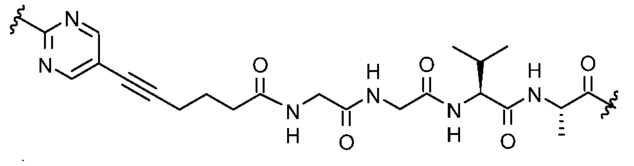

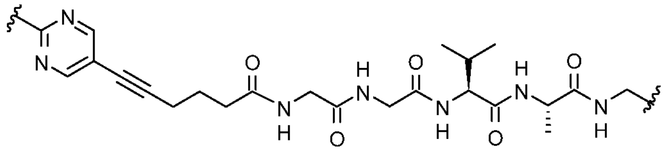

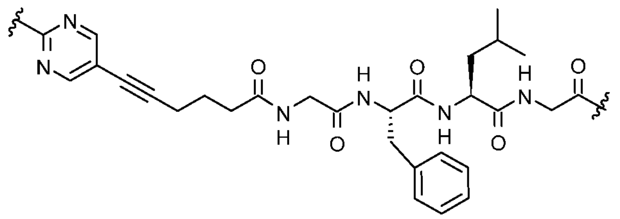

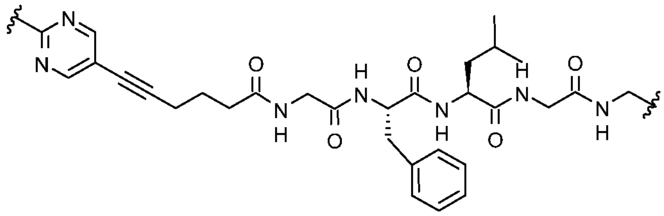

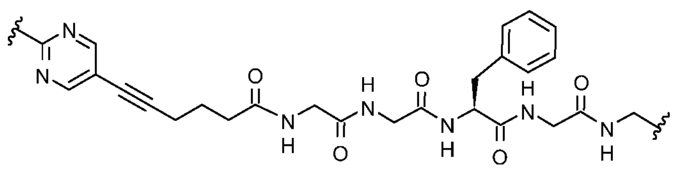

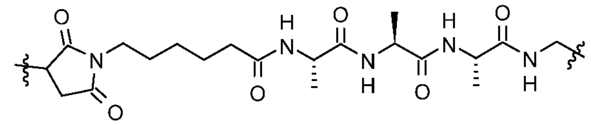

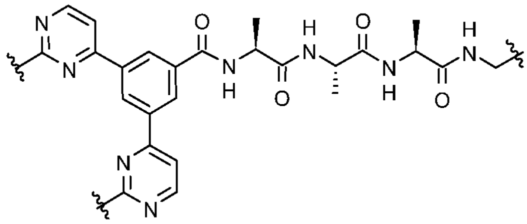

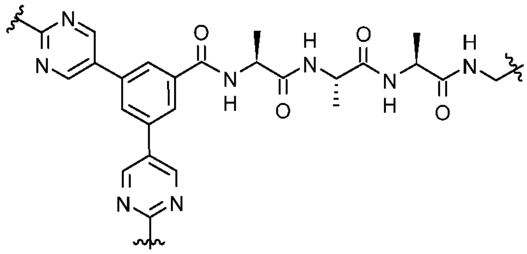

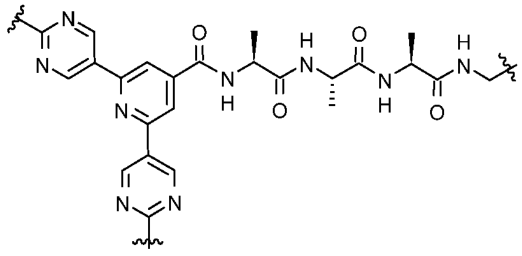

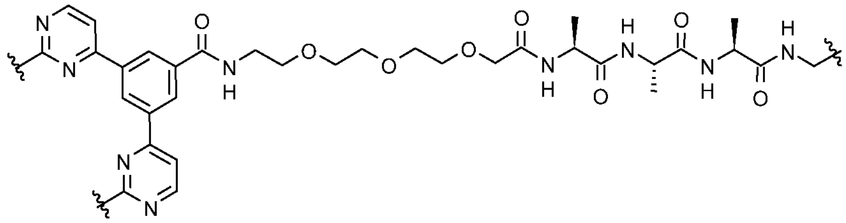

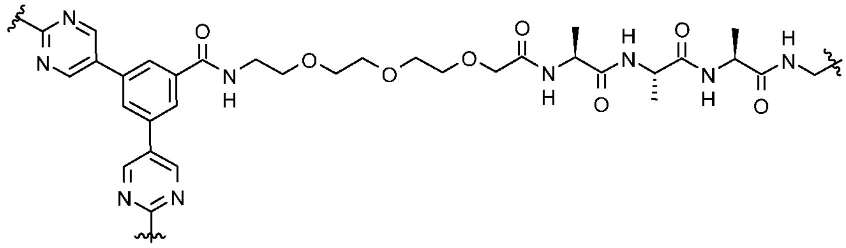

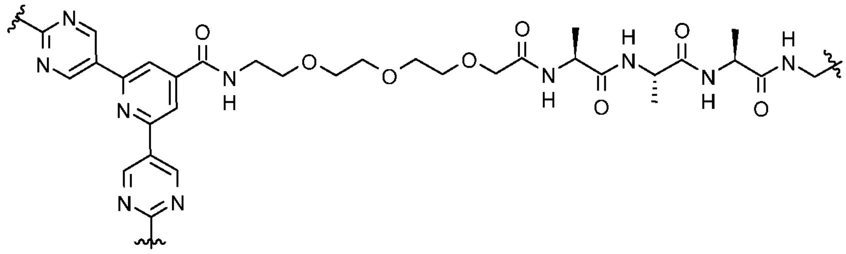

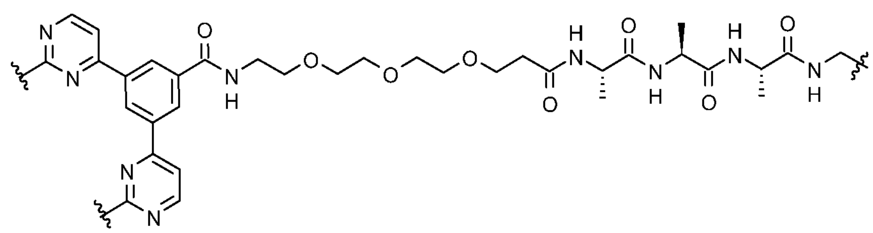

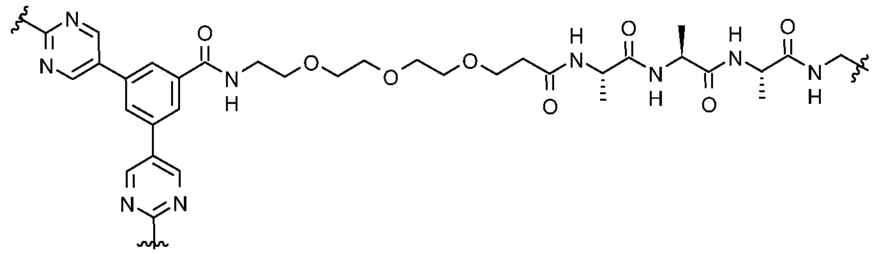

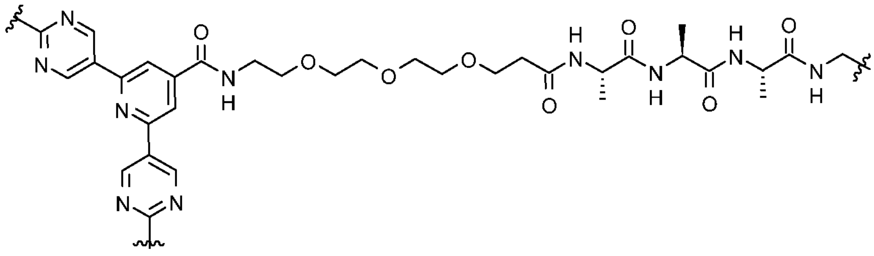

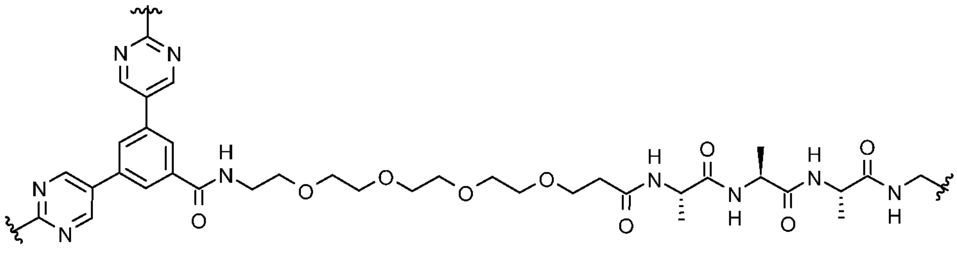

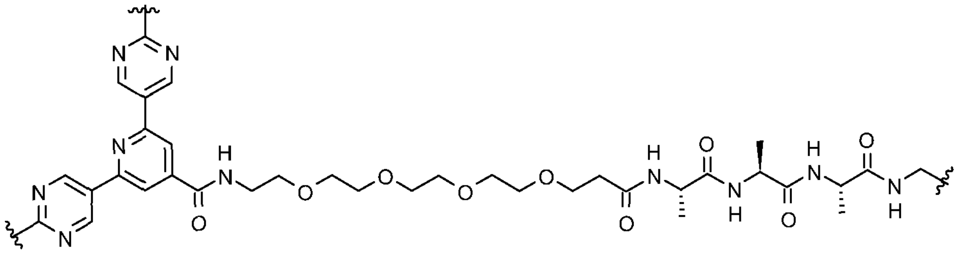

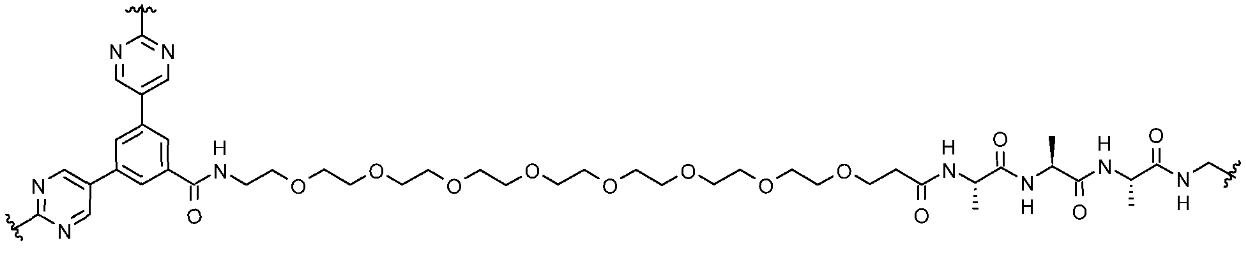

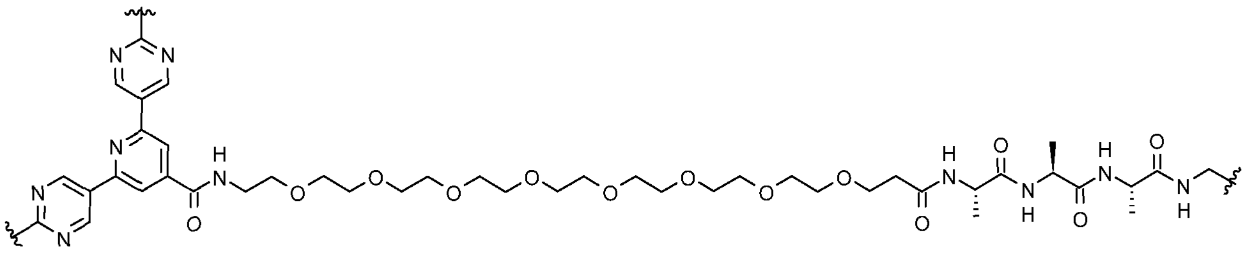

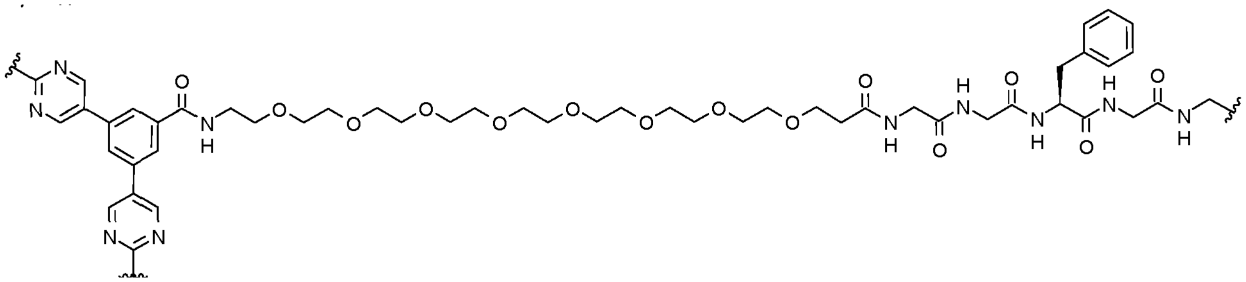

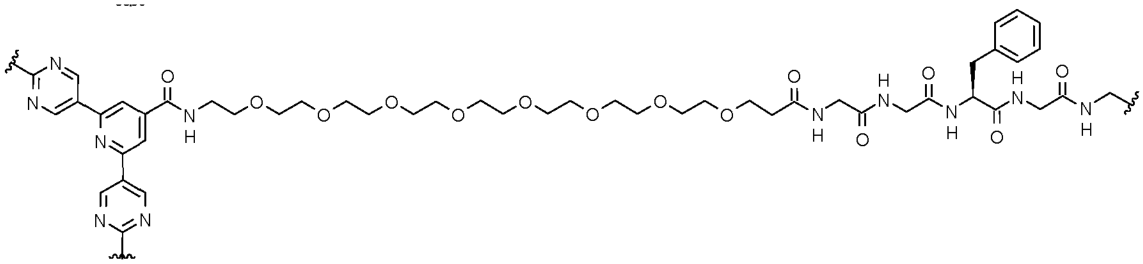

In some embodiments,

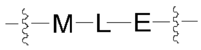

is a structure selected from the group consisting of:

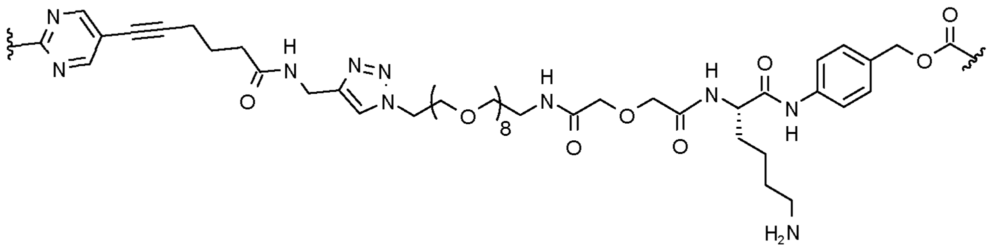

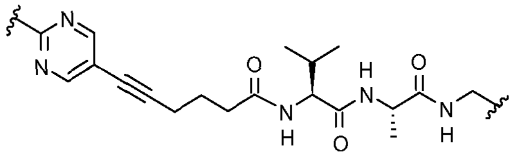

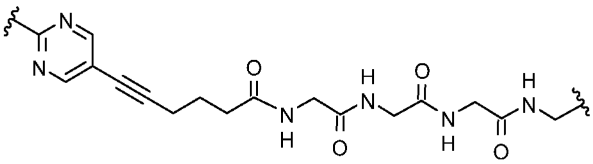

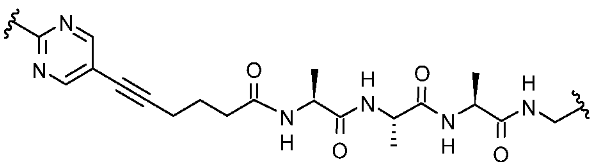

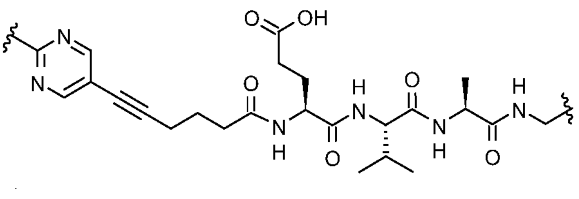

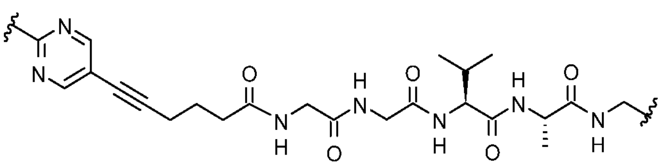

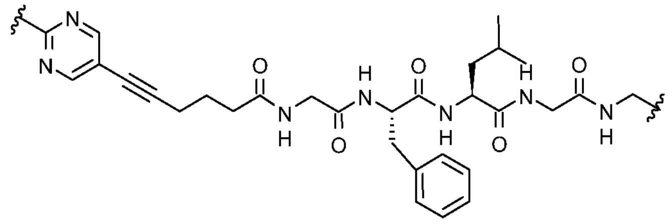

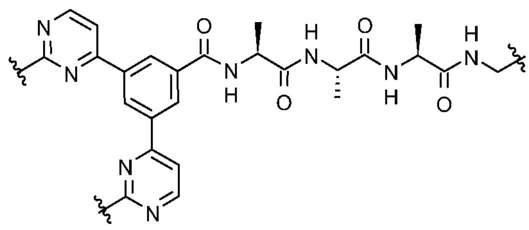

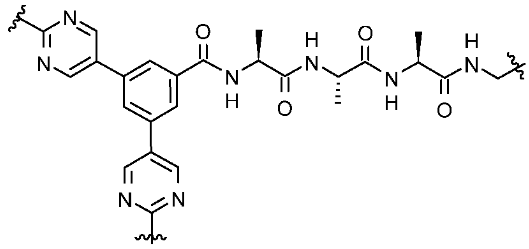

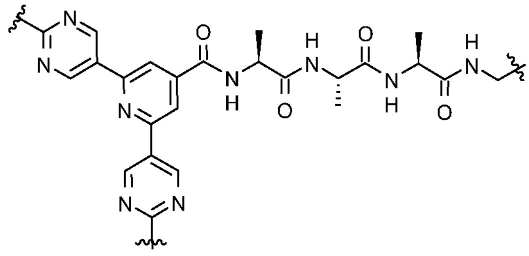

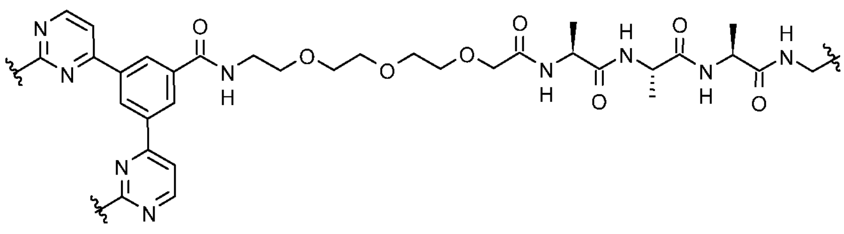

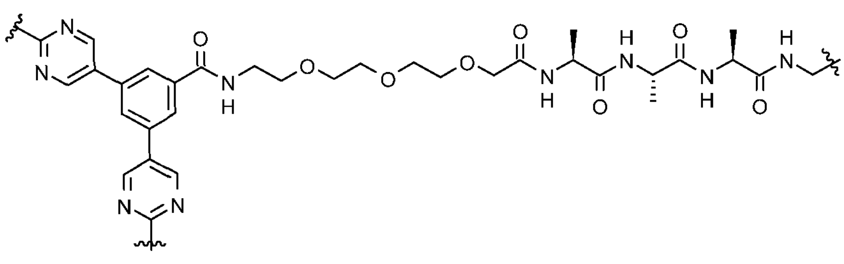

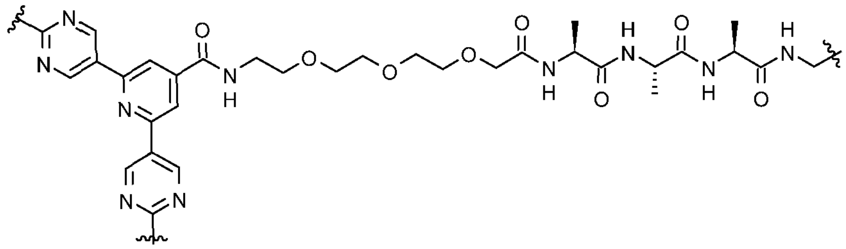

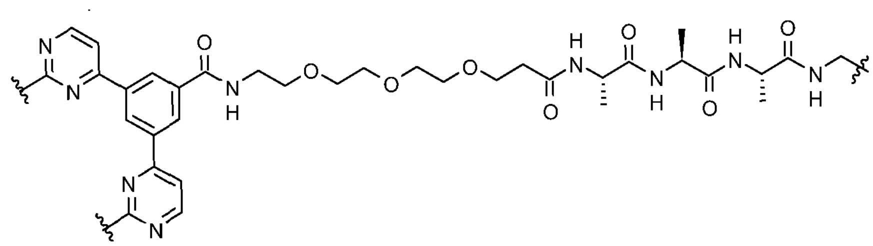

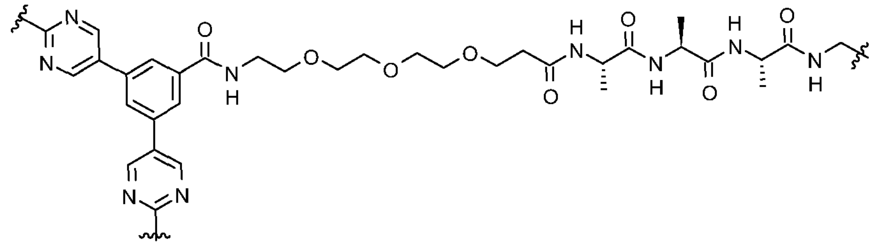

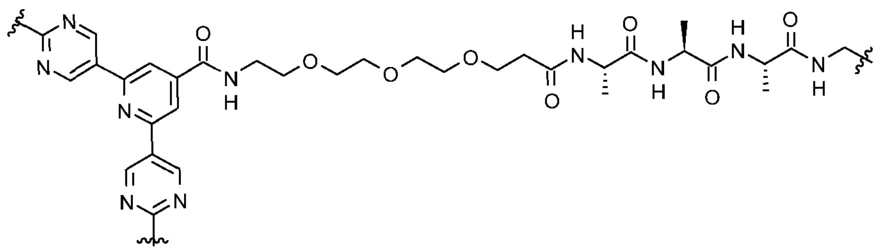

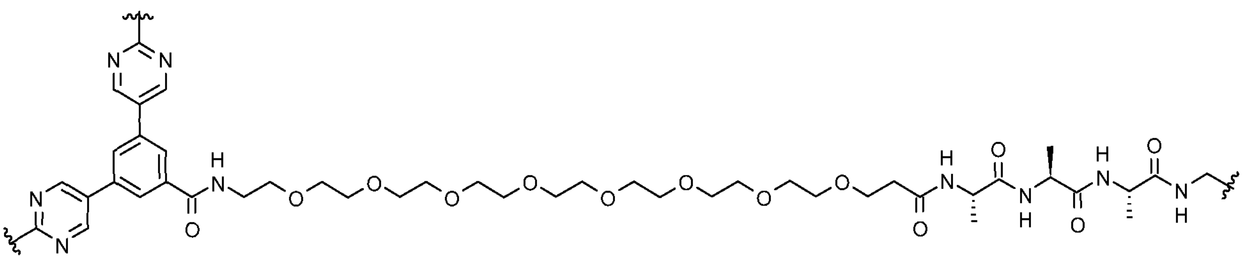

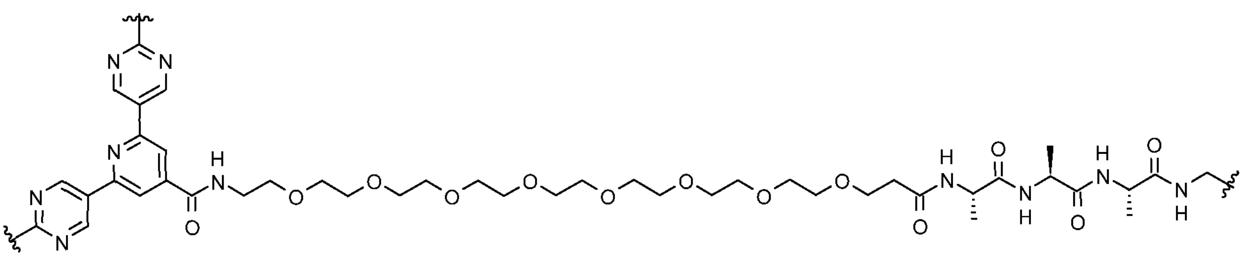

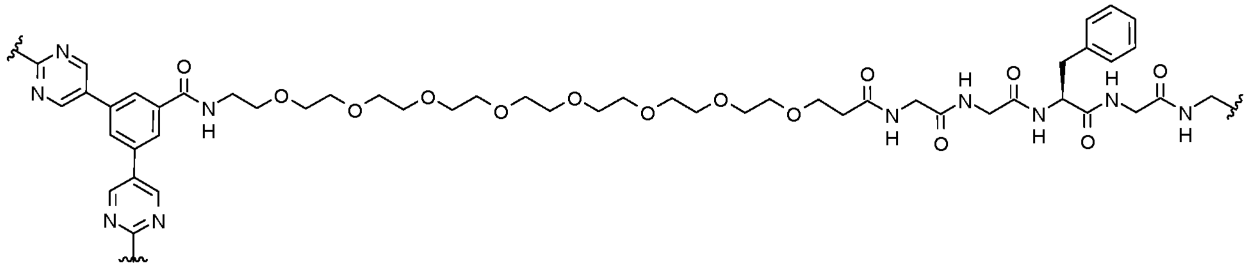

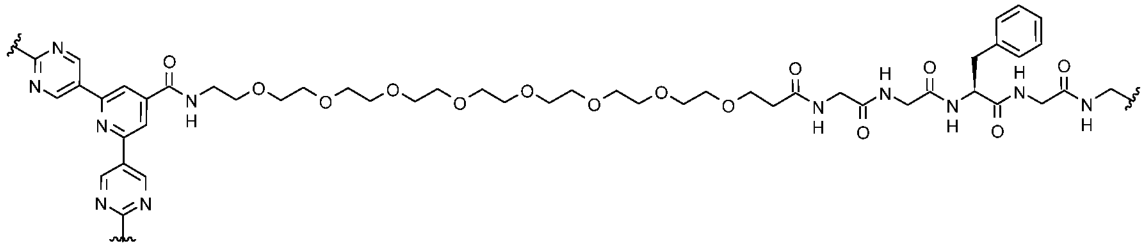

In some embodiments, the cytotoxic drug is selected from the group consisting of a tubulin inhibitor, a DNA intercalator, a DNA topoisomerase inhibitor and an RNA polymerase inhibitor.

In some embodiments, the tubulin inhibitor is an auristatin compound or a maytansine compound; the DNA intercalator is pyrrolobenzodiazepine (PBD); the DNA topoisomerase inhibitor is a topoisomerase I inhibitor (such as camptothecin, hydroxycamptothecin, 9-amino-camptothecin, SN-38, irinotecan, topotecan, belotecan, or rubitecan) or a topoisomerase II inhibitor (such as doxorubicin, PNU-159682, docamicin, daunorubicin, mitoxantrone, podophyllotoxin or etoposide); the RNA polymerase inhibitor is $\alpha$-amanitin or a pharmaceutically acceptable salt, ester or an analogue thereof.

The cytotoxic drug disclosed herein generally comprises a variety of functional groups, such as hydroxyl (-OH), carboxyl (-COOH), sulfhydryl (-SH), primary amino ($-NH_2$), secondary amino ($-NR_AH$) or tertiary amino ($-NR_BR_C$), wherein $R_A$, $R_B$ and $R_C$ here only represent non-hydrogen substituents on N, and the cytotoxic drug may be linked to the linker in the conjugate via these functional groups.

In some embodiments, the cytotoxic drug is linked to E in the antibody-drug conjugate via the -OH, -SH, primary amino, secondary amine or tertiary amino thereon.

In some embodiments, the cytotoxic drug is selected from the compounds of formula I and formula II or pharmaceutically acceptable salts, esters, stereoisomers, tautomers or prodrugs of the compounds of formula I and formula II:

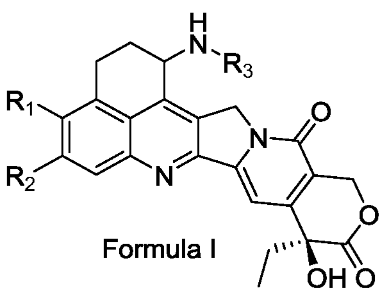

Formula I

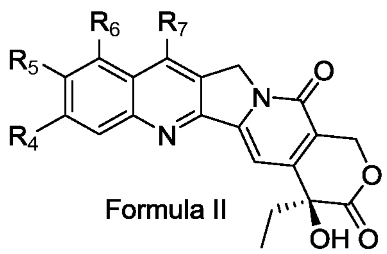

Formula II

wherein $R_1$ and $R_2$ are each independently selected from the group consisting of $C_{1-6}$ alkyl and halogen;

$R_3$ is selected from the group consisting of H and $-CO-CH_2OH$;

$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;

$R_6$ is selected from the group consisting of H or $-C_{1-4}$ alkylene-$NR_aR_b$;

$R_7$ is selected from the group consisting of $C_{1-6}$ alkyl and $-C_{1-4}$ alkylene-$NR_aR_b$;

wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence.

In some embodiments, the cytotoxic drug is selected from the following compounds or pharmaceutically acceptable salts, esters, stereoisomers, tautomers or prodrugs of the compounds:

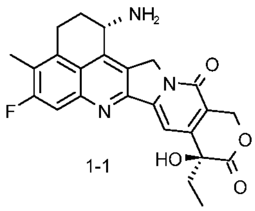
1-1

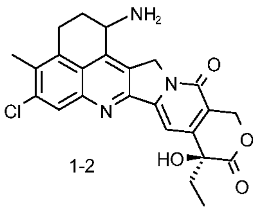
1-2

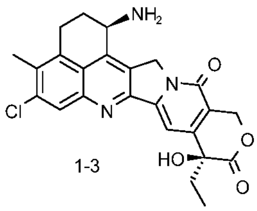
1-3

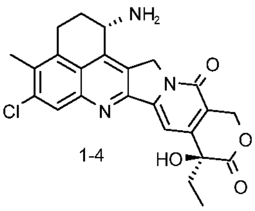
1-4

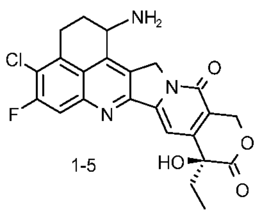
1-5

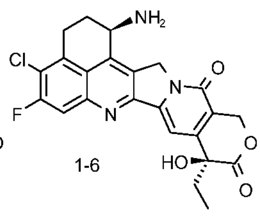
1-6

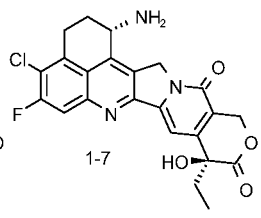
1-7

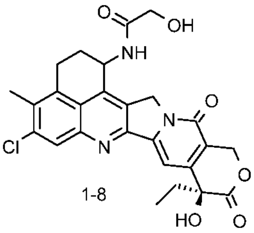
1-8

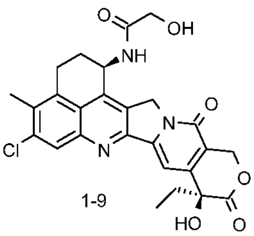
1-9

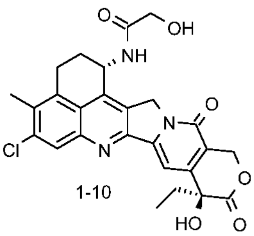
1-10

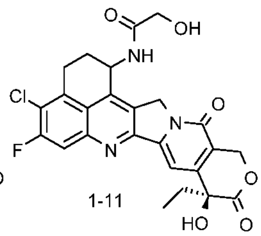
1-11

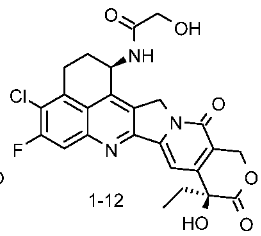
1-12

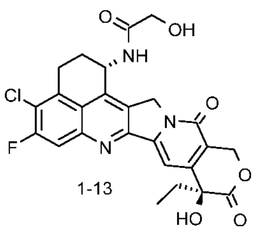
1-13

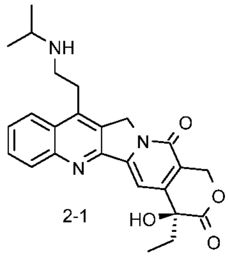
2-1

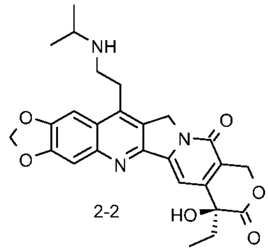
2-2

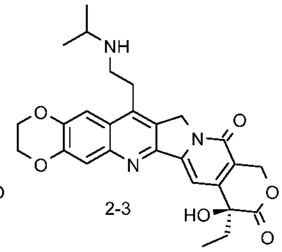
2-3

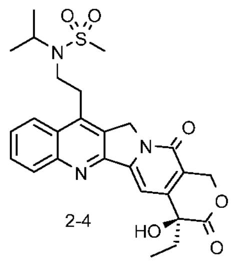
2-4

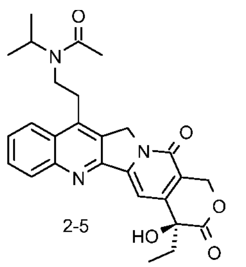
2-5

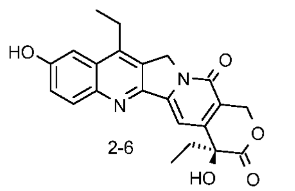
2-6

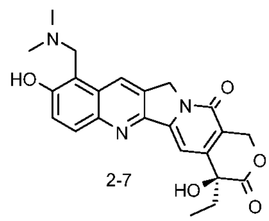
2-7

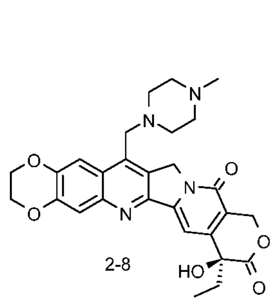
2-8

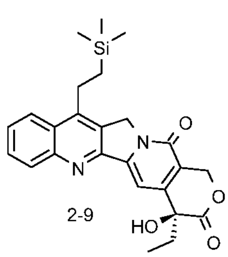
2-9

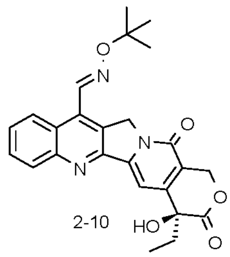
2-10

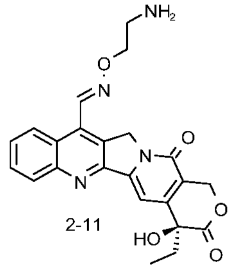
2-11

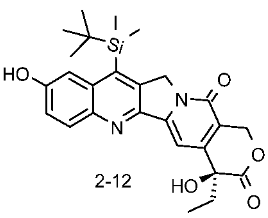
2-12
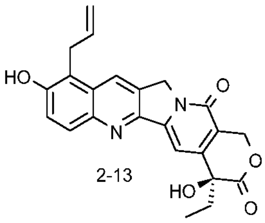
2-13
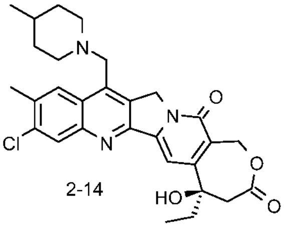
2-14
.

In some embodiments, the cytotoxic drug is selected from the following compounds or pharmaceutically acceptable salts, esters, stereoisomers, tautomers or prodrugs of the compounds:

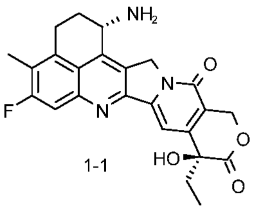
1-1
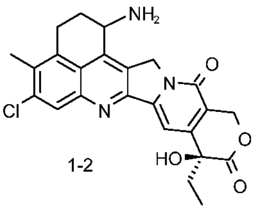
1-2
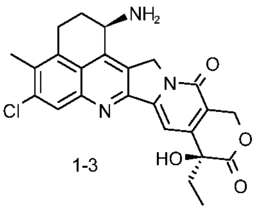
1-3
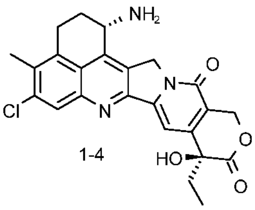
1-4
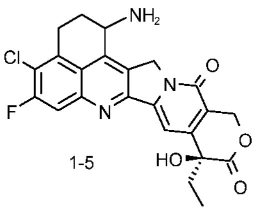
1-5
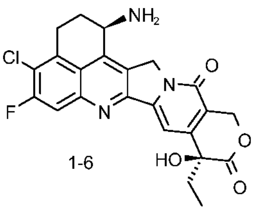
1-6
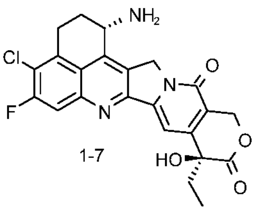
1-7
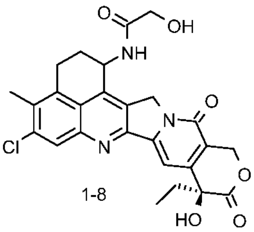
1-8
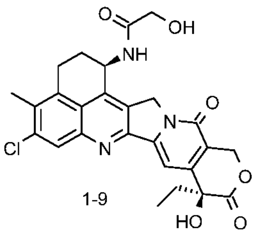
1-9
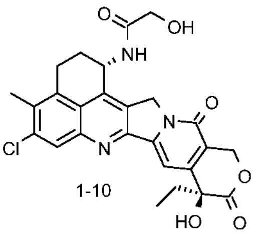
1-10
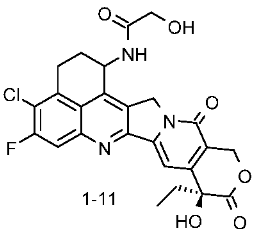
1-11
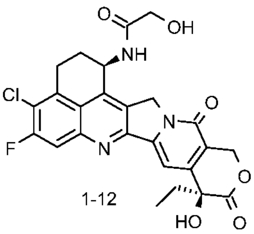
1-12
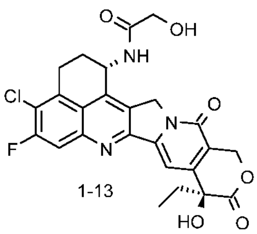
1-13
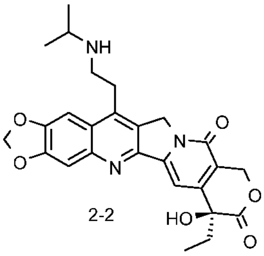
2-2
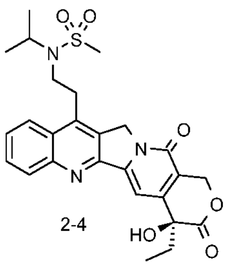
2-4
.

In some embodiments, the cytotoxic drug is selected from the following compounds or pharmaceutically acceptable salts, esters, stereoisomers, tautomers or prodrugs of the compounds:

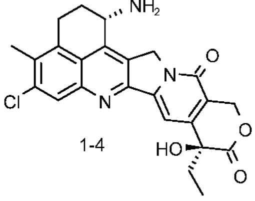
1-4
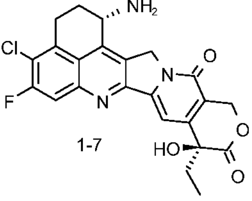
1-7
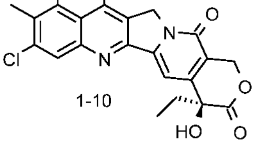
1-10
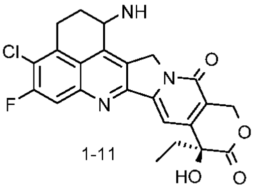
1-11

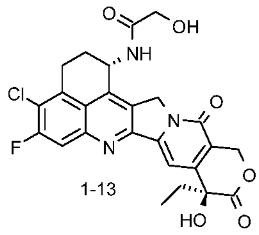
1-13
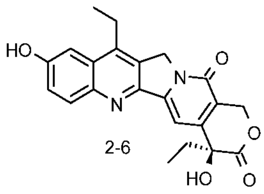
2-6
.

In some embodiments, the cytotoxic drug is selected from the following compounds or pharmaceutically acceptable salts, esters, stereoisomers, tautomers or prodrugs of the compounds:

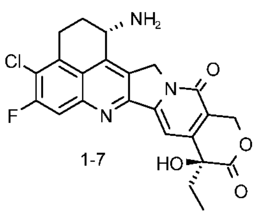
1-7
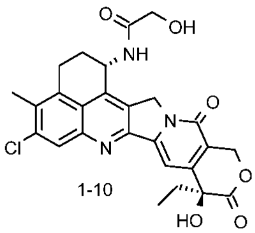
1-10
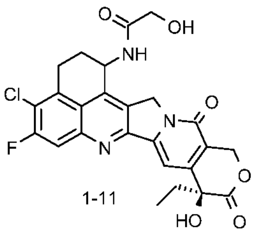
1-11

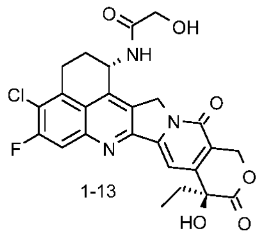
1-13
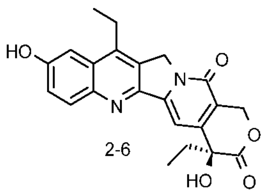
2-6
.

In some embodiments, the corresponding fragment of the cytotoxic drug obtained by linking the cytotoxic drug to the linker is D in the general formula; preferably, D is a monovalent structure obtained by losing one H from -OH, $-NH_2$ or a secondary amino on the cytotoxic drug.

In some embodiments, D is a structure selected from the group consisting of:

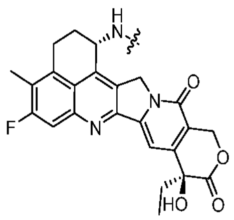
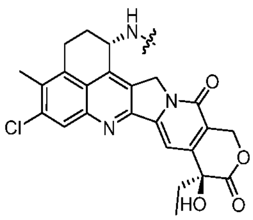
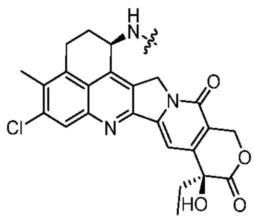
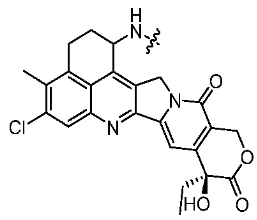

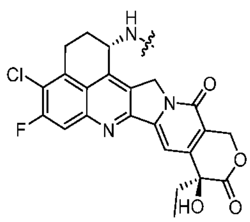
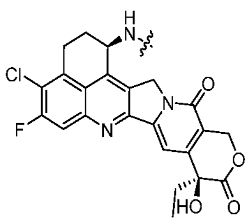
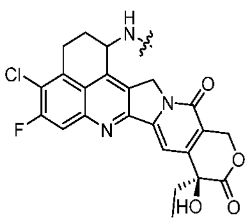
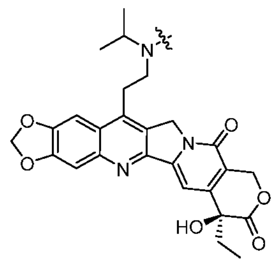

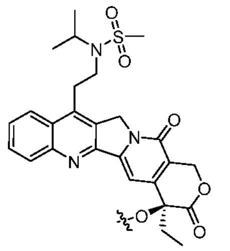
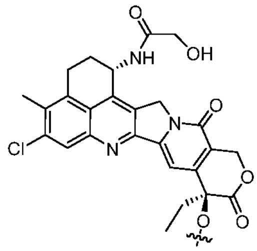
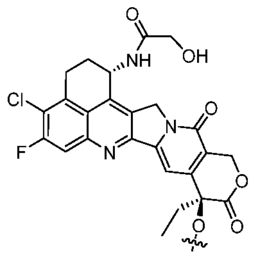
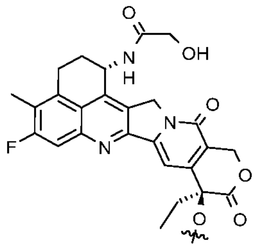

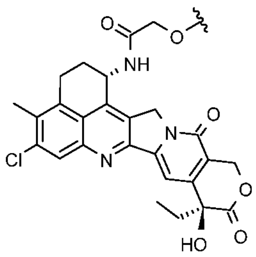
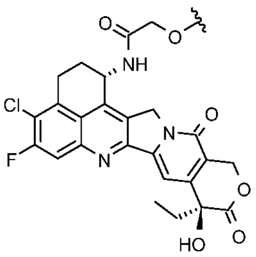
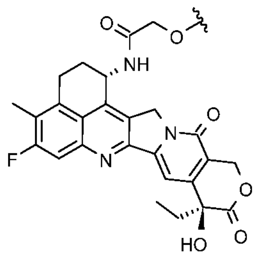.

In another aspect, the present invention provides a drug-linker compound having a structure represented by formula G-M-$[L-E-D]_x$, wherein

G is a functional group or leaving group capable of reacting with a specific amino acid or glycosyl in an antibody or an antigen-binding fragment; the G is preferably selected from the group consisting of halogen, halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, nitro, azido, cyano, alkenyl, alkynyl and an alkynyl-containing structural fragment;

M, which is a joint linked to G, is

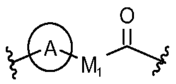,

wherein ring A is a 5- to 6-membered aliphatic heterocycle or a 5- to 20-membered aromatic ring system, and the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond and $C_{1-20}$ alkylene, $C_{2-20}$ alkenylene, $C_{2-20}$ alkynylene or amino;

preferably, M is

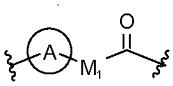,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, and the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, $C_{2-20}$ alkynylene or amino;

preferably, M is

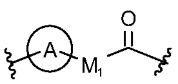,

wherein ring A is selected from the group consisting

of 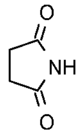, 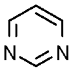, 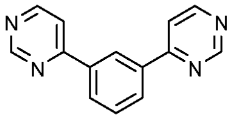, 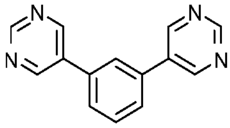, 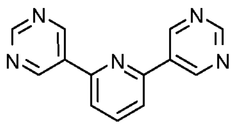, 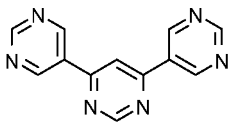

and

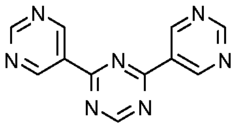;

$M_1$ is selected from the group consisting of a single bond and $C_{1-6}$ alkylene, $C_{2-6}$ alkenylene, $C_{2-6}$ alkynylene or amino; preferably, M is selected from the group consisting of

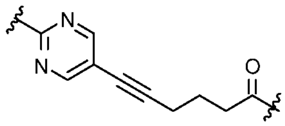

and

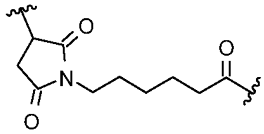;

preferably, M is

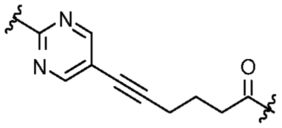;

preferably, M is selected from the group consisting of

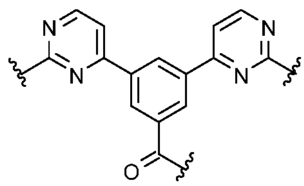,

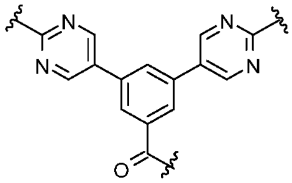, 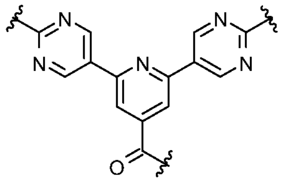, 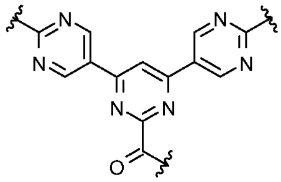, 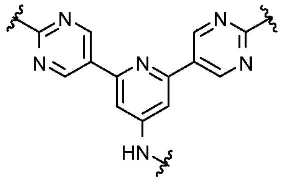

and

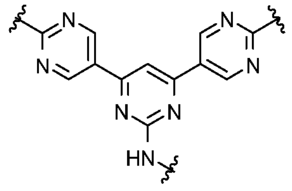;

preferably, M is selected from the group consisting of

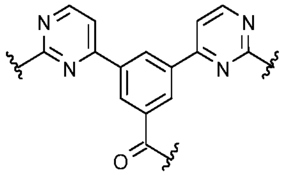,

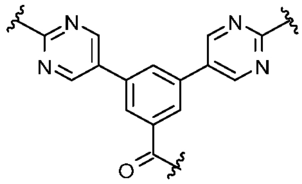 and 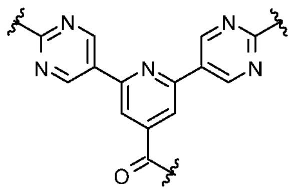

L is a linker between the joint M and E, and the L is selected from structures composed of one or more of the following: $C_{1-6}$ alkylene, -N(R')-, carbonyl, -O-, natural amino acids or non-natural amino acids and analogs thereof (such as Ala, Arg, Asn, Asp, Cit, Cys, Gln, Glu, Gly, His, Ile, Leu, Lys, Met, Phe, Pro, Ser, Thr, Trp, Tyr, Val, Lys($COCH_2CH_2(OCH_2CH_2)_rOCH_3$)), and short peptides composed of amino acids (such as Ala-Ala, Ala-Lys, Ala-Lys(Ac), Ala-Pro, Gly-Glu, Gly-Gly, Phe-Lys, Phe-Lys(Ac) , Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly),

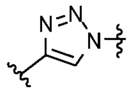, 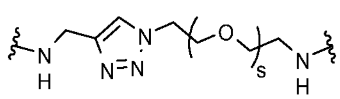, 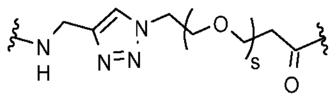,

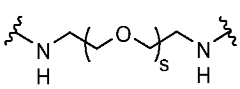, 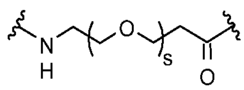, 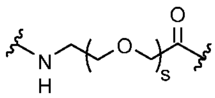, 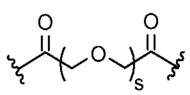,

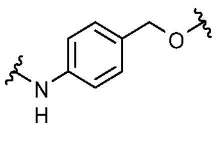, 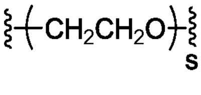 and 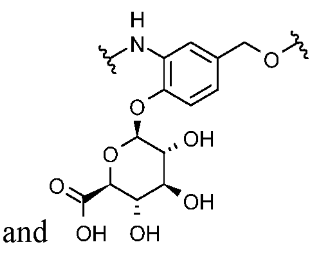;

wherein R' represents H, $C_{1-6}$ alkyl or a polyethylene glycol fragment containing 1-10 EO units; s is an integer from 1 to 20;

preferably, the L is selected from structures composed of one or more of the following: $C_{1-6}$ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly,

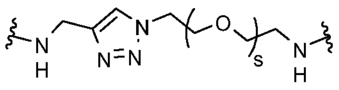 , 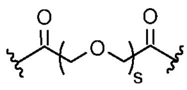 , 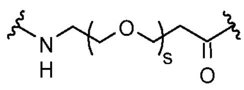 , 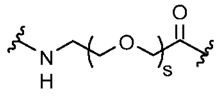

and

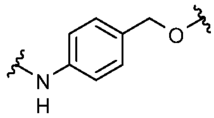 ;

wherein s is an integer from 1 to 20;
preferably, the L is selected from structures composed of one or more of the following:

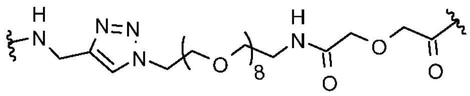 , 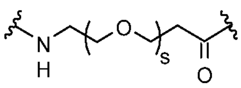 , 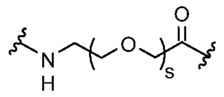 ,

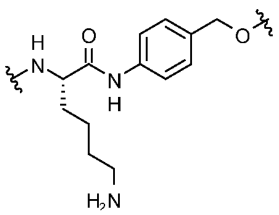 , 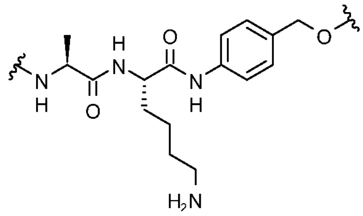 , 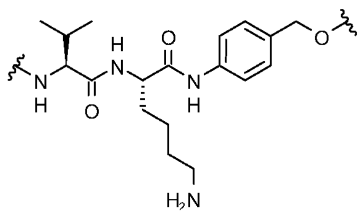 ,

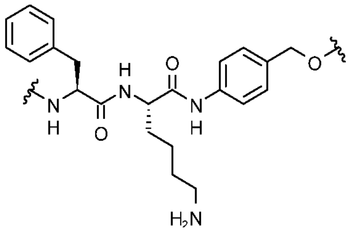 , 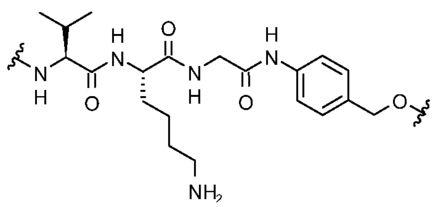 ,

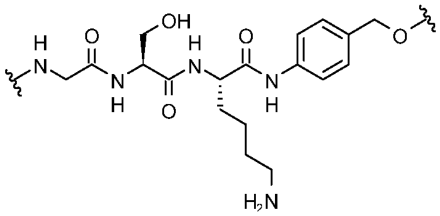 , 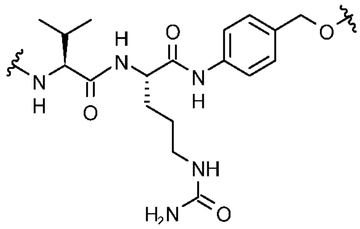 ,

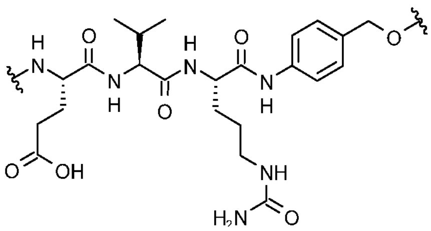 , 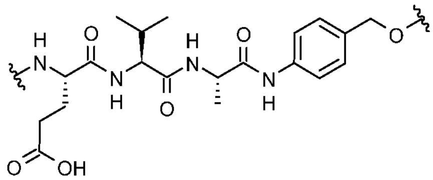 ,

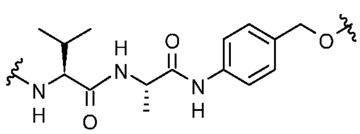,
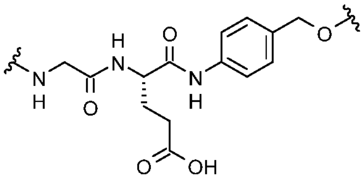,
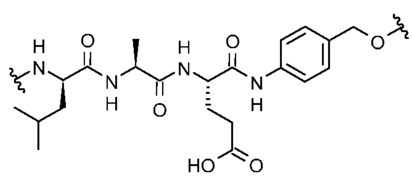,
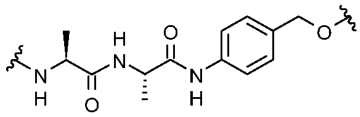,
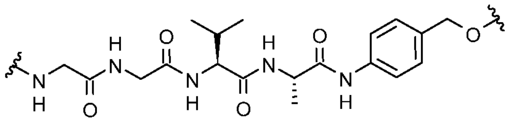,
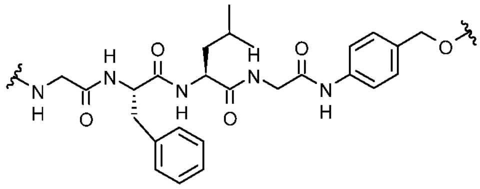,
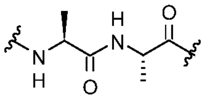,
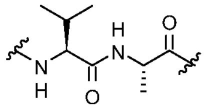,
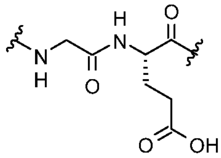,
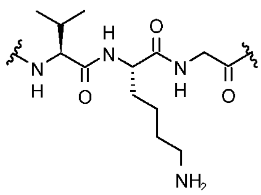,
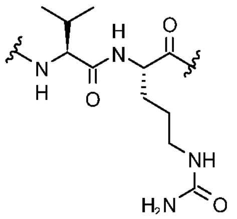,
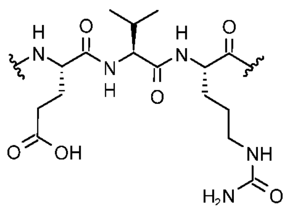,
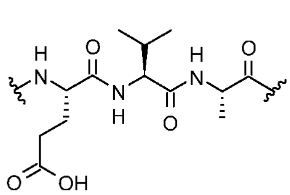,
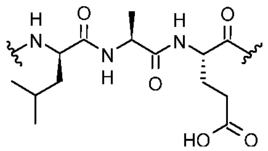,
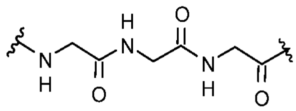,
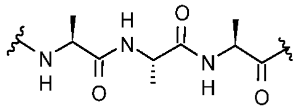,
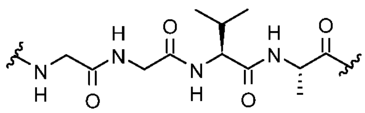,
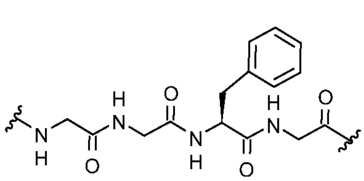 and
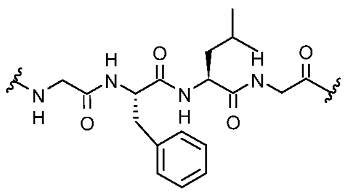;

preferably, the L is a structure selected from the group consisting of:

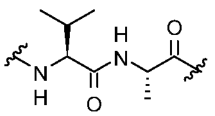,
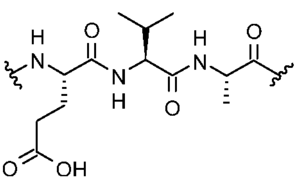,
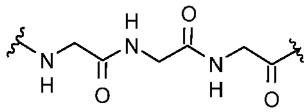.

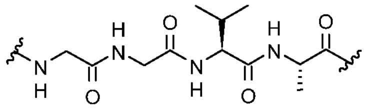,

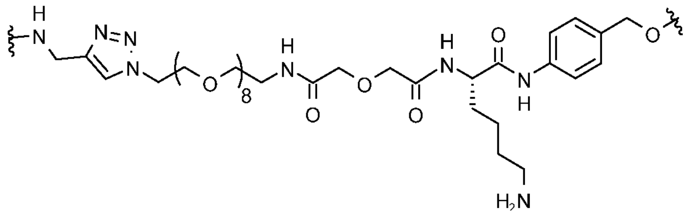,

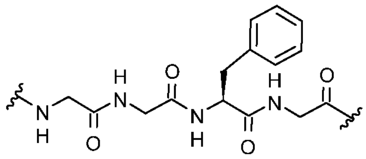,

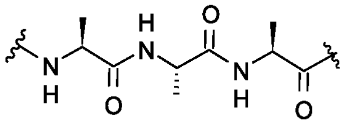,

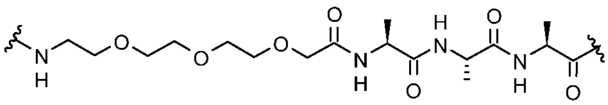

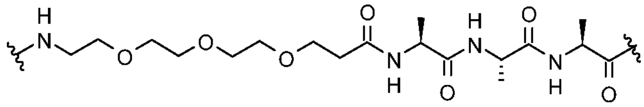,

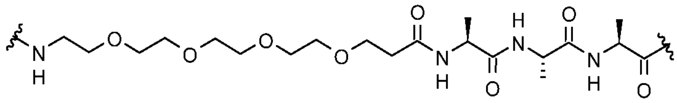,

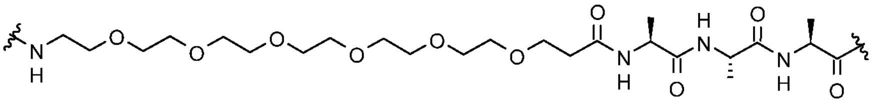

and

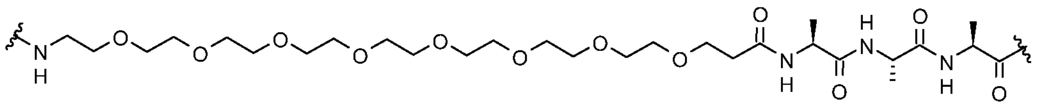;

preferably, the L is a structure selected from the group consisting of:

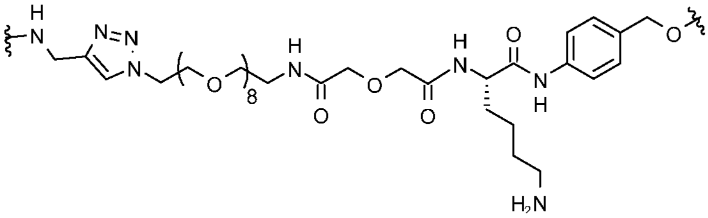,

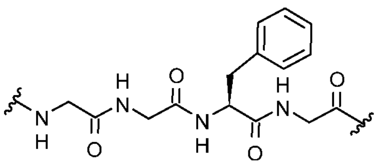

and

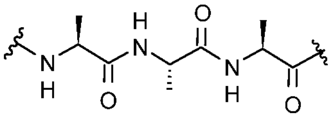;

preferably, the L is a structure selected from the group consisting of:

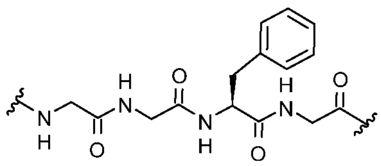 and 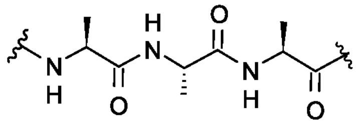 ;

preferably, the L is a structure selected from the group consisting of:

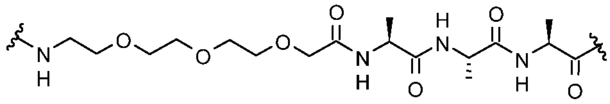

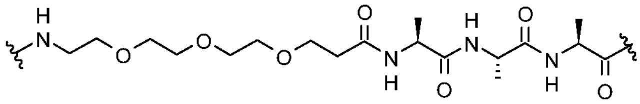

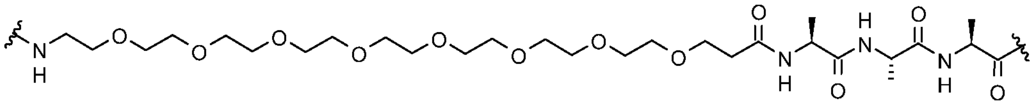 ;

E is a structural fragment connecting L and D, wherein E is a single bond, $-NHCH_2-$or a structure selected from the group consisting of:

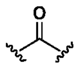, 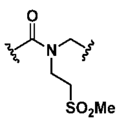, 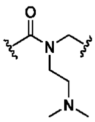 and 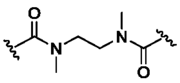 ;

preferably, E is a single bond, $-NHCH_2-$,

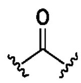 or 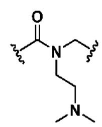

preferably, E is $-NHCH_2-$ or

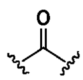;

preferably, E is $-NHCH_2-$;
preferably, E is a single bond;
preferably, E is

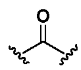;

D is a cytotoxic drug fragment, and the cytotoxic drug is selected from the group consisting of a tubulin inhibitor, a DNA intercalator, a DNA topoisomerase inhibitor and an RNA polymerase inhibitor; preferably, the tubulin inhibitor is an auristatin compound or a maytansine compound; preferably, the DNA intercalator is pyrrolobenzodiazepine (PBD); preferably, the DNA topoisomerase inhibitor is a topoisomerase I inhibitor (such as camptothecin, hydroxycamptothecin, 9-amino-camptothecin, SN-38, irinotecan, topotecan, belotecan, or rubitecan) or a topoisomerase II inhibitor

(such as doxorubicin, PNU-159682, docamicin, daunorubicin, mitoxantrone, podophyllotoxin or etoposide); preferably, the RNA polymerase inhibitor is α-amanitin or a pharmaceutically acceptable salt, ester or an analogue thereof;

preferably, the cytotoxic drug is selected from the compounds of formula I and formula II or pharmaceutically acceptable salts, esters, stereoisomers, tautomers or prodrugs of the compounds of formula I and formula II:

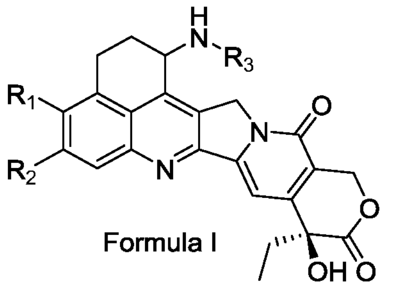
Formula I

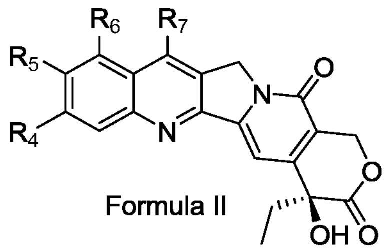
Formula II

wherein $R_1$ and $R_2$ are each independently selected from $C_{1-6}$ alkyl and halogen;

$R_3$ is selected from the group consisting of H and $-CO-CH_2OH$;

$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;

$R_6$ is selected from the group consisting of H or $-C_{1-4}$ alkylene-$NR_aR_b$;

$R_7$ is selected from the group consisting of $C_{1-6}$ alkyl and $-C_{1-4}$ alkylene-$NR_aR_b$;

wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence;

preferably, the cytotoxic drug is selected from the following compounds or pharmaceutically acceptable salts, esters, stereoisomers, tautomers or prodrugs of the compounds:

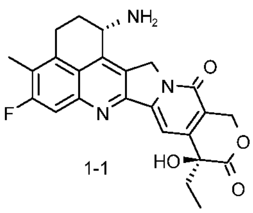
1-1

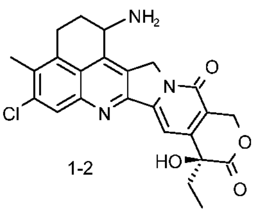
1-2

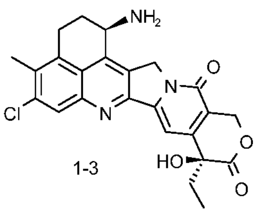
1-3

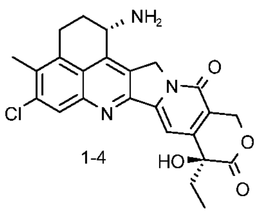
1-4

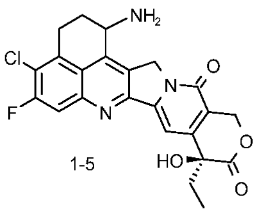
1-5

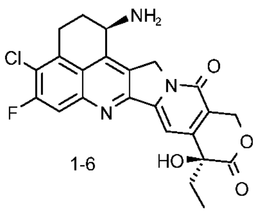
1-6

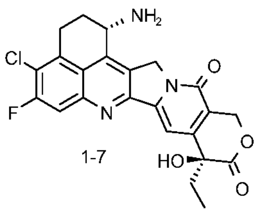
1-7

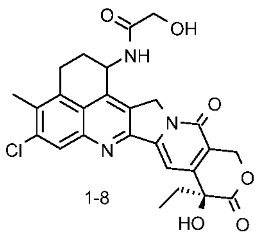
1-8

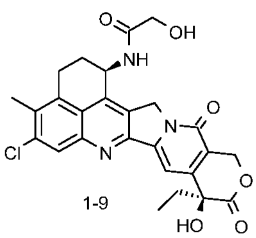
1-9

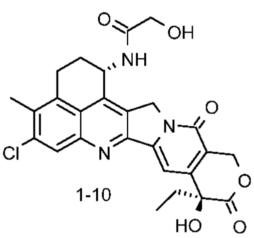
1-10

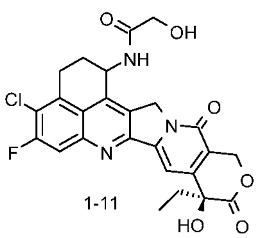
1-11

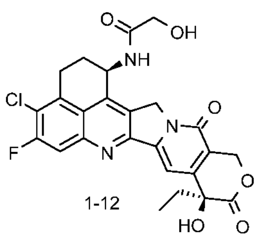
1-12

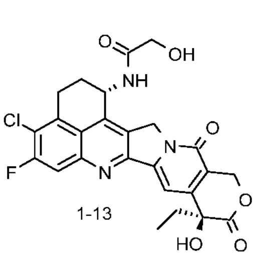
1-13

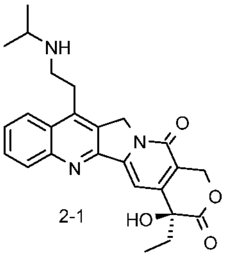
2-1

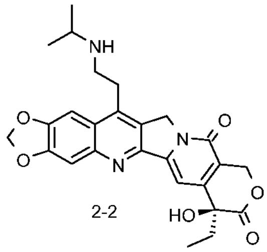
2-2

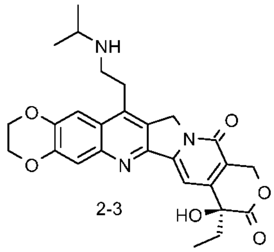
2-3

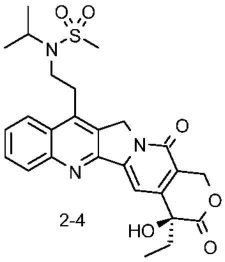
2-4

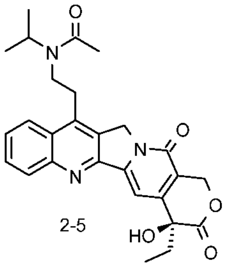
2-5

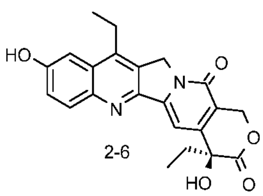
2-6

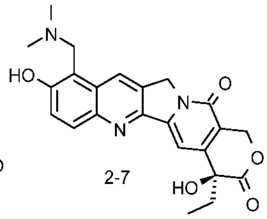
2-7

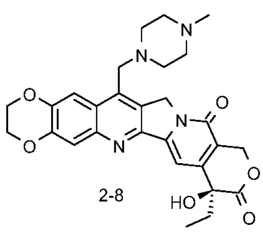
2-8

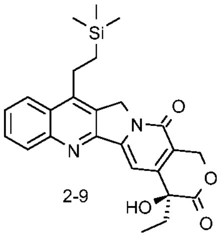
2-9

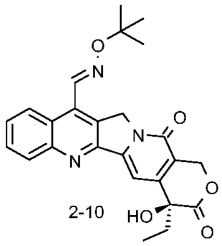
2-10

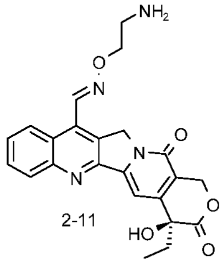
2-11

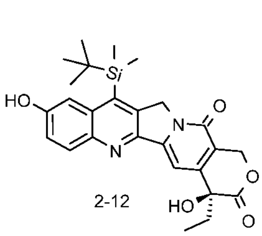
2-12

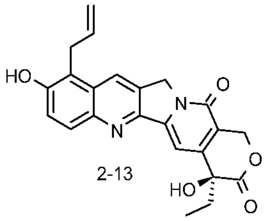
2-13

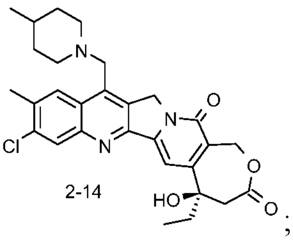
2-14

;

The corresponding fragment of the cytotoxic drug obtained by linking the cytotoxic drug to the linker is D in the general formula; preferably, D is a monovalent structure obtained by losing one H from -OH, $-NH_2$ or a secondary amino on the cytotoxic drug;

preferably, the cytotoxic drug is selected from the following compounds or pharmaceutically acceptable salts, esters, stereoisomers, tautomers or prodrugs of the compounds:

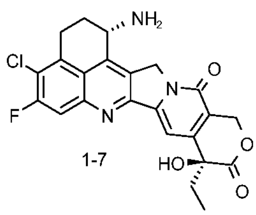
1-7

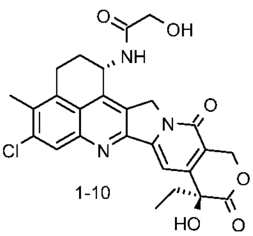
1-10

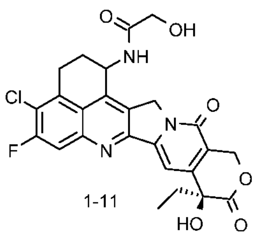
1-11

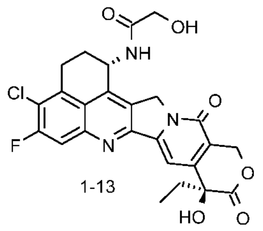

1-13

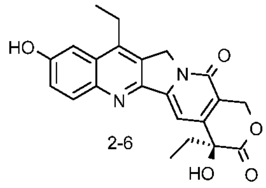

2-6

;

and/or
x is selected from 1 to 10.
preferably,

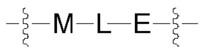

is a structure selected from the group consisting of:

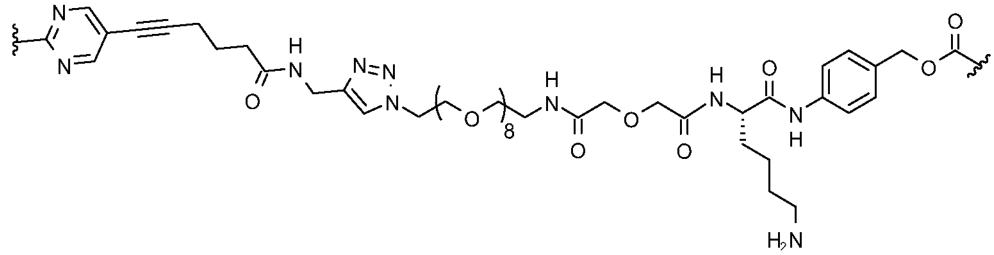

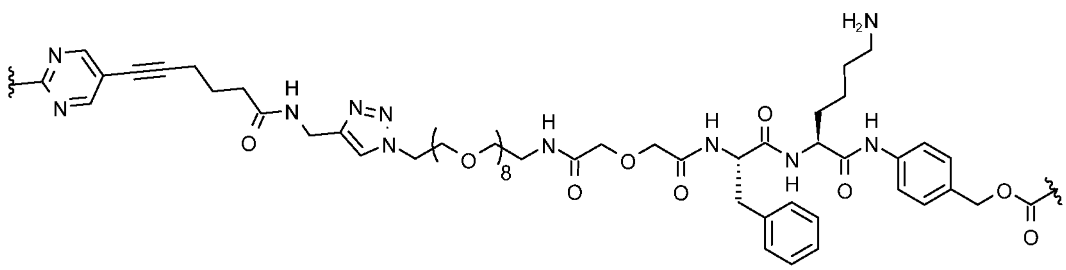

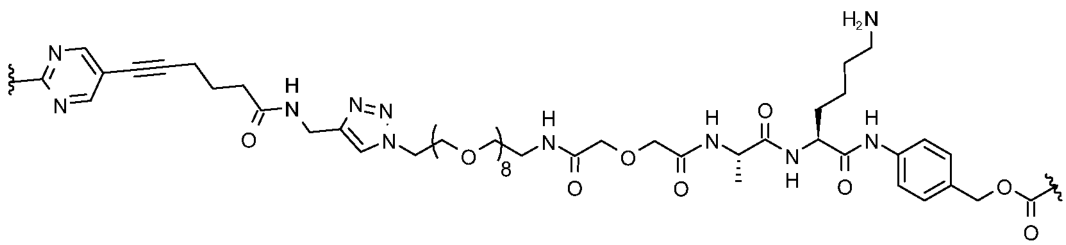

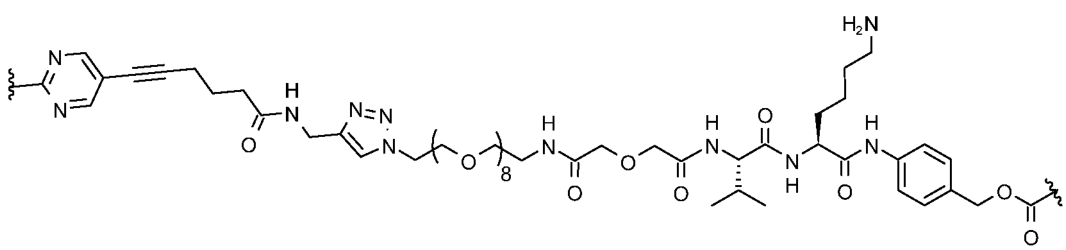

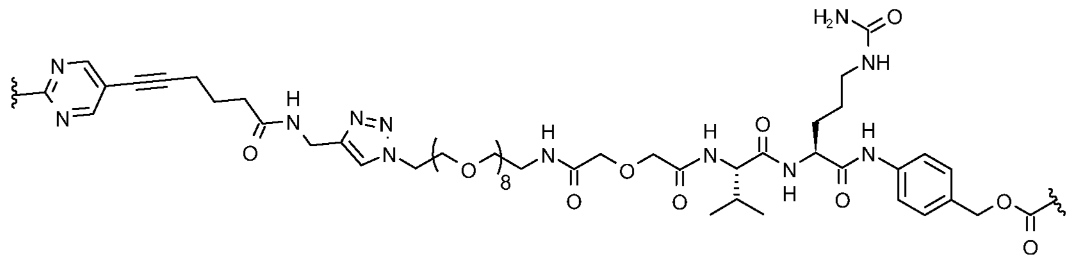

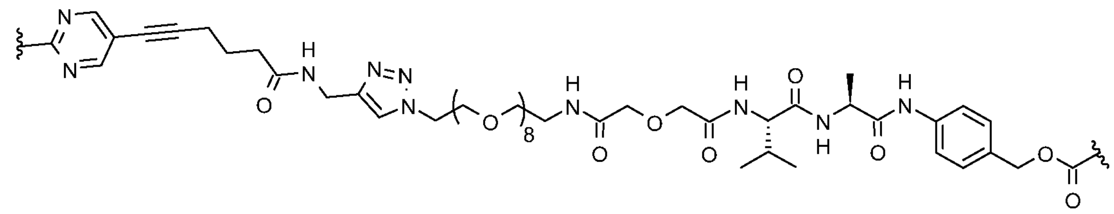

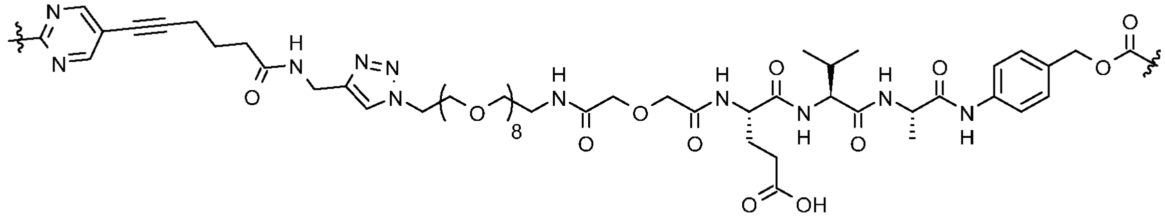

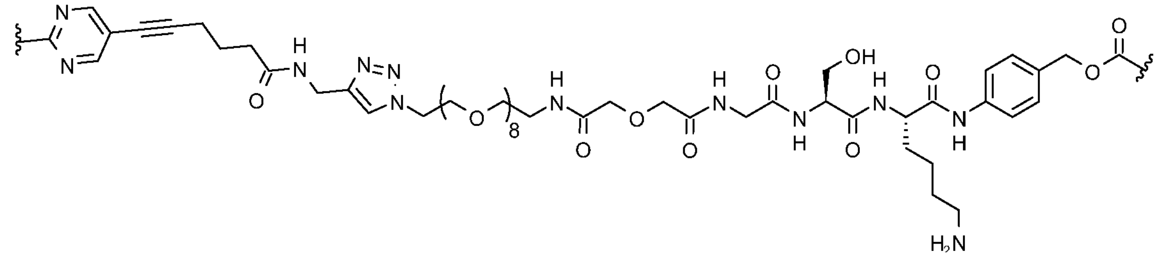

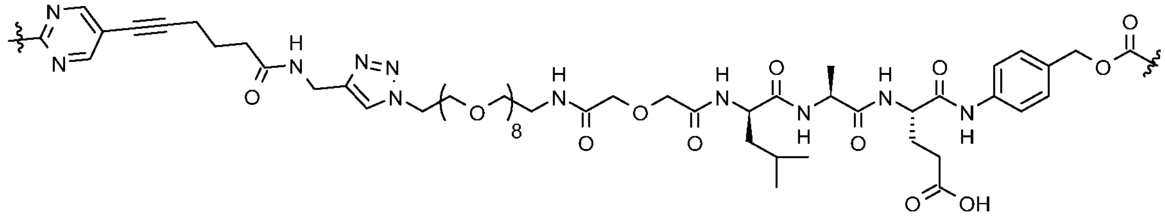

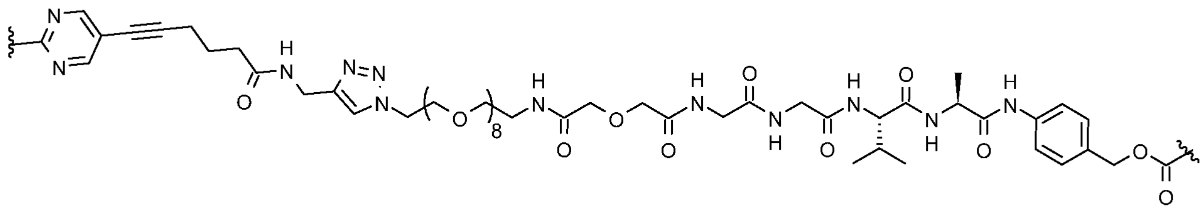

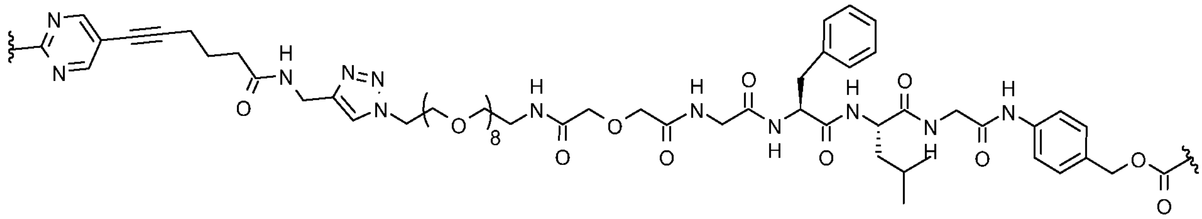

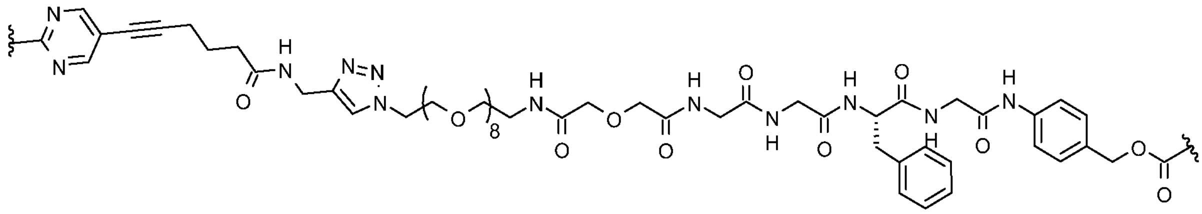

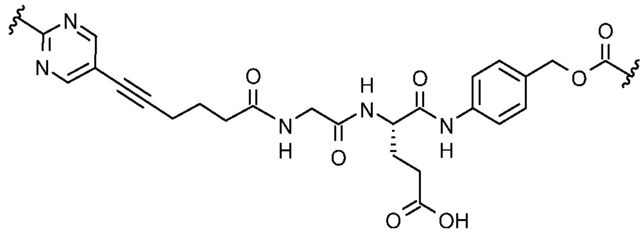

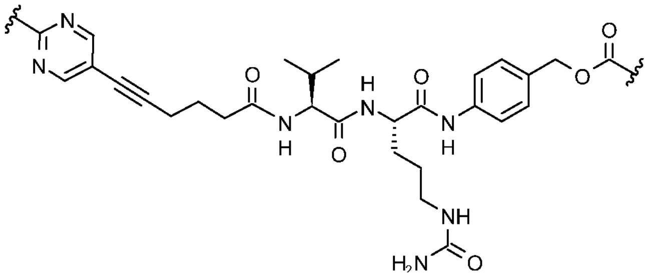

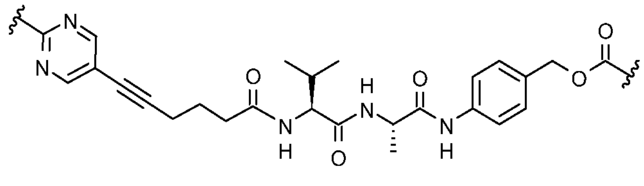

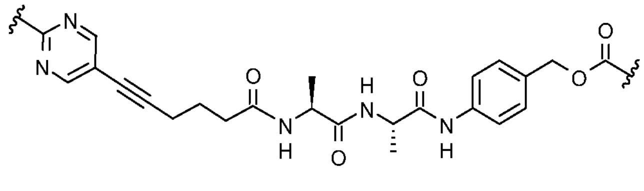

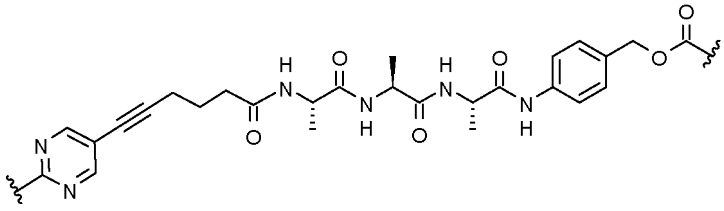

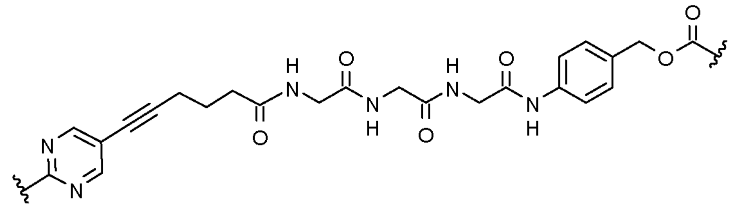

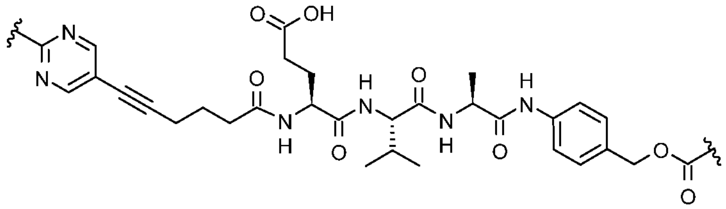

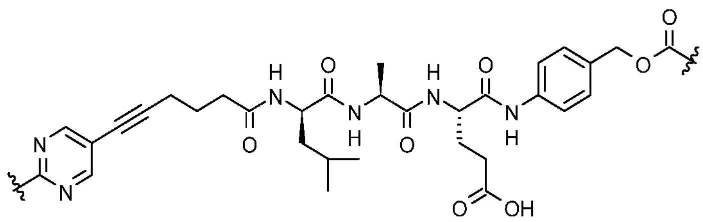

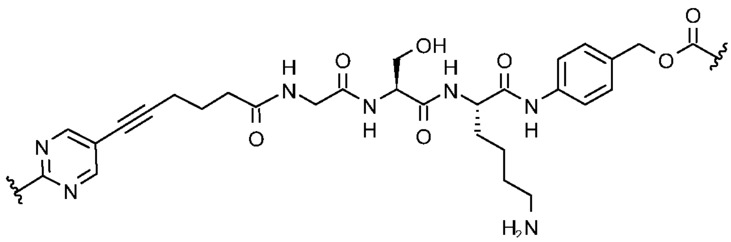

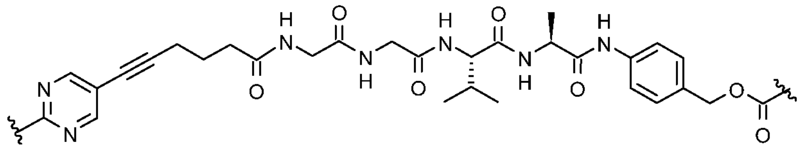

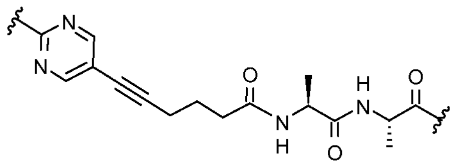

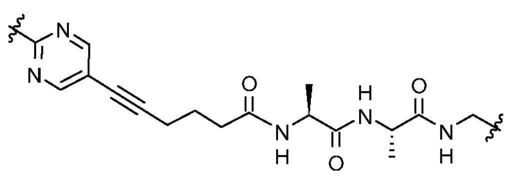

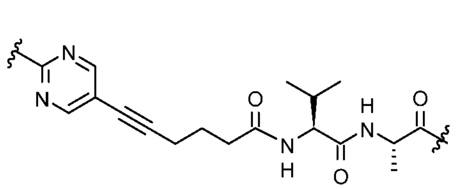

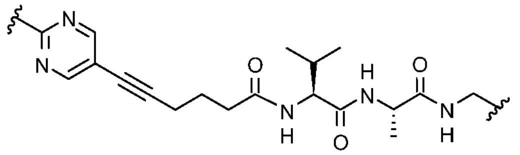

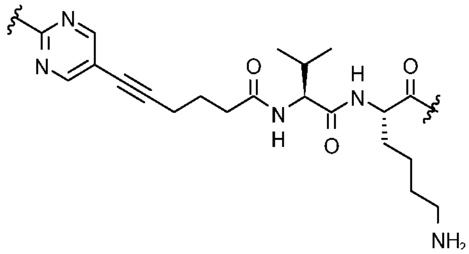

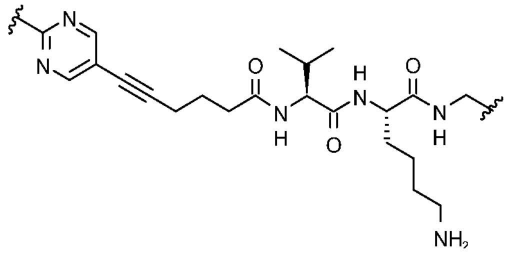

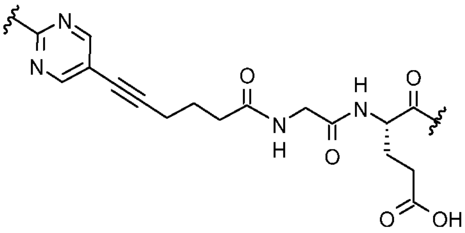

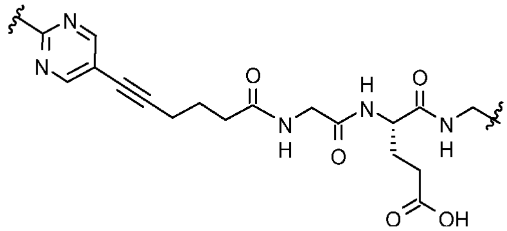

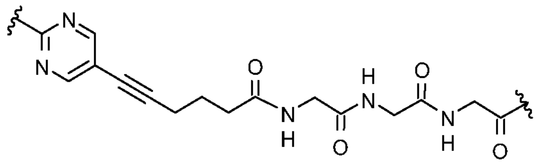

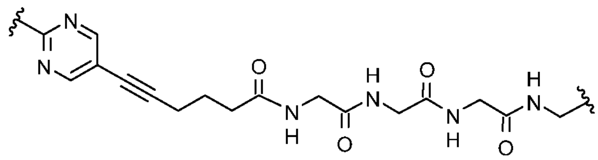

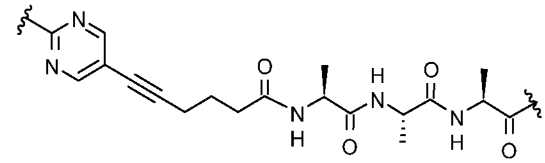

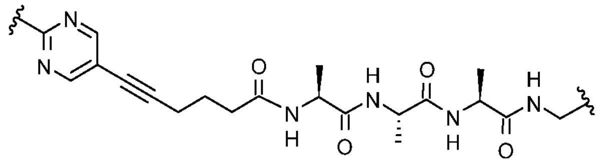

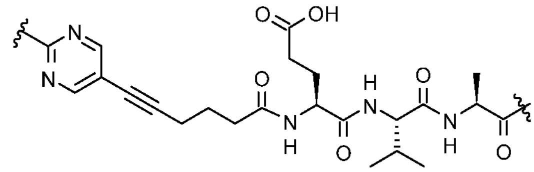

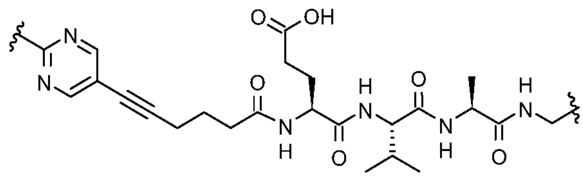

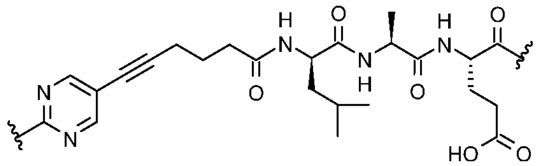

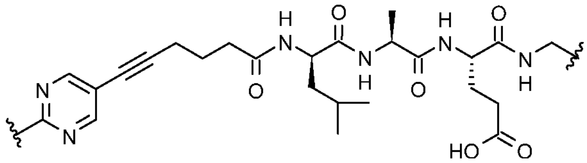

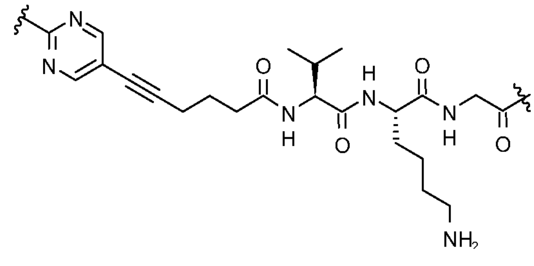

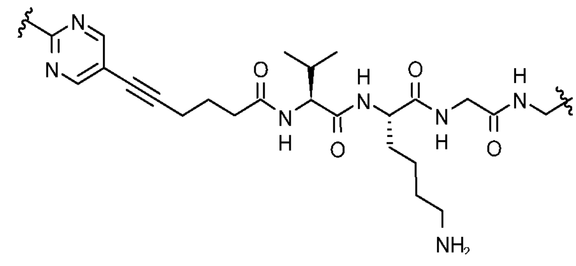

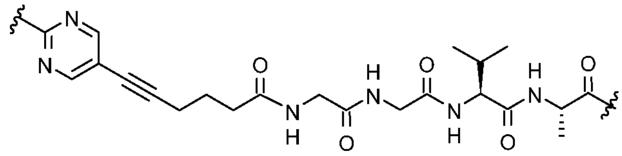

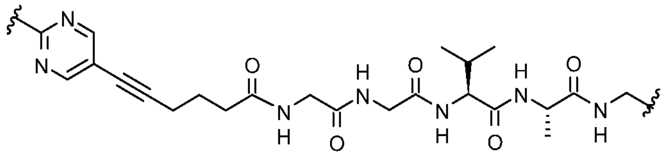

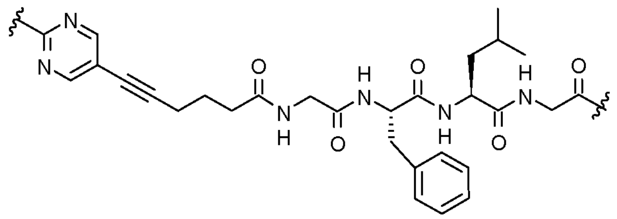

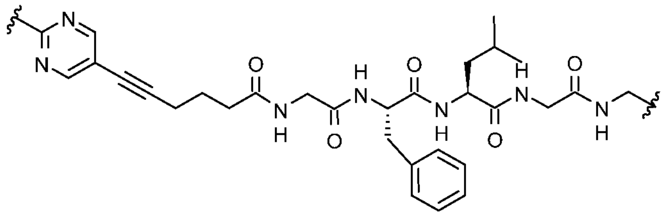

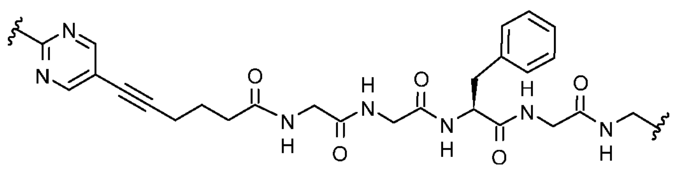

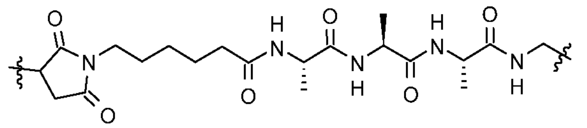

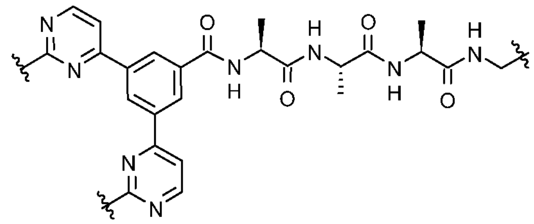

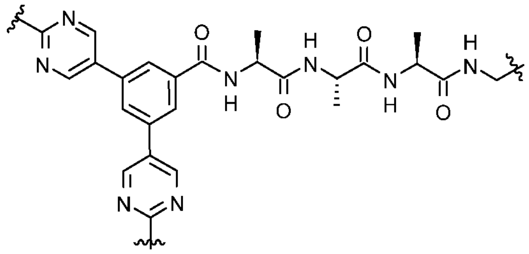

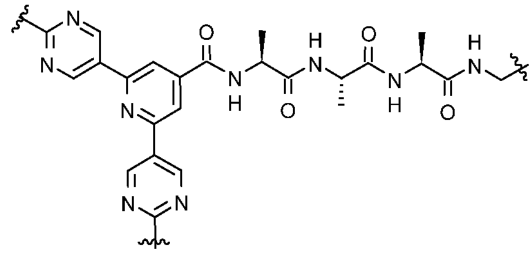

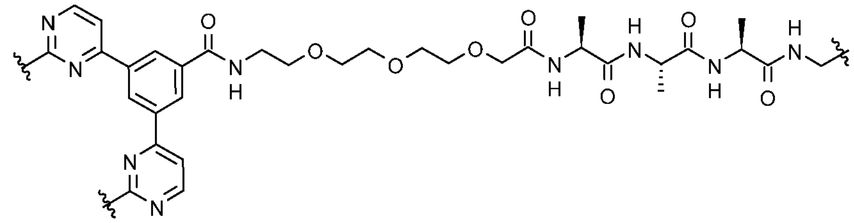

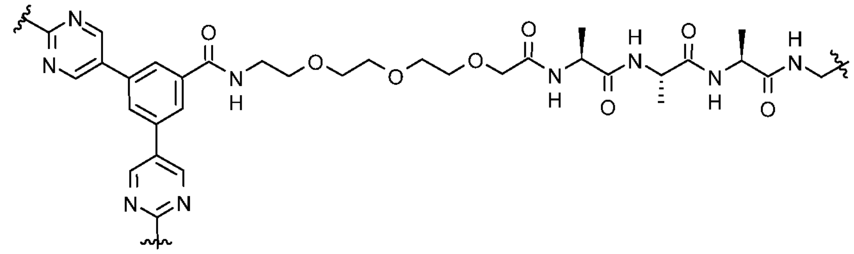

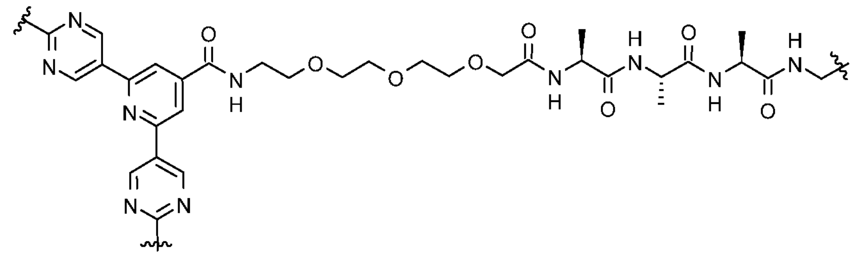

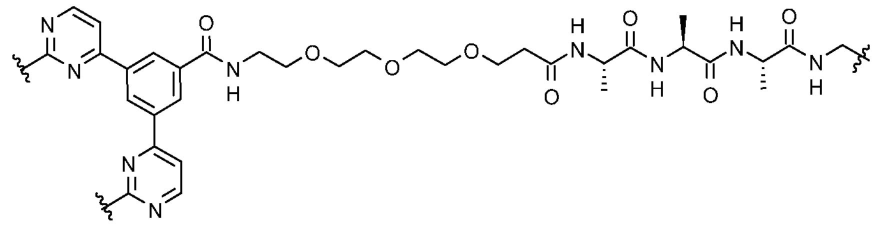

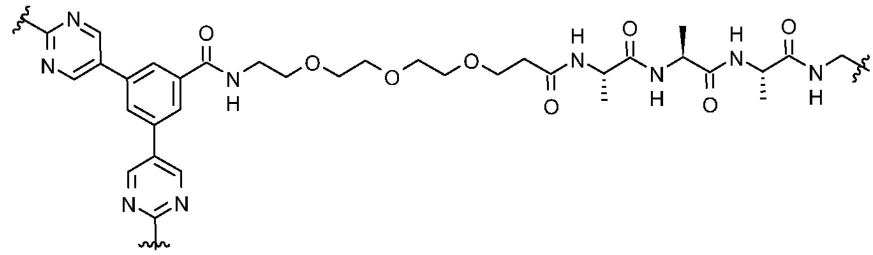

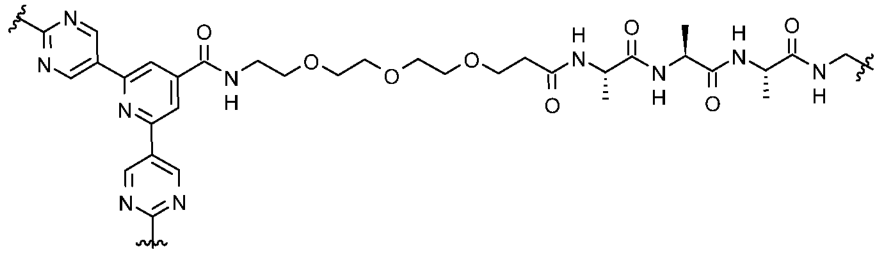

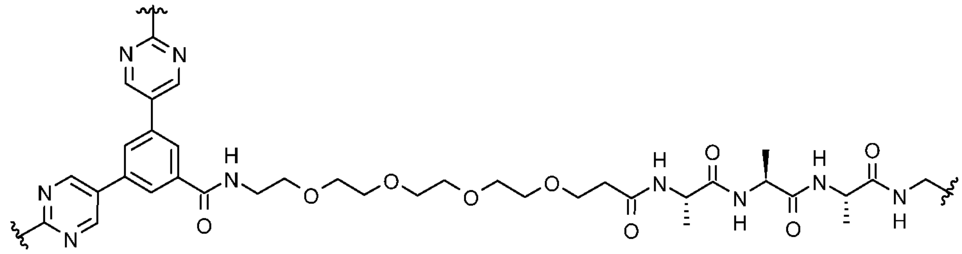

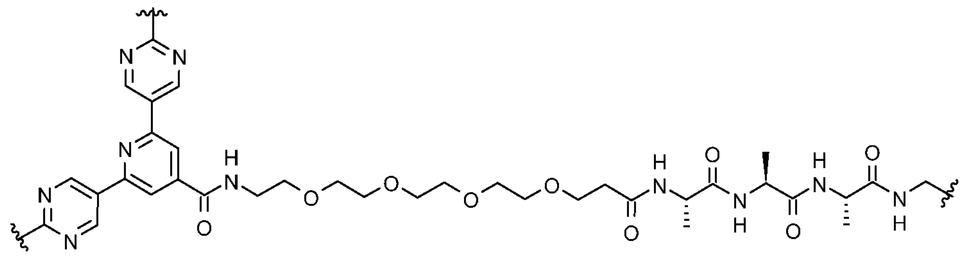

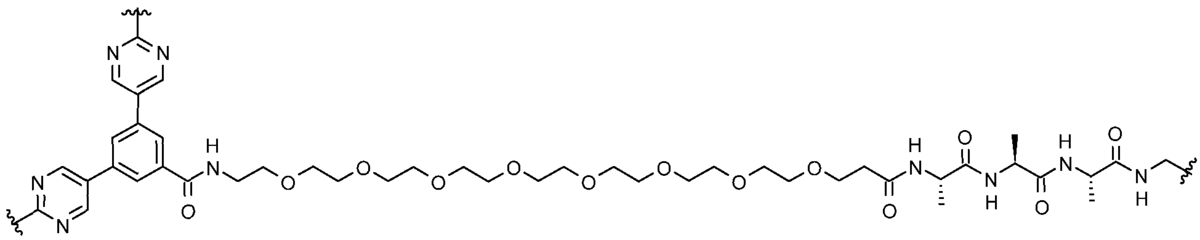

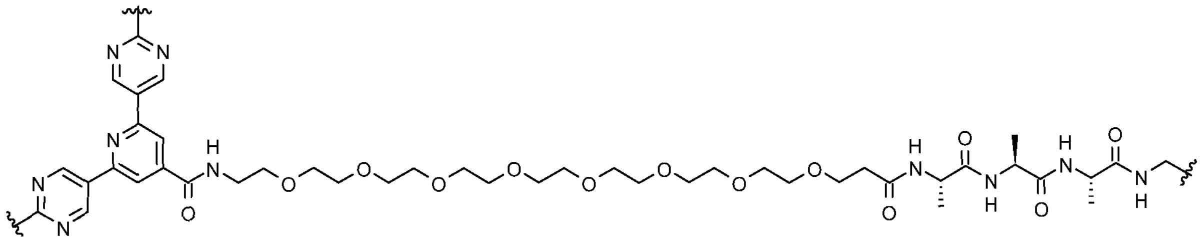

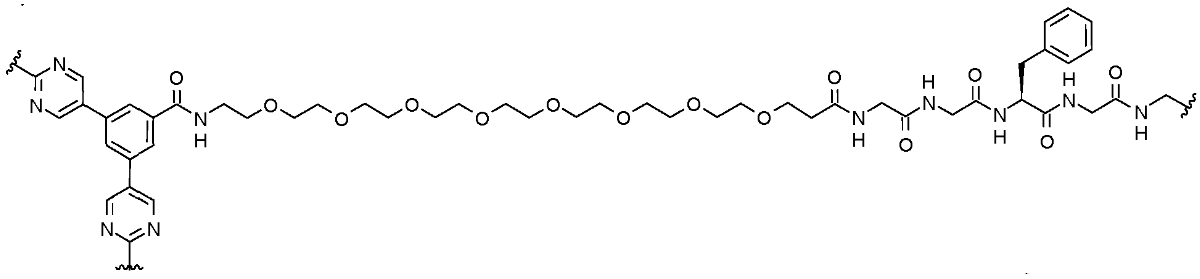

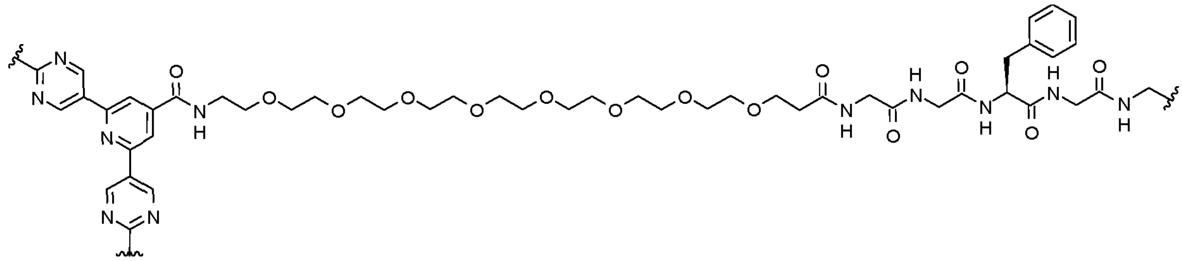

;

preferably,

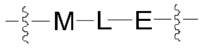

is a structure selected from the group consisting of:

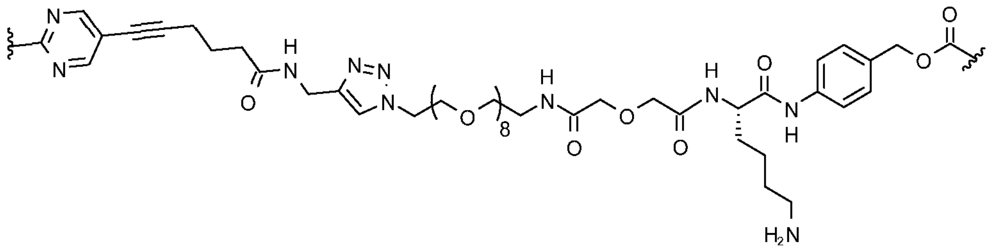

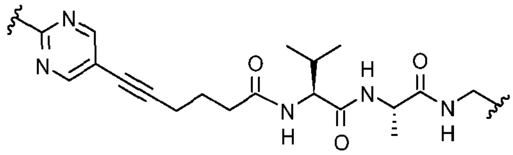

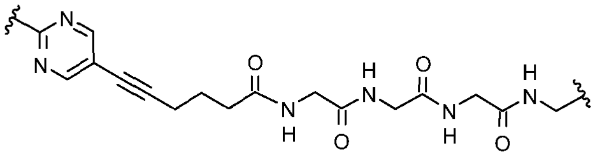

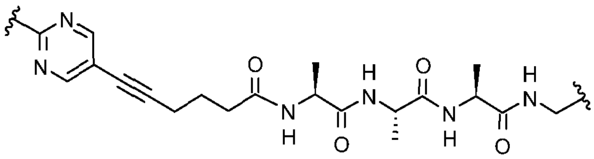

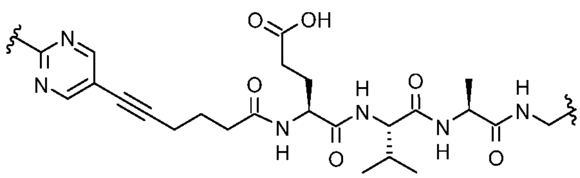

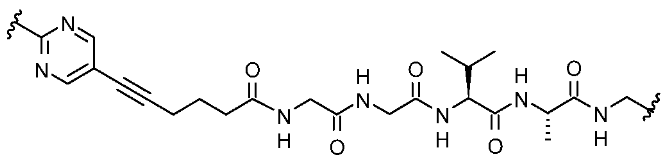

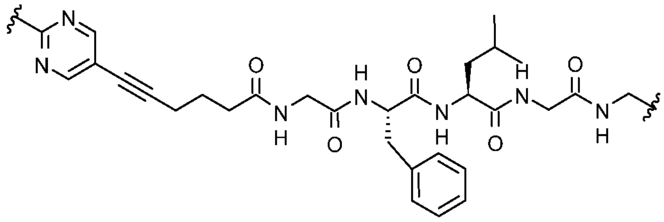

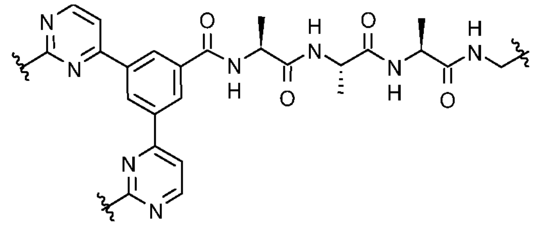

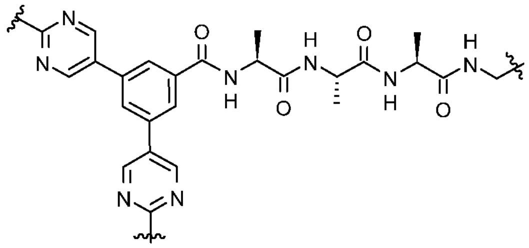

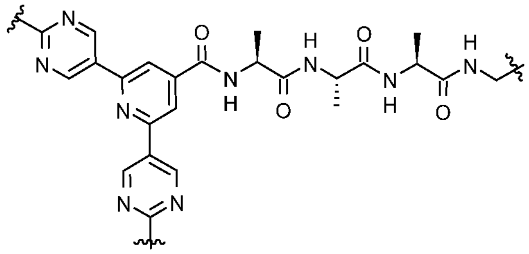

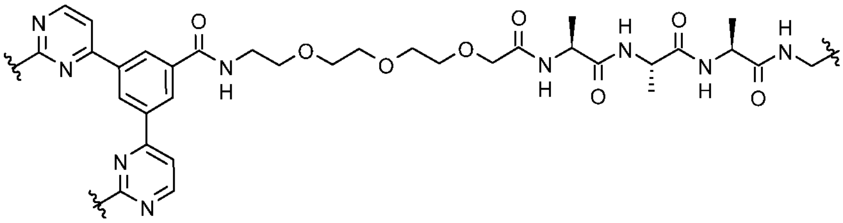

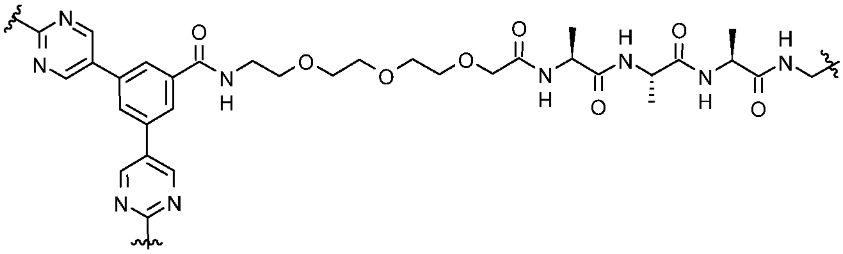

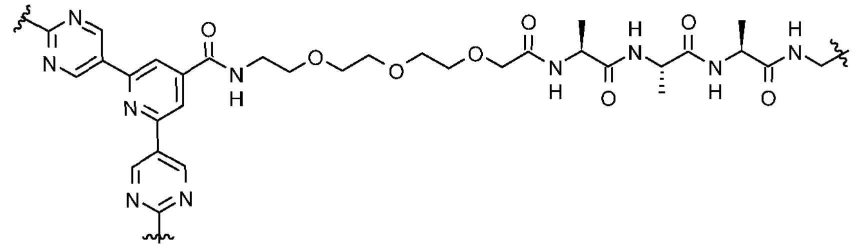

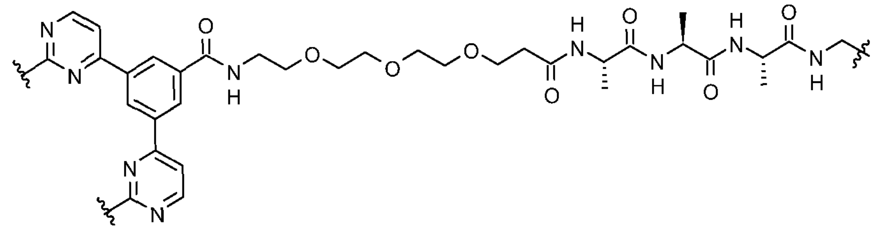

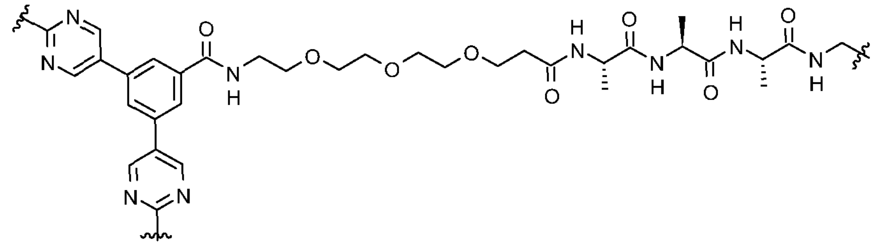

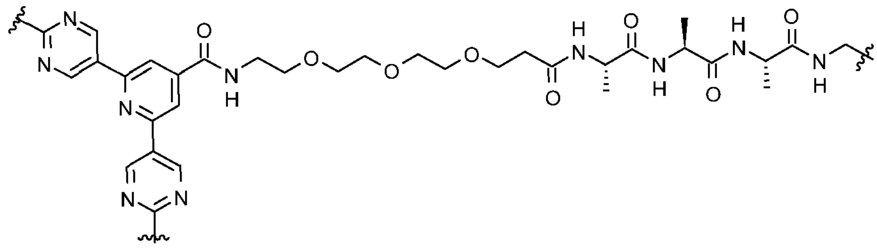

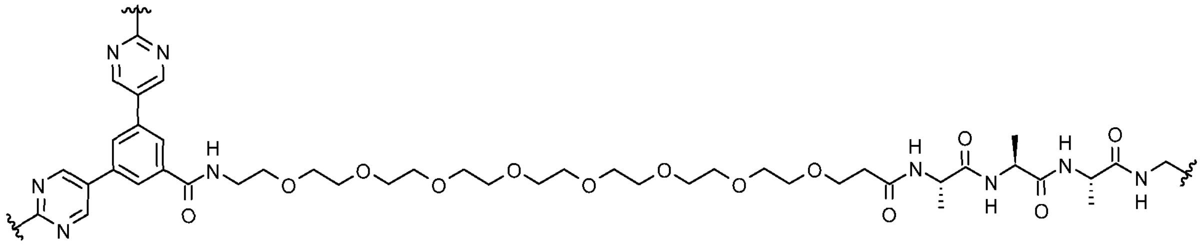

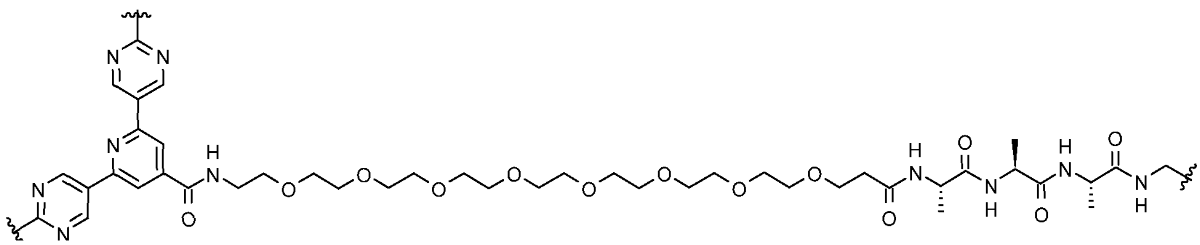

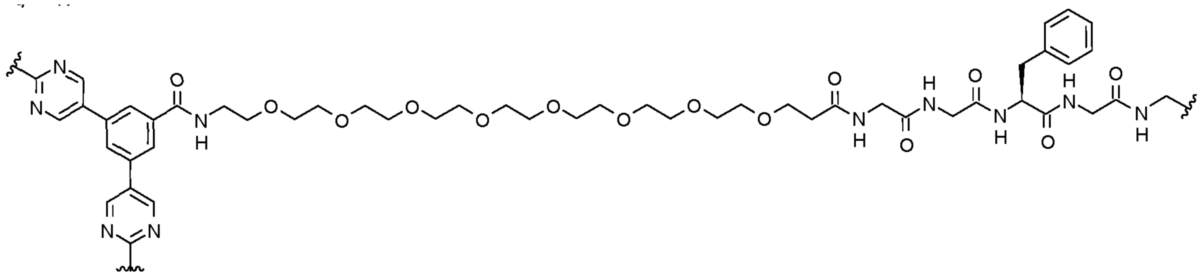

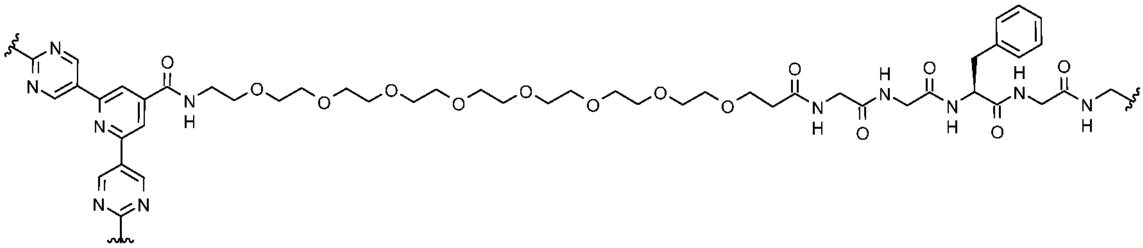

.

In another aspect, the present invention provides a drug-linker having a structure represented by formula G-M-[L-E-D]$_x$ (x is selected from 1 to 10), wherein
G-M is

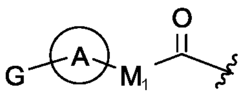

,

G is a leaving group of a nucleophilic substitution reaction (such as halogen, methylsulfonyl, fluorophenol or

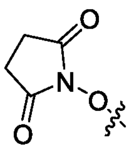

), or is hydroxyl (-OH), sulfhydryl (-SH) or amino ($-NH_2$); or, G forms an unsaturated double bond with an adjacent atom on ring A; ring A is a 5- to 6-membered aliphatic heterocycle or a 5- to 20-membered aromatic ring system, and the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond, $C_{1-20}$ alkylene, $C_{2-20}$ alkenylene, $C_{2-20}$ alkynylene or amino.

The structures of L, E and D are defined as above.

In some embodiments, G-M is

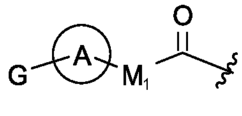

, G is methylsulfonyl, or, G forms a C-C double bond with an adjacent atom on ring A; ring A is a 5-membered aliphatic heterocycle, a 6-membered heteroaromatic ring, or a polycyclic ring formed by connecting one or more 6-membered heteroaromatic rings to a benzene ring or a 6-membered heteroaromatic ring via a single bond, and the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, $C_{2-10}$ alkynylene or amino.

In some embodiments, G-M is

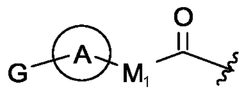 , and 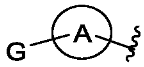

is selected from the group consisting of

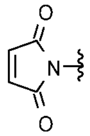 , 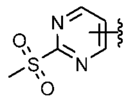 , 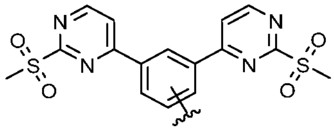 ,

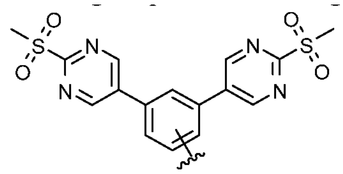 , 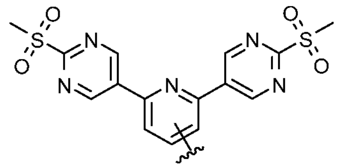 , 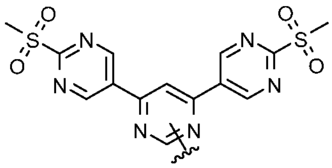

and

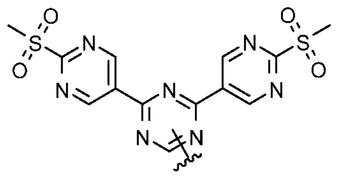 ;

$M_1$ is selected from the group consisting of a single bond, $C_{1-20}$ alkylene, $C_{2-20}$ alkenylene, or $C_{2-20}$ alkynylene or amino.

In some embodiments, the G-M is selected from the group consisting of

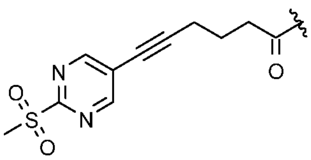 and 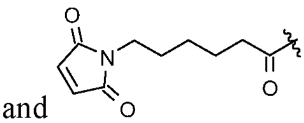 .

In some embodiments, the G-M is selected from the group consisting of

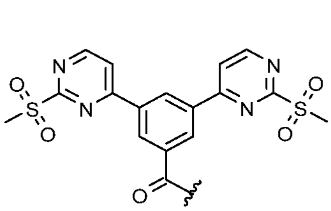 , 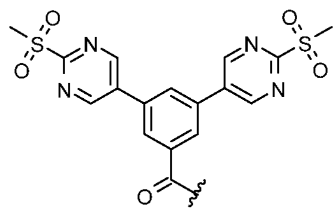 , 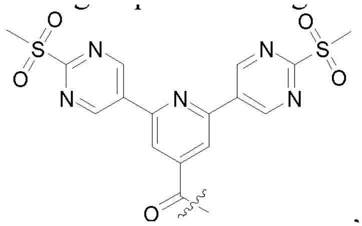 ,

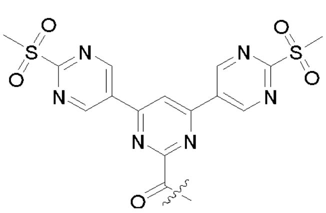 , 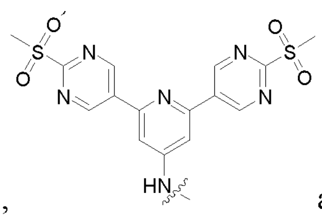 and 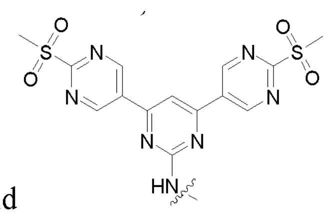 .

In some embodiments, the G-M is selected from the group consisting of

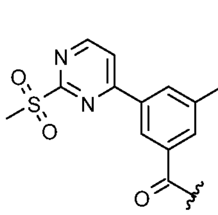
,
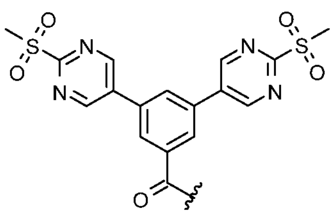
and
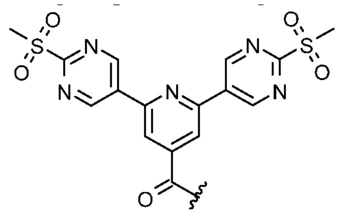
.

In some embodiments, x is selected from 1 to 10.

In some embodiments, the drug-linker compound is selected from A-01 to A-34, B-01 to B-07, and C-01 to C-28 shown below:

May 15, 2

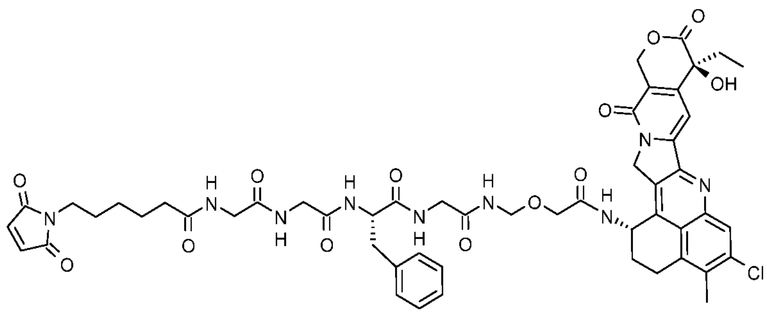
A-01

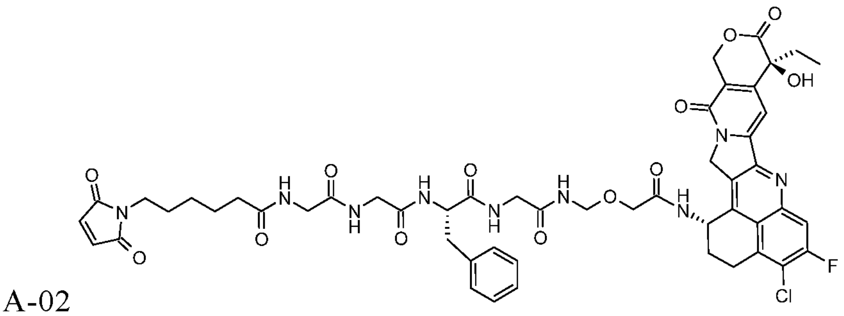
A-02

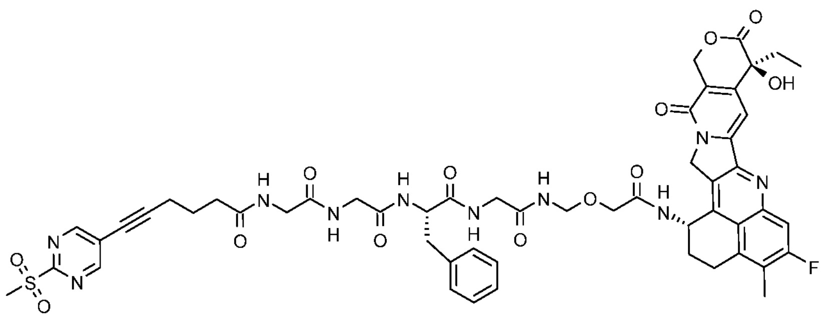
A-03

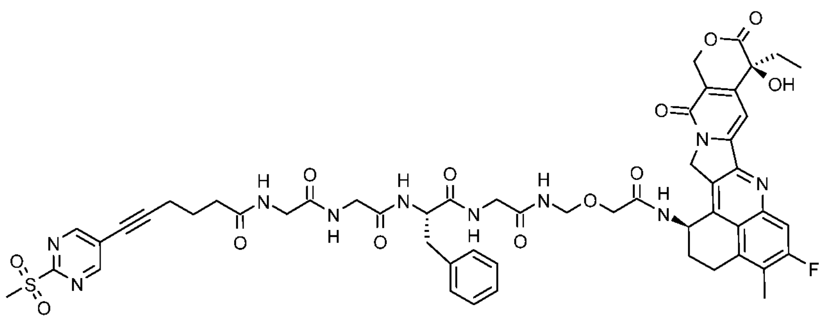
A-04

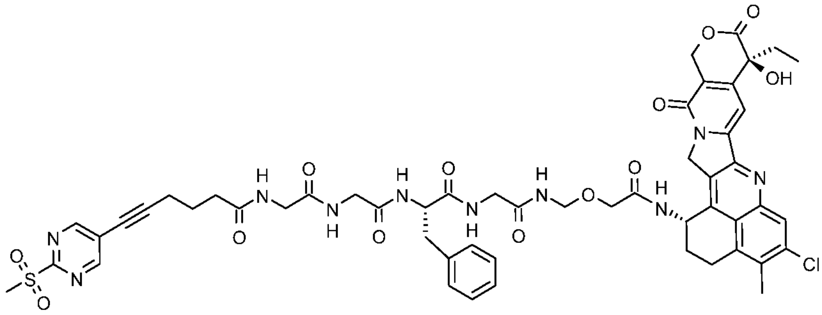

A-05

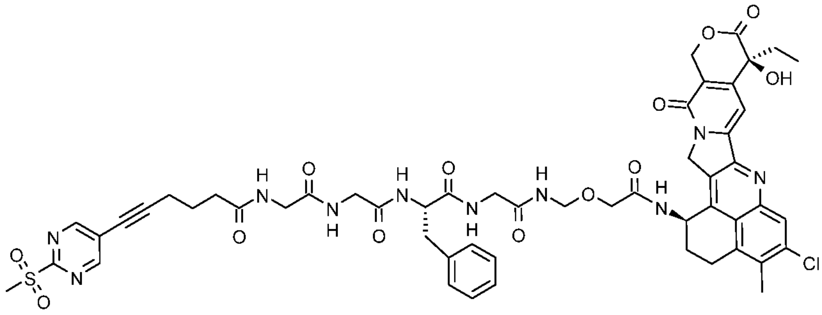

A-06

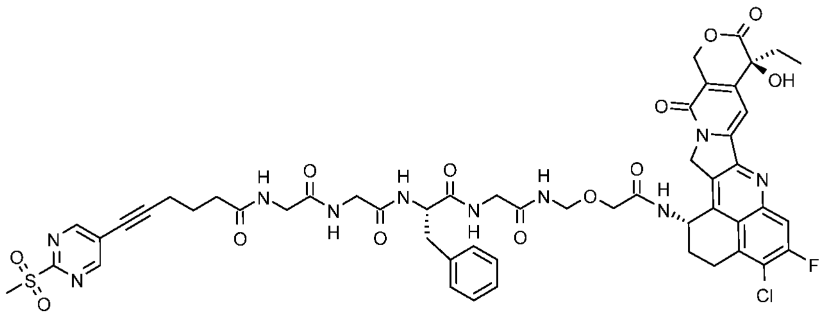

A-07

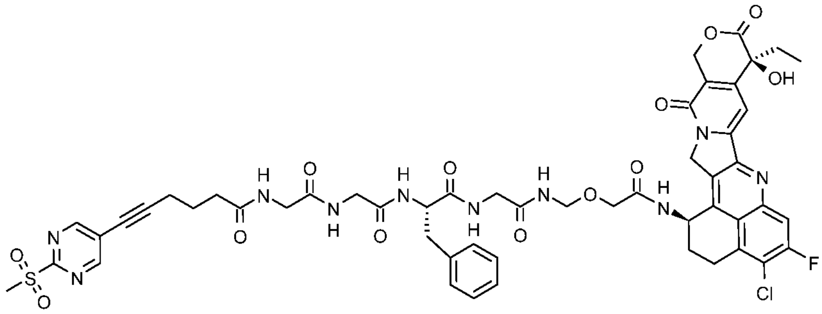

A-08

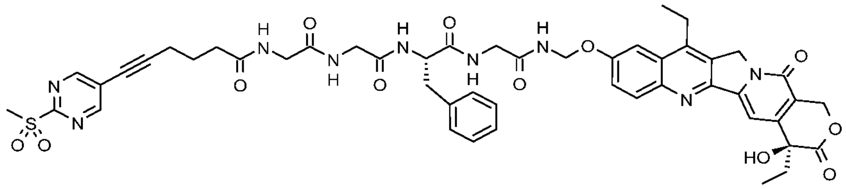

A-09

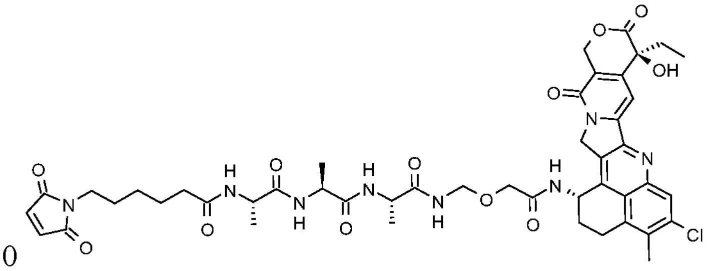

A-10

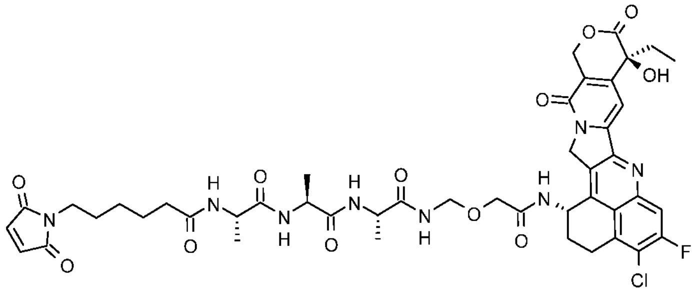

A-11

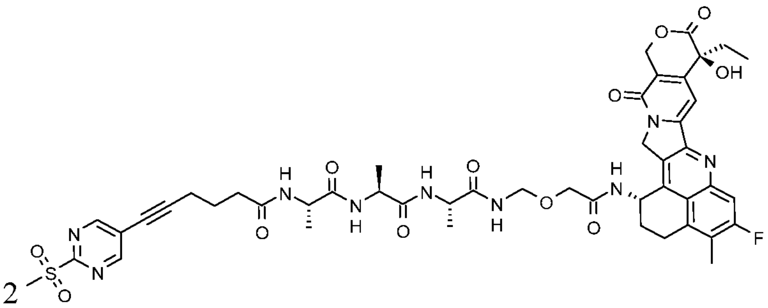

A-12

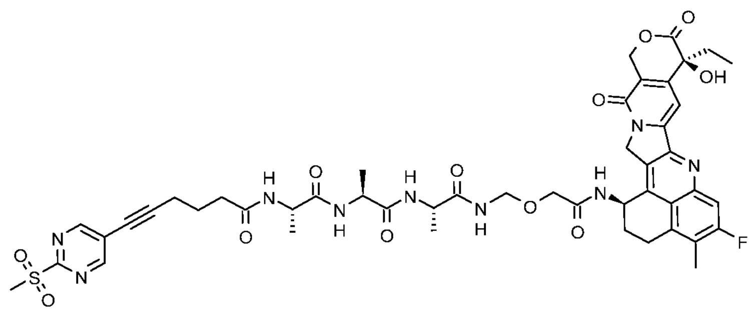

A-13

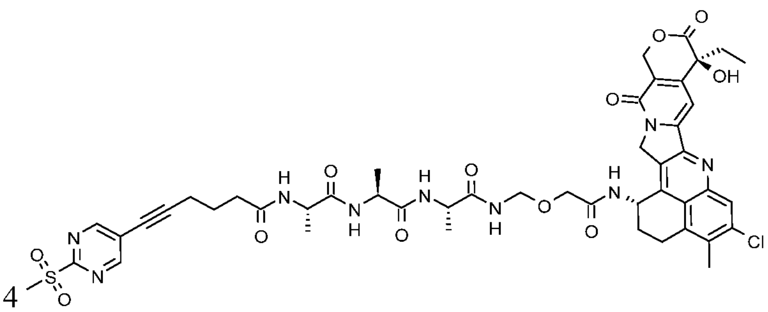

A-14

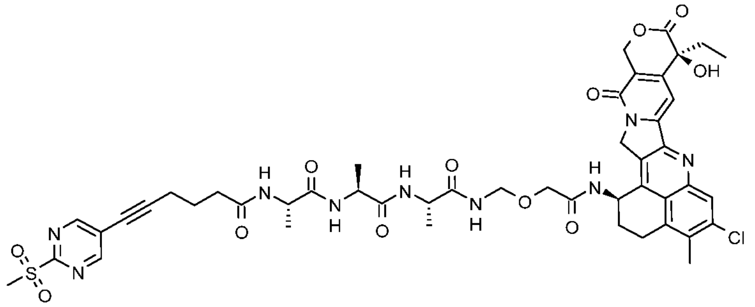

A-15

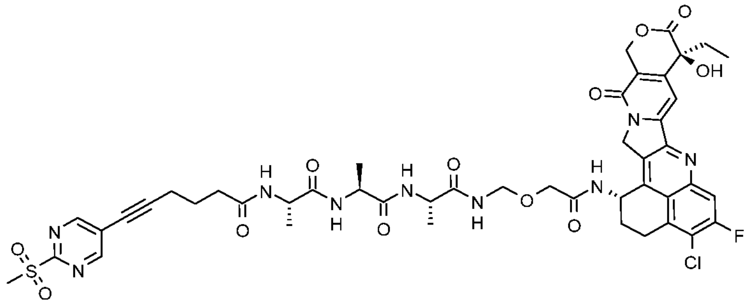

A-16

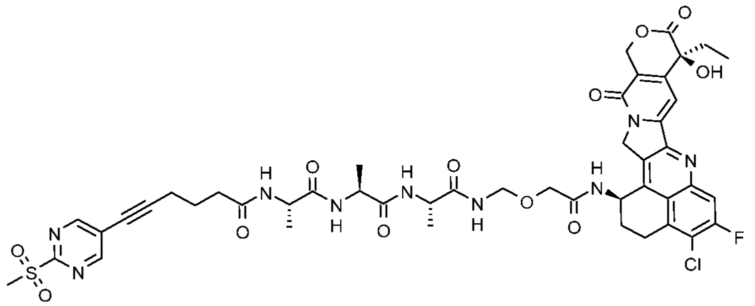

A-17

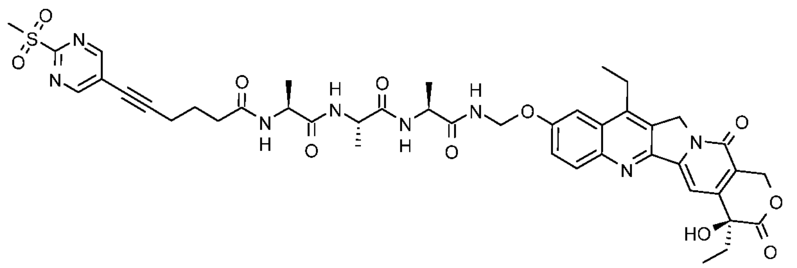

A-18

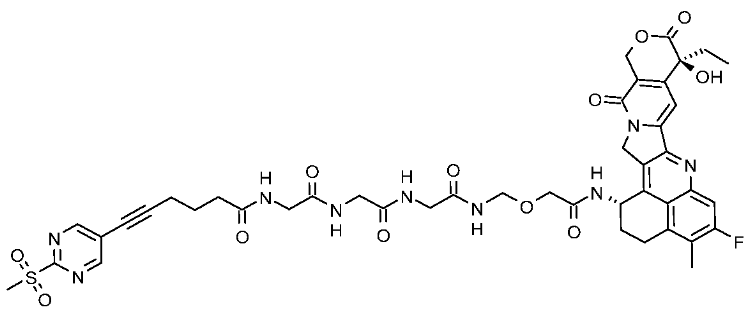

A-19

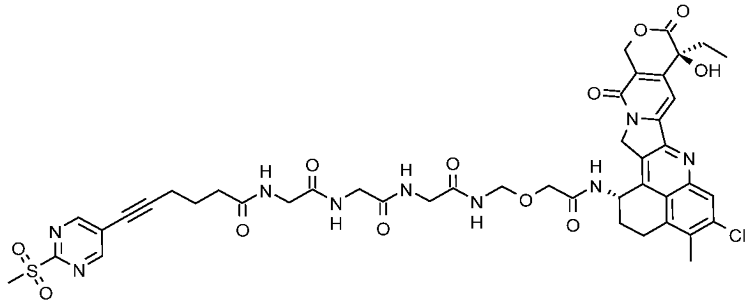

A-20

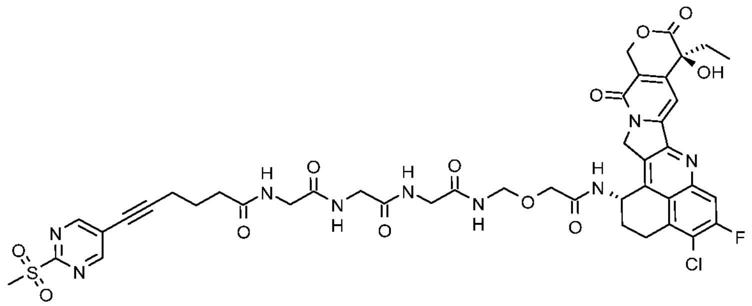

A-21

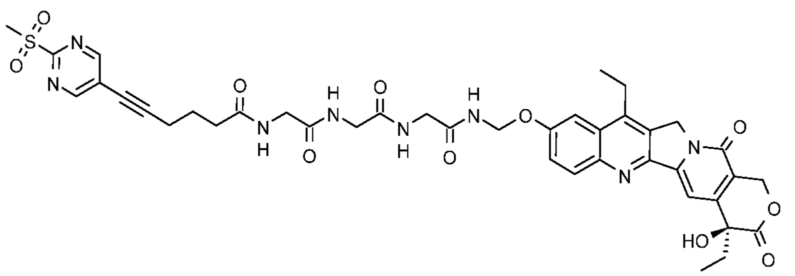

A-22

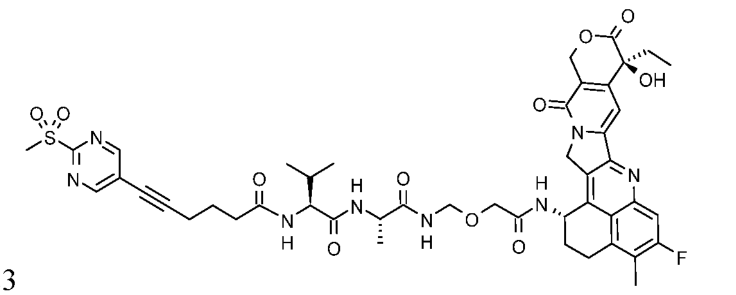

A-23

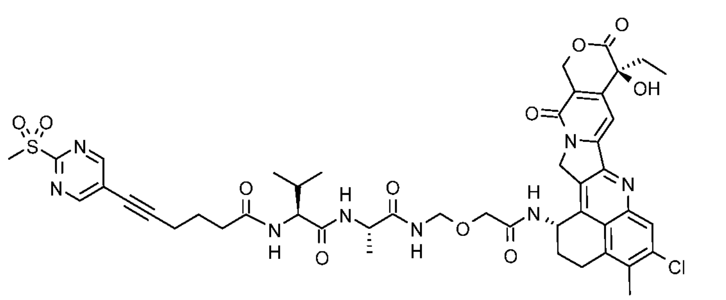

A-24

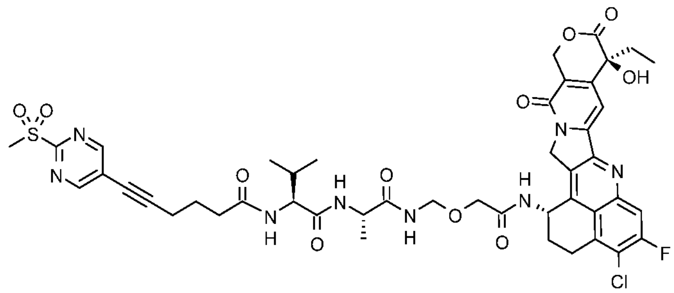

A-25

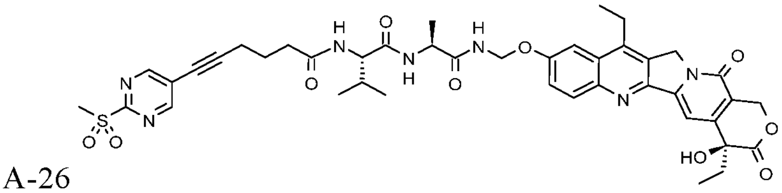

A-26

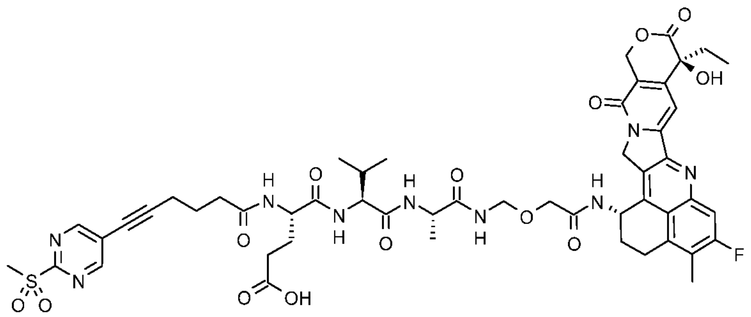

A-27

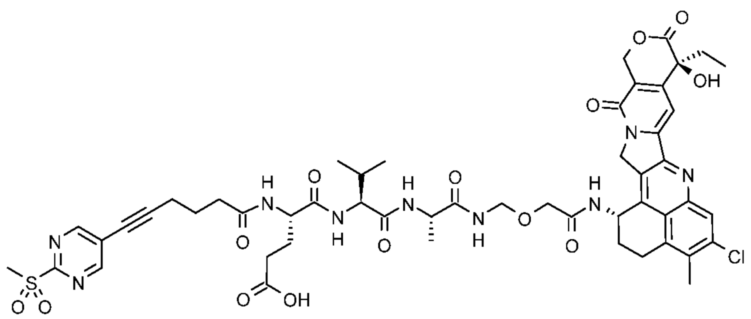

A-28

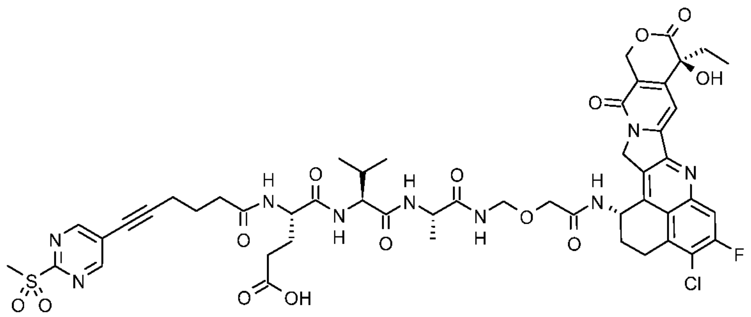

A-29

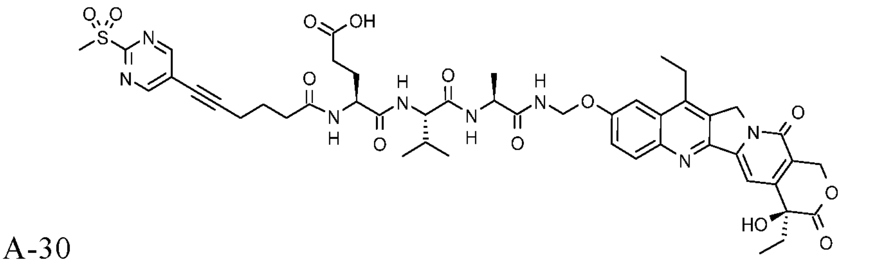

A-30

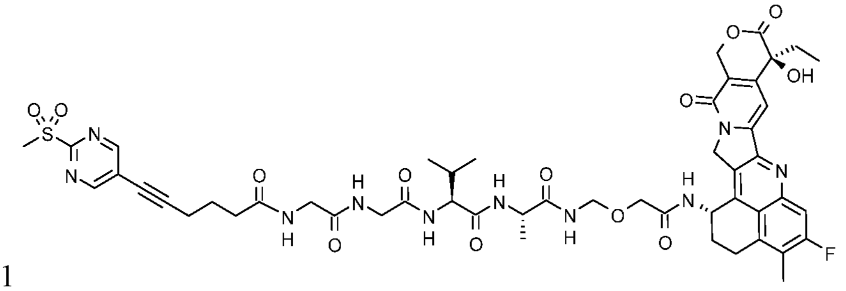

A-31

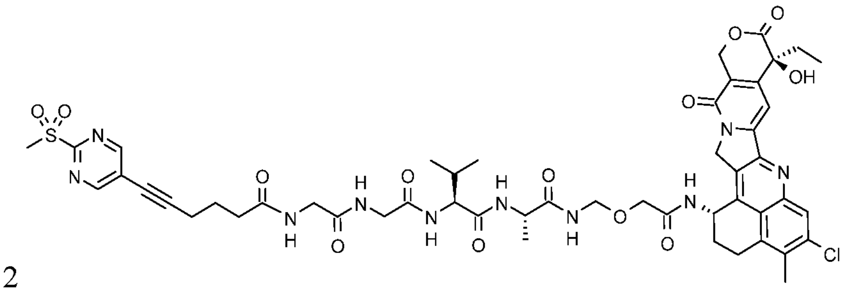

A-32

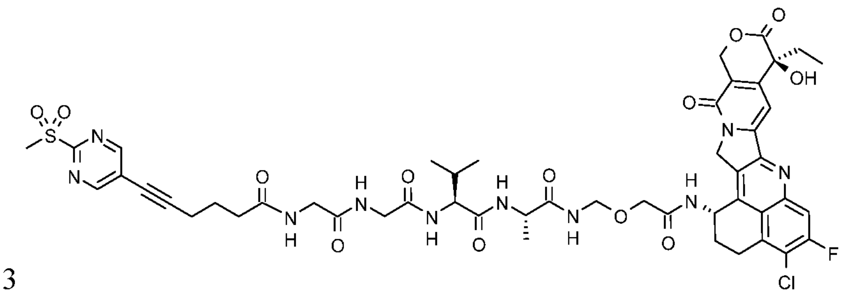

A-33

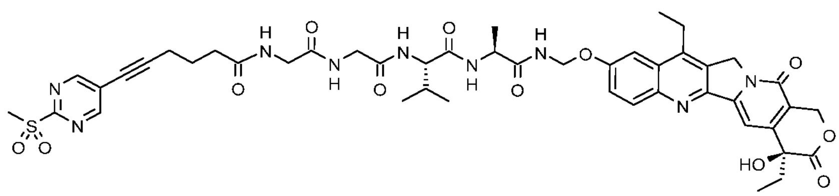

A-34

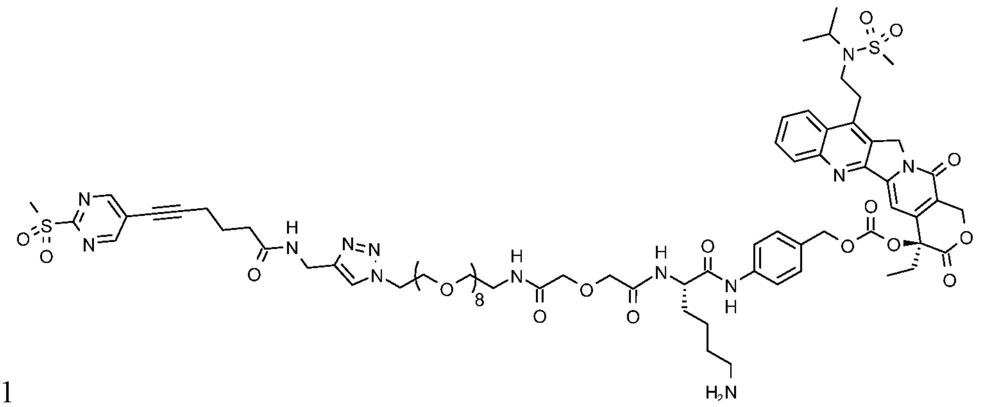

B-01

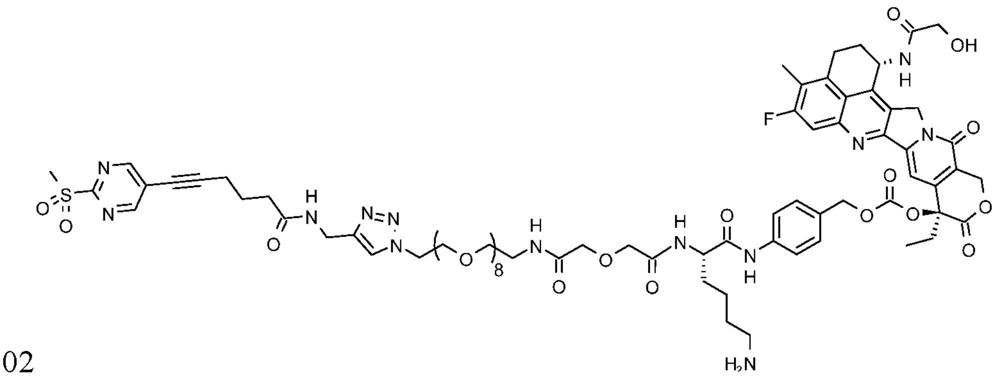

B-02

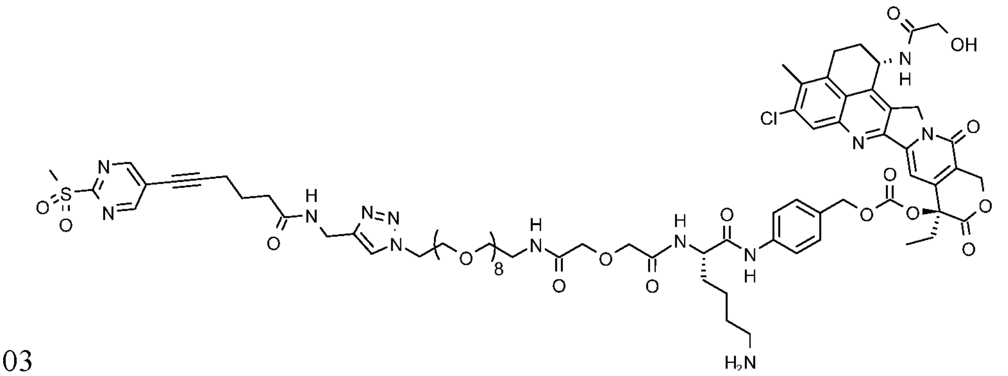

B-03

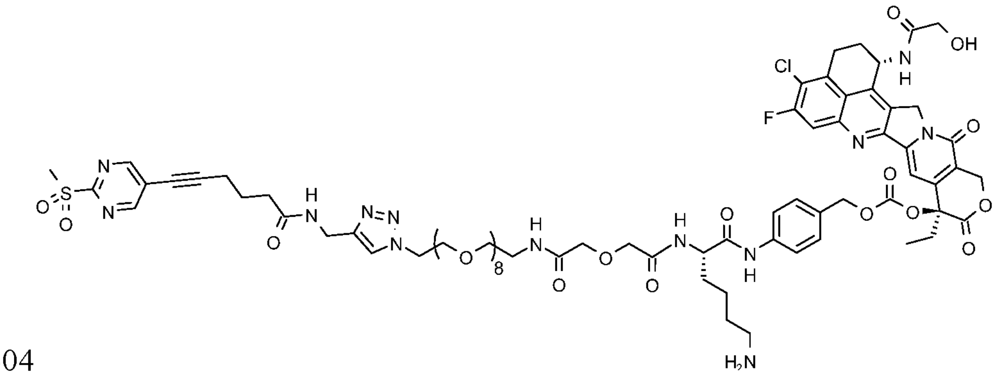

B-04

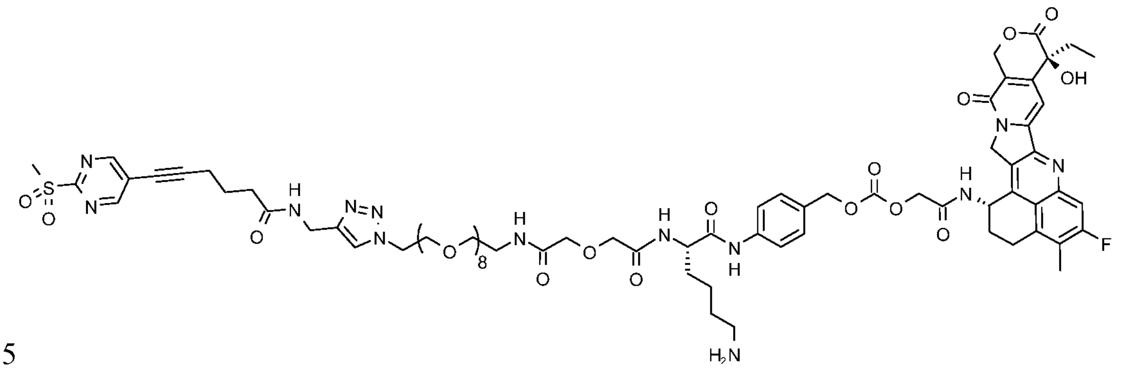

B-05

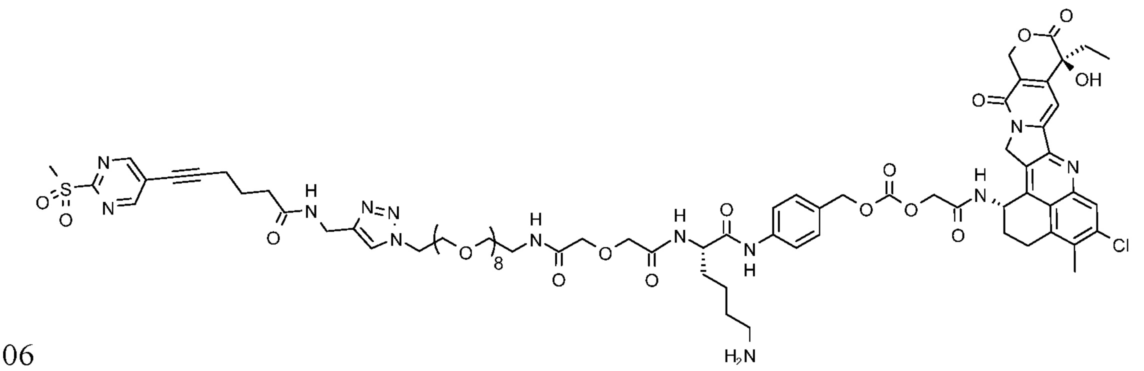

B-06

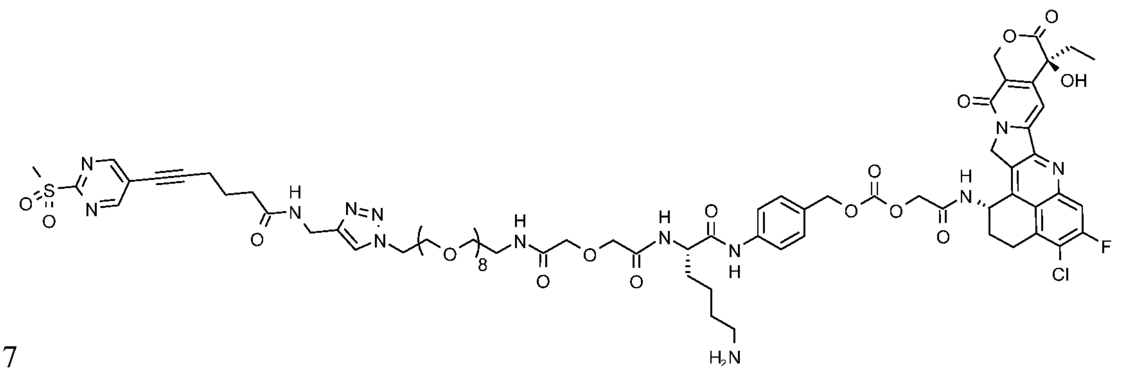

B-07

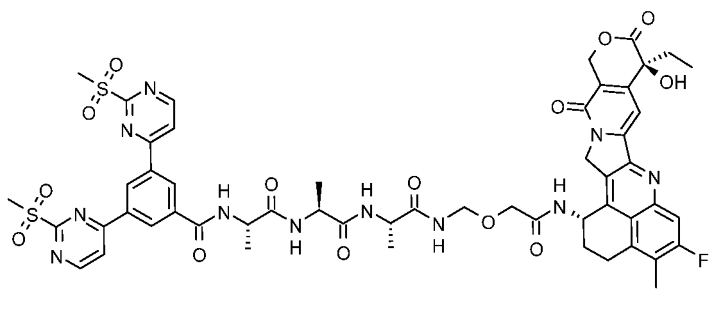

C-01

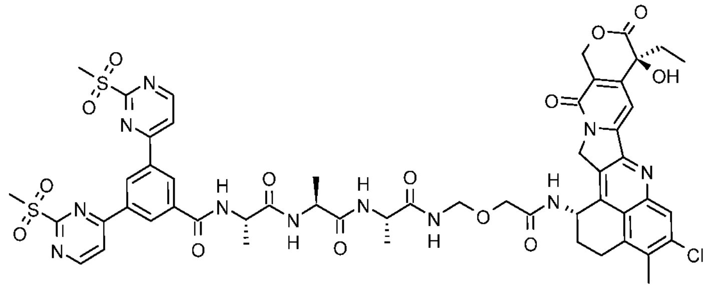

C-02

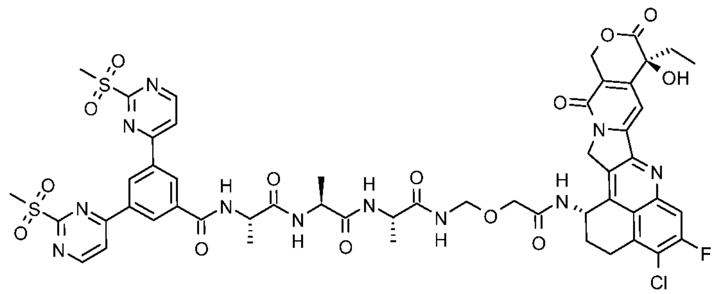

C-03

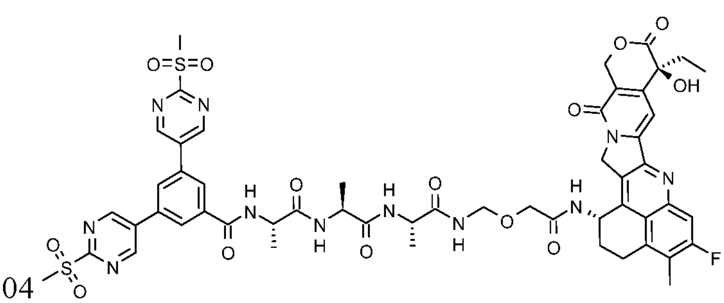

C-04

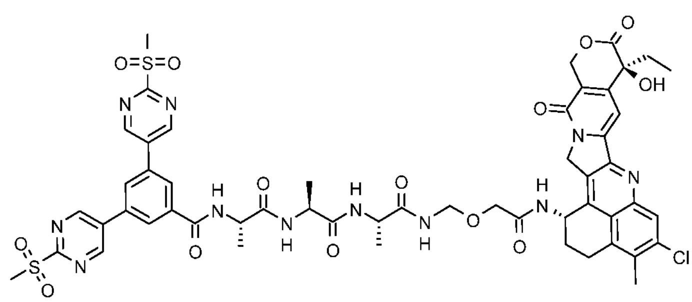

C-05

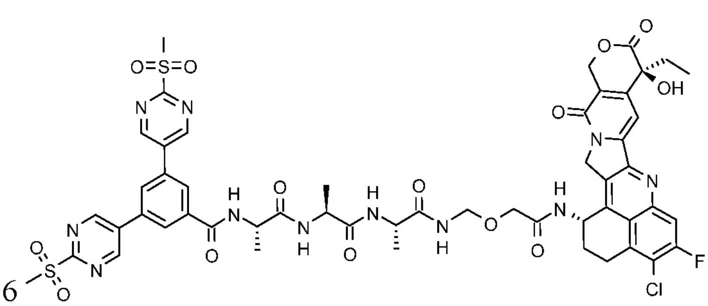

C-06

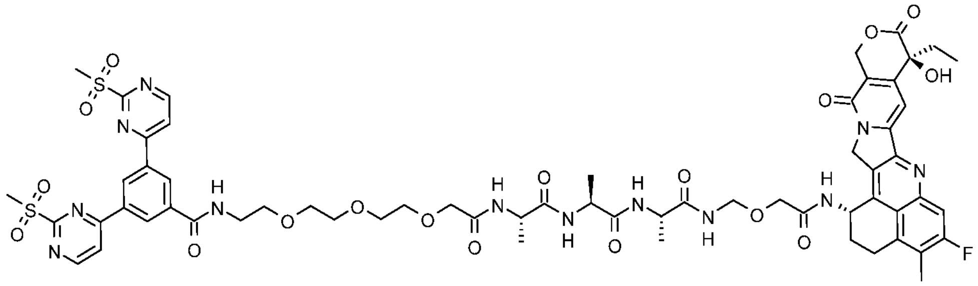

C-07

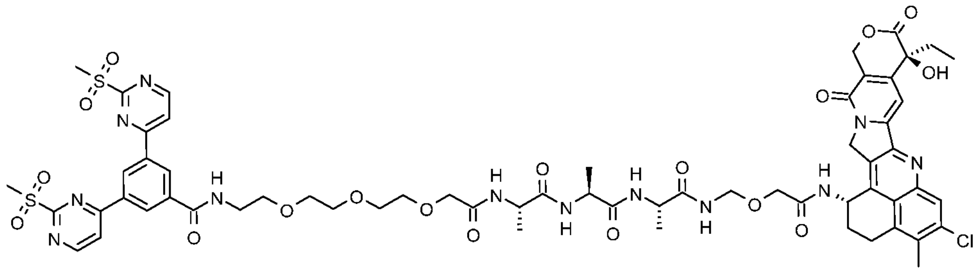

C-08

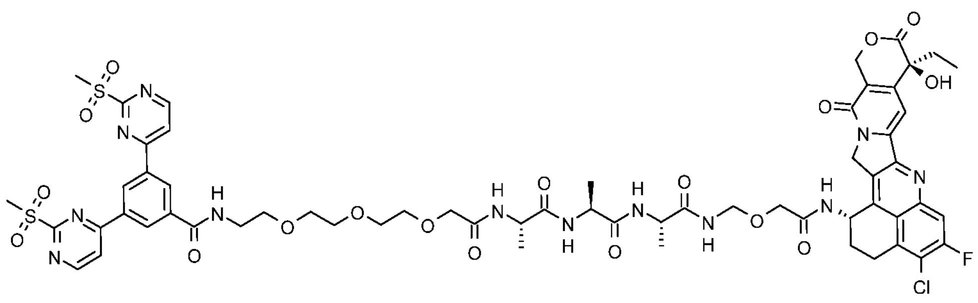

C-09

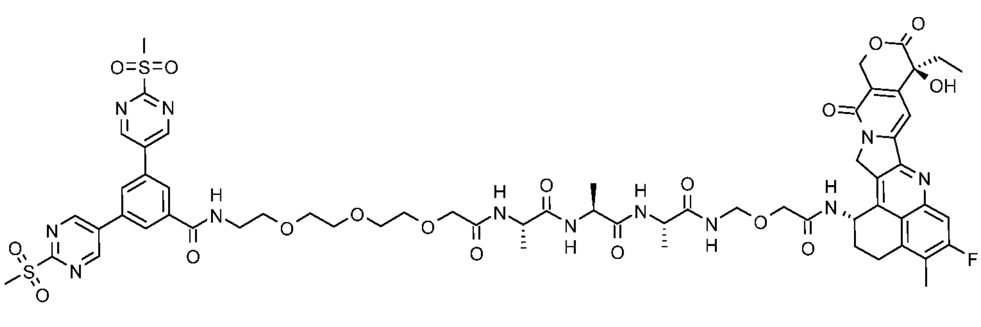

C-10

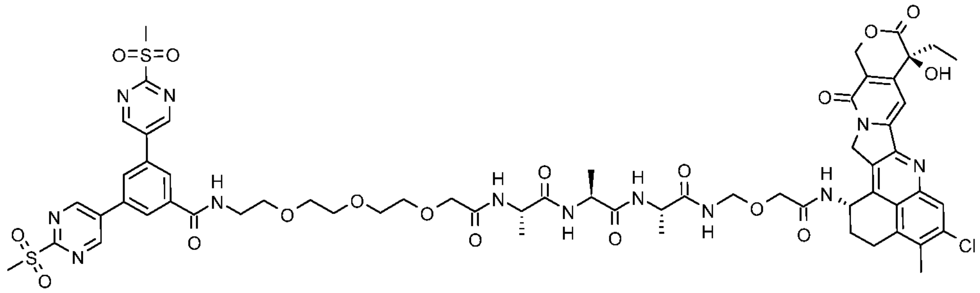

C-11

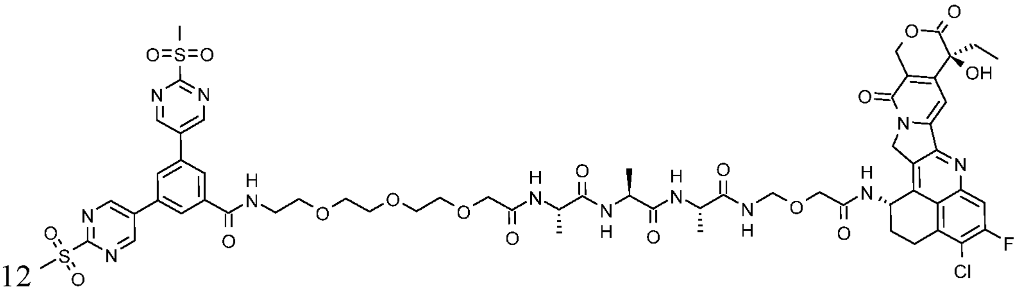

C-12

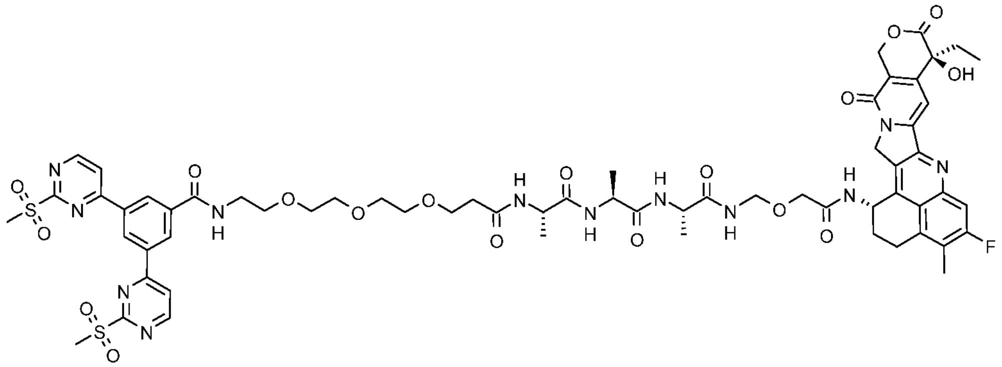

C-13

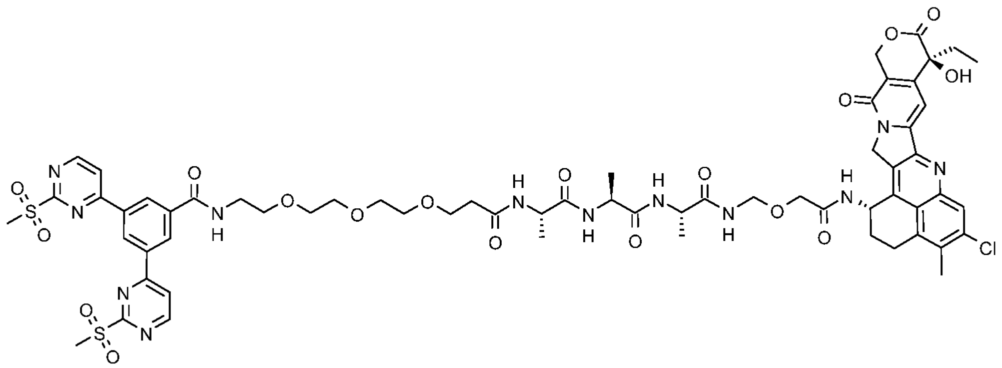

C-14

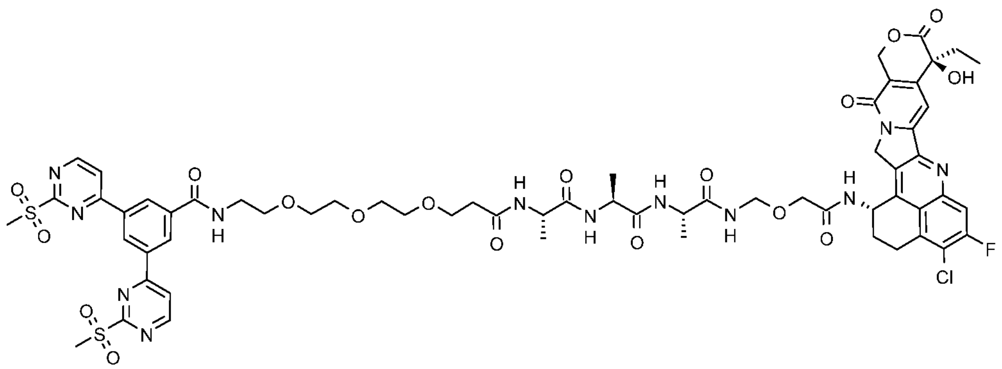

C-15

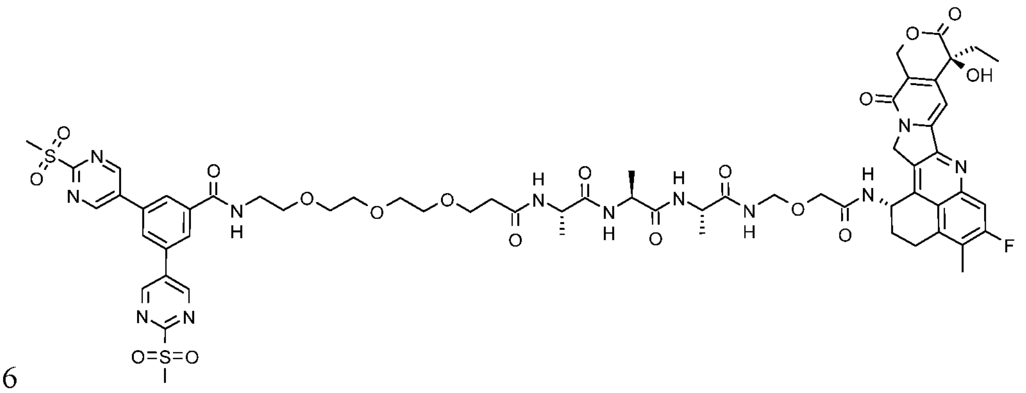

C-16

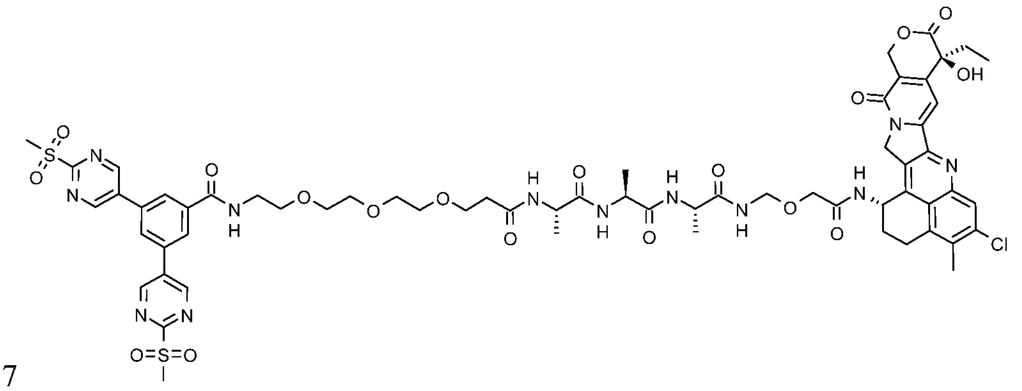

C-17

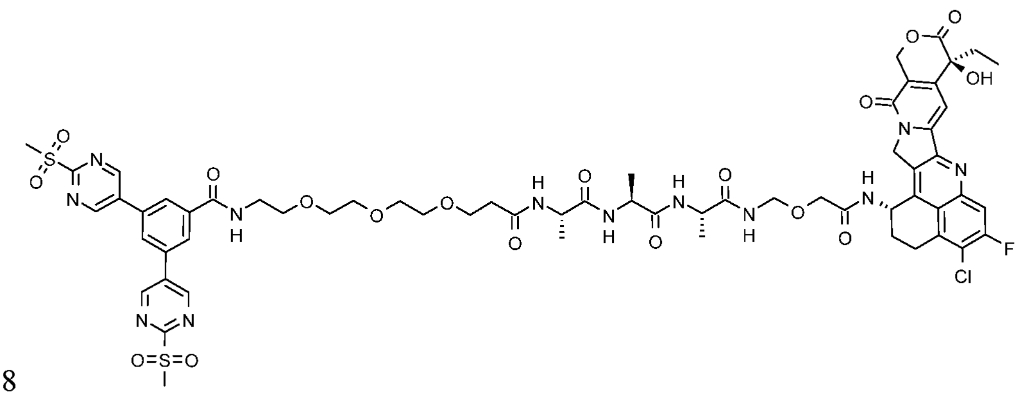

C-18

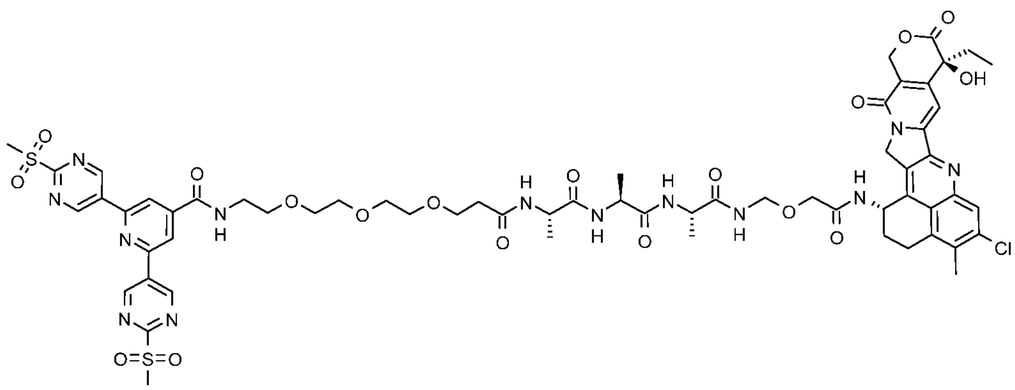

C-19

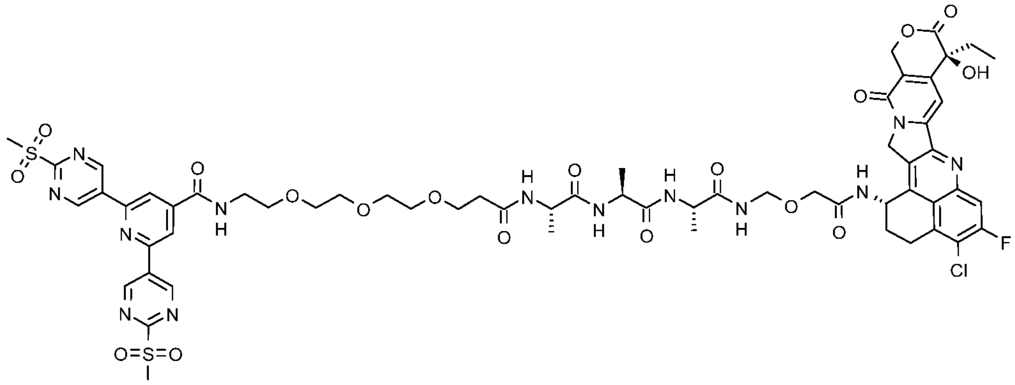

C-20

C-21

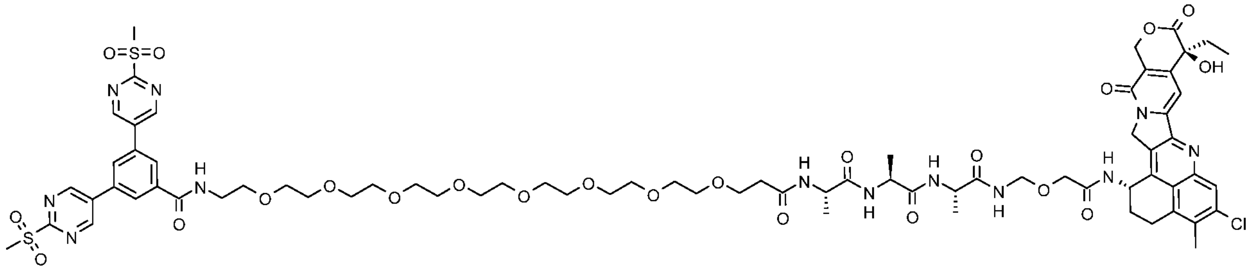

C-22

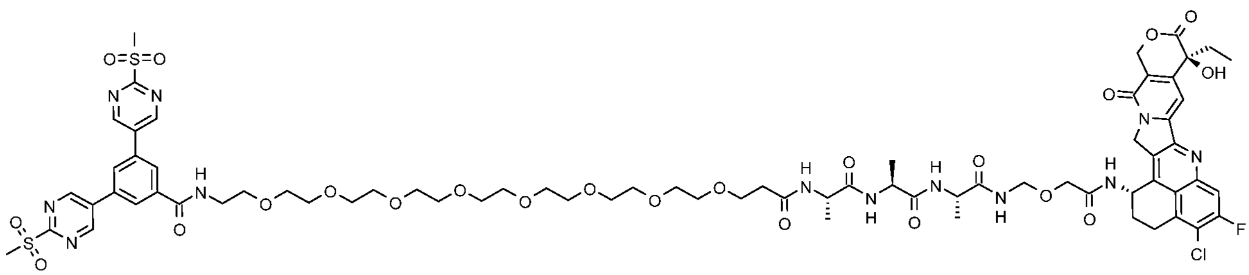

C-23

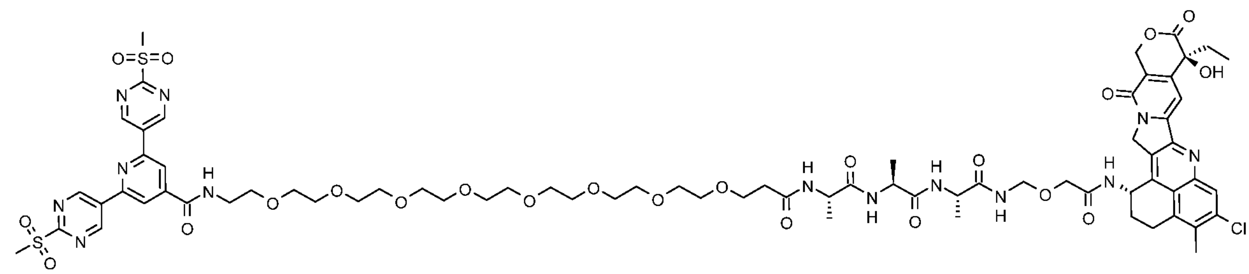

C-24

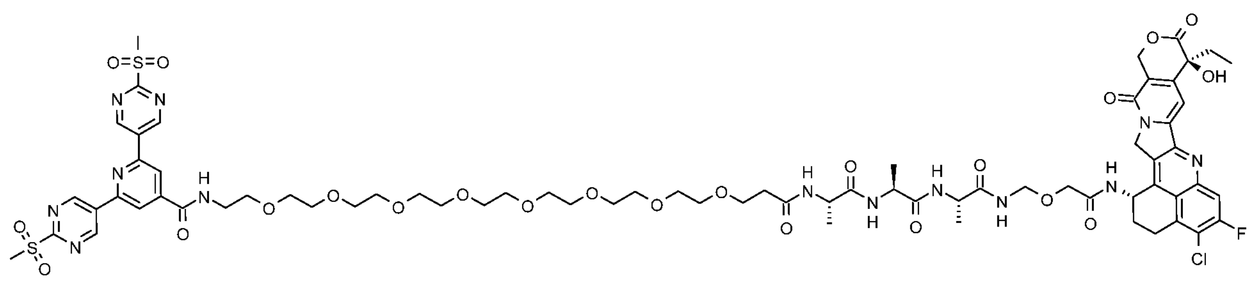

C-25

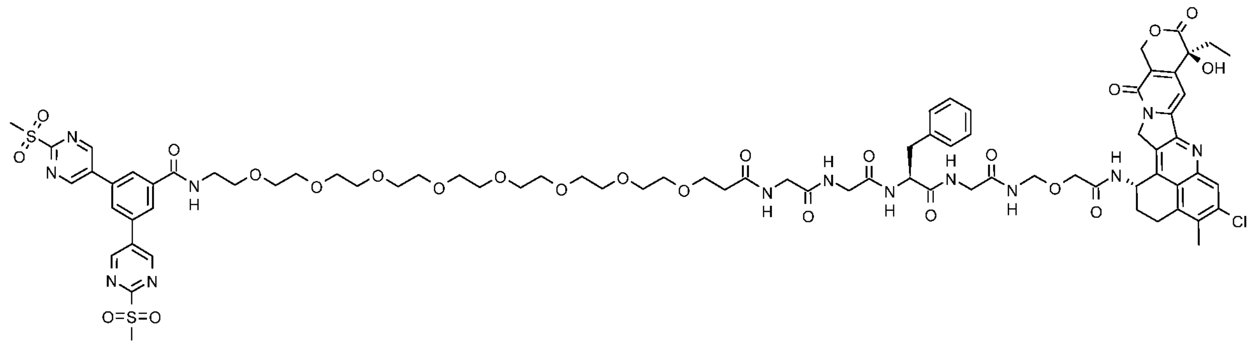

C-26

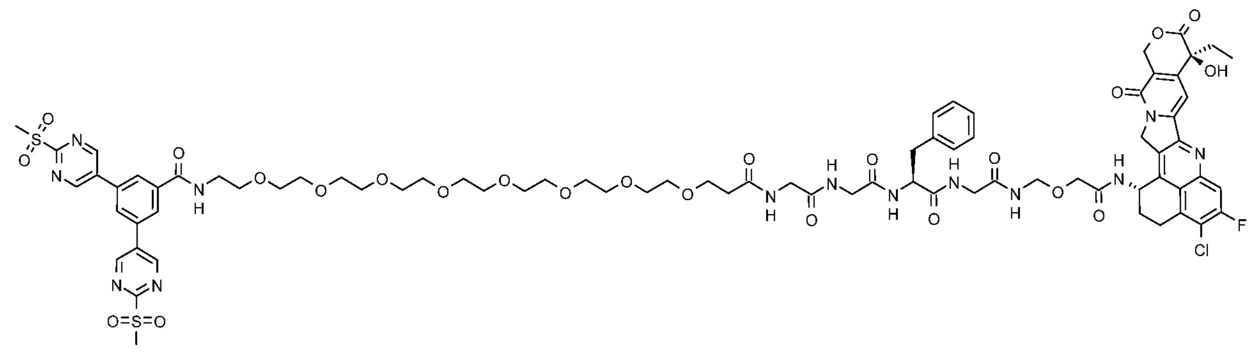

C-27

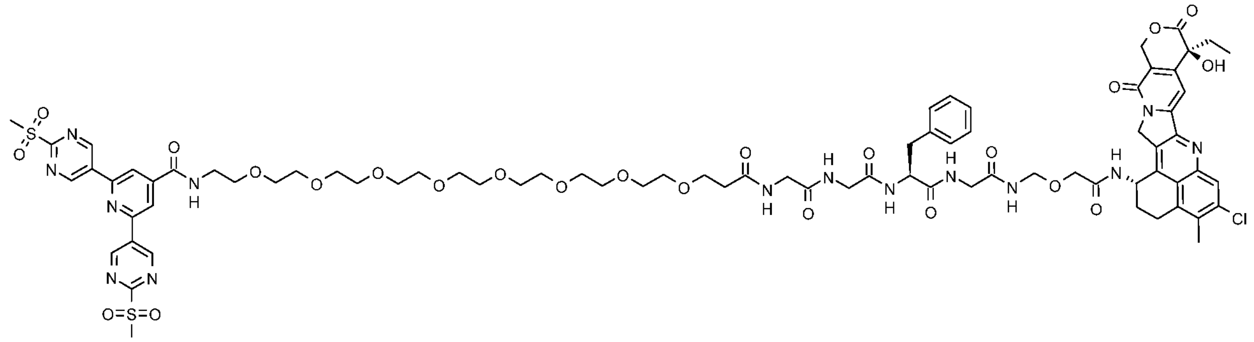

C-28

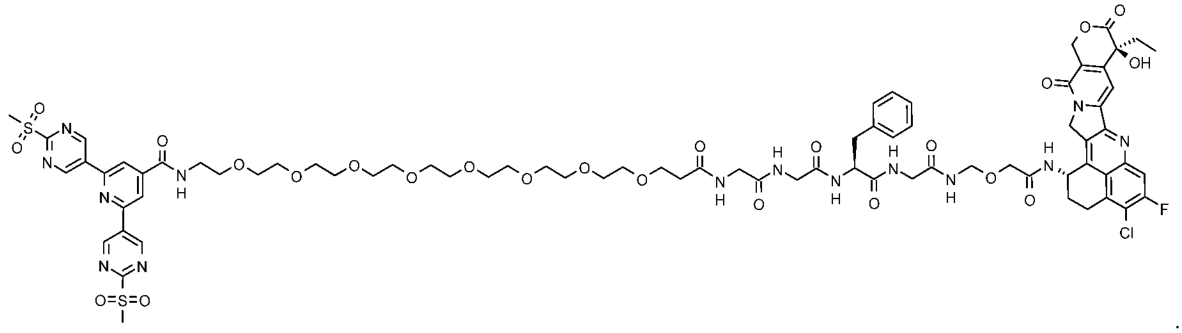

.

In some embodiments, the drug-linker compound of the present invention is selected from the group consisting of:

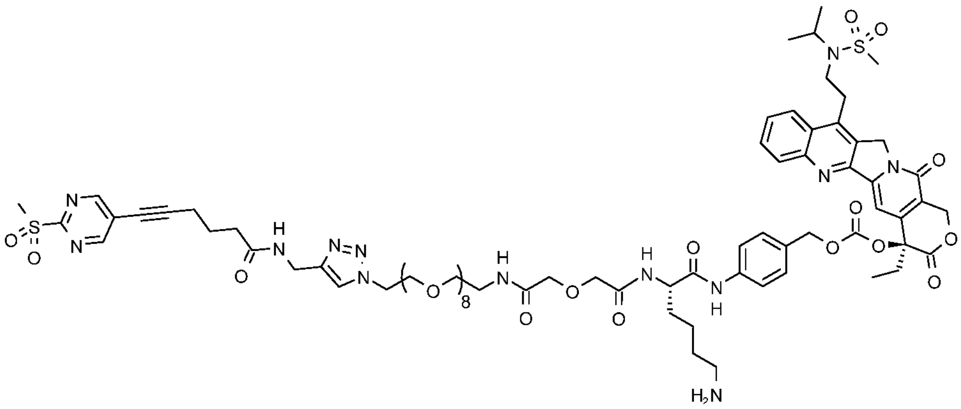

;

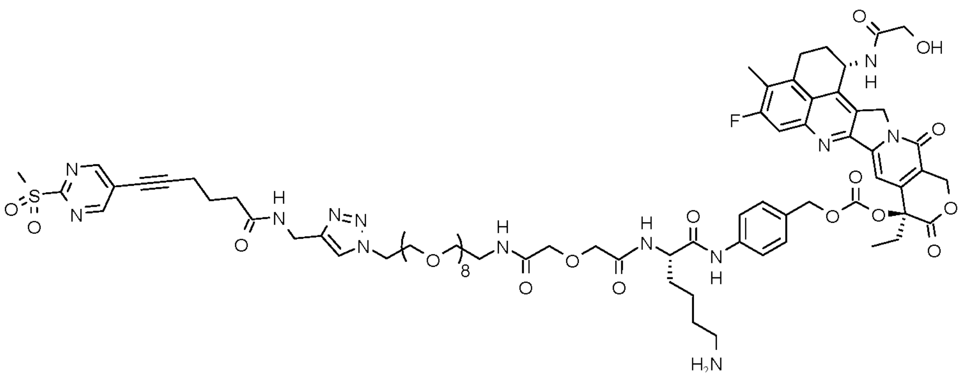

;

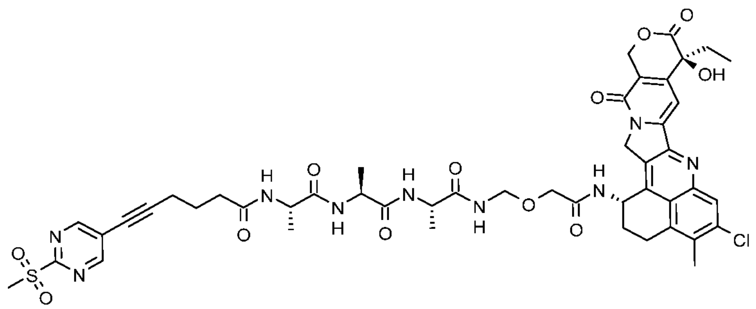

.

In some embodiments, the drug-linker compound described above may be optionally substituted by one or more suitable substituents.

In some embodiments, the drug-linker compound described above has the following structure:

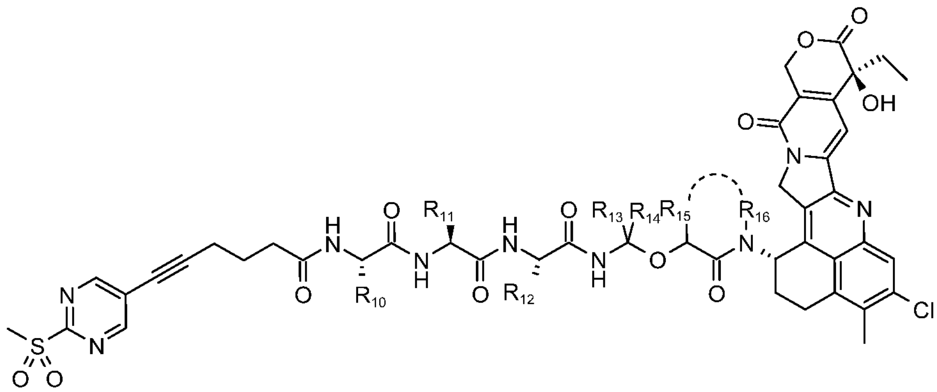

$R_{10}$, $R_{11}$ and $R_{12}$ are each independently selected from the group consisting of H, $C_{1-6}$ alkyl, $C_{3-6}$ cycloalkyl, 5- to 12-membered heterocyclyl, $C_{6-10}$ aryl, 5- to 12-membered heteroaryl, -$C_{1-6}$ alkyl-$C_{6-10}$ aryl and -$C_{1-6}$ alkyl-5- to 12-membered heteroaryl; the alkyl, cycloalkyl, heterocyclyl, aryl and heteroaryl are optionally substituted by one or more substituents selected from the group consisting of hydroxyl, CN, halogen, $C_{1-6}$ alkyl, halo $C_{1-6}$ alkyl, $C_{1-6}$ alkoxy, $C_{6-10}$ aryl, and 5- to 12-membered heteroaryl;

$R_{13}$ and $R_{14}$ are each independently selected from the group consisting of H, $C_{1-6}$ alkyl, $C_{3-6}$ cycloalkyl and 4- to 6-membered heterocyclyl; the alkyl, cycloalkyl and heterocyclyl are optionally substituted by one or more substituents selected from the group consisting of hydroxy, CN, halogen, $C_{1-6}$ alkyl, halo $C_{1-6}$ alkyl, $C_{1-6}$ alkoxy, $C_{6-10}$ aryl and 5- to 12-membered heteroaryl;

$R_{15}$ is selected from the group consisting of H, $C_{1-6}$ alkyl, $C_{3-6}$ cycloalkyl, halo $C_{1-6}$ alkyl, $C_{1-6}$ alkyl-O-$C_{1-6}$ alkyl, $C_{2-6}$ alkenyl, $C_{2-6}$ alkynyl and 3- to 6-membered heterocycloalkyl; $R_{16}$ is H; or, $R_{15}$ and $R_{16}$ and the atoms connected thereto jointly form a 4- to 7-membered ring; the 4- to 7-membered ring is optionally substituted by one or more substituents selected from the group consisting of hydroxyl, CN, halogen, $C_{1-6}$ alkyl, halo $C_{1-6}$ alkyl, $C_{1-6}$ alkoxy, $C_{6-10}$ aryl, and 5- to 12-membered heteroaryl.

In some embodiments, $R_{10}$, $R_{11}$ and $R_{12}$ are each independently selected from the group consisting of H, $C_{1-6}$ alkyl, $C_{3-6}$ cycloalkyl, $C_{6-10}$ aryl, benzyl, hydroxy-substituted benzyl, and indolyl-$C_{1-6}$ alkyl;

$R_{13}$ and $R_{14}$ are each independently selected from the group consisting of H, $C_{1-6}$ alkyl, $C_{3-6}$ cycloalkyl, and 4- to 6-membered heterocyclyl; and

$R_{15}$ is selected from the group consisting of H, $C_{1-6}$ alkyl, $C_{3-6}$ cycloalkyl, halo $C_{1-6}$ alkyl, $C_{1-6}$ alkyl-O-$C_{1-6}$ alkyl, $C_{2-6}$ alkenyl, $C_{2-6}$ alkynyl and 3- to 6-membered heterocycloalkyl; $R_{16}$ is H; or, $R_{15}$ and $R_{16}$ and the atoms connected thereto jointly form a 4- to 7-membered ring.

In some embodiments, $R_{10}$, $R_{11}$ and $R_{12}$ are each independently selected from the group consisting of H, $C_{1-4}$ alkyl, $C_{3-6}$ cycloalkyl, phenyl, benzyl, p-hydroxybenzyl, and indolylmethyl.

In some embodiments, $R_{13}$ and $R_{14}$ are each independently selected from the group consisting of H, $C_{1-4}$ alkyl, $C_{3-6}$ cycloalkyl, and 4- to 6-membered heterocyclyl.

In some embodiments, $R_{13}$ and $R_{14}$ are each independently selected from the group consisting of H, methyl, ethyl, propyl, isopropyl, butyl, isobutyl, tert-butyl, cyclopropyl, cyclobutyl, cyclopentyl, cyclohexyl and 4- to 6-membered heterocyclyl.

In some embodiments, $R_{15}$ is selected from the group consisting of H, $C_{1-4}$ alkyl, $C_{3-6}$ cycloalkyl, halo $C_{1-4}$ alkyl, $C_{1-4}$ alkyl-O-$C_{1-4}$ alkyl, $C_{2-6}$ alkenyl, $C_{2-6}$ alkynyl and 3- to 6-membered heterocycloalkyl; or, $R_{15}$ and $R_{16}$ and the atoms connected thereto jointly form a 4-to 7-membered heterocycloalkyl or a 4- to 7-membered heteroaryl.

In some embodiments, $R_{15}$ is selected from the group consisting of H, methyl, ethyl, propyl, isopropyl, butyl, isobutyl, tert-butyl, cyclopropyl, cyclobutyl, cyclopentyl, cyclohexyl, halo $C_{1-4}$ alkyl, $C_{1-4}$ alkyl-O-$C_{1-4}$ alkyl, $C_{2-6}$ alkenyl, $C_{2-6}$ alkynyl and 3- to 6-membered heterocycloalkyl; or, $R_{15}$ and $R_{16}$ and the atoms connected thereto jointly form a 4- to 7-membered heterocycloalkyl or a 4- to 7-membered heteroaryl.

In some embodiments, $R_{10}$, $R_{11}$ and $R_{12}$ are each independently selected from the group consisting of H, $C_{1-4}$ alkyl, $C_{3-6}$ cycloalkyl, phenyl, benzyl, p-hydroxybenzyl, and indolylmethyl;

$R_{13}$ and $R_{14}$ are each independently selected from the group consisting of H, methyl, ethyl, propyl, isopropyl, butyl, isobutyl, tert-butyl, cyclopropyl, cyclobutyl, cyclopentyl, cyclohexyl and 4- to 6-membered heterocyclyl;

$R_{15}$ is selected from the group consisting of H, methyl, ethyl, propyl, isopropyl, butyl, isobutyl, tert-butyl, cyclopropyl,

cyclobutyl, cyclopentyl, cyclohexyl, halo $C_{1-4}$ alkyl, $C_{1-4}$ alkyl-O-$C_{1-4}$ alkyl, $C_{2-6}$ alkenyl, $C_{2-6}$ alkynyl and 3- to 6-membered heterocycloalkyl; or, $R_{15}$ and $R_{16}$ and the atoms connected thereto jointly form a 4- to 7-membered heterocycloalkyl or a 4- to 7-membered heteroaryl.

In another aspect, the present invention provides an intermediate compound having a structure selected from the group consisting of:

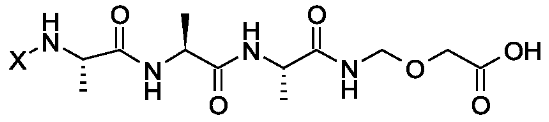
,
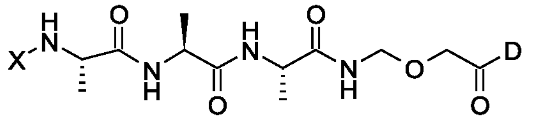
,

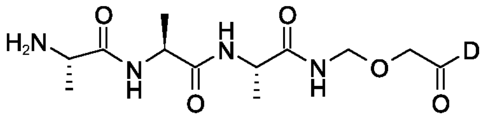
,
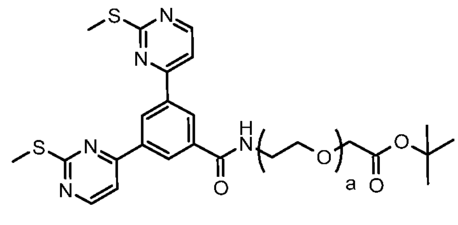
,

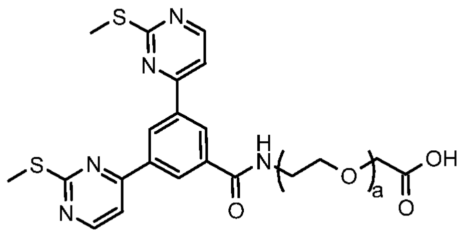
,
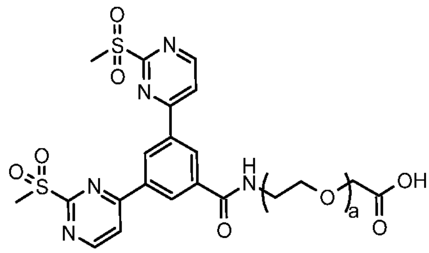
,

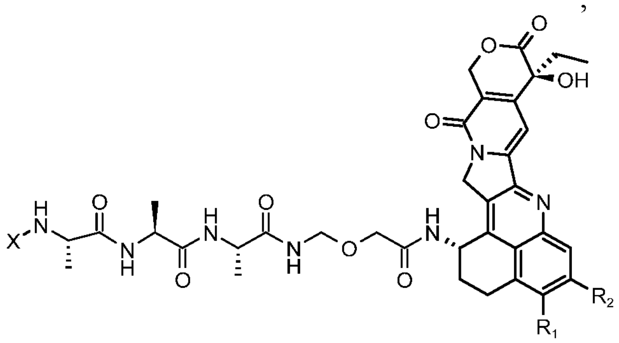
,
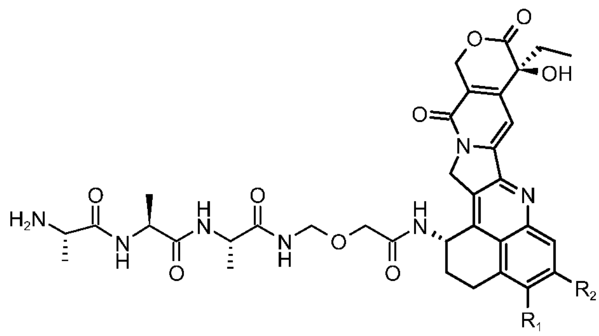
,

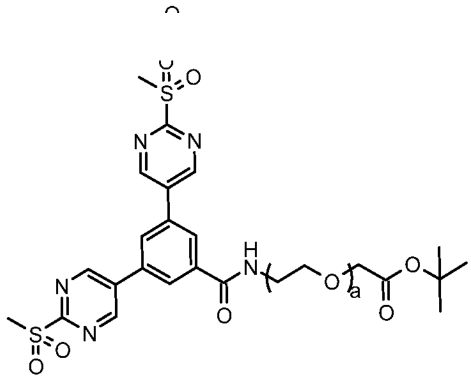
,
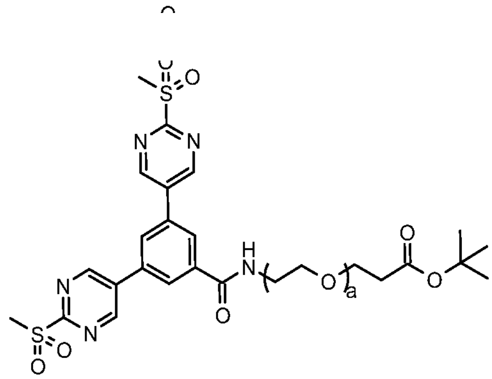
,

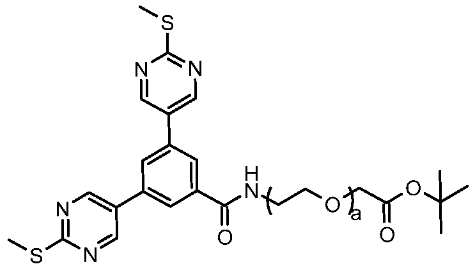

,

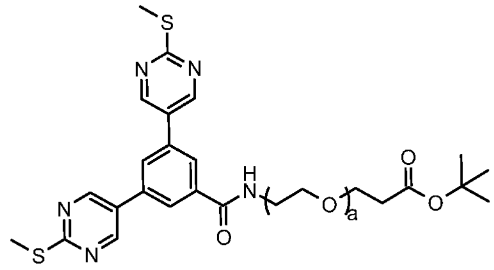

,

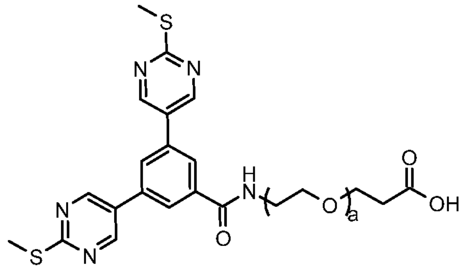

,

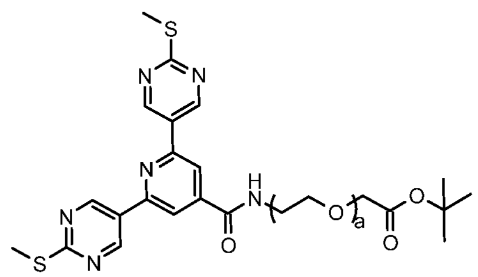

,

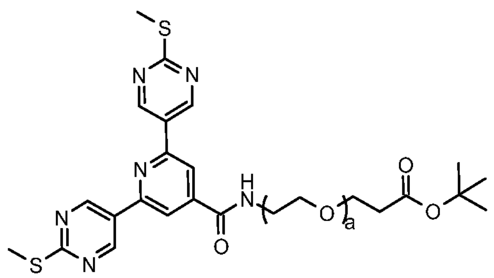

,

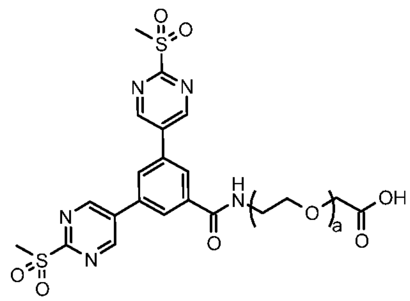

,

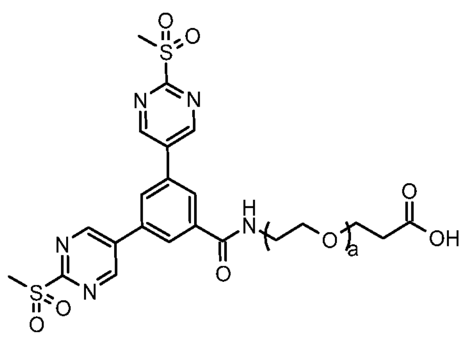

,

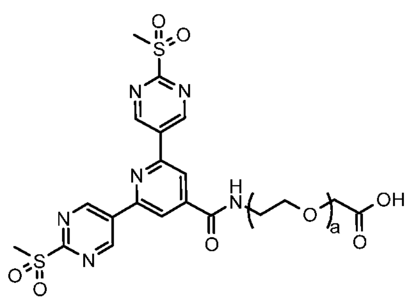

,

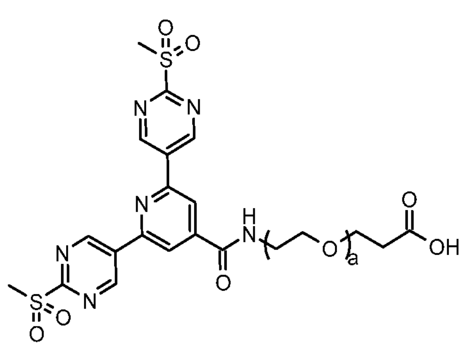

,

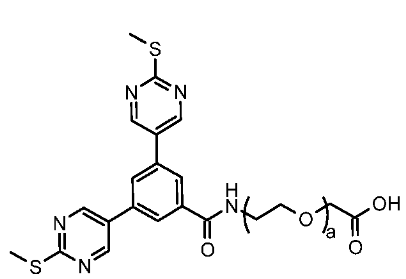

,

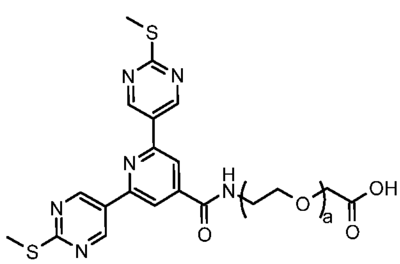

, and

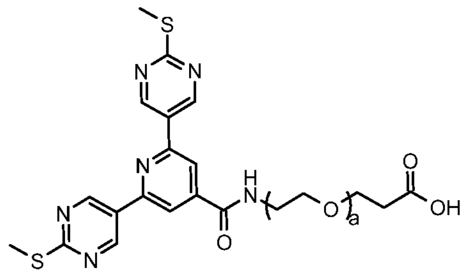

;

wherein X is selected from the group consisting of benzyloxycarbonyl, tertbutyloxycarbonyl, fluorenylmethoxycarbonyl, allyloxycarbonyl, trimethylsilylethoxycarbonyl, methoxycarbonyl, ethoxycarbonyl, phthaloyl, p-toluenesulfonyl, trifluoroacetyl, nitrobenzenesulfonyl, benzoyl, pivaloyl, triphenylmethyl, 4-methoxyphenyldiphenylmethyl, dimethoxytrityl, 2,4-dimethoxybenzyl, p-methoxybenzyl and benzyl; a is an integer from 1 to 10, preferably an integer from 3 to 8; $R_1$, $R_2$ and D are as defined above.

In some embodiments, x is Fmoc.

In some embodiments, D is selected from the group consisting of:

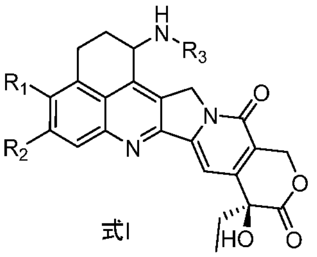

式I

and

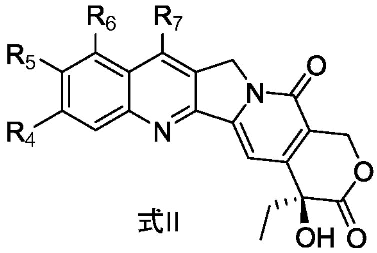

式II

wherein $R_1$ and $R_2$ are each independently selected from the group consisting of $C_{1-6}$ alkyl and halogen;

$R_3$ is selected from the group consisting of H and $-CO-CH_2OH$;

$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;

$R_6$ is selected from the group consisting of H or $-C_{1-4}$ alkylene-$NR_aR_b$;

$R_7$ is selected from the group consisting of $C_{1-6}$ alkyl and $-C_{1-4}$ alkylene-$NR_aR_b$;

wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence.

In another aspect, the present invention provides an intermediate compound having a structure selected from the group consisting of:

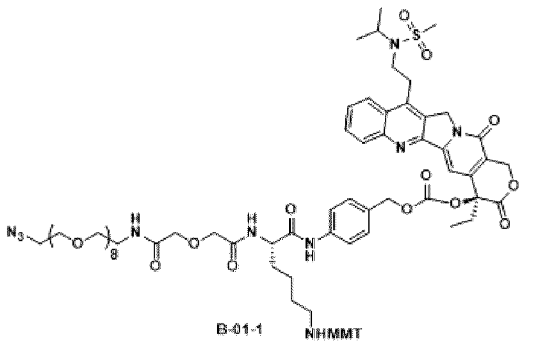

B-01-1

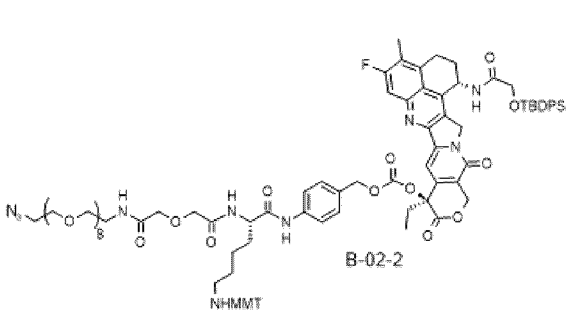

B-02-2

, ,

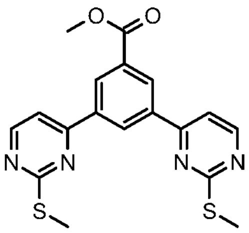

,

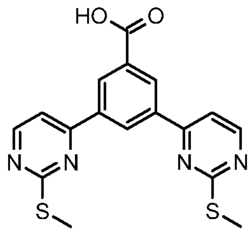

,

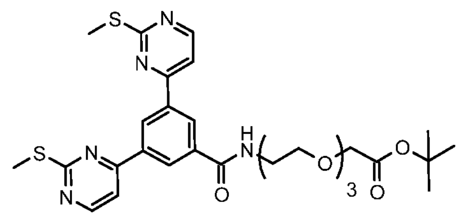

,

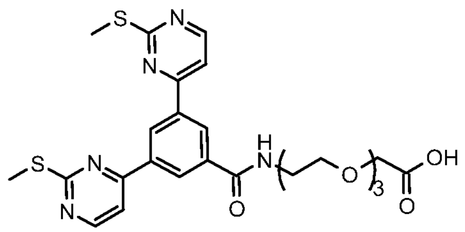

,

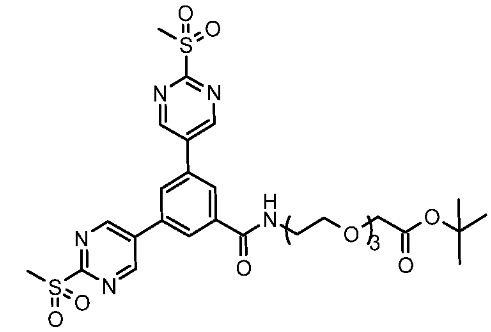

,

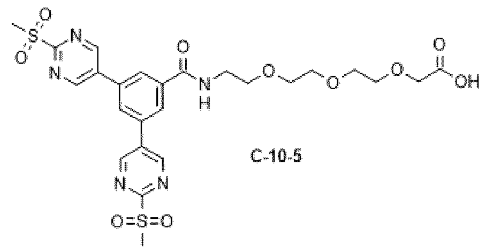

C-10-5

,

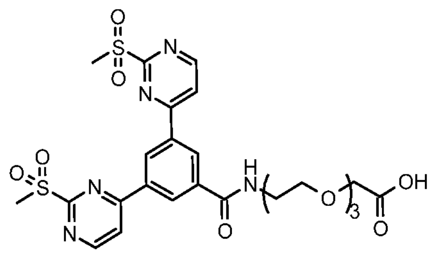

,

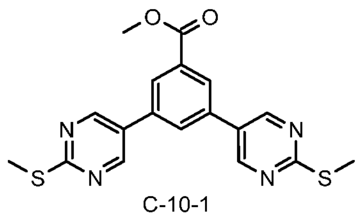

C-10-1

,

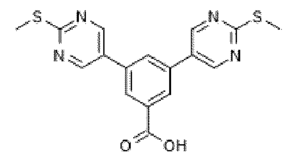

C-10-2

,

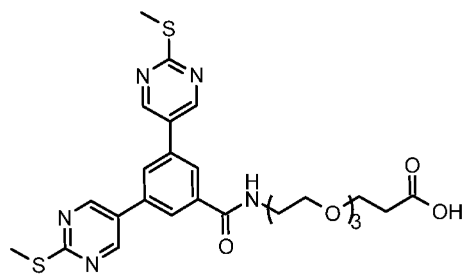

,

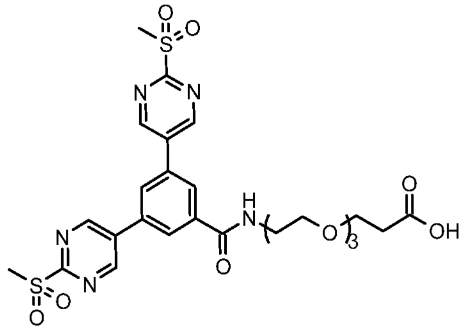

,

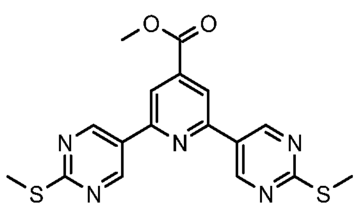

,

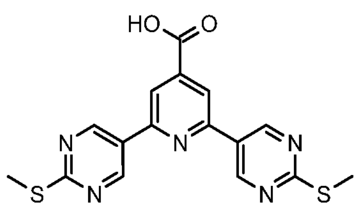

,

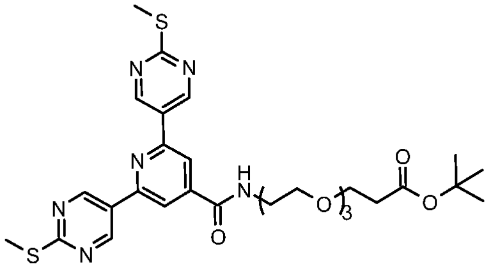

,

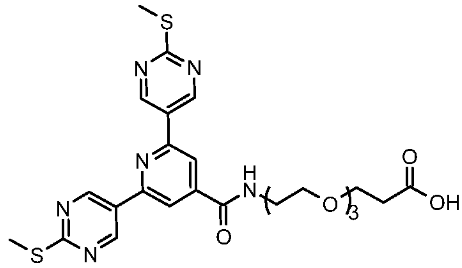

,

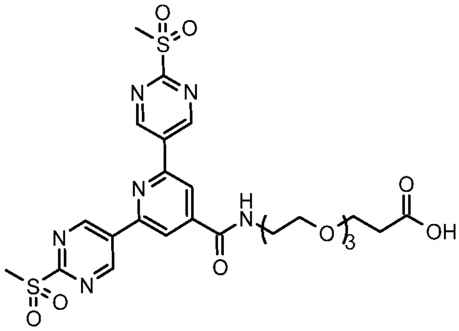

,

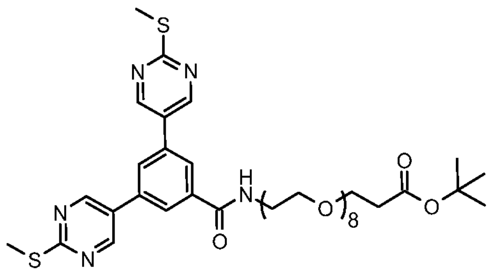

,

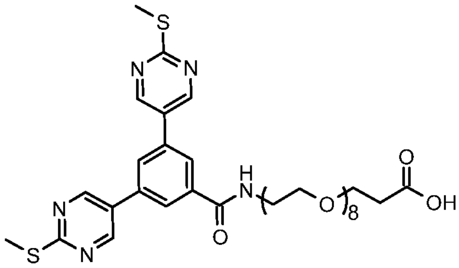

,

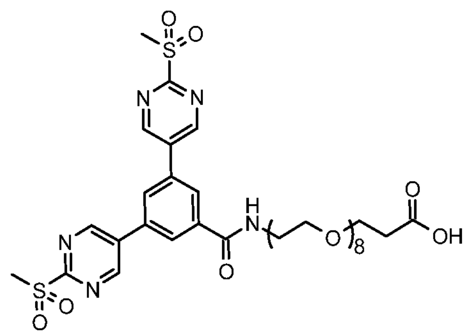

,

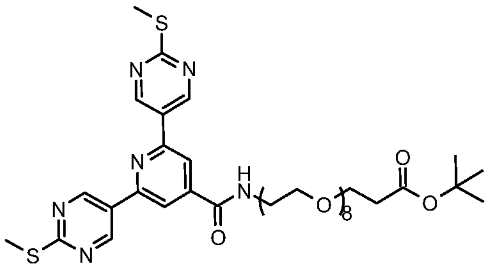

,

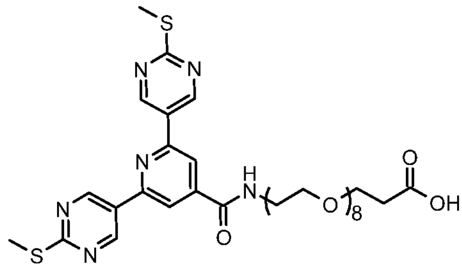

,

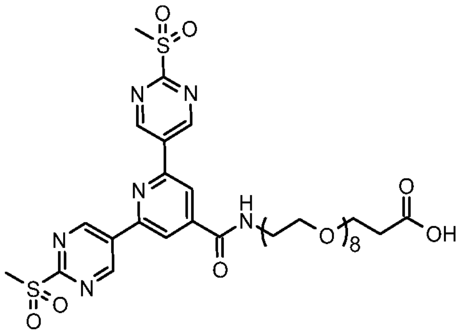

,

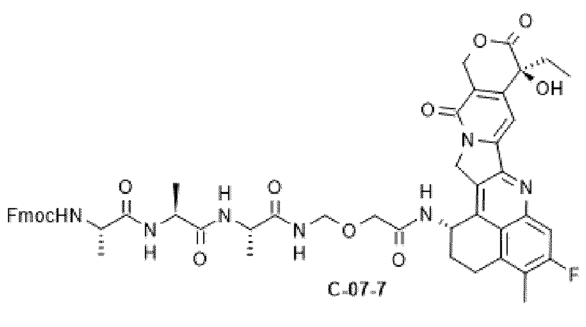

C-07-7

,

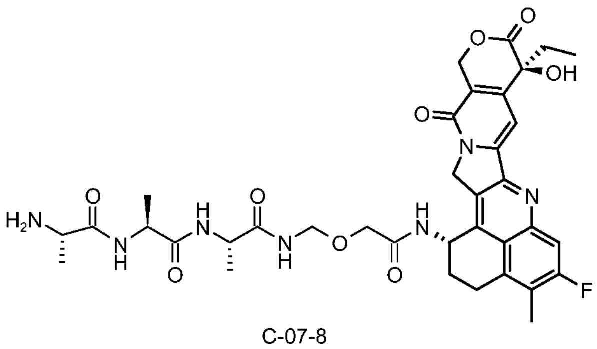

C-07-8

,

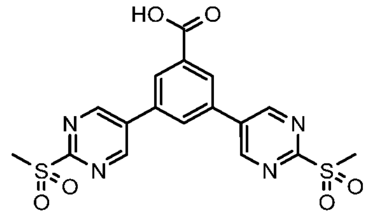

,

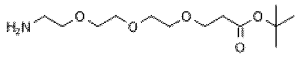

,

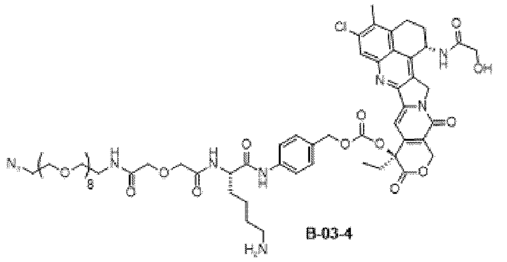

B-03-4

,

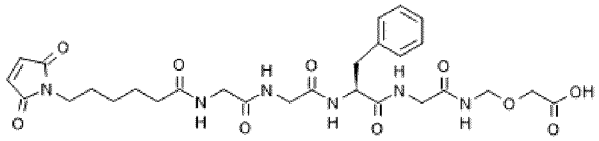

IM-1

,

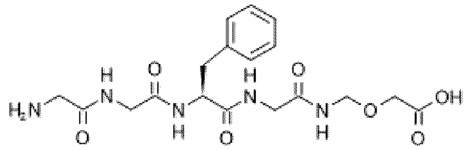

IM-3

,

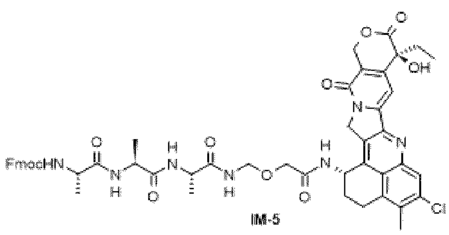

IM-5

,

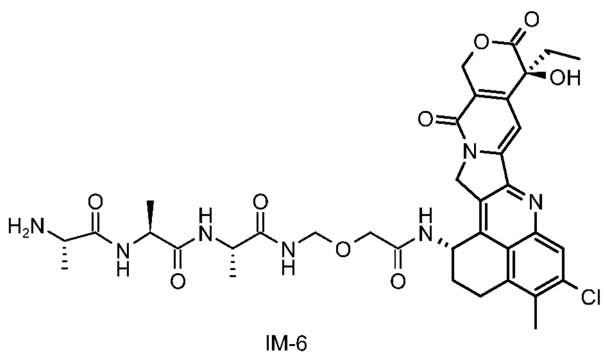

IM-6

,

and

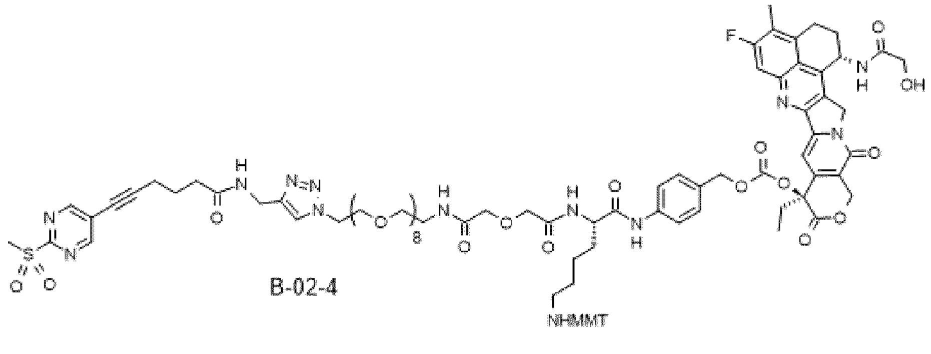

B-02-4

.

In another aspect, the present invention provides an antibody-drug conjugate having a structure represented by formula Ab-[M-L-E-D]$_x$, wherein M, L, E, D and x are as defined above, and Ab is an antibody capable of specifically binding to an antigen or an antigen-binding fragment thereof.

In some embodiments, the antibody-drug conjugate is selected from ADCA-01 to ADCA-34, ADCB-01 to ADCB-07, and ADCC-01 to ADCC-28 shown below: Ab in the formulas shown below is as defined above, wherein the sulfhydryl on the antibody and a drug-linker compound form a thioether bond through an addition reaction or a substitution reaction to obtain a complete antibody-drug conjugate, and x represents number of payload:

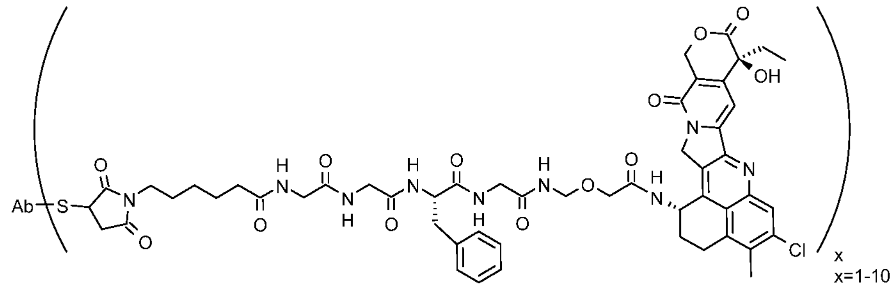

ADC A-01

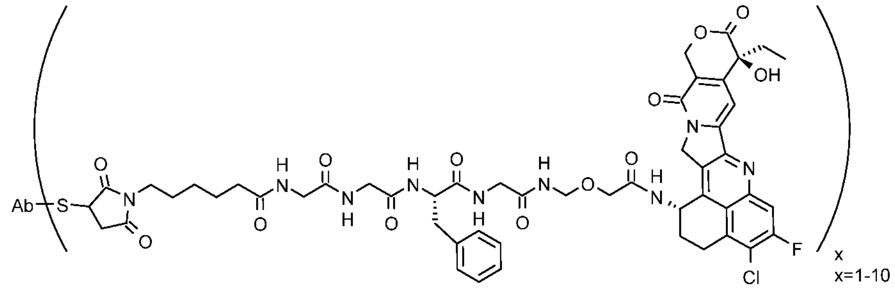

ADC A-02

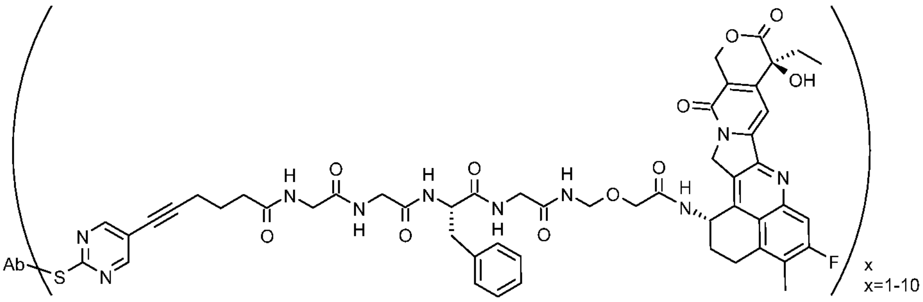

ADC A-03

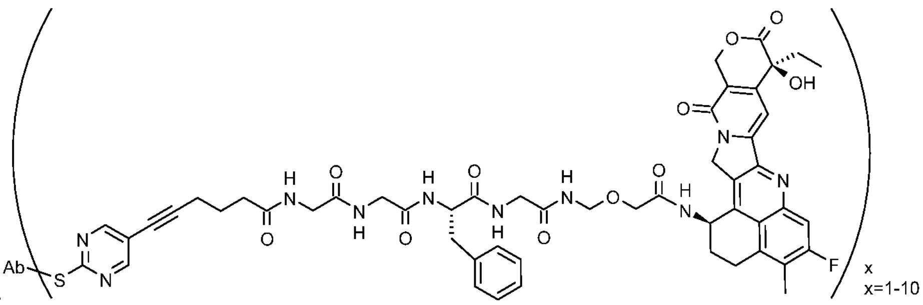

ADC A-04

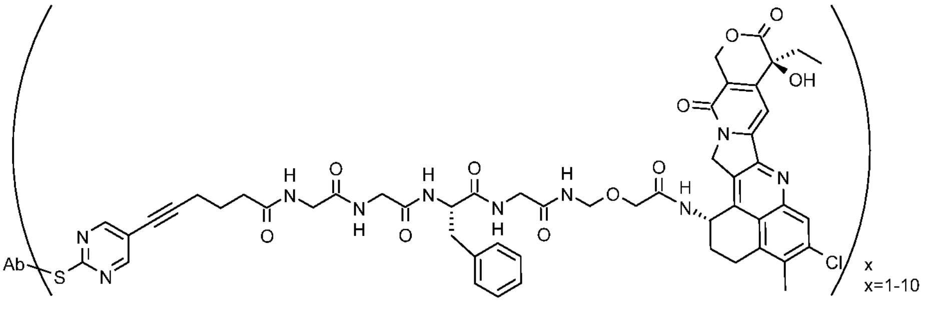

ADC A-05

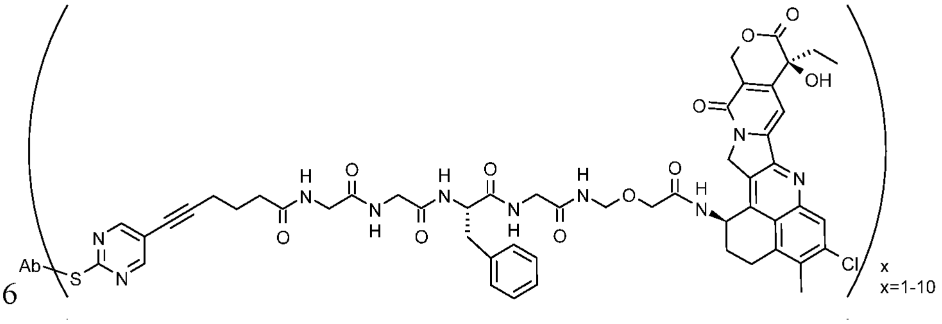

ADC A-06

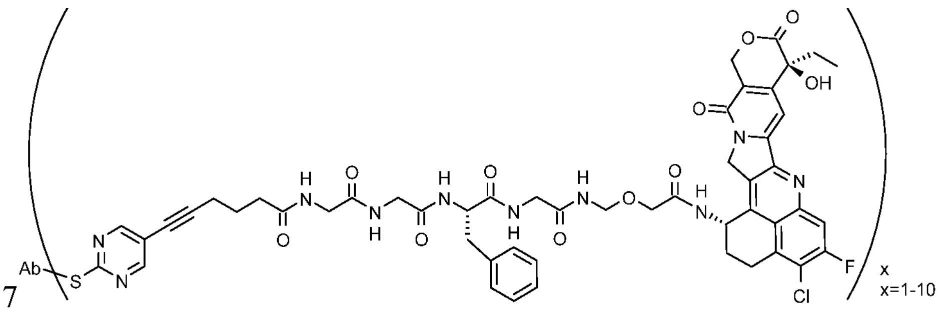

ADC A-07

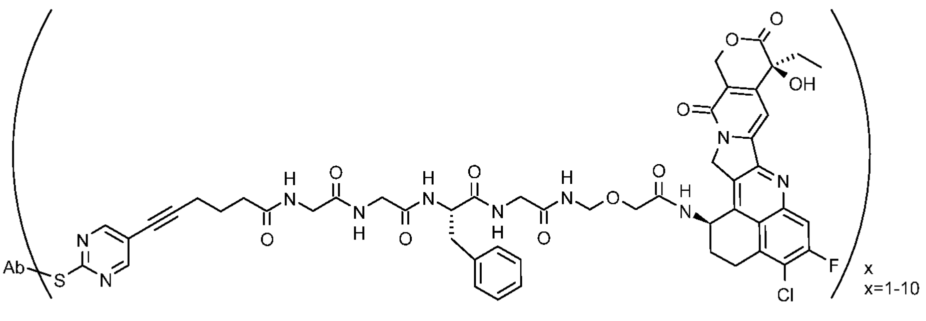

ADC A-08

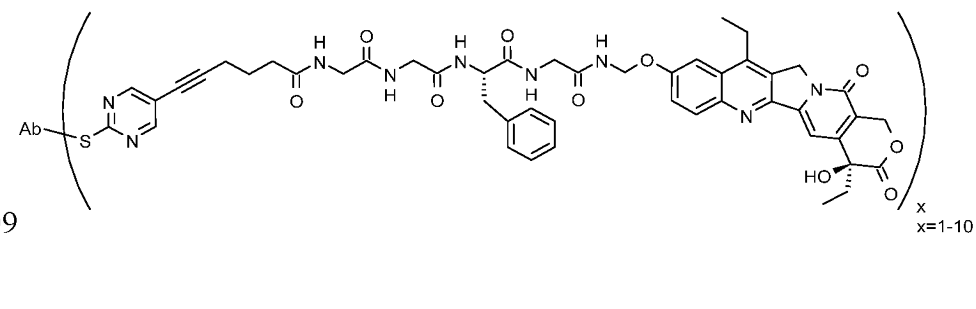

ADC A-09

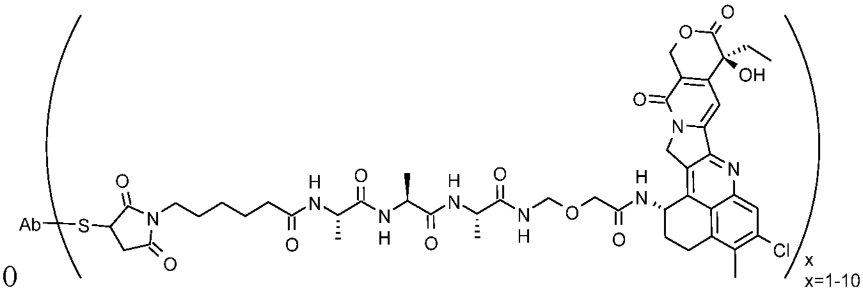

ADC A-10

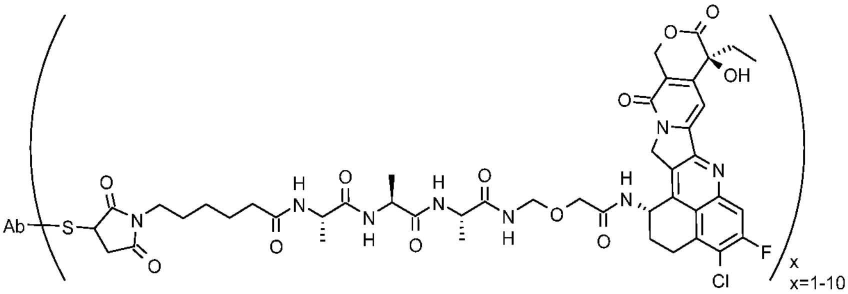

ADC A-11

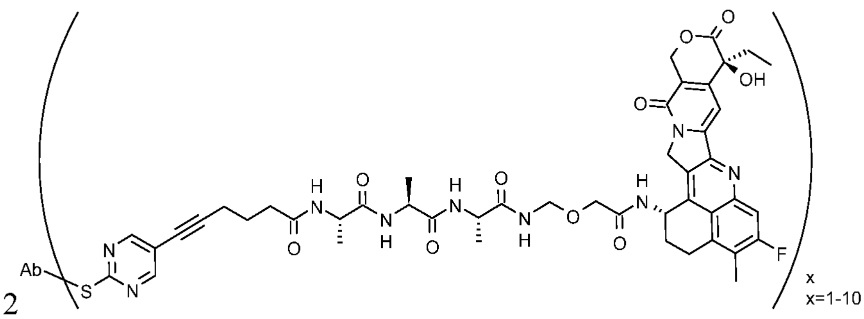

ADC A-12

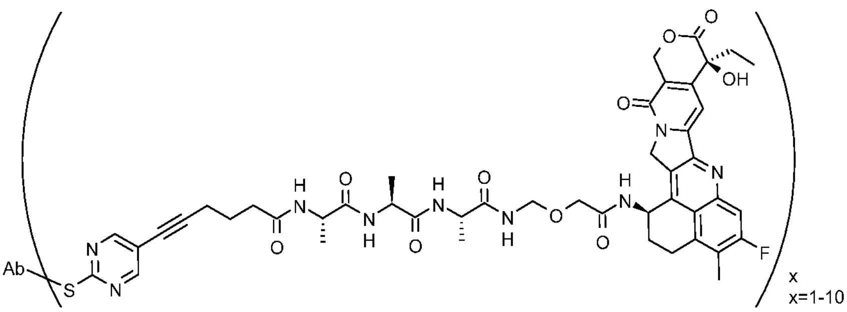

ADC A-13

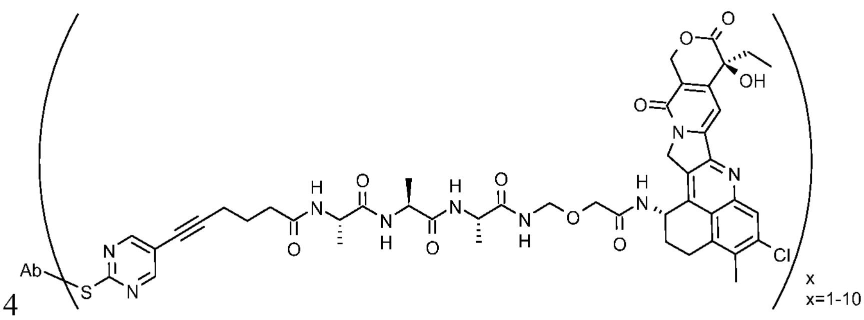

ADC A-14

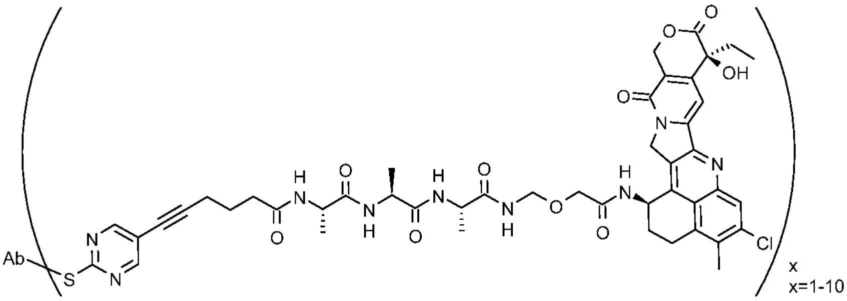

ADC A-15

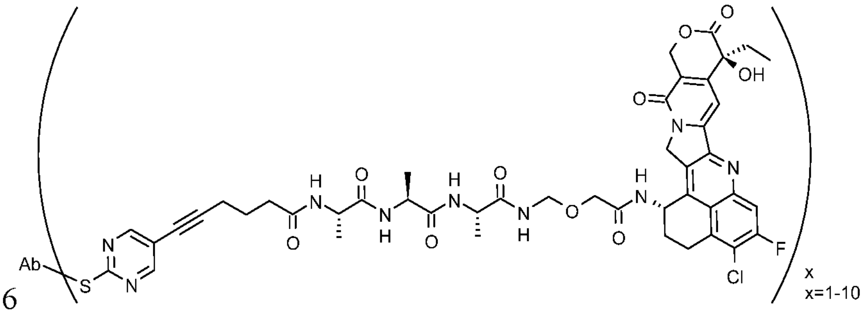

ADC A-16

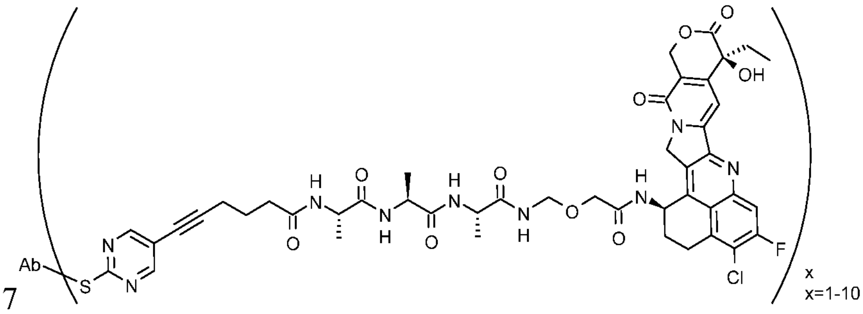

ADC A-17

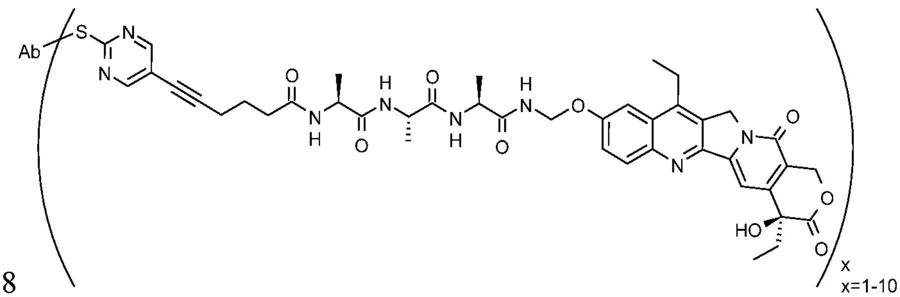

ADC A-18

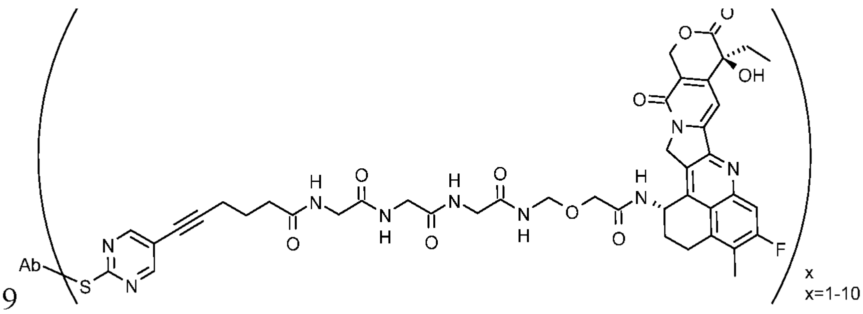

ADC A-19

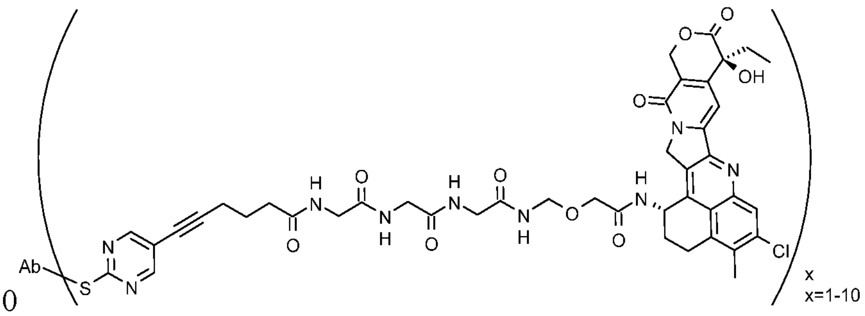

ADC A-20

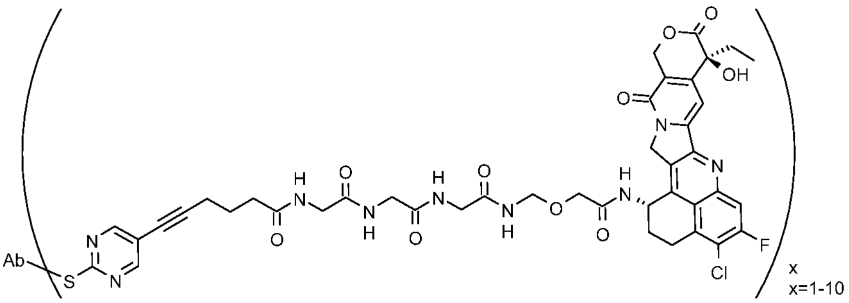

ADC A-21

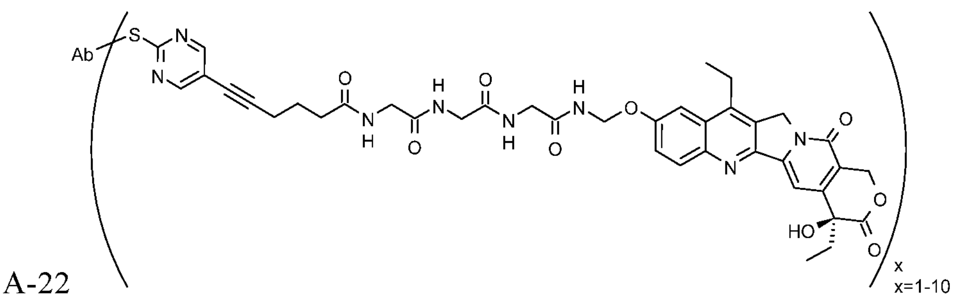

ADC A-22

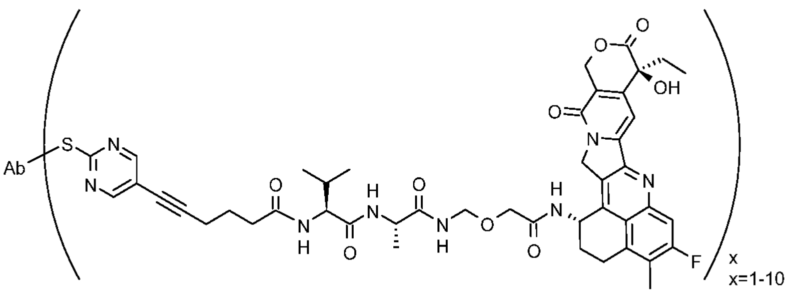

ADC A-23

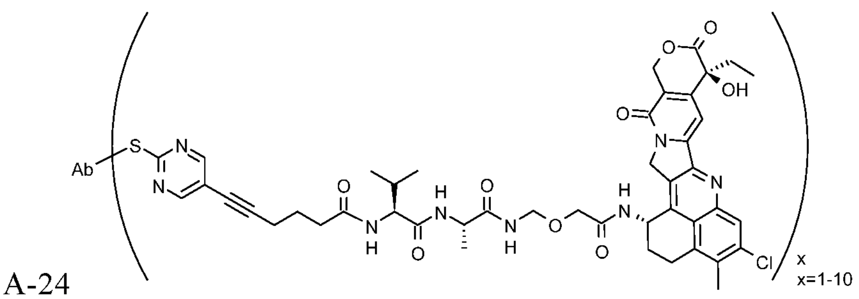

ADC A-24

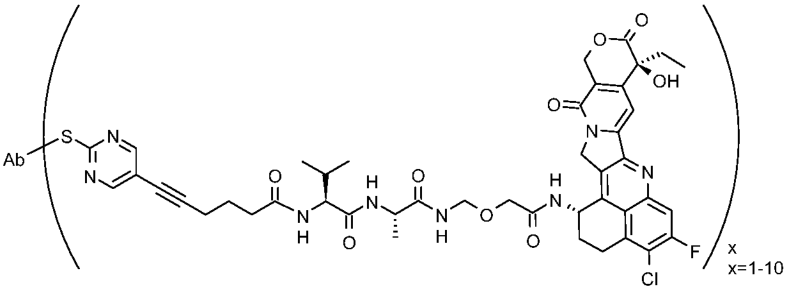

ADC A-25

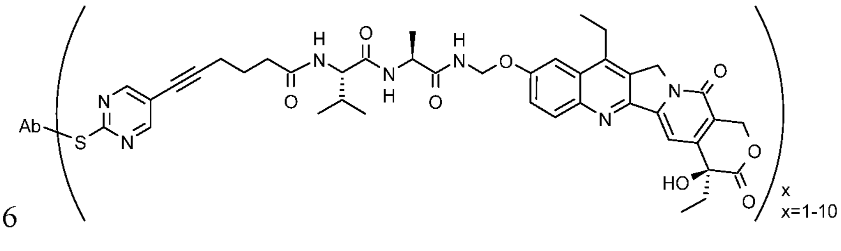

ADC A-26

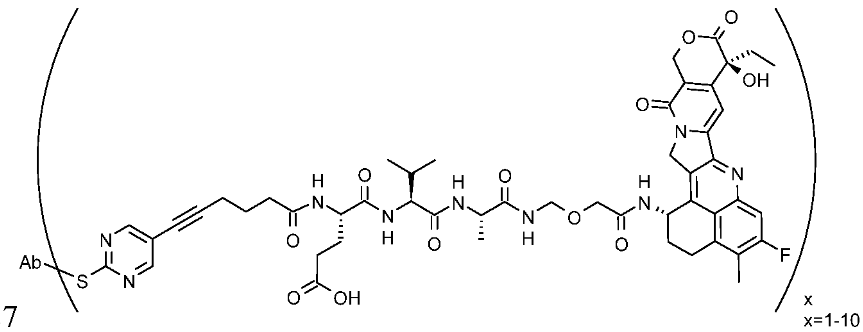

ADC A-27

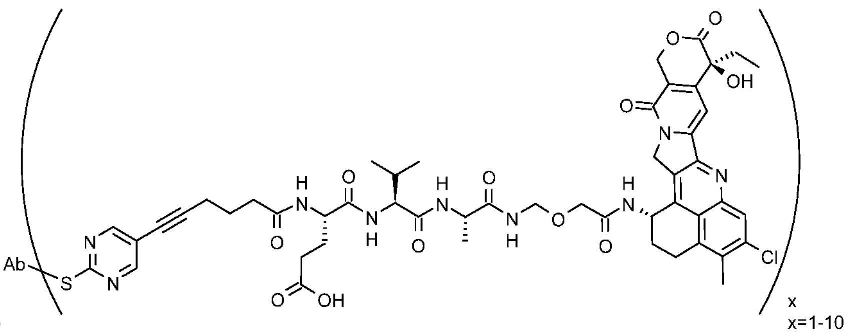

ADC A-28

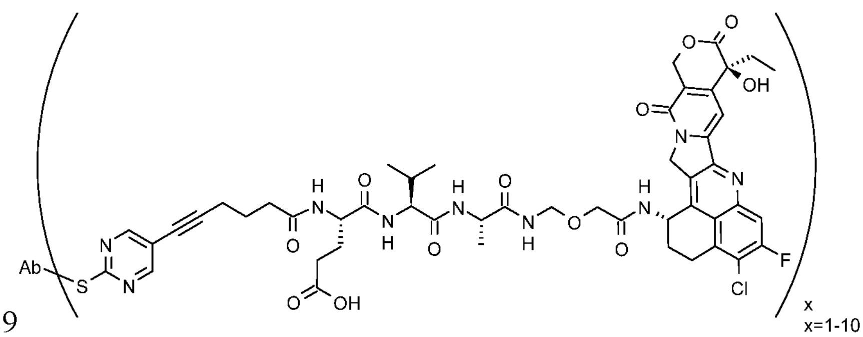

ADC A-29

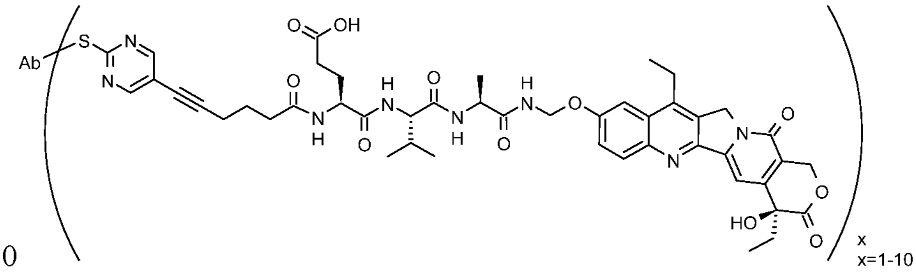

ADC A-30

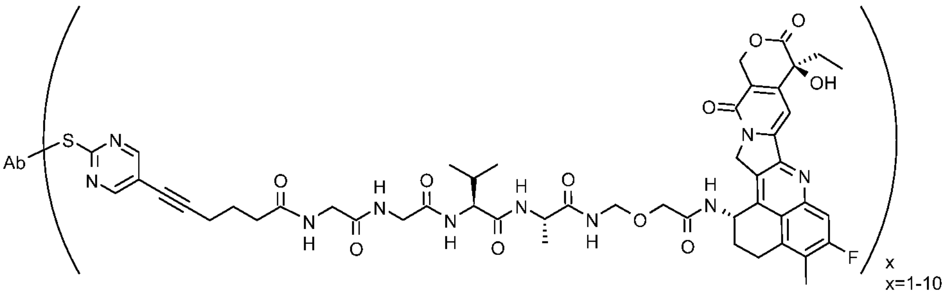

ADC A-31

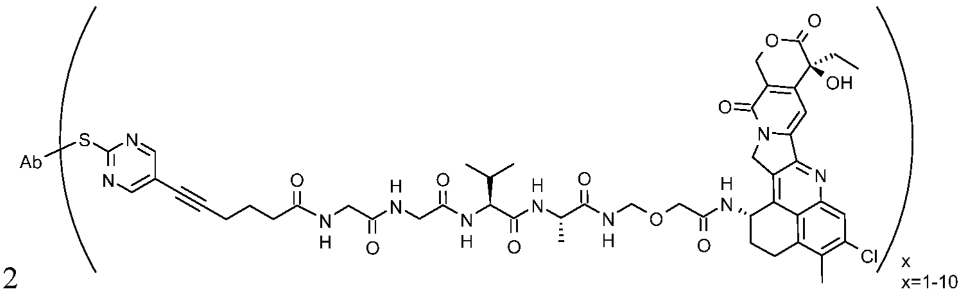

ADC A-32

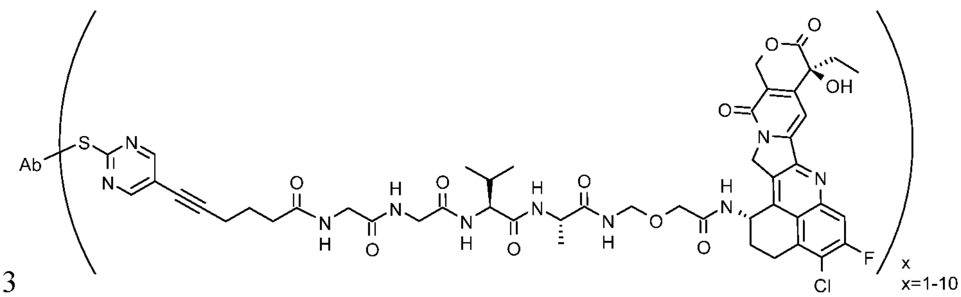

ADC A-33

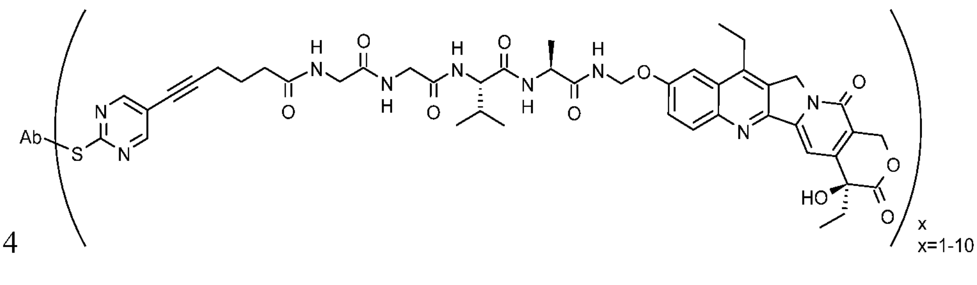

ADC A-34

ADC B-01

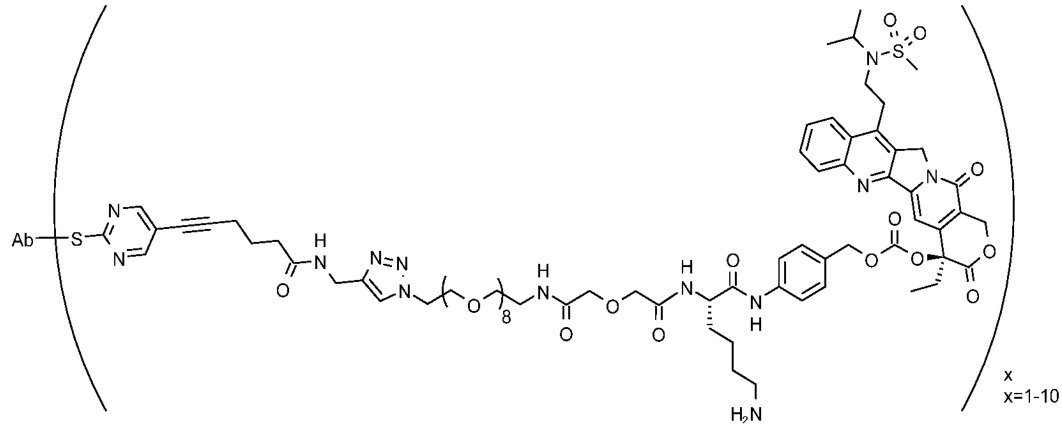

x=1-10

ADC B-02

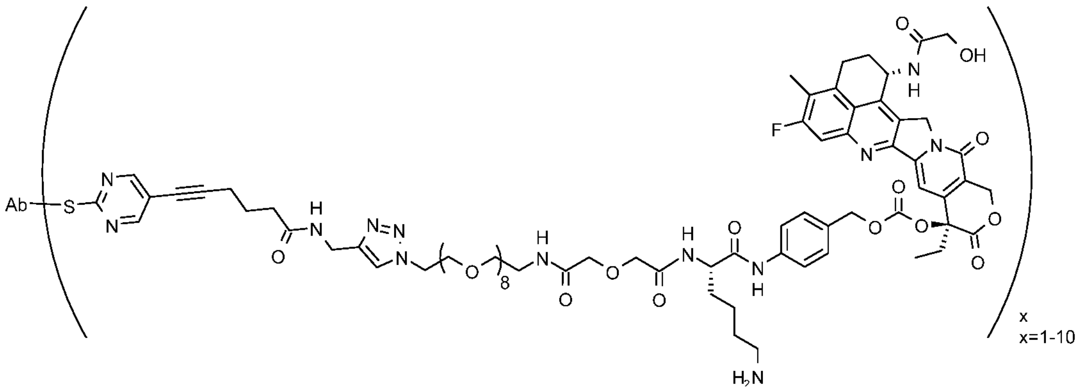

x=1-10

ADC B-03

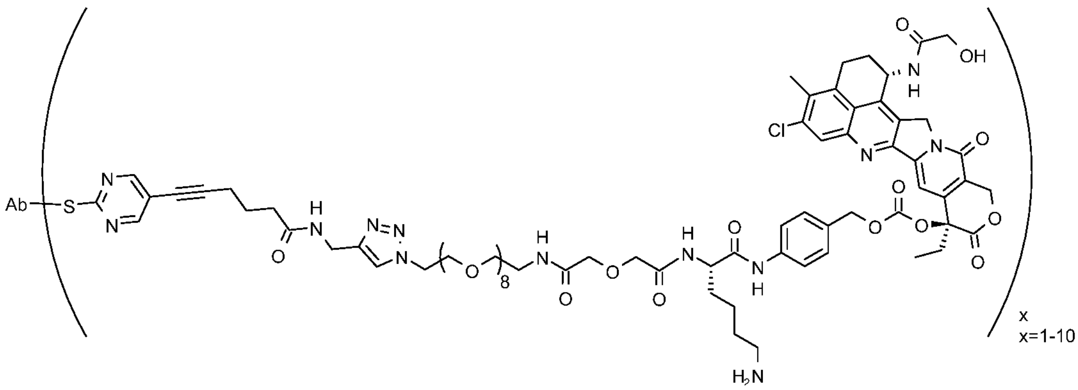

x=1-10

ADC B-04

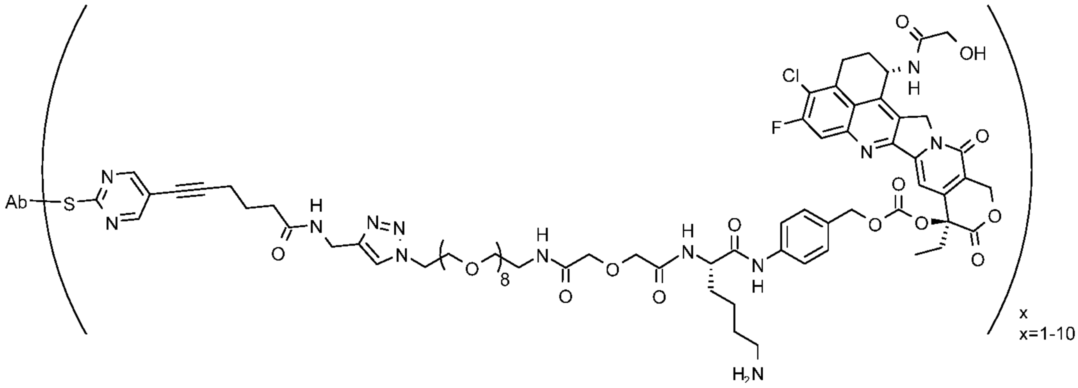

ADC B-05

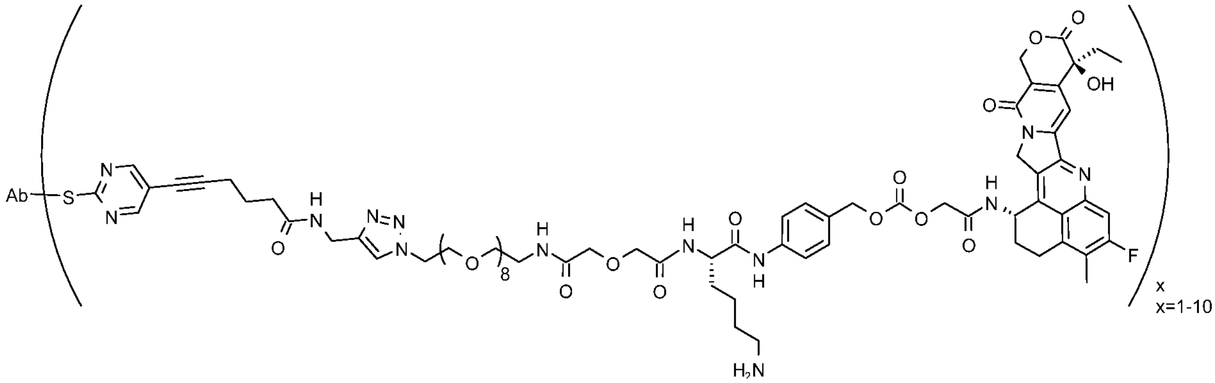

ADC B-06

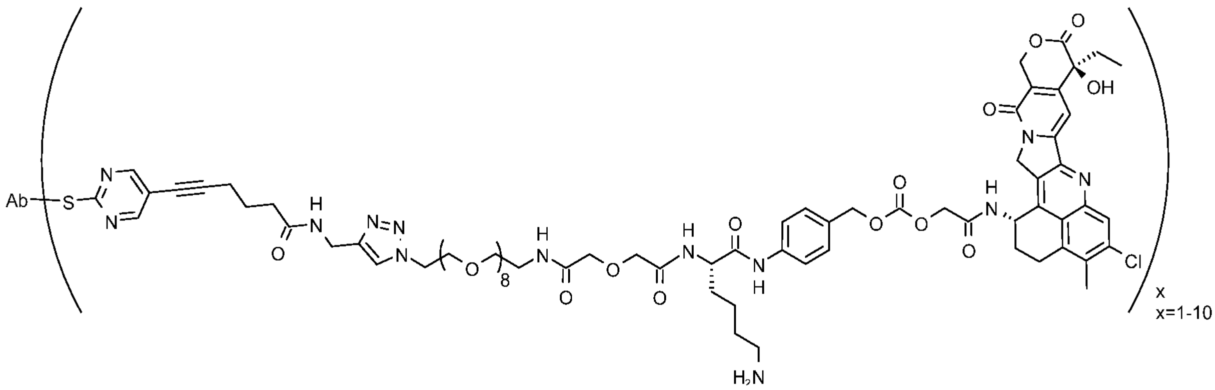

ADC B-07

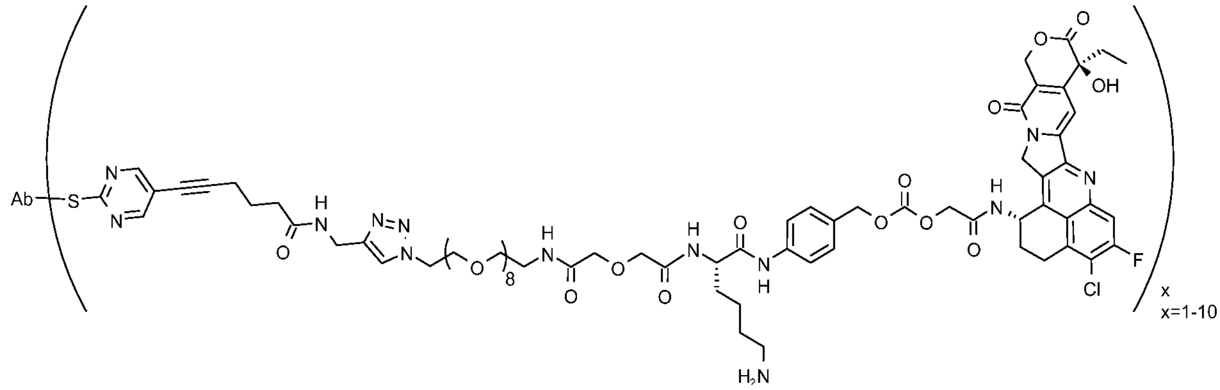

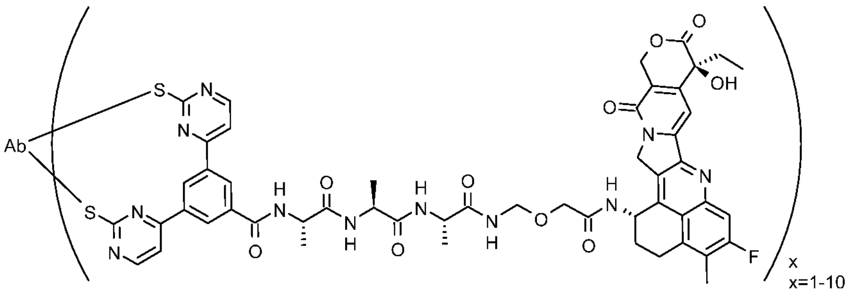

ADC C-01

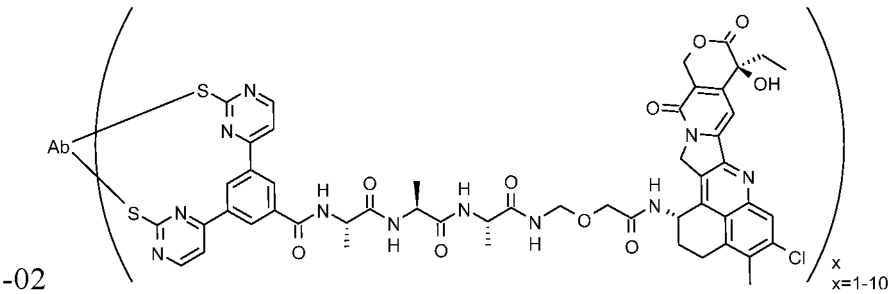

ADC C-02

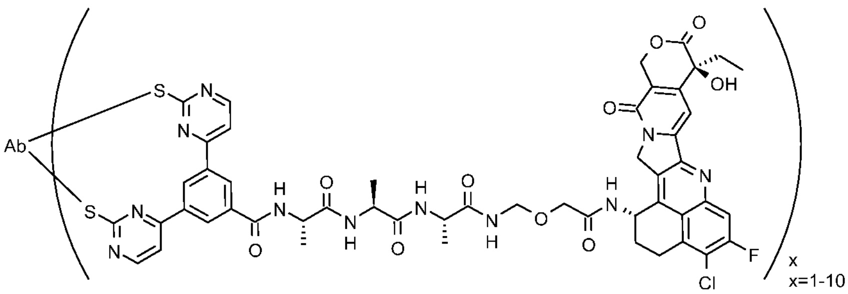

ADC C-03

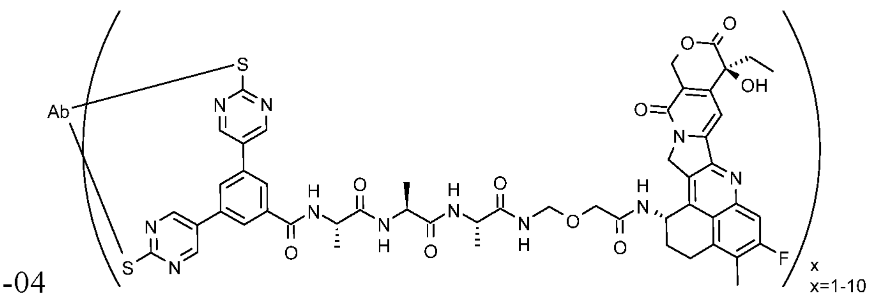

ADC C-04

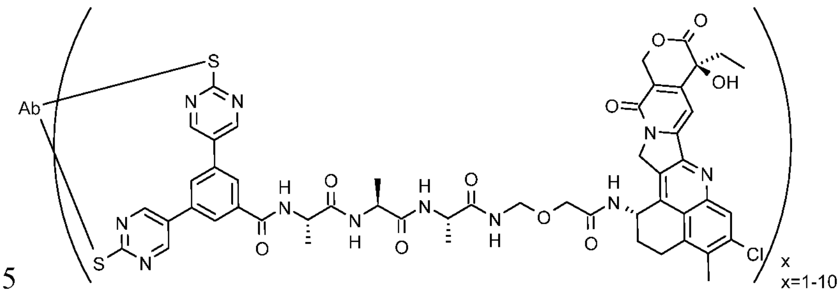

ADC C-05

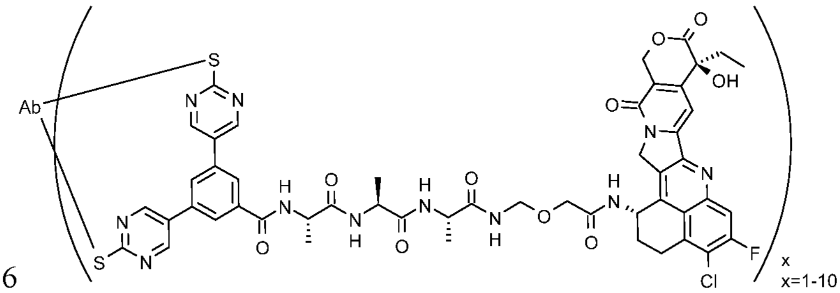

ADC C-06

ADC C-07

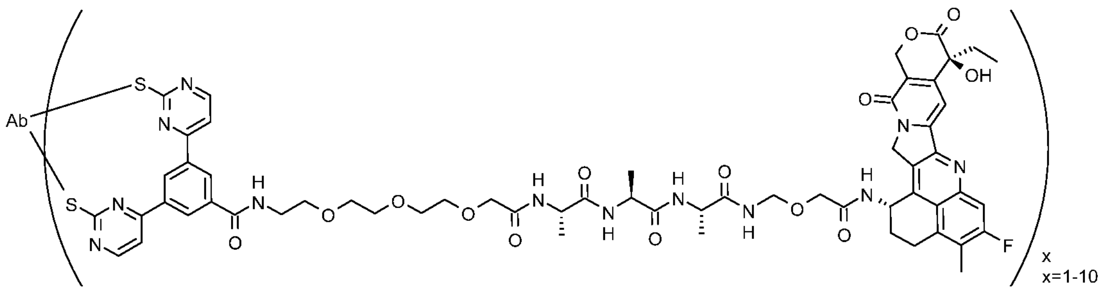

ADC C-08

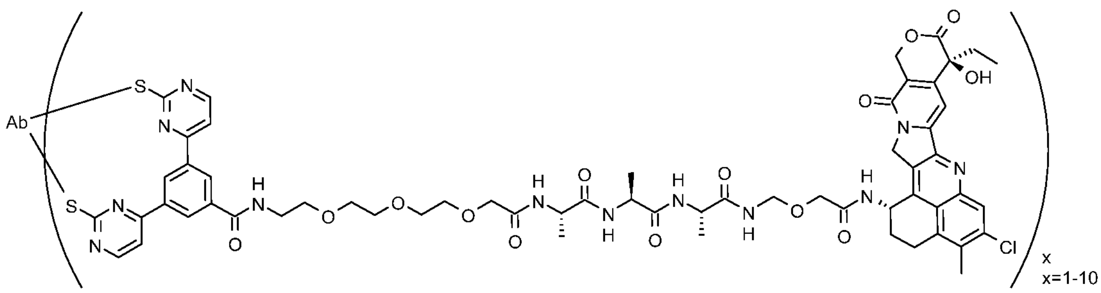

ADC C-09

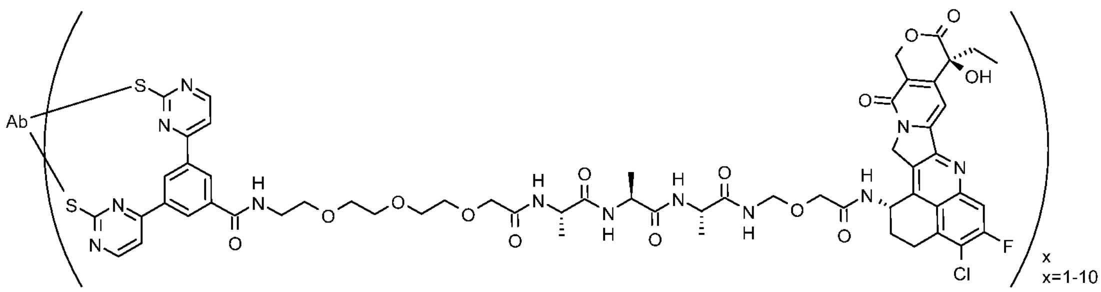

ADC C-10

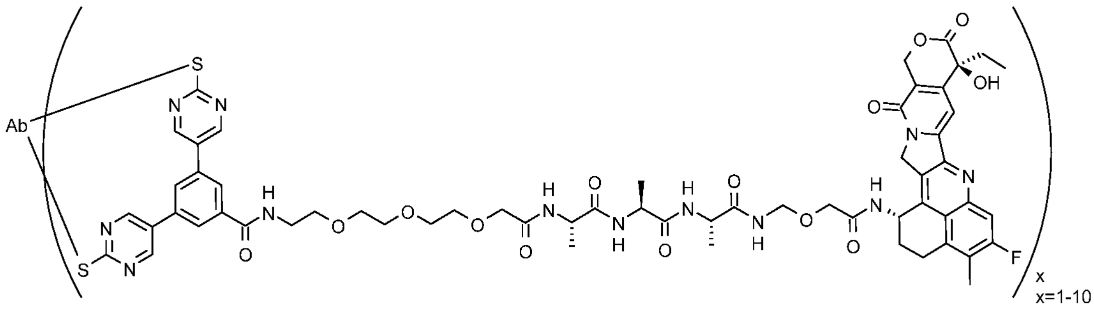

ADC C-11

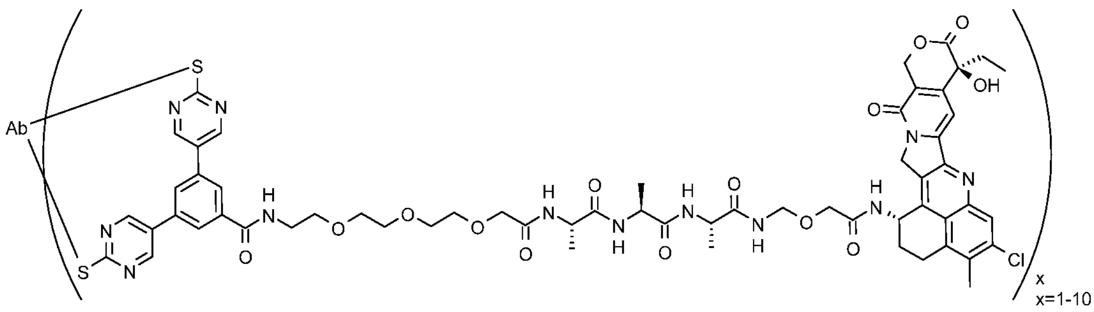

ADC C-12

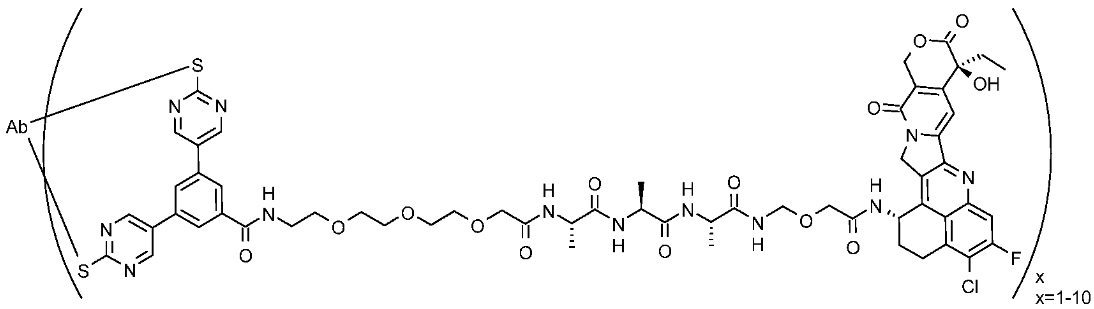

ADC C-13

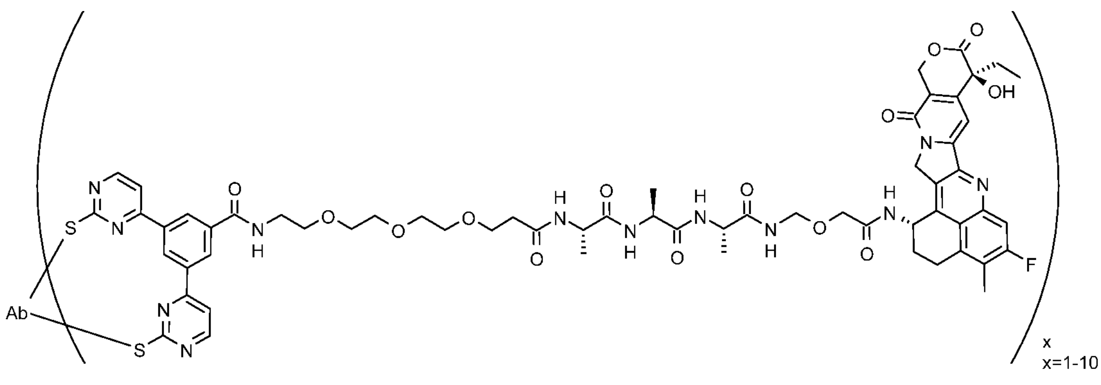

ADC C-14

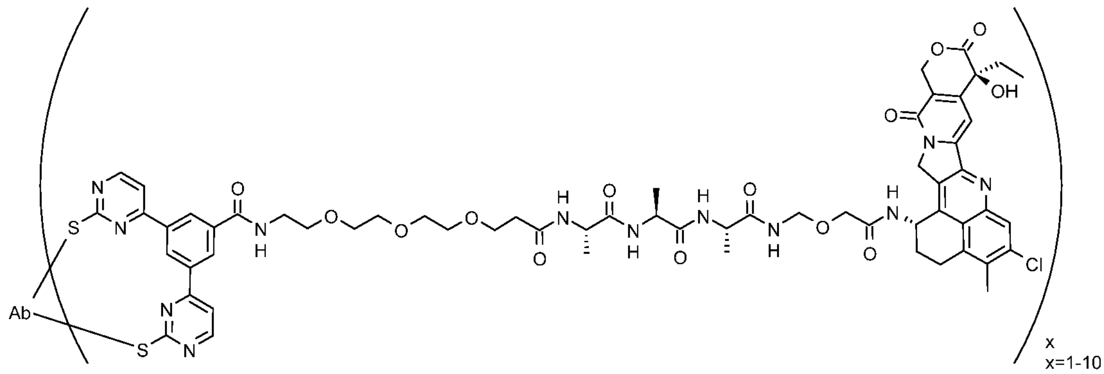

ADC C-15

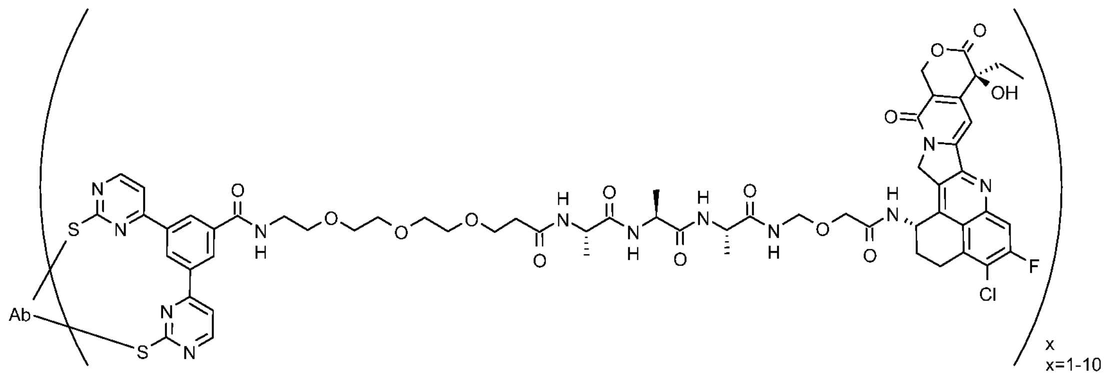

ADC C-16

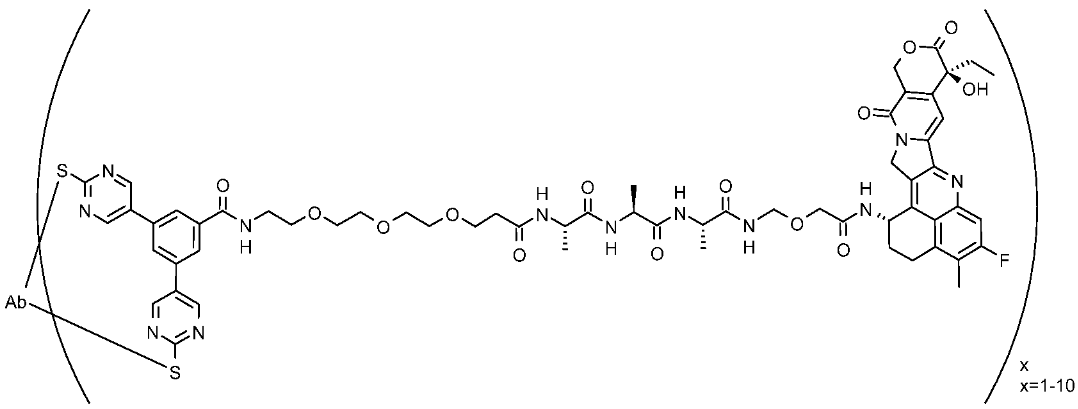

ADC C-17

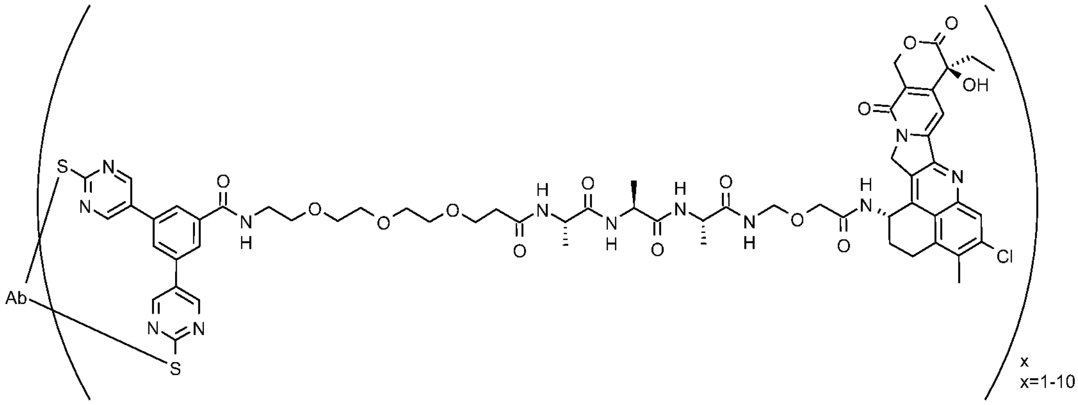

ADC C-18

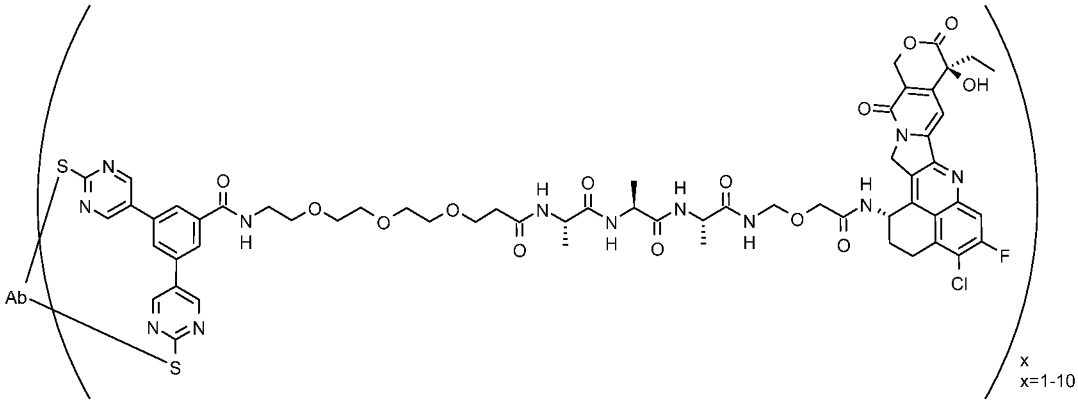

ADC C-19

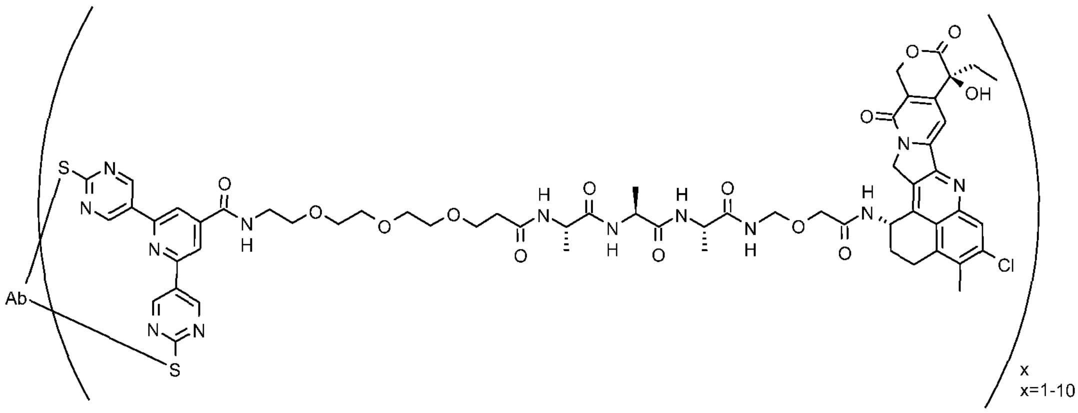

ADC C-20

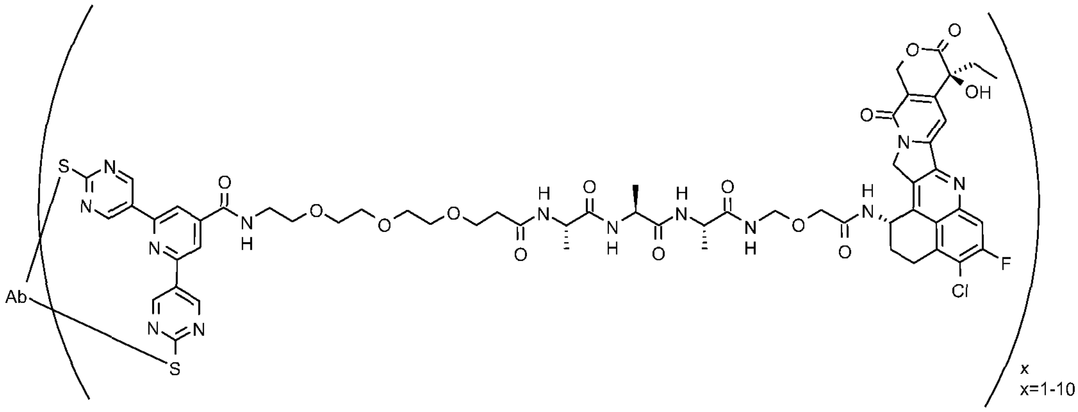

ADC C-21

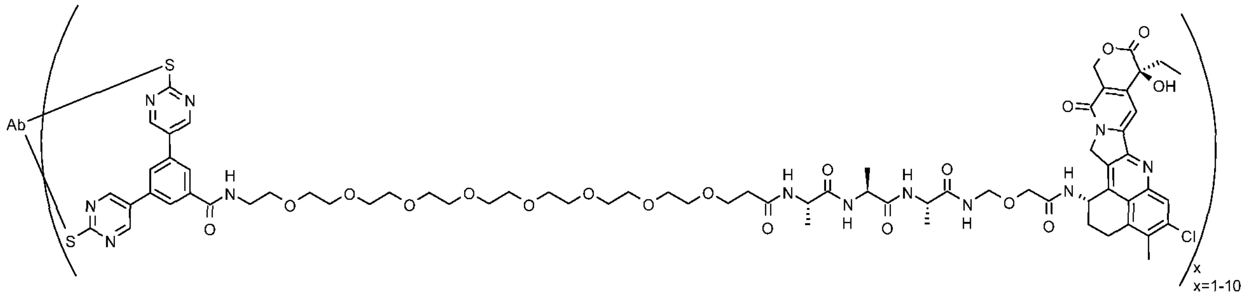

ADC C-22

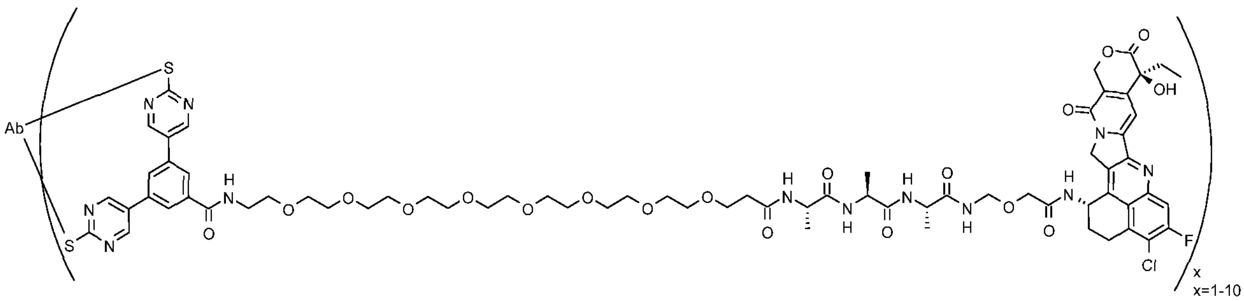

ADC C-23

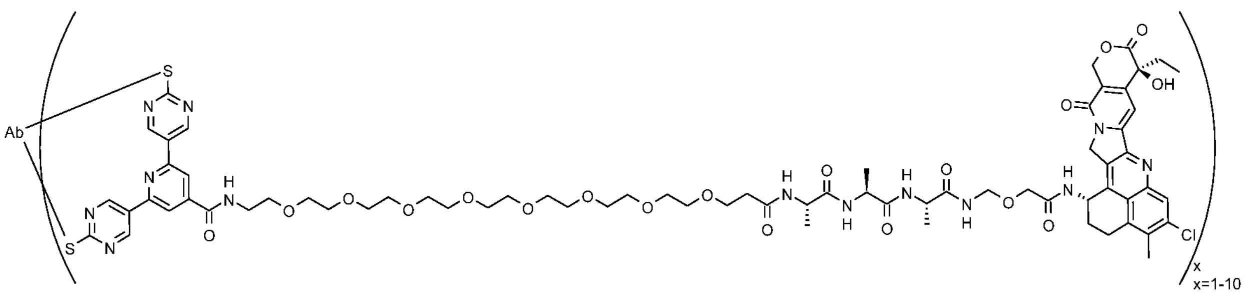

ADC C-24

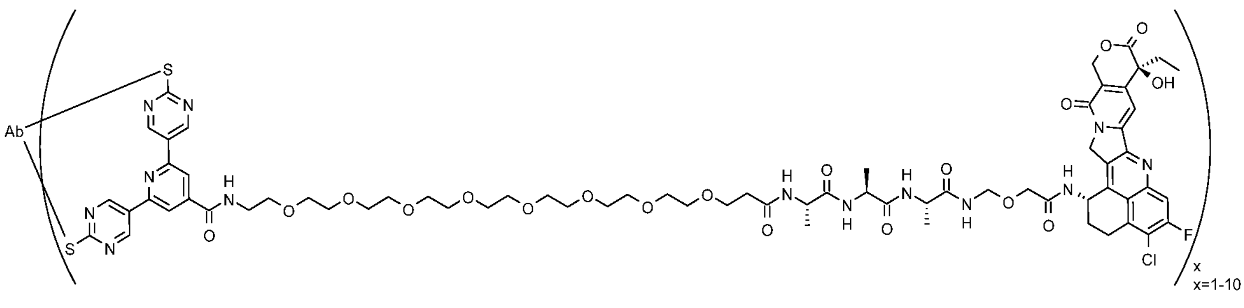

ADC C-25

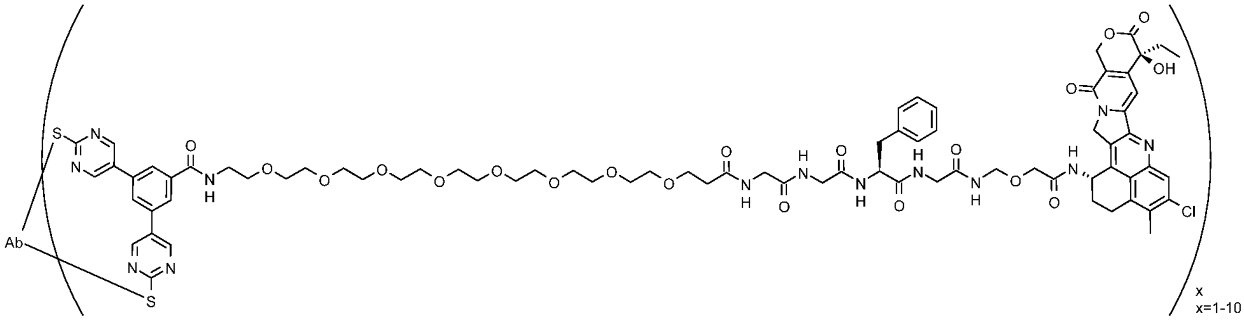

ADC C-26

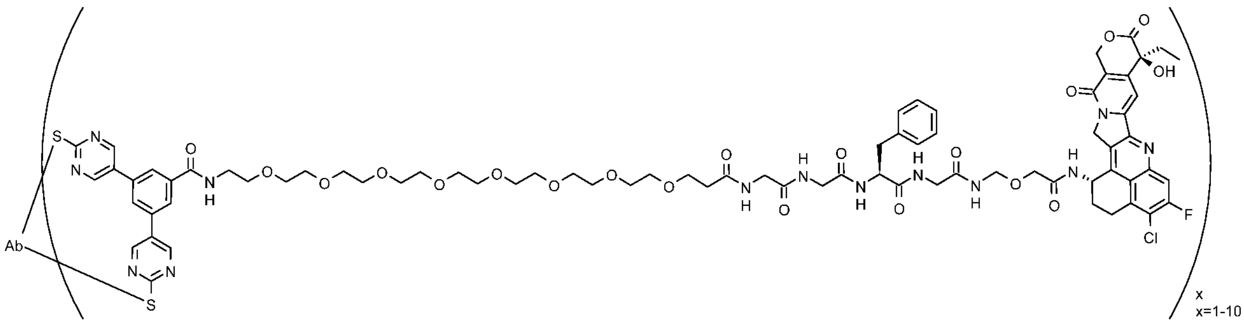

ADC C-27

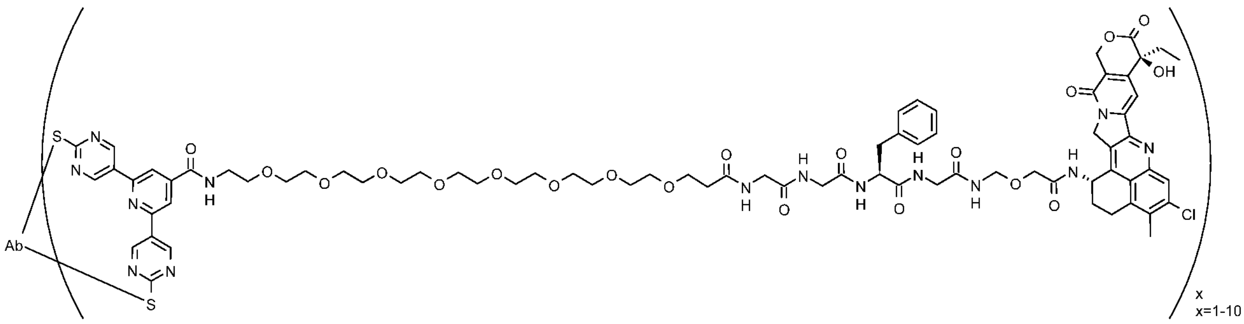

ADC C-28

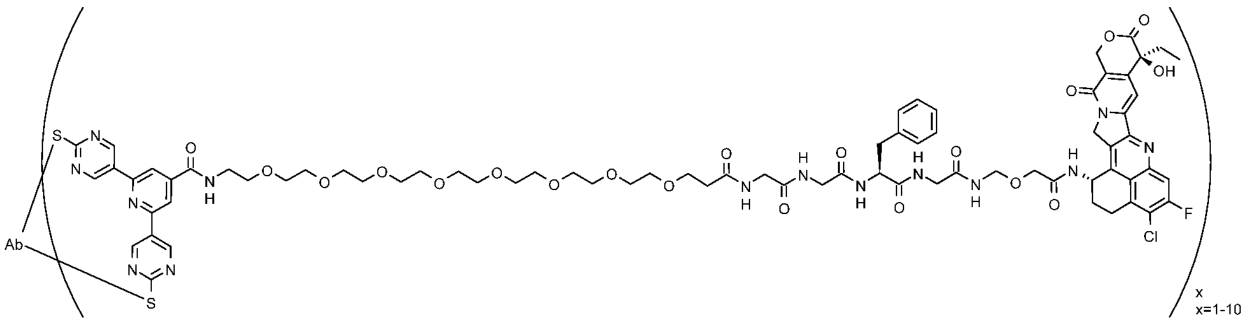

wherein

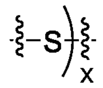

represents a specific connection mode between the sulfhydryl in the antibody or the antigen-binding fragment thereof and the linker.

Composition

In another aspect, the present invention provides a composition which may comprises a plurality of ADCs described herein. Each antibody molecule in the composition may be conjugated to 1, 2, 3, 4, 5, 6, 7, 8, 9 or 10 compounds described herein. In view of this, the composition is characterized by the drug-to-antibody ratio (DAR) in the range of about 1 to about 10. Methods for determining DAR are well known to the skilled person, including methods using reverse phase chromatography or HPLC-MS.

In some embodiments, the DAR value of the antibody-drug conjugate is 1-10, such as: 1-2, 1-3, 1-4, 1-5, 1-6, 1-7, 1-8, 1-9, 1-10, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, or 9-10, preferably 3-9, such as, 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 3.5-6.5, 3.5-7.0, 3.5-7.5, 3.5-8.0, 4.0-4.5, 4.0-5.0, 4.0-5.5, 4.0-6.0, 4.0-6.5, 4.0-7.0, 4.0-7.5, 4.0-8.0, 4.5-5.0, 4.5-5.5, 4.5-6.0, 4.5-6.5, 4.5-7.0, 4.5-7.5, 4.5-8.0, 5.0-5.5, 5.0-6.0, 5.0-6.5, 5.0-7.0, 5.0-7.5, 5.0-8.0, 5.5-6.0, 5.5-6.5, 5.5-7.0, 5.5-7.5, 5.5-8.0, 6.0-6.5, 6.0-7.0, 6.0-7.5, 6.0-8.5, 6.5-7.0, 6.5-7.5, 6.5-8.5, 7.0-7.5, 7.0-9.0 or 7.5-9.0.

In some embodiments, the DAR value of the antibody-drug conjugate is 4-8. Drug-linker (drug-linker compound) Those skilled in the art should understand that the antibody-drug conjugate described herein can be prepared by modularization. For example, a free form of "drug-linker" (which can be understood as G-M-[L-E-D]$_x$, wherein G-M is the structural form before covalently connecting with the antibody or the antigen-binding fragment thereof) is first obtained, and then the free form of "drug-linker" is covalently connected with the antibody or the antigen-binding fragment thereof to obtain the antibody-drug conjugate described herein. Accordingly, G-M in the free form of the "drug-linker" is connected to one or more sulfhydryl groups (-SH), amino groups ($-NH_2$) or carboxyl groups (-COOH) on the antibody or the antigen-binding fragment thereof by substitution reaction (for example, removing structures such as $-SO_2Me$ or -Br thereon) or by addition reaction, where x is selected from 1 to 10.

Pharmaceutical Composition

Pharmaceutical composition of antibody-drug conjugate and/or drug-linker

In another aspect, the present invention provides a pharmaceutical composition comprising the antibody-drug conjugate described in any one of the above solutions, or optionally the drug-linker described in any one of the above solutions, and one or more pharmaceutical excipients.

The antibody-drug conjugate described herein is usually formulated in a unit injectable form together with a pharmaceutically acceptable parenteral vehicle for parenteral use, such as injection, intravenous infusion, and intratumoral injection. Optionally, the antibody-drug conjugate with the desired purity is mixed with a pharmaceutically acceptable diluent, carrier, excipient or stabilizer in the form of a lyophilized agent or solution (Remington's Pharmaceutical Sciences (1980) 16th edition, Osol, A. Ed.). The antibody-drug conjugate described herein or the pharmaceutical composition comprising the antibody-drug conjugate can be administered by any route suitable for the individual to be treated.

In certain embodiments, the pharmaceutical composition may also comprise an additional pharmaceutical active agent.

In certain embodiments, the additional pharmaceutical active agent is a medicament with antitumor activity. In certain embodiments, the additional pharmaceutical active agent is selected from the group consisting of a B7-H3 inhibitor, an EGFR inhibitor, a HER2 inhibitor, a HER3 inhibitor, a HER4 inhibitor, an IGFR-1 inhibitor, an mTOR inhibitor, a PI3 kinase inhibitor, a c-met or VEGF inhibitor, a chemotherapeutic drug, or any combination thereof.

Application

1. Therapeutic use of antibody-drug conjugate and/or drug-linker

The antibody-drug conjugate, the drug-linker or the pharmaceutical composition described herein can be used to treat a variety of diseases or disorders, such as B7-H3 positive tumors and Her2 positive tumors.

In view of this, the present invention provides use of the antibody-drug conjugate, the drug-linker or the pharmaceutical composition comprising the same as described in any of the above solutions in preparation of a medicament for prevention and/or treatment and/or adjuvant therapy of a positive tumor.

The present invention provides use of the antibody-drug conjugate, the drug-linker or the pharmaceutical composition comprising the same as described in any of the above solutions in preparation of a medicament for prevention and/or treatment and/or adjuvant therapy of a B7-H3 positive tumor.

The present invention provides use of the antibody-drug conjugate, the drug-linker or the pharmaceutical composition comprising the same as described in any of the above solutions in preparation of a medicament for prevention and/or treatment and/or adjuvant therapy of a HER2 positive tumor.

Moreover, the present invention further provides a method for prevention and/or treatment and/or adjuvant therapy of a positive tumor, including a step of administering an effective amount of the antibody-drug conjugate, the drug-linker or the pharmaceutical composition comprising the same as described in any of the above solutions to a subject in need.

The present invention further provides a method for prevention and/or treatment and/or adjuvant therapy of a B7-H3 positive tumor, including a step of administering an effective amount of the antibody-drug conjugate, the drug-linker or the pharmaceutical composition comprising the same as described in any of the above solutions to a subject in need.

The present invention further provides a method for prevention and/or treatment and/or adjuvant therapy of a HER2 positive tumor, including a step of administering an effective amount of the antibody-drug conjugate, the drug-linker or the pharmaceutical composition comprising the same as described in any of the above solutions to a subject in need.

The present invention further provides use of the antibody-drug conjugate, the drug-linker or the pharmaceutical composition described in any of the above solutions in inhibiting proliferation of B7-H3 positive tumor cells.

The present invention further provides use of the antibody-drug conjugate, the drug-linker or the pharmaceutical composition described in any of the above solutions in inhibiting proliferation of Her2 positive tumor cells.

In certain embodiments, the antibody-drug conjugate, the drug-linker or the pharmaceutical composition is applied to an in vitro cell or a cell in a subject's body; for example, applied to the subject's body to inhibit the proliferation of a tumor cell in the subject's body; or, applied to an in vitro tumor cell (such as a cell line or a cell from a subject) to inhibit the proliferation of the in vitro tumor cell.

In the present invention, the B7-H3 positive tumors include solid tumors or hematological malignancies, such as colorectal cancer, gastric cancer, breast cancer, prostate cancer, head and neck squamous cell carcinoma, melanoma, neuroblastoma, sarcoma, lung cancer (such as small cell lung cancer and non-small cell lung cancer), kidney cancer, bladder cancer, thyroid cancer, mesothelioma, pancreatic cancer, ovarian cancer, endometrial cancer, esophageal cancer, liver cancer, salivary gland cancer, bile duct cancer, and meningioma.

In the present invention, the subject is preferably a mammal, such as a bovine, an equine, a porcine, a canine, a feline, a rodent, a primate; for example, a human.

In certain embodiments, the method further includes administration of a second therapy to the subject, wherein the second therapy is selected from the group consisting of surgery, chemotherapy, radiotherapy, immunotherapy, gene therapy, DNA therapy, RNA therapy, nanotherapy, viral therapy, adjuvant therapy and any combination thereof. In certain embodiments, the second therapy may be applied simultaneously, separately or sequentially with the method described above.

In certain embodiments, the tumor to which the pharmaceutical composition relates is selected from breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, and any combination thereof.

2. Use of drug-linker in the preparation of conjugate

The drug-linker described above is used for preparation of a conjugate. The conjugates of the present invention include antibody-drug conjugates.

3. Use of intermediate compound in preparation of drug-linker compound

The intermediate compound described above is used for preparation of a drug-linker compound.

Preparation method

The present invention further provides a preparation method for a drug-linker compound, comprising a step of deprotecting an intermediate compound.

Method 1

In some embodiments, the Method 1 includes a method for preparing compound A-05a, which includes a step of conjugation with the compound of formula I;

A-05a:

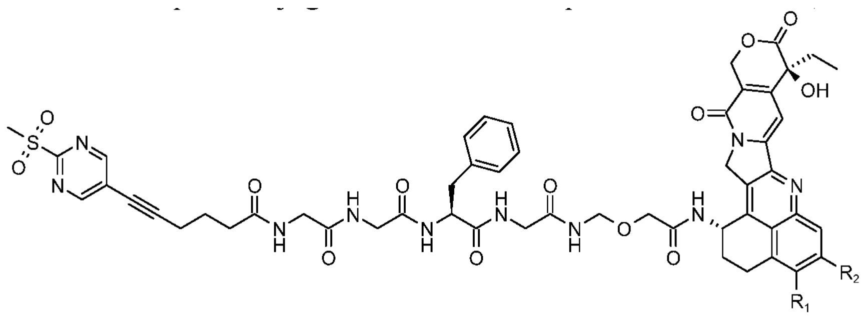

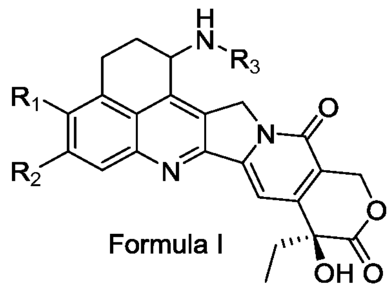

Formula I

wherein $R_1$ and $R_2$ are each independently selected from the group consisting of $C_{1-6}$ alkyl and halogen;
$R_3$ is selected from the group consisting of H and $-CO-CH_2OH$;
$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;
$R_6$ is selected from the group consisting of H or $-C_{1-4}$ alkylene-$NR_aR_b$;
$R_7$ is selected from the group consisting of $C_{1-6}$ alkyl and $-C_{1-4}$ alkylene-$NR_aR_b$;
wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence.

In some embodiments, $R_1$ and $R_2$ are each independently selected from the group consisting of $C_{1-4}$ alkyl, F, Cl, Br and I.

In some embodiments, $R_1$ is selected from the group consisting of halogen, methyl, ethyl, propyl and butyl, such as methyl and Cl.

In some embodiments, $R_2$ is selected from the group consisting of F, Cl, Br and I, such as F and Cl.

In some embodiments, $R_1$ is selected from the group consisting of methyl and Cl, and $R_2$ is selected from the group consisting of F and Cl.

Method 2

In some embodiments, the Method 2 includes a method for preparing compound A-14-a, which includes a step of deprotecting IM-5-a to obtain a compound of formula IM-6-a;

A-14-a: 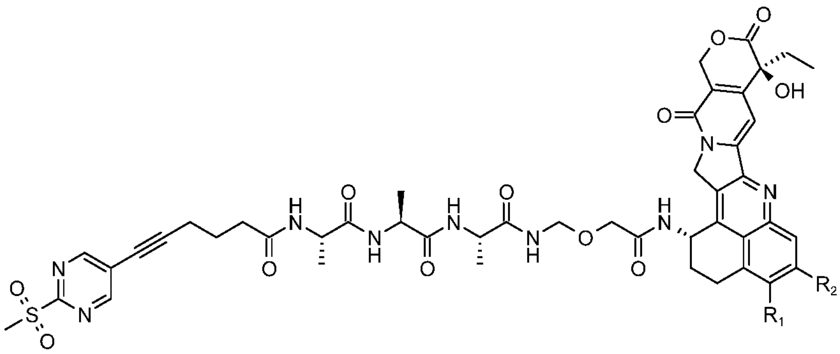

IM-5-a: 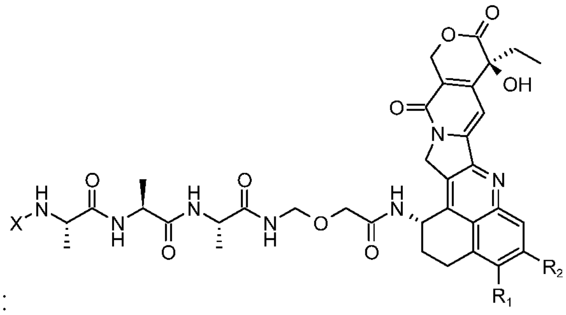

IM-6-a: 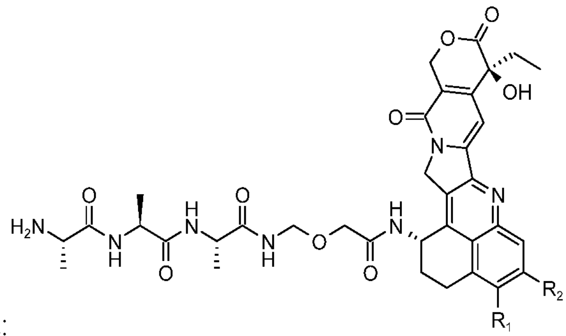

wherein X is defined as in the intermediate compound described above, and $R_1$ and $R_2$ are defined as in Method 1.

In some embodiments, in the deprotection reaction, the solvent is N,N-dimethylformamide, and an alkylamine compound, such as diethylamine, is added.

In some embodiments, the deprotection is carried out at room temperature, and the reaction time is 1-5h, such as 1-3h.

In some embodiments, the Method 2 further includes a step of reacting IM-6-a with IM-2 to obtain compound A-14-a.

In some embodiments, the solvent for the reaction is selected from the group consisting of N,N-dimethylformamide and N,N-dimethylacetamide.

In some embodiments, the reaction further includes a step of adding N,N-diisopropylethylamine.

Method 3

In some embodiments, the Method 3 includes a method for preparing compound B-02-a, which includes a step of deprotecting B-02-4a;

B-02-a:

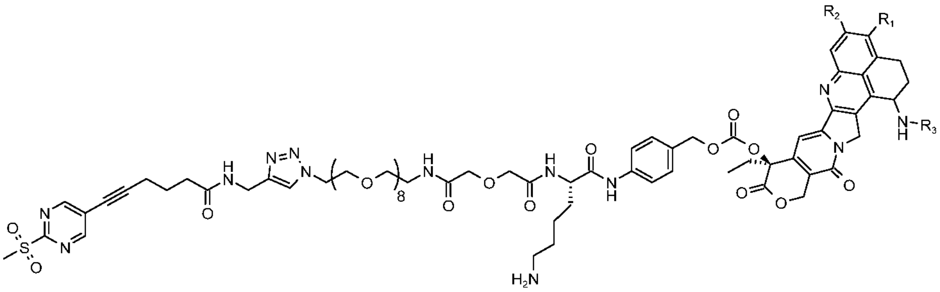

B-02-4a:

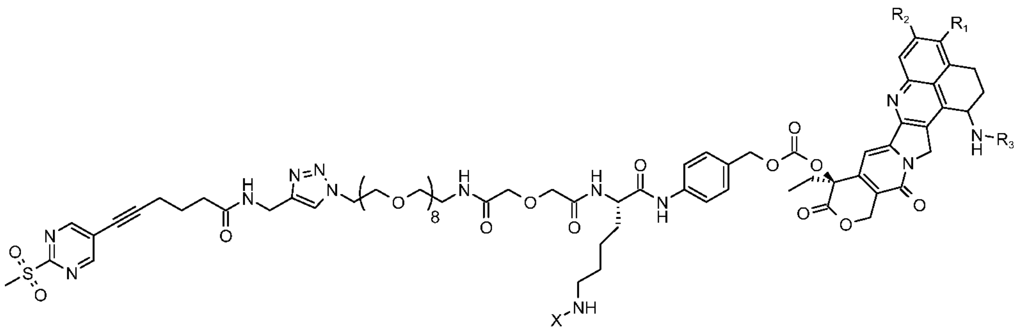

wherein X is defined as in the intermediate compound described above, and $R_1$, $R_2$ and $R_3$ are defined as in the compound of formula 1 described above.

Method 4

In some embodiments, the Method 4 includes a method for preparing compound C-07-a, which includes a step of reacting C-07-6 with C-07-8a;

C-07-a:

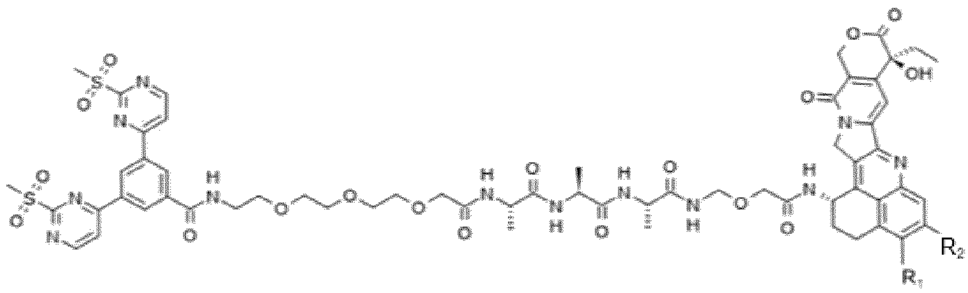

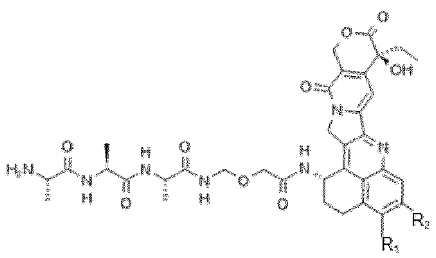

C-07-8a:

wherein $R_1$ and $R_2$ are defined as in Method 1.

In some embodiments, C-07-8a in the Method 4 is prepared by deprotection of C-07-7a;

C-07-7a: 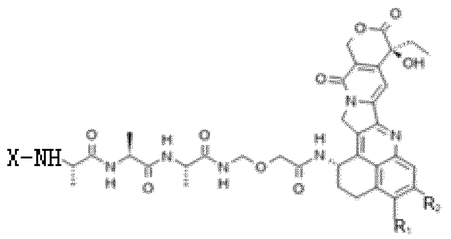,

wherein X is defined as in the intermediate compound described above, and $R_1$ and $R_2$ are defined as in Method 1.

Method 5

In some embodiments, the Method 5 includes a method for preparing compound C-10a, which includes a step of reacting C-10-5a with C-07-8a;

C-10-a:

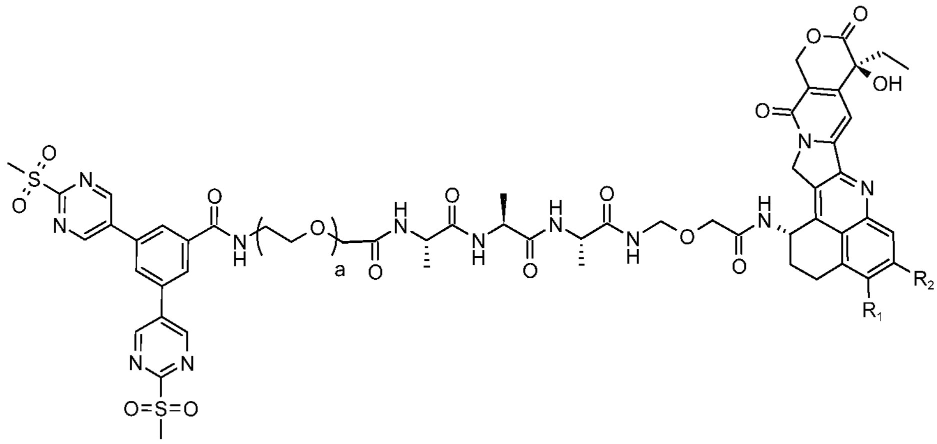

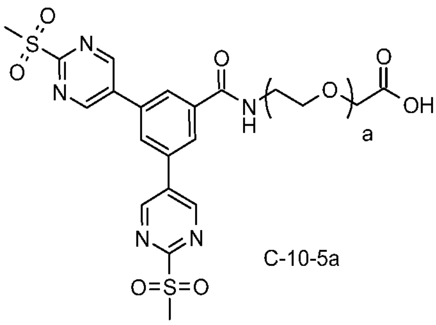

C-10-5a

,

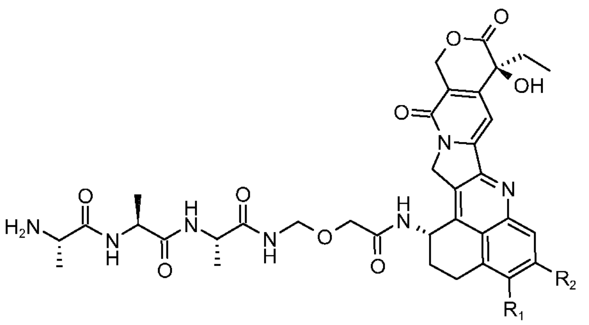

C-07-8a

;

wherein $R_1$, $R_2$ and a are as defined in any one of solutions described above.

In some embodiments, C-10-5a in Method 5 is prepared by deprotection of C-10-4a;

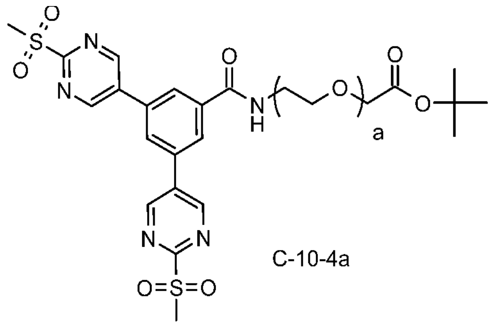

C-10-4a

In some embodiments, C-10-4a in Method 5 is prepared by reacting C-10-3 with compound A.

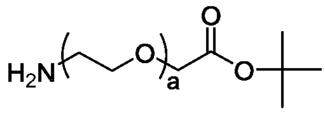

**Compound A**

Method 6

In some embodiments, the Method 6 includes a method for preparing compound C-17a, which includes a step of reacting C-17-3a with C-07-8a;

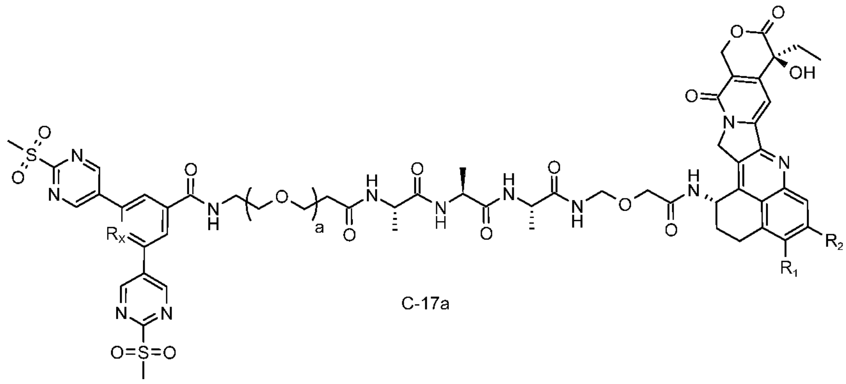

C-17a

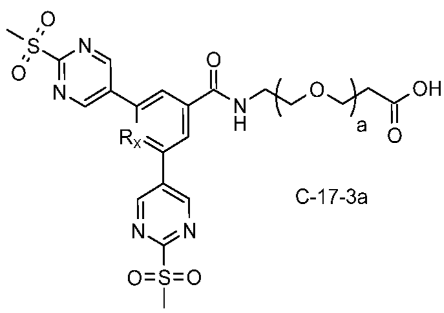

C-17-3a

wherein $R_1$, $R_2$ and a are as defined above; preferably, $R_1$ is methyl, $R_2$ is Cl and a is 3 or 8; $R_X$ is CH or N.

In some embodiments, C-17-3a in Method 6 is prepared by oxidation reaction of C-17-2a;

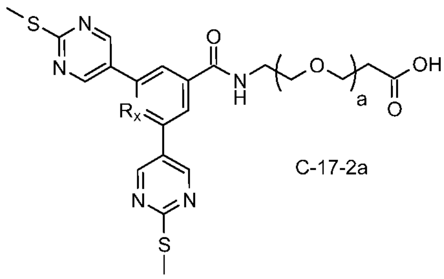

C-17-2a

In some embodiments, C-17-2a in Method 6 is prepared by deprotection of C-17-1a;

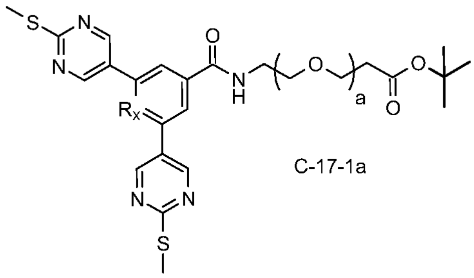

C-17-1a

In some embodiments, C-17-1a in Method 6 is prepared by conjugation reaction of C-10-2 or C-19-3 with compound A.

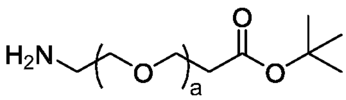

**Compound B**

Definitions

Unless otherwise defined below, all technical and scientific terms used herein are intended to have the same meaning as those generally understood by those skilled in the art. References to technology as used herein are intended to refer to technologies commonly understood in the art, including variations or substitutions of equivalent technologies that are obvious to those skilled in the art. In addition, the laboratory operation steps of genomics, nucleic acid chemistry, molecular biology, etc. used herein are all routine steps widely used in the corresponding fields. Although the following terms are believed to be well understood by those skilled in the art, the following definitions are set forth to better explain the present invention.

The term "antibody" refers to an immunoglobulin molecule that typically consists of two pairs of polypeptide chains, each having a light chain (LC) and a heavy chain (HC). Light chains of the antibody are classified as either kappa ($\kappa$) or lambda ($\lambda$) light chains. Heavy chains can be classified as $\mu$, $\delta$, $\gamma$, $\alpha$, or $\varepsilon$, and the isotypes of antibody can be defined as IgM, IgD, IgG, IgA and IgE, respectively. Within light and heavy chains, variable and constant regions are joined by a "J" segment of about 12 or more amino acids, the heavy chain also comprising a "D" segment of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain CL. The constant domains are not directly involved in the binding of antibodies and antigens, but exhibit a variety of effector functions, such as mediating the binding of immunoglobulins to host tissues or factors, including various cells (for example, effector cells) of immune system and the first component (Clq) of classical complement system. The VH and VL regions can also be subdivided into hypervariable regions (called complementary determining region (CDR)) interspersed with relatively conservative regions called framework regions (FR). The VH and VL regions can also be subdivided into hypervariable regions (called complementary determining region (CDR)) interspersed with relatively conservative regions called framework regions (FR). Each VH and VL consists of three CDRs and four FRs arranged from amino terminal to carboxyl terminal in the following order: FR1, CDR1, FR2, CDR2, FR3, CDR3, FR4. The variable regions (VH and VL) of each heavy chain/light chain pair form antigen-binding sites, respectively. The assignment of amino acids to various regions or domains can follow various numbering systems known in the art.

The term "complementary determining region" or "CDR" refers to the amino acid residue responsible for antigen

binding in the variable region of an antibody. There are three CDRs in each of the variable regions of the heavy and light chains, designated CDR1, CDR2, and CDR3. The precise boundaries of these CDRs may be defined in accordance with the various numbering systems known in the art, for example as defined in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al., (1989) Nature 342:878-883), the IMGT numbering system (Lefranc et al., Dev. Comparat. Immunol. 27: 55-77, 2003), or the AbM numbering system (Martin ACR, Cheetham JC, Rees AR (1989) Modelling antibody hypervariable loops:A combined algorithm. Proc Natl Acad Sci USA 86: 9268-9272). For a given antibody, a person skilled in the art will easily identify the CDRs defined by various numbering systems. And the corresponding relationship between different numbering systems is well known to those skilled in the art (for example, see Lefranc et al., Dev. Comparat. Immunol. 27: 55-77, 2003).

Herein, the CDRs contained in the antibody or the antigen-binding fragment thereof may be determined according to various numbering systems known in the art, such as Kabat, Chothia, IMGTor AbM. In certain embodiments, the CDRs contained in the antibody or the antigen-binding fragment thereof are defined by the Chothia numbering system.

The term "framework region" or "FR" residues refer to those amino acid residues in the variable region of an antibody other than the CDR residues as defined above.

The term "antigen-binding fragment" of an antibody refers to a polypeptide of a fragment of an antibody, such as a polypeptide of a fragment of a full-length antibody that maintains the ability to specifically bind to the same antigen that a full-length antibody binds to, and/or competes with a full-length antibody for specific binding to the antigen and which is also called an "antigen-binding moiety". In general, see Fundamental Immunology, Ch. 7 (Paul, W., ed., 2nd Edition, Raven Press, N.Y. (1989), which is incorporated herein by reference in its entirety for all purposes. Antigen-binding fragments of an antibody can be produced by recombinant DNA technologies or by enzymatic or chemical cleavage of an intact antibody. Non-limiting examples of antigen-binding fragments include Fab fragments, Fab' fragments, $F(ab)'_2$ fragments, $F(ab)'_3$ fragments, Fd, Fv, scFv, di-scFv, $(scFv)_2$, disulfide-stabilized Fv proteins ("dsFv"), single-domain antibodies (sdAb, nanobodies) and polypeptides that contain at least a portion of an antibody sufficient to confer specific antigen binding ability to the polypeptide. Engineering modified antibody variants are reviewed in Holliger et al., 2005; Nat Biotechnol, 23: 1126-1136.

The term "Fd" means an antibody fragment consisting of VH and CH1 domains; the term "dAb fragment" means an antibody fragment consisting of a VH domain (Ward et al., Nature 341: 544 546 (1989)); the term "Fab fragment" means an antibody fragment consisting of VL, VH, CL and CH1 domains; the term "$F(ab')_2$ fragment" means an antibody fragment comprising two Fab fragments linked by a disulfide bridge on the hinge region; the term "Fab' fragment" means a fragment obtained by reducing the disulfide bridge linking two heavy chain fragments in the $F(ab')_2$ fragment, which consists of a complete light chain and a Fd fragment (composed of VH and CH1 domains) of heavy chain.

The term "Fv" means an antibody fragment consisting of VL and VH domains of a single-arm of an antibody. The Fv fragment is generally considered to be the smallest antibody fragment that can form a complete antigen-binding site. It is generally thought that six CDRs confer antigen-binding specificity to an antibody. However, even one variable region (such as a Fd fragment, which contains only three antigen-specific CDRs) can identify and bind to antigen, although possibly having a lower affinity than a complete binding site.

The term "Fc" means an antibody fragment formed by linking the second and third constant regions of the first heavy chain of an antibody to the second and third constant regions of the second heavy chain of the antibody via a disulfide bridge. The Fc fragment of an antibody has many different functions, but does not involve in antigen binding.

The term "scFv" refers to a single polypeptide chain comprising VL and VH domains, wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Ed. Roseburg and Moore, Springer-Verlag, New York, pp. 269-315 (1994)). Such scFv molecules may have a general structure: $NH_2$-VL-Linker-VH-COOH or $NH_2$-VH-Linker-VL-COOH. A suitable linker in the art consists of repeated GGGGS amino acid sequences or variants thereof. For example, a linker having an amino acid sequence $(GGGGS)_4$ or a variant thereof can be used (Holliger et al. (1993), Proc. Natl. Acad. Sci. USA 90: 6444-6448). Other linkers that may be used in the invention are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56 and Roovers et al. (2001), Cancer Immunol. In some cases, a disulfide bridge may also be present between VH and VL of scFv. In certain embodiments, the VH and VL domains can be positioned relative to each other in any suitable arrangement. For example, a scFv comprising $NH_2$-VH-VH-COOH and $NH_2$-VL-VL-COOH.

The term "single-domain antibody (sdAb)" has the meaning generally understood by those skilled in the art, and it refers to an antibody fragment composed of a single monomer variable antibody domain (e.g., a single VH) that retains the ability to specifically bind to the same antigen as the full-length antibody (Holt, L. et al., Trends in Biotechnology, 21 (11): 484-490, 2003). Single-domain antibodies are also called nanobodies.

Each of the above antibody fragments retains the ability to specifically bind to the same antigen as bound by the full-length antibody, and/or competes with the full-length antibody for specific binding to an antigen.

Herein, unless clearly indicated otherwise in the context, reference to the term "antibody" includes not only an intact antibody but also an antigen-binding fragment of the antibody.

An antigen-binding fragment (e.g., the antibody fragment described above) may be obtained from a given antibody (e.g., the antibody provided by the invention) using conventional technologies known to those skilled in the art (e.g., recombinant DNA technologies or enzymatic or chemical cleavage methods), and the antigen-binding fragment of the antibody is specifically screened in the same manner as for an intact antibody.

The term "murine antibody" refers to an antibody obtained by the following method: fusing B cells of immunized mice with myeloma cells, and screening for murine hybrid fusion cells that can both proliferate indefinitely and secrete antibodies, followed by screening, antibody preparation and antibody purification. Or, the murine antibody refers to an antibody secreted by plasma cells formed by differentiation and proliferation of B cells after antigens invade the mouse body.

The term "humanized antibody" refers to a non-human antibody modified by genetic engineering, and the amino acid sequence of the antibody is modified to improve the homology with the sequence of human antibodies. Typically, all or some of the CDRs of a humanized antibody are derived from a non-human antibody (donor antibody), and all or some of the non-CDRs (e.g., FRs in the variable region and/or the constant region) are derived from a human immunoglobulin (receptor antibody). Humanized antibodies usually retain the expected properties of the donor antibody, including but not limited to antigen specificity, affinity, reactivity, ability to enhance immune cell activity, and ability to enhance immune response. The donor antibody can be a mouse, rat, rabbit or non-human primate (e.g., cynomolgus monkey) antibody having the desired properties (such as antigen specificity, affinity, reactivity, ability to enhance immune cell activity and/or ability to enhance immune response).

The term "identity" refers to the sequence matching degree between two polypeptides or two nucleic acids. When a position in each of two sequences for comparison is occupied by the same base or amino acid monomer subunit (for example, a position in each of two DNA molecules is occupied by adenine, or a position in each of two polypeptides is occupied by lysine), then the molecules are identical at that position. "% identity" between two sequences is a function that the number of matching positions shared by the two sequences $\div$ the number of positions to be compared $\times$ 100. For example, if six of ten positions in two sequences match, then the two sequences are 60% identical. For example, the DNA sequences CTGACT and CAGGTT have 50% identity (three of the total six positions match). Typically, comparison is made when two sequences are aligned to produce maximum identity. Such an alignment can be realized by using, for example, the method of Needleman et al. (1970) J. Mol. Biol. 48: 443-453, which can be conveniently performed by a computer program such as Align program (DNAstar, Inc). The % identity between two amino acid sequences can be determined by the algorithm of E. Meyers and W. Miller (Comput. Appl Biosci., 4: 11-17 (1988)) incorporated in the ALIGN program (version 2.0), using the PAM120 weight residue table, a gap length penalty of 12 and a gap penalty of 4. Additionally, the % identity between two amino acid sequences can be determined by the algorithm of Needleman and Wunsch (J Mol Biol. 48: 444-453 (1970)) in the GAP program incorporated in the GCG software package (available at www.gcg.com), using either a Blossum Matrix 62 or PAM250, and a gap weight of 16, 14, 12, 10, 8, 6 or 4 and a length weight of 1, 2, 3, 4, 5 or 6.

As used herein, the term "variant", in the context of a polypeptide (including a polypeptide), also refers to a polypeptide or peptide that contains an amino acid sequence that has been altered by the introduction of amino acid residues by substitution, deletion, or addition. In some cases, the term "variant" also refers to a polypeptide or peptide that has been modified (i.e., by covalently linking any type of molecule to a polypeptide or peptide). For example, but not restrictably, polypeptides may be modified, for example by glycosylation, acetylation, pegylation, phosphorylation, amidation, derivatization by known protective/blocking groups, proteolytic cleavage, attachment to cellular ligands or other proteins, etc. Derived polypeptides or peptides may be produced by chemical modifications using techniques known to those skilled in the art, including but not limited to specific chemical cleavage, acetylation, formylation, and metabolic synthesis of tunicamycin. In addition, a variant has similar, identical, or improved functions to the polypeptide or peptide from which the variant is derived.

As used herein, the term "specifically binding" refers to non-random binding reaction between two molecules, such as reaction between an antibody and an antigen against which it is. The strength or affinity of the specific binding interaction can be indicated by the dissociation equilibrium constant (KD) of the interaction or the median effective concentration ($EC_{50}$).

The specific binding properties between two molecules can be measured using methods well known in the art. One of the methods involves measuring the rate of formation and dissociation of antigen-binding site/antigen complexes. Both "binding rate constant" (ka or kon) and "dissociation rate constant" (kdis or koff) can be calculated from the concentration and the actual rates of association and dissociation (see Malmqvist M, Nature, 1993, 361: 186-187). The ratio of kdis/kon is equal to the dissociation constant KD (see Davies et al., Annual Rev Biochem, 1990; 59: 439-473). KD, kon and kdis values can be measured by any effective method. In certain embodiments, the dissociation constant can be measured by bioluminescence interferometry (e.g., ForteBio Octet assay). In addition, the dissociation constant can also be measured by surface plasmon resonance technology (such as Biacore) or Kinexa.

The term "conservative substitution" means an amino acid substitution that does not adversely affect or alter the expected properties of a protein/polypeptide comprising an amino acid sequence. For example, a conservative substitution may be introduced by standard technologies known in the art, such as site-directed mutagenesis and PCR-mediated mutagenesis. A conservative amino acid substitution includes a substitution of an amino acid residue with an amino acid residue having a similar side chain, e.g., with a residue that is physically or functionally similar to the corresponding amino acid residue (e.g., having similar size, shape, charge, chemical property, including the ability to form covalent or hydrogen bonds, etc.). Families of amino acid residues having similar side chains have been defined in the art. These families include amino acids having alkaline side chains (e.g., lysine, arginine and histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta branched side chains (e.g., threonine, valine, isoleucine) and aromatic side chains (e.g. tyrosine, phenylalanine, tryptophan, histidine). Therefore, it is preferred to replace a corresponding amino acid residue with another amino acid residue from the same side chain family. Methods for identifying conservative substitution of amino acids are well known in the art (see, for example, Brummell et al., Biochem. 32: 1180-1187 (1993); Kobayashi et al., Protein Eng. 12 (10): 879-884 (1999); and Burks et al., Proc. Natl Acad. Set USA 94: 412-417 (1997), which are incorporated herein by reference).

The compilation of twenty conventional amino acids involved herein follows the conventional usage. See, for example, Immunology-A Synthesis (2nd Edition, E. S. Golub and D. R. Gren, Eds., Sinauer Associates, Sunderland, Mass. (1991)), which is incorporated herein by reference. Herein, amino acids are generally expressed by single-letter and three-letter abbreviations well known in the art. For example, alanine may be expressed as A or Ala.

The term "include", "comprise", "has", "contain" or "relate to" and other similar forms used herein are inclusive or open-ended and do not exclude other unlisted elements or method steps.

The term "alkyl" refers to a group obtained by removing one H atom from a straight or branched hydrocarbyl, such as "$C_{1-20}$ alkyl", "$C_{1-10}$ alkyl", "$C_{1-6}$ alkyl", "$C_{1-4}$ alkyl" and "$C_{1-3}$ alkyl". Its specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1,2-dimethylpropyl.

The term "alkylene" refers to a group obtained by removing two H atoms from a straight or branched hydrocarbyl, such as "$C_{1-20}$ alkylene", "$C_{1-10}$ alkylene", "$C_{3-10}$ alkylene", "$C_{5-8}$ alkylene", "$C_{1-6}$ alkylene", "$C_{1-4}$ alkylene" and "$C_{1-3}$ alkylene". Its specific examples include but are not limited to: methylene, ethylene, 1,3-propylene, 1,4-butylene, 1,5-pentylene or 1,6-hexylene.

The term "alkenylene" refers to a divalent group obtained by losing two H atoms from a straight or branched hydrocarbyl containing at least one C-C double bond, and the alkenylene includes, for example, "$C_{2-20}$ alkenylene", "$C_{3-10}$ alkenylene", and "$C_{5-8}$ alkenylene". Its examples include, but are not limited to: vinylene, 1-propenylene, 2-propenylene, 1-butenylene, 2-butenylene, 1,3-butadienylene, 1-pentenylene, 2-pentenylene, 3-pentenylene, 1,3-pentadienylene, 1,4-pentadienylene, 1-hexenylene, 2-hexenylene, 3-hexenylene, and 1,4-hexadienylene.

The term "alkynylene" refers to a divalent group obtained by losing two hydrogen atoms from a straight or branched hydrocarbyl group containing at least one C-C triple bond. The hydrocarbyl includes, for example, "$C_{2-20}$ alkynylene", "$C_{3-10}$ alkynylene" and "$C_{5-8}$ alkynylene". Its examples include, but are not limited to, ethynylene, 1-propynylene, 2-propynylene, 1-butynylene, 2-butynylene, 1,3-butadiynylene, 1-pentynylene, 2-pentynylene, 3-pentynylene, 1,3-pentadiynylene, 1,4-pentadiynylene, 1-hexynylene, 2-hexynylene, 3-hexynylene, and 1,4-hexadiynylene.

The term "aliphatic heterocycle" refers to a saturated or partially saturated cyclic structure containing at least one (e.g., 1, 2 or 3) ring member selected from N, O and S. Its specific examples include, but are not limited to, 5- to 6-membered aliphatic heterocycles, 5- to 6-membered N-aliphatic heterocycles, and 5- to 6-membered oxo-aliphatic heterocycles, such as tetrahydrofuran, pyrrolidine, piperidine, and tetrahydropyran.

The term "heteroaromatic ring" refers to an aromatic ring structure containing at least one ring member selected from N, O and S. Its specific examples include, but are not limited to, 5- to 6-membered aromatic heterocycles, 5- to 6-membered N-aromatic heterocycles, 5- to 6-membered oxo-aromatic heterocycles, such as furan, thiophene, pyrrole, thiazole, isothiazole, thiadiazole, oxazole, isoxazole, oxadiazole, imidazole, pyrazole, 1,2,3-triazole, 1,2,4-triazole, 1,2,3-oxadiazole, 1,2,4 -oxadiazole, 1,2,5-oxadiazole, 1,3,4-oxadiazole, pyridine, pyrimidine, pyridazine, pyrazine, 1,2,3-triazine, 1,3,5-triazine, and 1,2,4,5-tetrazine.

The term "aromatic ring system" refers to a monocyclic or polycyclic system comprising at least one aromatic ring (e.g., benzene ring) or heteroaromatic ring (e.g., 5- to 6-membered aromatic heterocycle, such as 5- to 6-membered N-aromatic heterocycle, such as pyrimidine ring). Two or more aromatic rings and/or heteroaromatic rings can form a fused ring or be connected by a single bond (e.g., bipyrimidinylphenyl), and the aromatic ring system can be divalent or higher valent (e.g., trivalent or tetravalent), such as a 5- to 20-membered aromatic ring system.

As used herein, the term "suitable substituent" refers to modifications that can be made to a compound by a person skilled in the art according to the needs of the compound's substituent. "Suitable substituents" include oxo (=O), halogen, cyano, $NR^8R^9$, carboxyl, sulfhydryl, hydroxyl, ester group (e.g., -$C_{1-6}$ alkyl-C(=O)-O$C_{1-6}$ alkyl), $C_{1-6}$ alkyl, $C_{2-6}$

alkenyl, $C_{2-6}$ alkynyl, $C_{1-6}$ alkyl-O-$C_{1-6}$ alkyl, halo $C_{1-6}$ alkyl, $C_{1-6}$ alkoxy, $C_{3-6}$ cycloalkyl, 3- to 10-membered heterocyclyl, 5- to 10-membered heteroaryl, $C_{6-10}$ aryl, benzyl, hydroxy-substituted benzyl, indolyl methylene and $C_{1-6}$ haloalkoxy; $R^8$ and $R^9$ are each independently selected from the group consisting of H, $C_{1-6}$ alkyl, $C_{3-6}$ cycloalkyl, 3- to 10-membered heterocyclyl, 5- to 10-membered heteroaryl, $C_{6-10}$ aryl, $C_{1-6}$ alkoxy, halo $C_{1-6}$ alkyl, $C_{1-6}$ haloalkoxy, halogen, hydroxy, carboxyl and ester group (e.g., -$C_{1-6}$ alkyl-C(=O)-O$C_{1-6}$ alkyl).

As used herein, the term "pharmaceutically acceptable carrier and/or excipient" means a carrier and/or excipient that is pharmacologically and/or physiologically compatible with the subject and active ingredient, which are well known in the art (see, for example, Remington's Pharmaceutical Sciences. Edited by Gennaro AR, 19th ed. Pennsylvania: Mack Publishing Company, 1995) and include, but not limited to, pH modulators, surfactants, adjuvants, ionic strength enhancers, diluents, osmotic pressure maintaining reagents, delayed absorption reagents, preservatives. For example, the pH modulators include, but not limited to, phosphate buffers. The surfactants include, but not limited to cationic, anionic, or nonionic surfactants, such as Tween-80. The ionic strength enhancers include, but not limited to, sodium chloride. The preservatives include, but not limited to, various antibacterial and antifungal agents, such as p-hydroxybenzoate, trichloro-tert-butyl alcohol, phenol, sorbic acid, etc. The osmotic pressure maintaining reagents include, but not limited to, sugars, NaCl and the like. The delayed absorption reagents include, but not limited to, monostearates and gelatin. Diluents include, but not limited to, water, aqueous buffers (e.g., buffer saline), alcohols and polyols (e.g., glycerol), etc. The preservatives include, but not limited to, various antibacterial and antifungal agents, such as thiomersalate, 2-phenoxyethanol, p-hydroxybenzoate, trichloro-tert-butyl alcohol, phenol, sorbic acid, etc. The stabilizers have a meaning commonly understood by those skilled in the art, which can stabilize the desired activity of the active ingredient in drug, including but limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate).

As used herein, the term "prevention" refers to a process for preventing or delaying the onset of a disease or condition or symptom (e.g., a tumor) in vivo in a subject. As used herein, the term "treatment" refers to a process for achieving a beneficial or desired clinical result. For purposes of the invention, beneficial or desired clinical result includes, but not limited to, alleviation of a symptom, diminishment of extent of disease, stabilizing (i.e., not worsening) state of a disease, delay or slowing of disease progression, amelioration or palliation of disease state, and remission (whether partial or all) of symptoms, no matter detectable or undetectable. In addition, "treatment" can also mean prolonging survival as compared to the expected survival (if without treatment).

As used herein, the term "subject" refers to a mammal, such as a primate mammal, such as a human. In some embodiments, the subject (e.g., human) has a tumor, or is at a risk of suffering from such disease.

As used herein, the term "effective amount" refers to an amount sufficient to achieve or at least partially achieve a desired effect. For example, an effective amount for preventing a disease is an amount sufficient to prevent, stop, or delay the onset of the disease (e.g., a tumor); a therapeutic effective amount refers to an amount sufficient to cure or at least partially prevent a disease and its complications of a patient who have already suffered from the disease. To determine such effective amount is within the scope of capability of those skilled in the art. For example, the amount effective for a therapeutic use will depend on the severity of the disease to be treated, the general state of the patient's own immune system, the general condition of the patient such as age, weight and gender, route of administration, and other treatments administered concurrently, etc.

The terms "cancer" and "tumor", used interchangeably, refer to a large group of diseases characterized by the uncontrolled growth of abnormal cells in the body. Unregulated cell division may lead to the formation of malignant tumors or cells that invade neighboring tissues and may metastasize to distant parts of the body through the lymphatic system or bloodstream. Cancers include benign and malignant cancers as well as dormant tumors or micrometastases. Cancer also includes hematologic malignancies.

The term "hematologic malignancies" includes lymphoma, leukemia, myeloma, or lymphoid malignancies, as well as splenic and lymph node tumors. Example lymphomas include B-cell lymphomas and T-cell lymphomas. B-cell lymphomas include, for example, Hodgkin's lymphoma. T-cell lymphomas, include, for example, cutaneous T-cell lymphoma. Hematological malignancies also include leukemia, such as secondary leukemia or acute lymphoblastic leukemia. Hematologic malignancies also include myeloma (e.g., multiple myeloma) and other hematological and/or B-cell or T-cell related cancers.

**DETAILED DESCRIPTION OF EMBODIMENTS**

The invention is further described below through the description of specific embodiments, but this is not intended to limit the invention. Based on the teachings of the invention, those skilled in the art can make various modifications or improvements without departing from the basic concept and scope of the invention.

Information of the sequences to which the invention relates is shown in the table below:

| SEQ ID NO: | Description | |
|---|---|---|
| 1 | Amino acid sequence of VH in 20G11G6/2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSS |
| 2 | Amino acid sequence of VL in 20G11G6/2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIK |
| 3 | Amino acid sequence of VH in 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSS |
| 4 | Amino acid sequence of VL in 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIK |
| 5 | CDR-H1 of 20G11G6/2# (IMGT) | GGSNSSSNW |
| 6 | CDR-H2 of 20G11G6/2# (IMGT) | ISPTGFT |
| 7 | CDR-H3 of 20G11G6/2# (IMGT) | ARDRLYFDF |
| 8 | CDR-L1 of 20G11G6/2#/2#8890 (IMGT) | QSVSSN |
| 9 | CDR-L2 of 20G11G6/2#/2#8890 (IMGT) | GAS |
| 10 | CDR-L3 of 20G11G6/2#/2#8890 (IMGT/Chothia/Kabat/ AbM) | QQYNNWPIT |
| 11 | CDR-H1 of 20G11G6/2# (Chothia) | GGSNSSSN |
| 12 | CDR-H2 of 20G11G6/2# (Chothia) | SPTGF |
| 13 | CDR-H3 of 20G11G6/2# (Chothia/Kabat/AbM) | DRLYFDF |
| 14 | CDR-L1 of 20G11G6/2#/2#8890 (Chothia/Kabat/AbM) | RASQSVSSNLA |
| 15 | CDR-L2 of 20G11G6/2#/2#8890 (Chothia/Kabat/AbM) | GASTRAT |
| 16 | CDR-H1 of 20G11G6/2# (Kabat) | SSNWWT |

(continued)

| SEQ ID NO: | Description | |
|---|---|---|
| 17 | CDR-H2 of 20G11G6/2# (Kabat) | EISPTGFTSYSPSLKS |
| 18 | CDR-H1 of 2#8890 (IMGT) | GGSDSSTNW |
| 19 | CDR-H2 of 2#8890 (IMGT) | ISPIGFT |
| 20 | CDR-H3 of 2#8890 (IMGT) | ARDRLYFAF |
| 21 | CDR-H1 of 2#8890 (Chothia) | GGSDSSTN |
| 22 | CDR-H2 of 2#8890 (Chothia) | SPIGF |
| 23 | CDR-H3 of 2#8890 (Chothia/ Kabat/AbM) | DRLYFAF |
| 24 | CDR-H1 of 2#8890 (Kabat) | STNWWT |
| 25 | CDR-H2 of 2#8890 (Kabat) | EISPIGFTSYSPSLRS |
| 26 | CDR-H1 of 20G11G6/2# (AbM) | GGSNSSSNWWT |
| 27 | CDR-H2 of 20G11G6/2# (AbM) | EISPTGFTS |
| 28 | CDR-H1 of 2#8890 (AbM) | GGSDSSTNWWT |
| 29 | CDR-H2 of 2#8890 (AbM) | EISPIGFTS |
| 30 | Amino acid sequence of CH of IgG1 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APELLGGPSVFLFPPKPKDTLMISRTPEVTCVVVDVSH EDPEVKFNWYVDGVEVHNAKTKPREEQYNSTYRVVS VLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| 31 | Amino acid sequence of mutated CH of IgG1 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APEAAGAPSVFLFPPKPKDTLMISRTPEVTCVVVDVSH EDPEVKFNWYVDGVEVHNAKTKPREEQYNSTYRVVS VLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |

(continued)

| SEQ ID NO: | Description | |
|---|---|---|
| 32 | Amino acid sequence of human light chain κ constant region | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 33 | Nucleotide sequence of VH of 20G11G6/2# | CAGGTTCAATTGCAAGAAAGCGGCCCCGGGCTGGTAAAACCAAGTGGCACCCTGAGCCTGACGTGTGCTGTGTCTGGGGGGAGCAATTCCTCAAGCAATTGGTGGACATGGGTGAGGCAGTCCCCCGGAAAGGGCTTGGAATGGATCGGCGAGATCAGCCCTACAGGTTTTACCAGCTACTCCCCTAGCCTGAAATCCAGGGTGACGATCAGTGTCGACAAGTCTAAAACACAGTTCTCTCTGAACCTTTCAAGTGTCACTGCAGCCGATACTGCTGTGTATTACTGCGCCAGAGATCGGCTGTATTTTGACTTTTGGGGCCAGGGCACTCTGGTCACAGTGTCTAGC |
| 34 | Nucleotide sequence of VL of 20G1 1G6/2# | GAAATAGTGATGACCCAATCCCCCGCAACTCTGTCTGTGTCCCCTGGGAAGAGAGCCACCCTTTCCTGTCGCGCCTCACAATCCGTGAGCTCAAATCTGGCATGGTATCAACAGAAACCAGGCCAAGCGCCTCGGCTGCTGATTTACGGCGCCTCTACCCGCGCTACCGGAATCCCAGCACGCTTTAGCGGCTCAGGGTCCGGCACTGAGTTCACCTTGACAATTAGTAGTCTGCAGTCCGAAGATTTTGCCGTCTATTACTGCCAGCAGTATAATAACTGGCCAATCACCTTTGGCCAGGGCACACGCCTGGAGATCAAG |
| 35 | Nucleotide sequence of VH of 2#8890 | CAAGTACAACTCCAAGAAAGTGGGCCAGGACTCGTGCAACCATCTGGCACCCTCTCTCTCACCTGCGCAGTGTCTGGCGGCTCCGATTCTTCCACCAACTGGTGGACCTGGGTGAGACAGTCCCCAGGCCAAGGATTGGAATGGATTGGCGAGATTTCCCCTATTGGGTTCACCAGCTACAGCCCCAGCCTGAGGAGCAGGGTGACTATCAGCGTGGATAAGTCTAAAACCCAGTTCAGTCTCAAACTGAGTAGTGTGACTGCCGCGGACACGGCAGTGTATTATTGCGCCCGGGACCGCCTTTATTTCGCCTTCTGGGGGCAGGGCACTTTGGTGACCGTCAGTAGC |
| 36 | Nucleotide sequence of VL of 2#8890 | GAGATTGTCATGACACAGAGTCCTGCTACCTTGTCAGTTAGTCCTGGGGAGAGAGCAACACTGTCCTGCCGCGCTAGCCAAAGTGTTAGCTCCAACCTGGCCTGGTACCAGCAGAAACCCGGGCAGGCACCCAGACTCCTGATTTACGGAGCCTCTACACGCGCAACTGGAATCCCTGACCGGTTCTCAGGATCCGGGAGTGGCACGGAATTCACCTTGACCATTAGCAGCCTGGAATCCGAAGATTTTGCAGTGTATTACTGTCAGCAGTACAACAACTGGCCTATCACGTTCGGGCAGGGGACCAGATTGGAAATCAAG |

(continued)

| SEQ ID NO: | Description | |
|---|---|---|
| 37 | Nucleotide sequence of CH of IgG1 | GCATCTACTAAAGGCCCAAGTGTTTTTCCTCTGGCCC<br>CATCCTCAAAAAGCACCTCCGGAGGCACAGCTGCGC<br>TGGGATGCCTCGTGAAAGATTATTTCCCTGAACCTGT<br>TACCGTGTCATGGAACAGCGGCGCTCTCACTTCTGG<br>CGTGCACACATTTCCCGCTGTGTTGCAGTCCAGCGG<br>ACTCTATAGCCTGAGCTCCGTGGTTACAGTGCCCTCT<br>TCATCTTTGGGCACCCAGACCTACATATGCAATGTGA<br>ATCATAAACCGAGCAATACCAAGGTAGATAAAAAGG<br>TAGAGCCAAAAAGTTGCGATAAGACTCATACATGCC<br>CACCTTGTCCTGCCCCGGAGCTGCTCGGAGGACCAA<br>GCGTGTTTCTCTTCCCCCCCAAGCCAAAGGATACCTT<br>GATGATTAGTCGCACCCCAGAAGTGACATGCGTGGT<br>GGTTGACGTCAGCCACGAGGACCCAGAGGTGAAGT<br>TTAATTGGTACGTGGACGGTGTGGAAGTCCACAATG<br>CAAAAACCAAGCCTCGCGAAGAACAGTATAACAGT<br>ACCTACAGGGTCGTCAGTGTCCTGACCGTGCTGCAC<br>CAGGACTGGCTCAATGGAAAGGAATACAAGTGTAAG<br>GTCTCCAACAAGGCCTTGCCAGCTCCCATTGAAAAG<br>ACCATTAGCAAGGCCAAAGGTCAGCCTCGCGAACCA<br>CAAGTGTACACACTGCCTCCGTCACGCGATGAACTT<br>ACCAAAAATCAGGTATCACTGACCTGCCTTGTAAAG<br>GGCTTTTACCCGAGTGACATAGCAGTCGAGTGGGAA<br>TCCAACGGTCAGCCGGAGAACAACTATAAAACCACT<br>CCCCCAGTGCTTGATTCCGATGGCAGCTTCTTCCTGT<br>ACAGTAAACTGACTGTGGATAAGTCTCGCTGGCAGC<br>AGGGCAATGTGTTTTCTTGCTCTGTCATGCACGAGG<br>CACTTCACAACCACTACACACAGAAAAGTCTCAGTC<br>TGAGTCCAGGGAAA |
| 38 | Nucleotide sequence of human light chain κ constant region | AGAACAGTGGCCGCACCAAGCGTGTTTATCTTTCCT<br>CCCTCTGACGAACAATTGAAGAGTGGGACCGCCTCA<br>GTGGTGTGTCTGCTGAACAATTTCTACCCCCGGGAG<br>GCAAAGGTGCAGTGGAAGGTGGATAATGCACTCCAG<br>TCCGGGAATAGTCAGGAAAGCGTGACTGAGCAAGA<br>CAGCAAAGACTCTACATATTCTCTGTCCAGTACCCTG<br>ACCCTCAGCAAGGCTGATTATGAGAAGCACAAGGTG<br>TACGCCTGTGAGGTCACTCACCAGGGACTGAGTTCA<br>CCGGTCACTAAAAGCTTTAACAGAGGAGAGTGC |

(continued)

| SEQ ID NO: | Description | |
|---|---|---|
| 39 | Nucleotide sequence of mutated CH of IgG1 | GCCTCTACAAAAGGACCATCAGTCTTTCCATTGGCTC CGAGTAGCAAAAGCACCAGCGGTGGAACAGCTGCT CTGGGGTGCCTGGTCAAGGACTATTTCCCCGAGCCT GTGACCGTCTCTTGGAACAGCGGGGCACTGACCTCC GGTGTCCATACATTTCCTGCAGTGCTGCAAAGTTCTG GATTGTATTCACTCTCCTCTGTCGTGACGGTTCCTAG TTCATCCCTGGGCACCCAGACTTATATTTGCAATGTC AACCACAAACCCAGCAATACTAAAGTGGATAAGAAG GTCGAACCCAAGTCTTGCGATAAGACACACACATGC CCTCCTTGTCCCGCACCAGAGGCTGCTGGAGCACCA TCAGTGTTTCTTTTCCCACCTAAGCCCAAGGATACAC TGATGATTAGTCGCACACCCGAAGTGACATGCGTCG TCGTAGACGTGAGCCACGAGGACCCTGAGGTGAAAT TTAACTGGTACGTGGATGGAGTGGAGGTACATAATG CAAAGACAAAACCCCGAGAAGAGCAGTACAACAGC ACTTACAGGGTGGTGTCCGTTCTGACAGTCCTGCAT CAGGATTGGCTTAACGGTAAAGAATATAAGTGTAAA GTTAGTAATAAGGCCCTGCCCGCCCCCATTGAGAAG ACTATTAGTAAGGCCAAGGGACAGCCCAGGGAACCT CAGGTGTATACCCTGCCACCCTCCCGGGACGAACTG ACAAAAAATCAGGTATCATTGACCTGCCTCGTGAAG GGCTTTTATCCCTCAGATATAGCCGTGGAATGGGAGT CAAATGGCCAGCCTGAGAACAATTACAAGACTACGC CTCCCGTGCTGGATTCTGATGGATCATTTTTCTGTAC AGCAAGCTGACCGTGGATAAGTCCCGGTGGCAGCA GGGGAACGTGTTCTCTTGCTCCGTGATGCACGAGGC ACTTCATAACCATTATACCCAAAAGTCTCTCTCACTG TCCCCCGGCAAA |
| 40 | Amino acid sequence of heavy chain of 2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPELLGGPSVFLFPPKPKDTLMISRTPEVTCVVV DVSHEDPEVKFNWYVDGVEVHNAKTKPREEQYNSTY RVVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLTV DKSRWQQGNVFSCSVMHEALHNHYTQKSLSLSPGK |

(continued)

| SEQ ID NO: | Description | |
|---|---|---|
| 41 | Amino acid sequence of light chain of 2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |
| 42 | Amino acid sequence of heavy chain of 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPEAAGAPSVFLFPPKPKDTLMISRTPEVTCVV VDVSHEDPEVKFNWYVDGVEVHNAKTKPREEQYNST YRVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKLT VDKSRWQQGNVFSCSVMHEALHNHYTQKSLSLSPGK |
| 43 | Amino acid sequence of light chain of 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |

The abbreviations used herein have the following meanings:

| Abbreviati ons | Meanings |
|---|---|
| CDR | Complementary determining region in the variable region of an immunoglobulin |
| FR | Antibody framework region: amino acid residues, other than CDR residues, in the variable region of an antibody |
| VH | Heavy chain variable region of an antibody |
| VL | Light chain variable region of an antibody |
| IgG | Immunoglobulin G |
| IMGT | A numbering system based on the international ImMunoGeneTics information System® (IMGT) initiated by Lefranc et al., see Lefranc et al., Dev. Comparat. Immunol. 27: 55-77, 2003. |
| Kabat | Immunoglobulin alignment and numbering system proposed by Elvin A. Kabat (see, for example, Kabat et al., Sequences of Proteins of Immunological Interest, 5th Ed. Public Health Service, National Institutions of Health, Bethesda, Md., 1991). |
| Chothia | Immunoglobulin numbering system proposed by Chothia et al., which is a classical rule for identifying CDR boundaries based on the location of structural ring regions (see, for example, Chothia & Lesk (1987) J. Mol. Biol. 196: 901-917; Chothia et al. (1989) Nature 342: 878-883). |

(continued)

| Abbreviati ons | Meanings |
|---|---|
| AbM | Definition of CDR comes from Martin's study (Martin ACR, Cheetham JC, Rees AR (1989) Modelling antibody hypervariable loops: A combined algorithm. Proc Natl Acad Sci USA 86: 9268-9272). |
| mAb | Monoclonal antibody |
| EC50 | A concentration that produces 50% efficacy or binding |
| IC50 | A concentration that produces 50% inhibition |
| ELISA | Enzyme-linked immunosorbent assay |
| PCR | Polymerase chain reaction |
| HRP | Horseradish peroxidase |
| $K_D$ | Dissociation equilibrium constant |
| Ka | Association rate constant |
| Kd | Dissociation rate constant |
| ADCC | Antibody-dependent cell-mediated cytotoxicity |
| CDC | Complement-dependent cytotoxicity |
| FACS | Fluorescence activated Cell Sorting |
| CDR-H1 | Complementary determining region 1 in the heavy variable region of immunoglobulin |
| CDR-H2 | Complementary determining region 2 in the heavy variable region of immunoglobulin |
| CDR-H3 | Complementary determining region 3 in the heavy variable region of immunoglobulin |
| CDR-L1 | Complementary determining region 1 in the light variable region of immunoglobulin |
| CDR-L2 | Complementary determining region 2 in the light variable region of immunoglobulin |
| CDR-L3 | Complementary determining region 3 in the light variable region of immunoglobulin |

| Abbreviations | Meaning | Abbreviations | Meaning |
|---|---|---|---|
| HATU | N,N,N',N'-tetramethyl-O-(7-azabenzotriazole-1-yl)urea hexafluorophosphate | HOBt | 1-Hydroxybenzotriazole |
| DIPEA | Diisopropylethylamine | EDCI | 1- (3-Dimethylaminopropyl) -3-ethylcarbodiimide hydrochloride |
| TFA | Trifluoroacetic acid | DMF | N,N-Dimethylformamide |
| Pd/C | Palladium-carbon | DMTMM | 4-(4,6-Dimethoxytriazin-2-yl)-4-methylmorpholine hydrochloride |
| NBS | N-bromosuccinimide | IPA | Isopropyl alcohol |
| $NaIO_4$ | Sodium periodate | $RuCl_3.H_2O$ | Ruthenium trichloride hydrate |
| XPhos Pd G3 | (2-dicyclohexylphosphino-2',4',6'-tri-isopropyl-1,1'-biphenyl(2'-amino-1,1 '-biphenyl-2-yl) methanesulfonate Palladium (II) | $LiOH.H_2O$ | Lithium hydroxide hydrate |
| $K_3PO_4$ | Potassium phosphate | m-CPBA | m-Chloroperoxybenzoic acid |
| THF | Tetrahydrofuran | DMSO | Dimethyl sulfoxide |
| DCM | Dichloromethane | MeOH | Methanol |
| HPLC | High performance liquid chromatography | LC-MS | Liquid chromatography-mass spectrometry |

(continued)

| Abbreviations | Meaning | Abbreviations | Meaning |
|---|---|---|---|
| TCEP | Tris(2-carboxyethyl)phosphine | $Na_2HPO_4$ | Disodium hydrogen phosphate |
| EDTA | Ethylenediaminetetraacetic acid | PB | Phosphate buffer |
| DAR | Drug loading ratio | | |

The structures of the compounds described in the following examples were confirmed by nuclear magnetic resonance (1H NMR) or mass spectrometry (MS).

Nuclear magnetic resonance ($^1$H NMR) was measured by using a Bruker 400 MHz NMR instrument; the deuterated reagent was hexadeuterated dimethyl sulfoxide (DMSO-d6); and the internal standard substance was tetramethylsilane (TMS).

Abbreviations in nuclear magnetic resonance (NMR) spectra used in the examples were shown as below.

s: singlet, d: doublet, t: triplet, q: quartet, m: multiplet, br: broad, J: Coupling constant, Hz: Hertz, and DMSO-d6: deuterated dimethyl sulfoxide. δ values were expressed in ppm values.

Mass spectrometry (MS) was determined by using an Agilent (ESI) mass spectrometer, model Agilent 6120B.

Example 1 N-((S)-10-benzyl-1-((1S,9S)-9-ethyl-5-fluoro-9-hydroxy-4-methyl-10,13-diox o-2,3,9,10,13,15-hexahydro-1H,12H-benzo[de]pyrano[3',4':6,7]indolizino[1,2-b]quinolin-1-yl)a mino)-1,6,9,12,15-pentaoxo-3-oxa-5,8,11,14-tetraazahexadecan-16-yl-6-(2,5-dioxo-2,5-dihydro-1-H-pyrrol-1-yl)hexanamide (M-01)

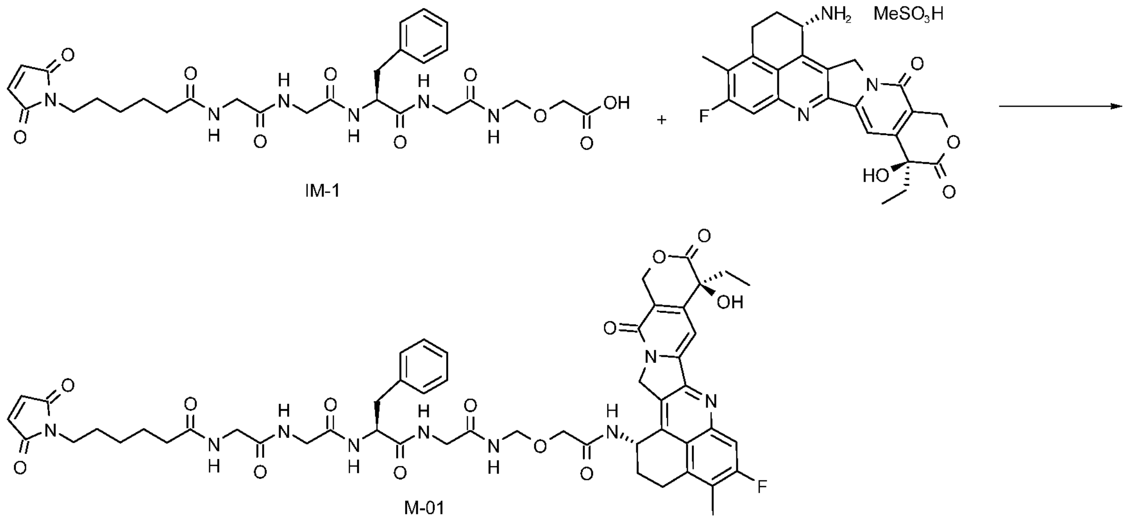

IM-1

M-01

Compound IM-1 (0.40 g, 640.59 μmol, the synthesis refers to patent CN 111936169A), and exitecan mesylate (0.37 g, 704.65 μmol) was dissolved in DMF (8 mL), followed by addition of HATU (0.32 g, 832.77 μmol) and DIPEA (0.25 g, 1.92 mmol), and the resulting solution reacted at 25°C for 4 h. DIPEA was removed under reduced pressure; lyophilization was performed after adding water to remove most of the DMF to obtain a crude product. The crude product was purified by preparative high performance liquid chromatography under the following conditions to obtain 273 mg of M-01 compound.

Chromatographic column: Waters XBridge Prep C18 OBD 45 mm×450 mm×8.0 μm

Mobile phase A: Acetonitrile; Mobile phase B:Water (0.05% Trifluoroacetic acid)

| Time [min] | Mobile phase A [%] | Mobile phase B [%] | Flow rate [mL/min] |
|---|---|---|---|
| 0.00 | 30 | 70 | 70 |
| 5.00 | 30 | 70 | 70 |
| 60.00 | 70 | 30 | 70 |

The structural characterization data of M-01 were as follows:
ESI-MS (m/z):1034.4[M+H]$^+$.

Example 2 N-((S)-10-benzyl-1-(((1S,9S)-5-chloro-9-ethyl-9-hydroxy-4-methyl-10,13-dio xo-2,3,9,10,13,15-hexahydro-1H,12H-benzo[de]pyran[3',4':6,7]indolizine[1,2-b]quinolin-1-yl)a mino)-1,6,9,12,15-pentaoxo-3-oxa-5,8,11,14-tetraazahexadecan-16-yl)-6-(2-(methylsulfonyl)pyr imidin-5-yl)hexan-5-amide (A-05)

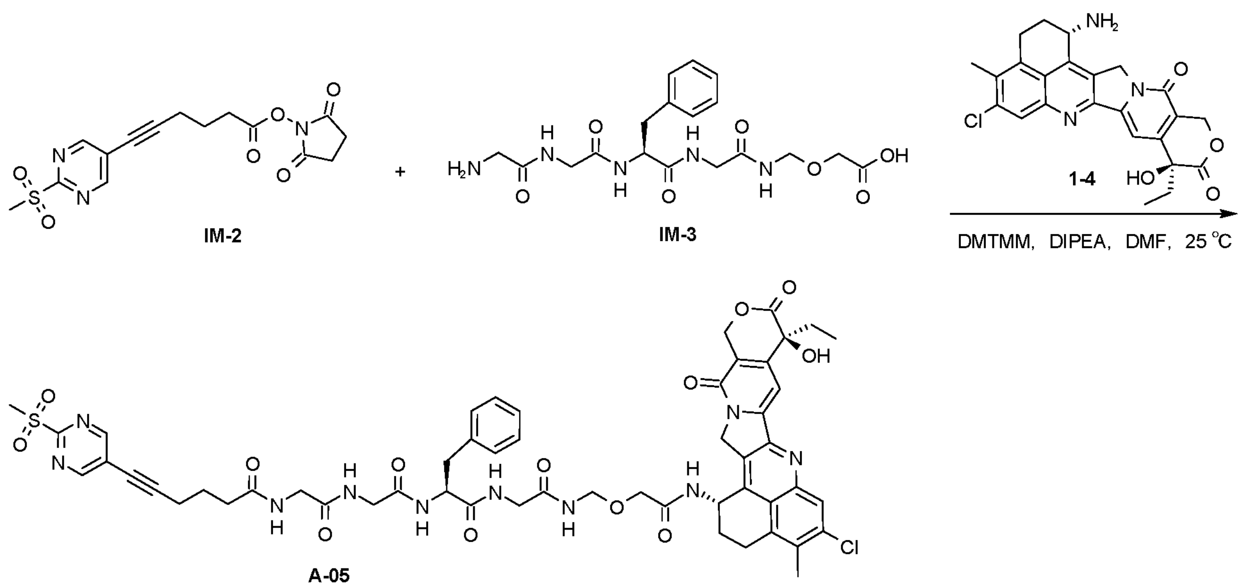


IM-2 + IM-3 → A-05 (1-4)

Under the protection of nitrogen, 2,5-dioxopyrrolidin-1-yl-6-(2-(methylsulfonyl)pyrimid in-5-yl)hexyl-5-ynate (IM-2,0.66 g,1.80 mmol) and (R)-16-amino-10-benzyl-6,9,12,15-tetrao xo-3-oxa-5,8,11,14-tetraazapentadecanoic acid (IM-3, 0.75 g, 1.77 mmol) were added into DMF (19 mL), the resulting solution was heated to 35 °C to react for 16 h, and then (1S,9S)-1-amino-5-chloro-9-ethyl-9-hydroxy-4-methyl-1,2,3,9,12,15-hexahydro-10H,13H-benzo [de]pyran [3',4':6,7]indolizino[1,2-b]quinolin-10,13-diketone (1-4, 1.00 g, 1.77 mmol) was a dded to the reaction system. The reaction solution was cooled to 5~15°C with ice water, DMTMM (0.98 g, 3.53 mmol) was added, and DIPEA (1.14 g, 8.84 mmol) was then ad ded dropwise, the resulting solution reacted at 25°C for 16 h. The reaction solution was poured into a mixed solution of DCM (600 mL), IPA (60 mL) and water (100 mL) and stirred for 10 minutes. The DCM phase was isolated, washed with brine (100 ml), and c oncentrated to obtain a crude product. The crude product was purified by preparative hig h performance liquid chromatography and then freeze-dried to obtain 0.98 mg of Compo und A-05.

The separation and purification method of A-05 was as follows:

Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 80 | 20 | 24 |

The structural characterization data of A-05 were as follows:

MS m/z (ESI): 1107.3 [M+H]+
$^1$H NMR (400 MHz, DMSO) δ 9.10 (s, 2H), 8.66 - 8.63 (m, 1H), 8.51 (d, J = 8.8 Hz, 1H), 8. 34 - 8.31 (m, 1H), 8.21 - 8.19 (m, 1H), 8.17 - 8.09 (m, 2H), 8.08 - 8.04 (m, 1H), 7.30 (s, 1H), 7.2 6 - 7.15 (m, 5H), 6.55 (s, 1H), 5.56 - 5.55 (m, 1H), 5.48 - 5.35 (m, 2H), 5.25 - 5.10 (m, 2H), 4.6 4 (d, J = 6.4 Hz, 2H), 4.45 - 4.44 (m, 1H), 4.06 - 3.98 (m, 2H), 3.77 - 3.52 (m, 6H), 3.41 (s, 3H), 3.25 - 3.12 (m, 2H), 3.03 - 3.00 (m, 1H), 2.83 - 2.72 (m, 1H), 2.58 - 2.56 (m, 2H), 2.48 (s, 3H), 2 .33 - 2.30 (m,

2H), 2.21 - 2.13 (m, 2H), 1.91 - 1.76 (m, 4H), 0.87 (t, J = 7.2 Hz, 3H).

Example 3 N-((S)-10-benzyl-1-(((1S,9S)-5-fluoro-9-ethyl-9-hydroxy-4-chloro-10,13-di oxo-2,3,9,10,13,15-hexahydro-1H,12H-benzo[de]pyran[3',4':6,7]indolizine[1,2-b]quinolin-1-yl)a mino)-1,6,9,12,15-pentaoxo-3-oxo-5,8,11,14-tetraazahexadecan-16-yl)-6-(2-(methylsulfo)pyrimi din-5-yl)hexan-5-amid(A-07)

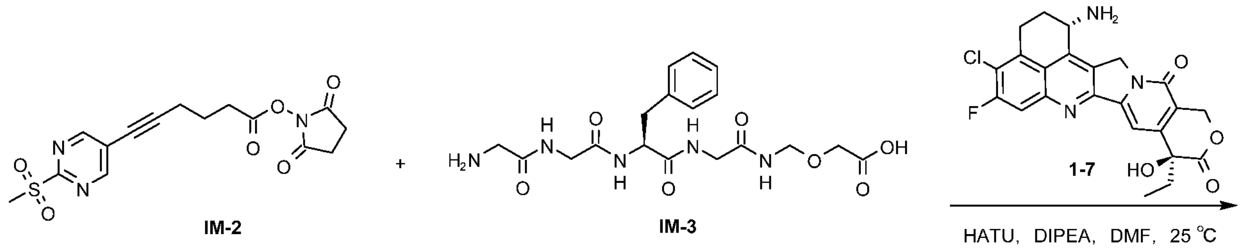

IM-2 + IM-3

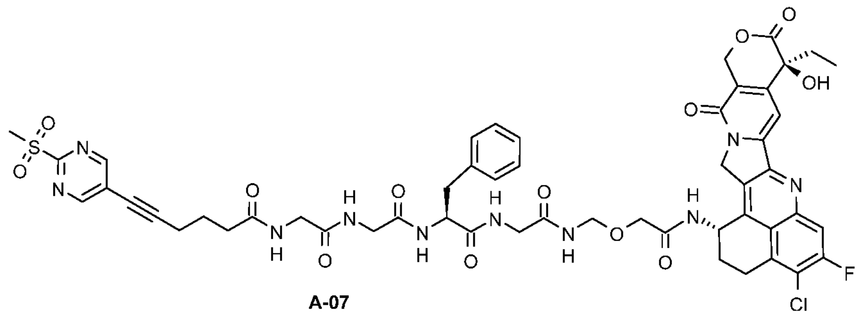
A-07

Under the protection of nitrogen, 2,5-dioxopyrrolidin-1-yl-6-(2-(methylsulfonyl)pyrimid in-5-yl)hexyl-5-ynate (IM-2,21.6 mg, 0.059 mmol) and (R)-16-amino-10-benzyl-6,9,12,15-te traoxo-3-oxo-5,8,11,14-tetraazapentadecanoic acid (IM-3, 24.5 mg, 0.058 mmol) were adde d to DMF (1 mL), and the resulting solution was heated to 35°C to react for 16 h. (1S, 9S)-1-amino-5-fluoro-9-ethyl-9-hydroxy-4-chloro-1,2,3,9,12,15-hexahydro-10H,13H-benzo[de] pyran[3',4':6,7] indolizino[1,2-b]quinolin-10,13-dione trifluoroacetate (30.0 mg, 0.053 mmol), HATU (30 mg, 0.079 mmol) and DIPEA (27.2 mg, 0.21 mmol) were added to the reac tion system to have a reaction at 25 °C for 16 h. The reaction solution was directly pur ified by preparative high performance liquid chromatography and then freeze-dried to obt ain 26.4 mg of Compound A-07.

The separation and purification method of A-07 was as follows:

Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 30 | 70 | 28 |
| 2 | 30 | 70 | 28 |
| 18 | 90 | 10 | 28 |

The structural characterization data of A-07 were as follows:
ESI-MS (m/z):1111.3[M+H]$^+$.

Example 4 N-((7S,10S,13S)-1-(((1S,9S)-5-chloro-9-ethyl-9-hydroxy-4-methyl-10,13-di oxo-2,3,9,10,13,15-hexahydro-1H,12H-benzo[de]pyran[3',4':6,7]indolizino[1,2-b]quinolin-1-yl)a mino)-7,10-dimethyl-1,6,9,12-tetraoxo-3-oxo-5,8,11-triazotetradecane-13-yl)-6-(2-(methylsulfo) pyrimidin-5-yl)hexan-5-amid (A-14)

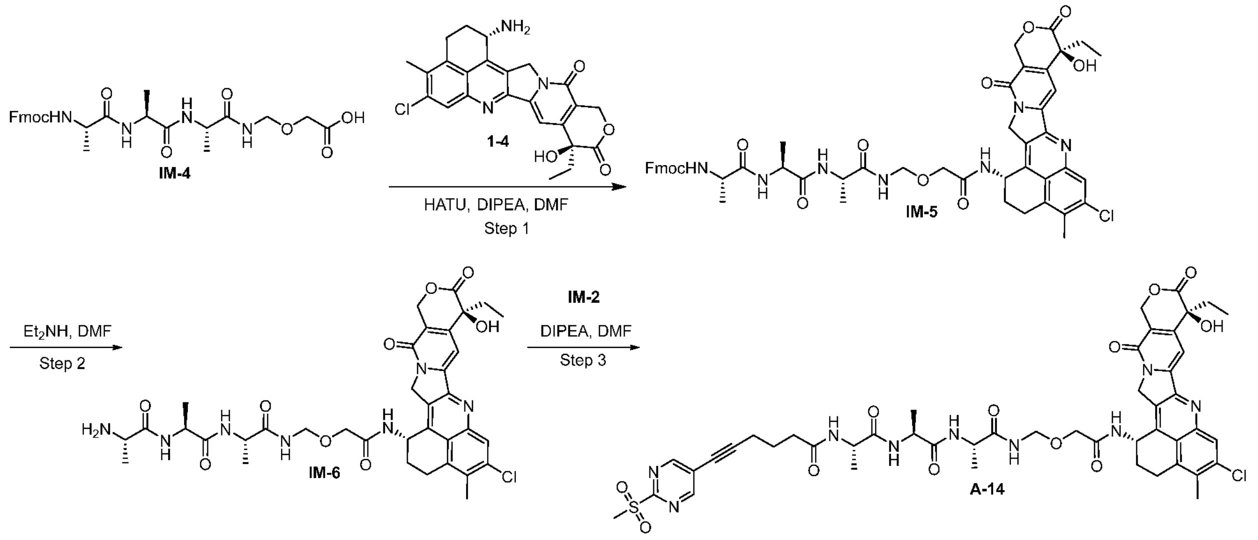


Step 1:

Compound IM-4 (657 mg, 1.22 mmol) and Compound 1-4 (500 mg, 1.11 mmol) were dissolved in N, N-dimethylformamide (10 mL), followed by addition of HATU (630.67 mg, 1.66 mmol) and N, N-diisopropylethylamin (428 mg, 3.32 mmol), and the resulting solution was stirred at room temperature for 1 h. After the reaction was completed, the reaction solution was directly purified by preparative high performance liquid chromatography and then freeze-dried to obtain 700 mg of Compound IM-5.

The preparation method was as follows:

Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

Step 2:

Compound IM-5 (500 mg, 0.513 mmol) was dissolved in N, N-dimethylformamide (2 mL), followed by addition of diethylamine (75.05 mg, 1.03 mmol), and the resulting solution reacted at room temperature for 1 h. After the reaction completed, the reaction solution was directly purified by preparative high performance liquid chromatography and then freeze-dried to obtain 307 mg of Compound IM-6.

The preparation method was as follows:

Chromatographic column: Waters SunFire Prep C18 OBD (5 μm*19 mm*150 mm)
Mobile phase A: Acetonitrile: Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2.00 | 20 | 80 | 24 |
| 18.00 | 80 | 20 | 24 |

Step 3:

IM-6 (170 mg, 0.226 mmol) and Compound IM-2 (90.83 mg, 0.249 mmol) were dissolved in N, N-dimethylformamide (10 mL), followed by addition of N, N-diisopropylethylamin (29.21 mg, 0.226 mmol), and the resulting solution was stirred at room temperature for 16 h. The reaction solution was directly purified by preparative high performance liquid chromatography and then freeze-dried to obtain 50.56 mg of Compound A-14.

The structural characterization data were as follows:

MS m/z (ESI): 1002.4 [M+H]+

The preparation method was as follows:

Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

$^1$H NMR (400 MHz, DMSO) δ 9.11 (s, 2H), 8.68 (t, J = 6.4 Hz, 1H), 8.49 (d, J = 8.8 Hz, 1H), 8.1 6 (s, 1H), 8.10 (d, J = 7.2 Hz, 1H), 8.01 (d, J = 7.2 Hz, 1H), 7.91 (d, J = 6.8 Hz, 1H), 7.31 (s, 1H) , 6.55 (s, 1H), 5.65-5.55 (m, 1H), 5.43 (s, 2H), 5.21 (s, 2H), 4.67-4.55 (m, 2H), 4.29-4.15 (m, 3H), 3.98 (s, 2H), 3.41 (s, 3H), 3.25-3.15 (m, 2H), 2.57-2.56 (m, 2H), 2.35-2.27 (m, 2H), 2.22-2.12 (m, 2H), 1.91-1.75 (m, 4H), 1.23-1.09 (m, 9H), 0.87 (t, J = 7.2 Hz, 3H).

Example 5 Synthesis of 4-((S)-2-(4-aminobutyl)-35-(4-((6-(2-(methylsulfonyl)pyrimid in-5-yl)hexan-5-alkynylamido) methyl)-1H-1,2,3-triazol-1-yl)-4,8-dioxo-6,12,15,18,21,24,27,30, 33-nonaoxa-3,9-diazapentatriacontanamido)benzyl((S)-4-ethyl-11-(2-(N-isopropylmethylsulfona mido)ethyl)-3,14-dioxo-3,4,12,14-tetrahydro-1H-pyrano[3',4':6,7]indolizino[1,2-b]quinolin-4-yl) carbonate (B-01)

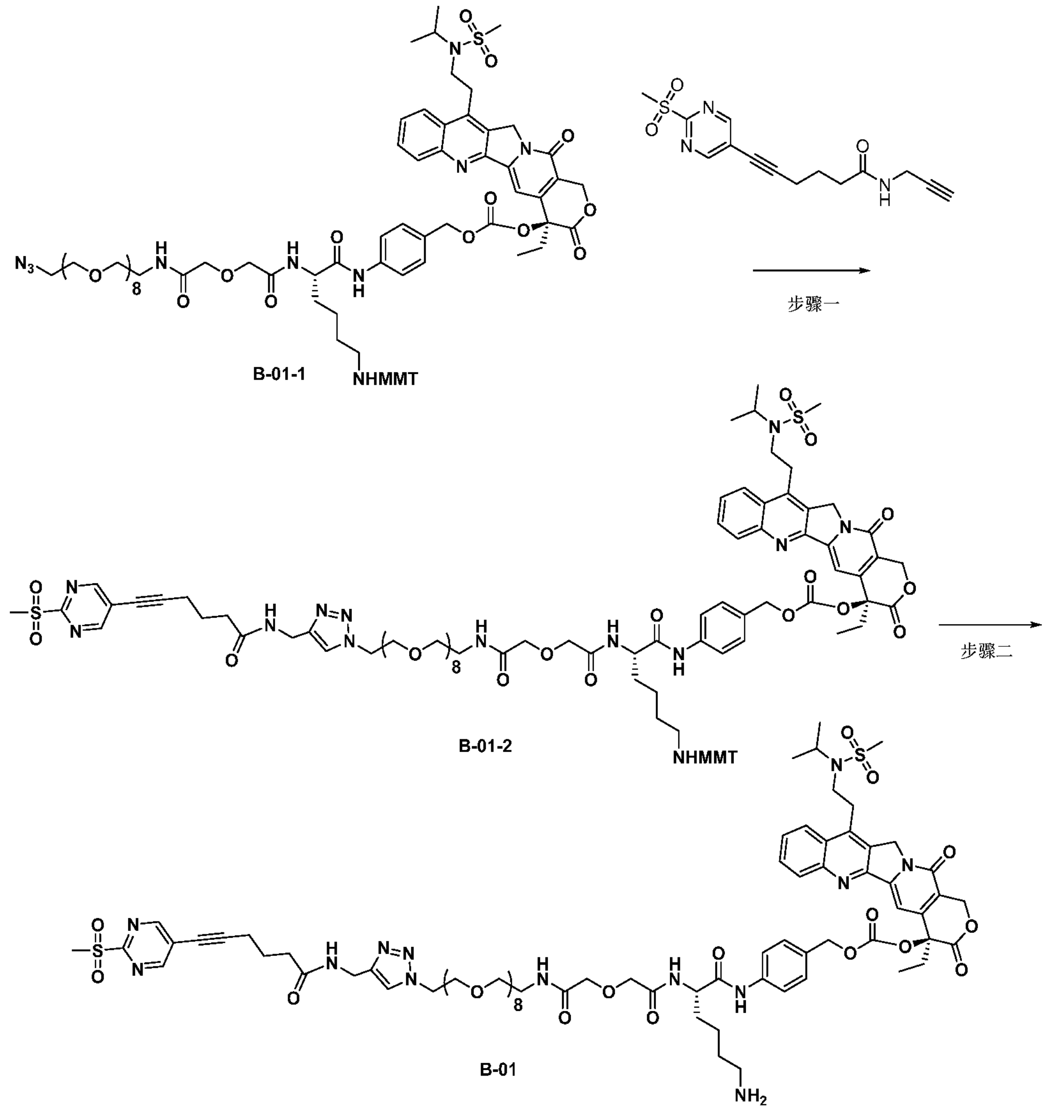


B-01-1

B-01-2

B-01

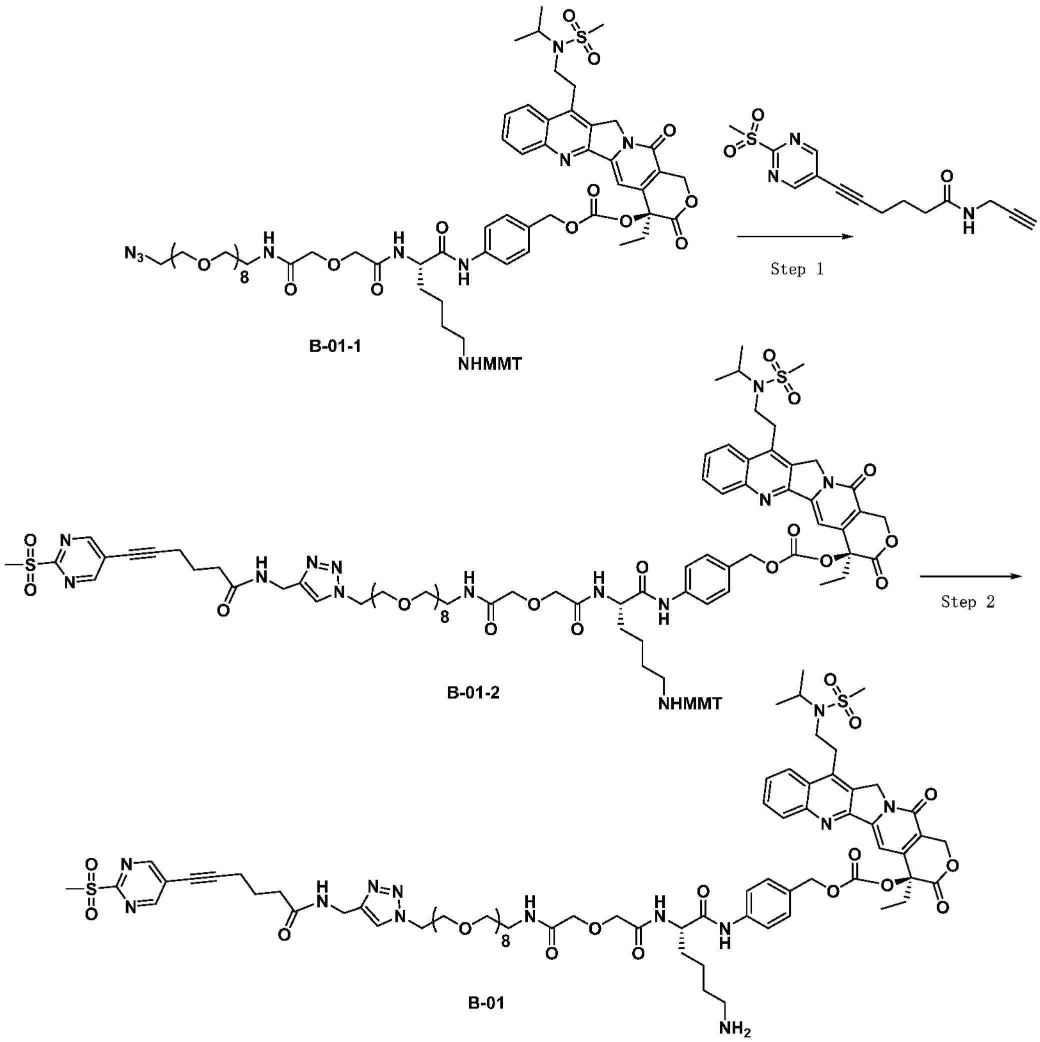


B-01-1

B-01-2

B-01

Step 1:

Compound B-01-1 (413.40 mg, 0.251 mmol, the synthesis refers to patent CN111295389B) was dissolved in dimethyl sulfoxide and water (2.0 mL: 0.5 mL) at room temperature, followed by addition of cuprous bromide (72.95 mg, 0.503 mmol) and 6-(2-(methylsulfonyl)pyrimidin-5-yl)-N-(prop-2-yn-1-yl)-hexan-5-yneamide (95.10 mg, 0.302 mmol), and the resulting solution was stirred to react for 1 h and filtered. The filtrate was purified by preparative high performance liquid chromatography (the conditions were as follows) to obtain 30.00 mg of Compound B-01-2.

Chromatographic column: SunFire Prep C18 OBD 19 mm×150 mm×5.0 μm
Mobile phase A: Acetonitrile: Mobile phase B: Water

| Time [min] | Mobile phase A [%] | Mobile phase B [%] | Flow rate [mL/min] |
|---|---|---|---|
| 0.00 | 40 | 60 | 28 |
| 8.00 | 40 | 60 | 28 |
| 50.00 | 90 | 10 | 28 |

Step 2:

Compound B-01-2 (30.00 mg, 0.02 mmol) was dissolved in dichloromethane (1.0 mL), then trifluoroacetic acid (0.2 mL) was added to the reaction solution, and reaction was allowed at room temperature for 30 min. The reaction solution was concentrated under reduced pressure and purified by preparative high performance liquid chromatography

(the conditions were as follows) to obtain 20.00 mg of trifluoroacetate of Compound B-01.

Chromatographic column: SunFire Prep C18 OBD 19 mm×150 mm×5.0 μm
Mobile phase A: Acetonitrile; Mobile phase B: Water (0.05% Trifluoroacetic acid)

| Time [min] | Mobile phase A [%] | Mobile phase B [%] | Flow rate [mL/min] |
|---|---|---|---|
| 0.00 | 15 | 85 | 28 |
| 8.00 | 15 | 85 | 28 |
| 50.00 | 60 | 40 | 28 |

The structural characterization data were as follows: ESI-MS (m/z):1631.7$[M+H]^+$, 816.0$[M/2+H]^+$.

Example 6 4-((S)-2-(4-aminobutyl)-35-(4-((6-(2-(methylsulfonyl)pyrimidin-5-yl)hexan-5-alkynylamido) methyl)-1H-1,2,3-triazol-1-yl)-4,8-dioxo-6,12,15,18,21,24,27,30,33-nonaoxa-3, 9-diazapentatriacontanamido)benzyl((1S,9R)-9-ethyl-5-fluoro-1-(2-hydroxyacetamido)-4-methyl -10,13-dioxo-2,3,9,10,13,15-hexahydro-1H,12H-benzo [de]pyrano[3',4':6,7]indolizino[1,2-b]quin olin-9-yl)carbonate (B-02)

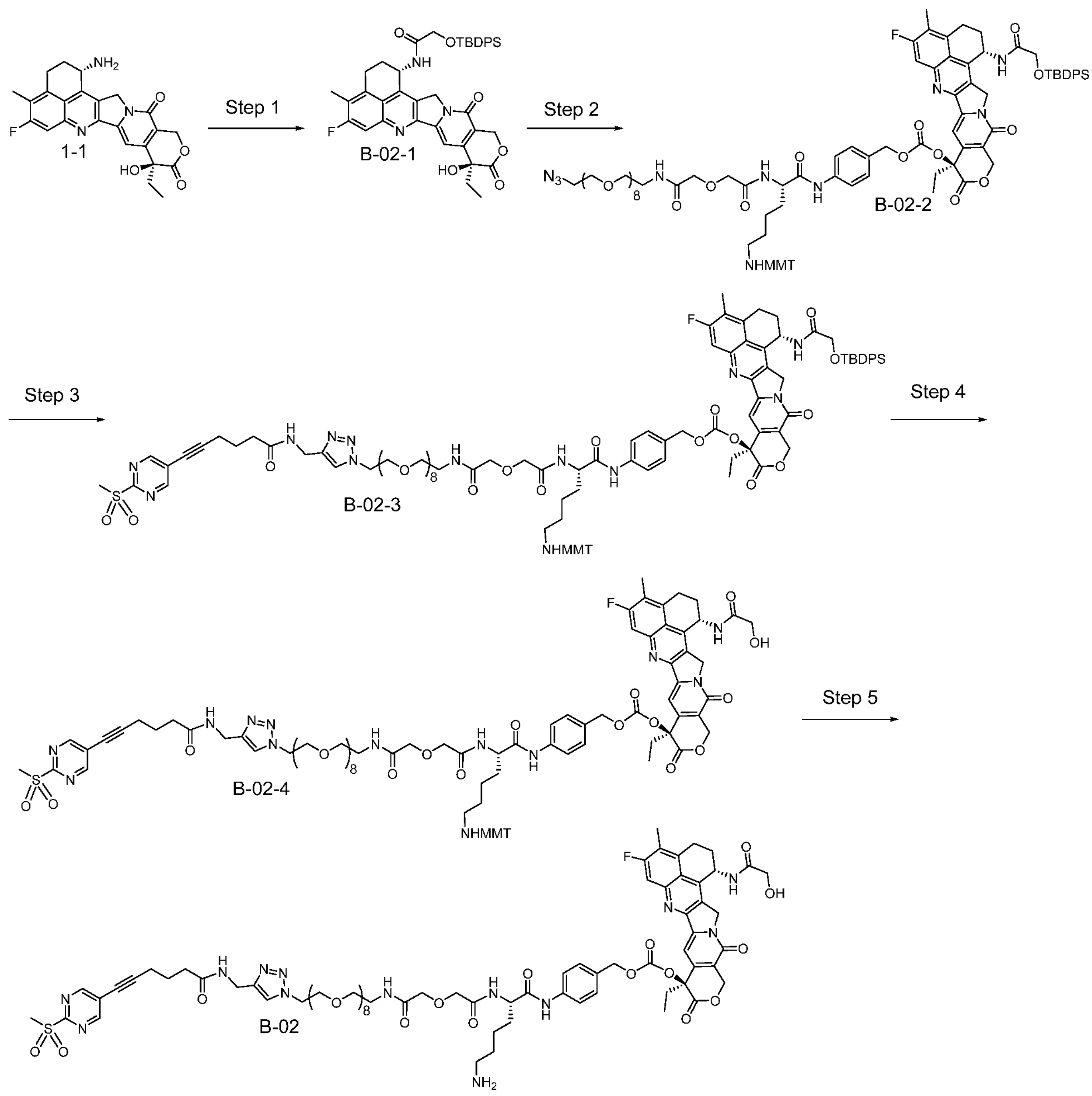


Step 1:

Methanesulfonate of 1-1 (30.00 mg, 56.44 μmol) was dissolved in N, N-dimethylformamide (1 mL) at 25 °C, followed by addition of 1H-benzotriazol-1-oxytripyrtyl hexafluorophosphate (58.74 mg, 112.88 μmol), N,N-diisopropylethylamin (43.76 mg, 338.63 μmol) and 2-((tert-butyldiphenylsilyl)oxo)acetic acid (26.62 mg, 84.66 μmol), and the resulting solution reacted at 25 °C for 1 h. The reaction was monitored by liquid chromatography-mass spectrometry. After the reaction completed, water and ethyl acetate were added into the reaction solution for extraction; the organic phases were combined, dried with sodium sulfate and concentrated under reduced pressure; and the crude product was isolated by thin layer chromatography (dichloromethane: methanol=15:1) to obtain 27.00 mg of Compound B-02-1.

Step 2:

B-02-1 (20 mg, 27.33 μmol) was dissolved in dichloromethane (2 mL) at 0 °C, followed by addition of 4-dimethylaminopyridine (26.71 mg, 218.61 μmol) and triphosgene (8.11 mg, 27.33 μmol) in dichloromethane solution (0.5 mL), and the resulting solution reacted at 0 °C for 0.5 h; after the residual triphosgene was replaced with nitrogen, (S)-2-(32-azido-5-oxo-3,9,12,15,18,21,24,27,30-nonaoxa-3,9-diazapentatriacontanamido)-N-(4-(hydroxymethyl)phenyl)-6-(((4-methoxyphenyl)benzhydryl)amino)hexanamide (43.46 mg, 40.99 μmol) in dichloromethane solution (1 mL)

was added dropwise, and the resulting solution reacted at 0 °C for 0.5 h; and the reaction was monitored by liquid chromatography-mass spectrometry. After the reaction completed, the reaction solution was concentrated; and the crude product was isolated by thin layer chromatography (dichloromethane: methanol=15:1) and purified to obtain 30.00 mg of Compound B-02-2.

Step 3:

B-02-2 (250.00 mg, 137.51 μmol) was dissolved in a mixed solvent of DMSO (2 mL) and water (0.4 mL) at 25 °C, followed by addition of 6-(2-(methylsulfonyl)pyrimidin-5-yl)-N-(prop-2-yne-1-yl) hexan-5-yneamid (62.98 mg, 206.26 μmol) and cuprous bromide (39.45 mg, 275.01 μmol), and the resulting solution reacted at 25 °C for 1 h; the reaction was monitored by liquid chromatography-mass spectrometry; after the reaction completed, the reaction solution was purified by preparative high performance liquid chromatography (the conditions were as follows) to obtain 150.00 mg of Compound B-02-3.

Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 18 | 90 | 10 | 28 |

Step 4:

B-02-3 (150 mg, 49.45 μmol) was dissolved in tetrahydrofuran (1 mL) at 25 °C, a mixed solution of tetrabutylammonium fluoride (1M of tetrahydrofuran solution)/glacial acetic acid (v/v=13/1) (50 uL) was added dropwise, and the resulting solution reacted at 25 °C for 0.5 h. The reaction was monitored by liquid chromatography-mass spectrometry; after the reaction completed, the reaction solution was purified by preparative high performance liquid chromatography (the conditions were as follows), and the prepared solution was lyophilized to obtain 50.00 mg of Compound B-02-4.

Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 20 | 90 | 10 | 28 |

Step 5:

B-02-4 (50 mg, 26.52 μmol) was dissolved in dichloromethane (1 mL) at 25 °C, followed by addition of trifluoroacetic acid (60.49 mg, 530.49 μmol), and the resulting solution reacted at 25 °C for 0.5 h; the reaction was monitored by liquid chromatography-mass spectrometry; after the reaction completed, the reaction solution was concentrated, and the crude product was purified by preparative high performance liquid chromatography (the conditions were as follows). The prepared solution was lyophilized to obtain 23.69 mg of Compound B-02.

Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 18 | 90 | 10 | 28 |

The structural characterization data of B-02 were as follows:

ESI-MS (m/z):1613.6[M+H]+.

Example 7 N-((7S,10S,13S)-1-(((1S,9S)-9-ethyl-5-fluoro-9-hydroxy-4-methyl-10,13-di oxo-2,3,9,10,13,15-hexahydro-1H,12H-benzo[de]pyran[3',4':6,7]indolizino[1,2-b]quinolin-1-yl)a mino)-7,10,13-trimethyl-1,6,9,12,15-pentaoxo-3,17,20,23-tetraoxo-5,8,11,14-tetraazapentane-25 -yl)-3,5-bis(2-(methylsulfo)pyrimidin-4-yl)benzamide (C-07)

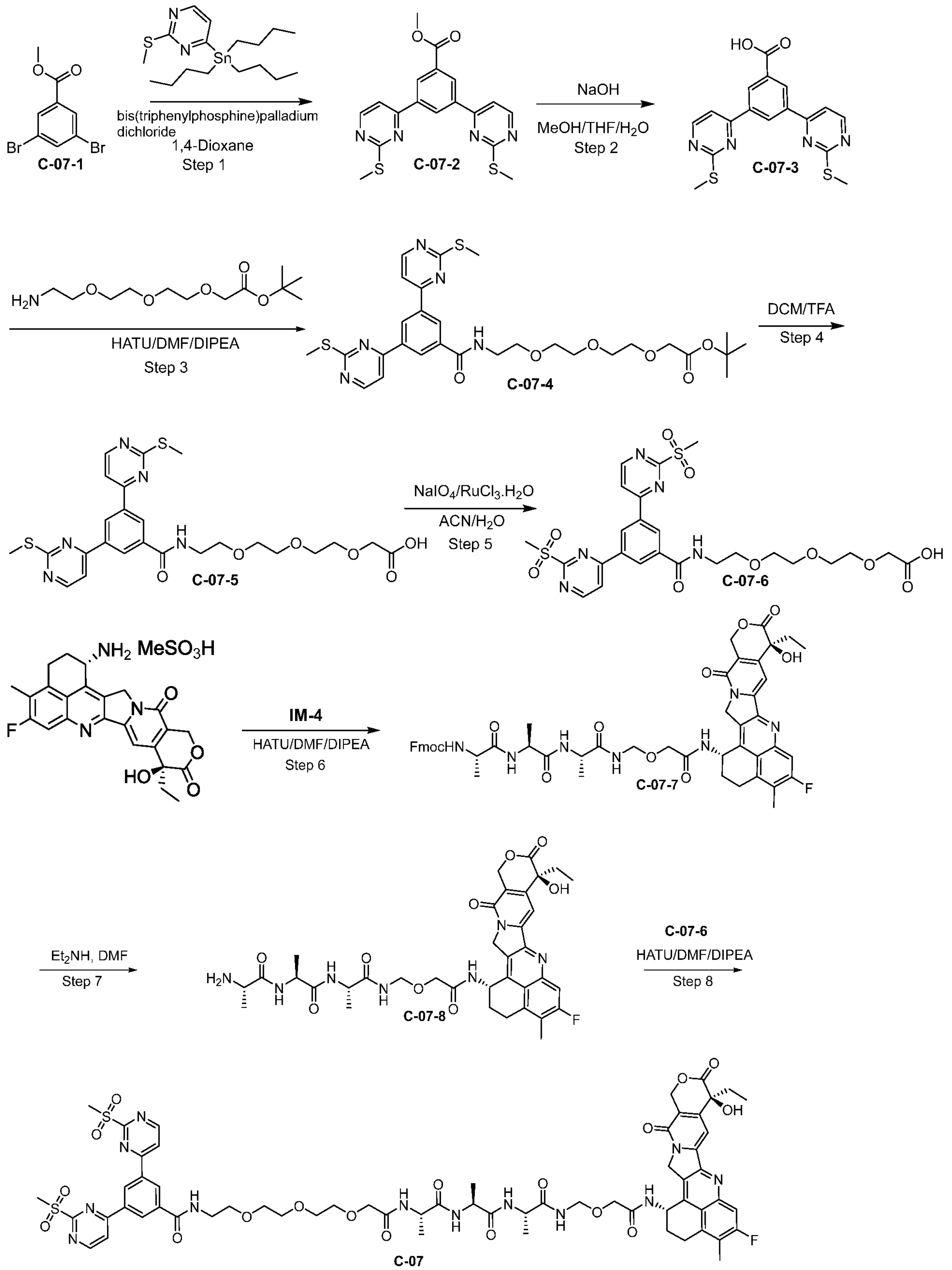


Step 1:

Raw materials C-07-1 (4.80 g, 16.33 mmol), tributyl(2-methylsulfamoylpyrimidine-4-yl) tin (16.27 g, 39.19 mmol)

and bis(triphenylphosphine)palladium dichloride (2.29 g, 3.27 mmol) were dissolved in 1,4-dioxane (100 mL), and the reaction system was stirred at 110 °C to react for 5 hours in a nitrogen atmosphere. The reaction was monitored by LC-MS, and the reaction system was concentrated and purified by column chromatography (EA/PE = 0-50%) to obtain 1.36 g of Compound C-07-2.

Step 2:

Compound C-07-2 (510 mg,1.33 mol) and NaOH (212.24 mg, 5.31 mmol) were dissolved in THF (12.5 mL), MeOH (12.5 mL) and $H_2O$ (2.5 mL), and the resulting solution was stirred at 25 °C to react for 2 h. The reaction was monitored by LC-MS, and the pH of the system was adjusted to about 2 with 3 N HCl; a large amount of solid was precipitated and filtered; and the filter cake was collected and dried to obtain 380 mg of Compound C-07-3.

Step 3:

Compound C-07-3 (315 mg, 850.32 μmol), tert-butyl 2-[2-[2-(2-aminoethoxy)ethoxy] ethoxyacetate (246.31 mg, 935.35 μmol), HATU (484.99 mg, 1.28 mmol) and DIPEA (329.69 mg, 2.55 mmol) were added to DMF (3 mL), and the resulting solution reacted at 25 °C for 2 h. The reaction was monitored by LC-MS. The reaction solution was directly purified by preparative high performance liquid chromatography and then freeze-dried to obtain 40 mg of Compound C-07-3.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 55 | 45 | 24 |
| 2.00 | 55 | 45 | 24 |
| 18.00 | 100 | 0 | 24 |

Step 4:

Compound C-07-4 (40 mg, 64.96 μmol) was dissolved in DCM (3 mL) and TFA (1.5 mL), and the resulting solution reacted at 25 °C for 1.5 h. The reaction was monitored by LC-MS, and the reaction system was concentrated to dry to obtain 36 mg of Compound C-07-5.

Step 5:

Compound C-07-5 (26 mg, 46.46 μmol), Sodium periodate (99.37 mg, 464.57 μmol) and $RuCl_3 \cdot H_2O$ (9.64 mg, 46.46 μmol) were dissolved in ACN (15 mL) and water (7.5 mL), and the resulting solution reacted at 25 °C for 40 min. The reaction was monitored by LC-MS. Extraction with water and ethyl acetate was carried out, and ethyl acetate layer was concentrated to obtain 28 mg of Compound C-07-6.

Step 6:

Compound exitecan mesylate (600 mg, 1.13 mmol), (5S,8S,11S)-1-(9H-fluoren-9-yl)-5,8,11-trimethyl-3,6,9,12-tetraoxy-2,15-dioxo-4,7,10,13-tetraazaheptane-17-carboxylic acid (IM-4, 610.17 mg, 1.13 mmol), HATU (643.81 mg, 1.69 mmol) and DIPEA (437.65 mg, 3.39 mmol) were added to DMF (6 mL), and the resulting solution reacted at 25°C for 16 h. The reaction was monitored by LC-MS. Water was added into the reaction solution, a large amount of solid was precipitated, collected by filtration, and dissolved with DCM, and a crude product was obtained by concentration. The crude product was purified by column chromatography (DCM/MeOH=0-10%) to obtain 660 mg of Compound C-07-7.

Step 7:

Compound C-07-7 (660 mg, 688.94 μmmol) was dissolved in N, N-dimethylformamide (6 mL), followed by addition of diethylamine (251.94 mg, 3.44 mmol), and the resulting solution reacted at room temperature for 1 h. After the reaction completed, the reaction solution was directly purified by preparative high performance liquid chromatography and then freeze-dried to obtain 325 mg of Compound C-07-8.

The preparation method was as follows:

Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 10 | 90 | 28 |
| 4.00 | 10 | 90 | 28 |
| 20.00 | 90 | 10 | 28 |

Step 8:

Compound C-07-6 (15.95 mg, 25.58 μmol), C-07-8 (20 mg, 25.58 μmol), HATU (14.59 mg, 38.37 μmol) and DIPEA (9.92 mg, 76.75 μmol) were added to DMF (3 mL), and the resulting solution reacted at 25 °C for 2 h. The reaction was monitored by LC-MS. The reaction solution was directly purified by preparative high performance liquid chromatography and then freeze-dried to obtain 7 mg of Compound C-07.

The structural characterization data were as follows:
ESI-MS (m/z):1342.4 $[M+H]^+$.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2 | 20 | 80 | 24 |
| 18.00 | 90 | 10 | 24 |

Example 8 N-((7S,10S,13S)-1-(((1S,9S)-9-ethyl-5-fluoro-9-hydroxy-4-methyl-10,13-d ioxo-2,3,9,10,13,15-hexahydro-1H,12H-benzo[de]pyran[3',4':6,7]indolizino[1,2-b]quinolin-1-yl) amino)-7,10,13-trimethyl-1,6,9,12,15-pentaoxo-3,17,20,23-tetraoxo-5,8,11,14-tetraazapentane-2 5-yl)-3,5-bis(2-(methylsulfo)pyrimidin-5-yl)benzamide (C-10)

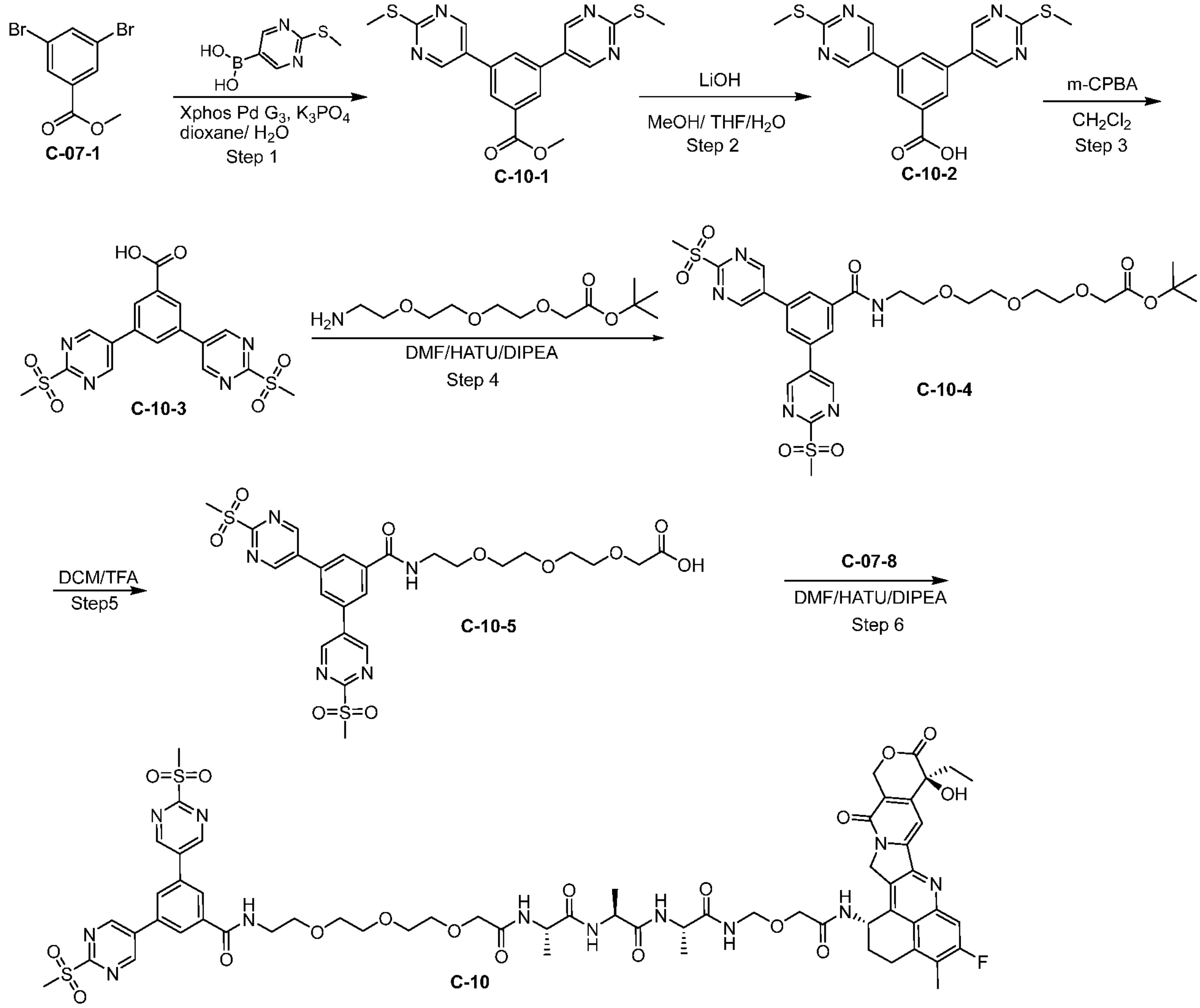


Step 1:

Raw materials C-10-1 (720 mg, 2.45 mmol), 2-methylthiopyrimidine-5-boronic acid (874 mg, 5.14 mmol), XPhosPd G3 (207 mg, 245 μmol) and $K_3PO_4$ (1.56 g, 7.35 mmol) were added into dioxane (12 mL) and $H_2O$ (4 mL), and the reaction system was stirred at 90 °C to react for 3 h in a nitrogen atmosphere. The reaction was monitored by LC-MS, and the reaction solution was filtered with diatomaceous earth. The filtrate was extracted with water and ethyl acetate, then concentrated to obtain a crude product, and the crude product was purified by column chromatography (EA/PE=0-25%) to obtain 710 mg of Compound C-10-1.

Step 2:

Compound C-10-1 (650 mg,1.69 mol) and lithium hydroxide (121 mg, 5.07 mmol) were dissolved in THF (2 mL), MeOH (2 mL) and $H_2O$ (2 mL), and the resulting solution was stirred at 25 °C to react for 2 h. The reaction was monitored by LC-MS. The pH of the system was adjusted to about 2 with 1N HCl, and a large amount of solid was precipitated. Filtration was carried out and filter cake was collected and dried to obtain 560 mg of Compound C-10-2.

Step 3:

Compound C-10-2 (450.80 mg, 1.22 mmol) was dissolved in DCM (10 mL), m-CPBA (2.46 g, 12.1 mmol, with a purity of 85% ) was added to the reaction system, and the resulting solution reacted at 25 °C for 12 h. The reaction was monitored by LC-MS. The solvent was blowed to dry with nitrogen flow to obtain a crude product. The crude product was purified by preparative high performance liquid chromatography and then freeze-dried to obtain 153 mg of Compound C-10-3.

The preparation method was as follows:

Chromatographic column: Phenomenex Luna C18 200*40mm*10um.
Mobile phase A: Acetonitrile; Mobile phase B: Water (0.05% hydrochloric acid)
Mobile phase: [water(HCl)-ACN]; B%: 13%-43%, 10min).

| Time [min] | Mobile phase A [%] | Mobile phase B [%] | Flow rate [mL/min] |
|---|---|---|---|
| 0.00 | 13 | 87 | 25 |
| 10.00 | 43 | 57 | 25 |
| 18.00 | 90 | 10 | 25 |

Step 4:

Compound C-10-3 (140 mg, 322.25 μmol), tert-butyl 2-[2-[2-(2-aminoethoxy)ethoxy] ethoxyacetate(84.86 mg, 322.25 μmol), HATU (183.80 mg, 483.37 μmol) and DIPEA (124.94 mg, 966.75 μmol) were added to DMF (4 mL), and the resulting solution reacted at 25 °C for 12 h. The reaction was monitored by LC-MS. The reaction solution was purified by preparative high performance liquid chromatography and then freeze-dried to obtain 51 mg of Compound C-10-4.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 90 | 10 | 24 |

Step 5:

Compound C-10-4 (50 mg, 73.56 μmol) was added to DCM (2 mL) and TFA (1 mL), and the resulting solution reacted at 25 °C for 1 h. The reaction was monitored by LC-MS. The reaction system was concentrated to dryness to obtain 45 mg of Compound C-10-5.

Step 6:

Compound C-10-5 (31.91 mg, 51.17 μmol), C-07-8 (40 mg, 51.17 μmol), HATU (29.18 mg, 76.75 μmol) and DIPEA (19.84 mg, 153.50 μmol) were added to DMF (3 mL), and the resulting solution reacted at 25 °C for 2 h. The reaction was monitored by LC-MS. The reaction solution was purified by preparative high performance liquid chromatography and then freeze-dried to obtain 13 mg of Compound C-10.

The structural characterization data were as follows:
ESI-MS (m/z):1342.5 [M+H]+.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2.00 | 20 | 80 | 24 |
| 18.00 | 90 | 10 | 24 |

Example 9 N-((7S,10S,13S)-1-(((1S,9S)-9-ethyl-5-chloro-9-hydroxy-4-methyl-10, 13-dioxo-2,3,9,10,13,15-hexahydro-1H,12H-benzo[de]pyran[3',4':6,7]indolizino[1,2-b]quin olin-1-yl)amino)-7,10,13-trimethyl-1,6,9,12,15-pentaoxo-3,17,20,23-tetraoxo-5,8,11,14-tetra azapentane-25-yl)-3,5-bis(2-(methylsulfo)pyrimidin-5-yl)benzamide (C-17)

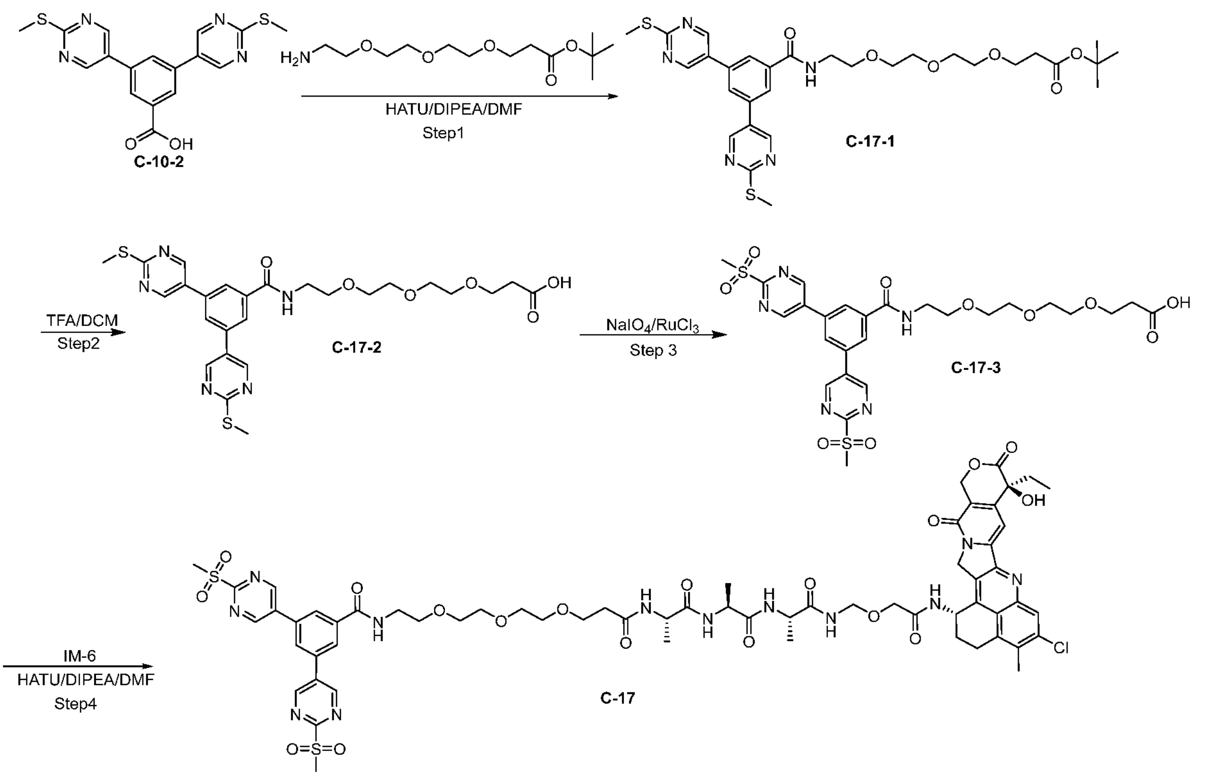


Step 1:

C-10-2 (3.00 g, 8.10 mmol) and tert-butyl 3-[2-[2-(2-aminoethoxy)ethoxy]ethoxy]-propionate (2.25 g, 8.10 mmol) were added to DMF (3 mL); HOBt (3.28 g, 24.3 mmol), EDCI (4.66 g, 24.3 mmol) and DIPEA (4.19 g, 32.4 mmol, 5.64 mL) were added in sequence, and the resulting solution was heated to 60 °C to react for 2 h. Water (50 mL) was added to the reaction solution, and extraction with ethyl acetate (30 mL x 3) was carried out. The organic phases were combined and dried with anhydrous sodium sulfate, and the reaction solution was filtered and concentrated to obtain a crude product C-17-1 (3.8 g, 4.75 mmol),which was directly used for the next step without purification.

Step 2:

C-17-1 (3.40 g, 5.40 mmol) was dissolved in dichloromethane (30 mL),followed by addition of trifluoroacetic acid (10.8 g, 94.2 mmol, 7 mL), and the reaction was stirred at 25 °C for 2 h. The reaction solution was directly concentrated and purified by preparative high performance liquid chromatography and then freeze-dried to obtain C-17-2 (2.09 g, 3.64 mmol).

The preparation method was as follows:

Chromatographic column: Phenomenex luna C18 (250 mm*70mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 22.00 | 60 | 40 | 30 |

Step 3:

C-17-2 (56 mg, 97.61 μmol) was added to acetonitrile (6 mL) and water (3 mL), and then sodium periodate (208.79 mg, 976.15μmol) and ruthenium trichloride hydrate (8.10 mg, 39.05 μmol) were added to the reaction system in sequence, and the resulting solution was stirred at 25 °C to react for 30 min. The reaction was monitored by LC-MS. Water and ethyl acetate were added for extraction and concentration to obtain C-17-3(60 mg).

Step 4:

IM-6 (20 mg, 25.06 μmol), C-17-3 (16 mg, 25.06 μmol), HATU (19.05 mg, 50.11 μmol) and DIPEA (16.19 mg, 125.28 μmol) were added in sequence to DMF (3 mL), and the reaction system reacted at 25 °C for 1 h. The reaction solution was directly purified by preparative high performance liquid chromatography and then freeze-dried to obtain Compound C-17 (16 mg).

The structural characterization data were as follows:
ESI-MS (m/z):1371.4 $[M+H]^+$.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 80 | 20 | 30 |

Example 10 N-((7S,10S,13S)-1-(((1S,9S)-5-chloro-9-ethyl-9-hydroxy-4-methyl-1 0,13-dioxo-2,3,9,10,13,15-hexahydro-1H,12H-benzo[de]pyrano[3',4':6,7]indolizino[1,2-b]qui nolin-1-yl)amino)-7,10,13-trimethyl-1,6,9,12,15-pentaoxo-3,18,21,24-tetraoxo-5,8,11,14-tetr aazahexane-26-yl)-2,6-bis(2-(methylsulfonyl)pyrimidin-5-yl)isonicotinamide (C-19)

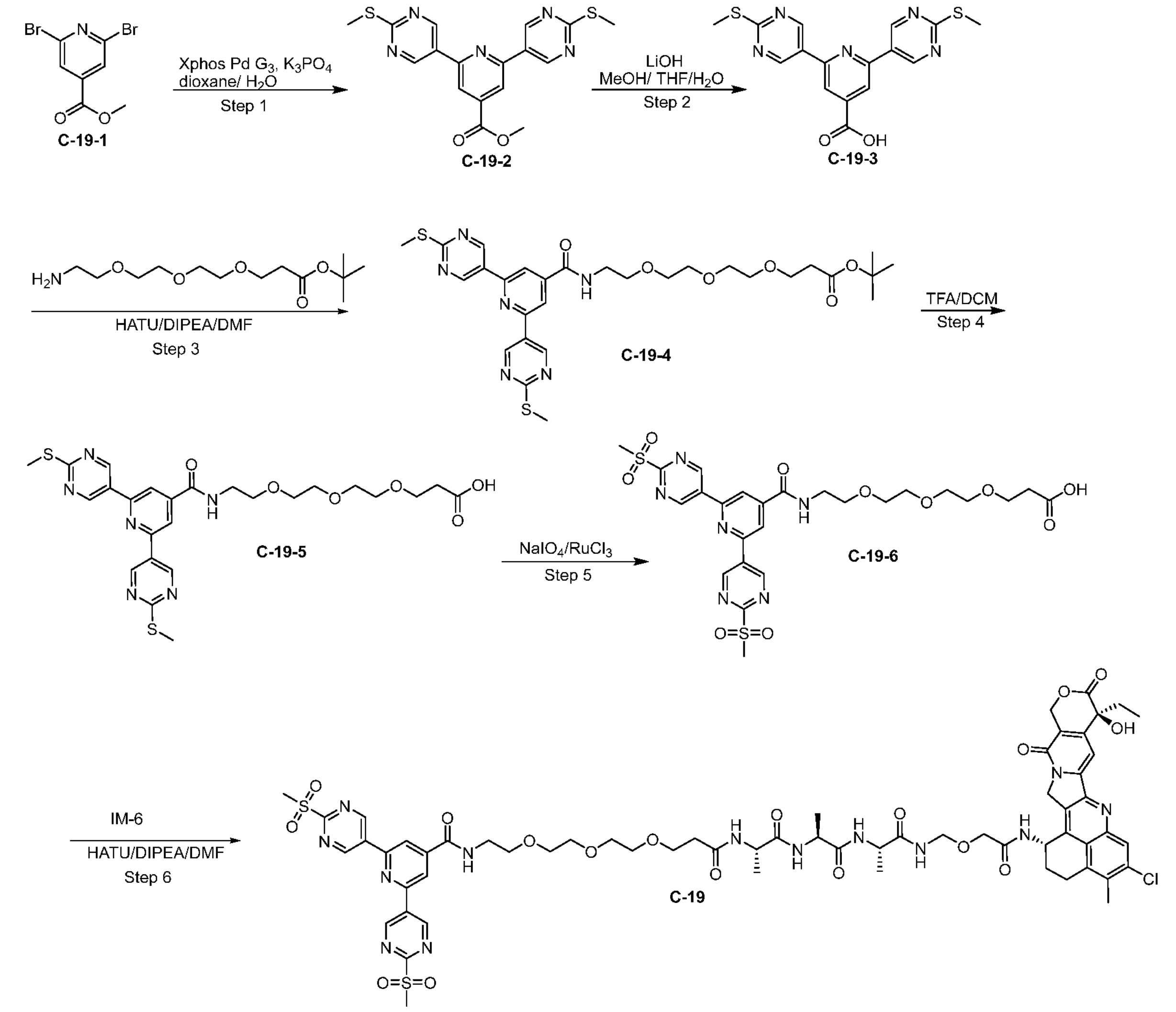


Step 1:

C-19-1 (5.00 g, 16.9 mmol), (2-(methylthio)pyrimidin-5-yl)boronic acid (6.34 g, 37.3 mmol), XPhos Pd G3 (1.44 g, 1.70 mmol) and potassium phosphate (10.80 g, 50.9 mmol) were added to 1,4-dioxane (51.0 mL) and water (17.0 mL). The reaction system was replaced with nitrogen three times and then reacted at 100 °C for 5 h. After the reaction system was cooled to room temperature, water (50.0 mL) was added to the reaction solution. The reaction solution was filtered, and the filtrate was concentrated to obtain a crude product. Trituration with petroleum ether and then filtered again, and the filter cake was vacuum dried to obtain C-19-2 (5.65 g).

Step 2:

C-19-2 (5.26 g, 13.7 mmol) was dissolved in THF (30.0 mL), MeOH (30.0 mL) and water (30.0 mL), followed by addition of LiOH•$H_2O$ (1.72 g, 40.9 mmol), and the resulting solution was stirred at 25°C for 2 h. The pH of the reaction solution was adjusted to 3 with 1 N aqueous hydrochloric acid. The solid was precipitated and collected by filtration, and the filter cake was vacuum dried to obtain C-19-3 (4.20 g).

Step 3:

C-19-3 (1.50 g, 4.04 mmol) and tert-butyl 3-(2-(2-aminoethoxy)ethoxyethoxyethyl) propionate (1.12 g, 4.04 mmol) were dissolved in DMF (20.0 mL); HOBt (1.64 g, 12.1 mmol), EDCI (2.32 g, 12.1 mmol) and DIPEA (2.09 g, 16.2 mmol) were added in sequence, and the resulting solution was heated to 60 °C and stirred for 2 h. After the reaction system was cooled to room temperature, water (10.0 mL) and ethyl acetate (20.0 mL) were added to the reaction solution. The aqueous phase was extracted twice with ethyl acetate (25.0 mL * 2). The organic phases were combined, dried with

anhydrous sodium sulfate and filtered. The filtrate was concentrated to obtain a crude product C-19-4 (2.50 g), which was used in the next step without purification.

Step 4:

C-19-4 (2.50 g, 3.96 mmol) was dissolved in dichloromethane (3.00 mL), followed by addition of TFA (4.61 g, 40.4 mmol), and the reaction system was stirred at 25 °C for 12 h. The reaction solution was directly concentrated and purified by preparative high performance liquid chromatography and then freeze-dried to obtain C-19-5 (1.20 g).

The preparation method was as follows:

Chromatographic column: Phenomenex luna C18 (150 mm*25mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 36 | 64 | 30 |
| 15.00 | 66 | 34 | 30 |

Step 5:

C-19-5 (1.10 g, 1.91 mmol) was dissolved in a mixed solvent of acetonitrile (30 mL) and water (15 mL), followed by addition of ruthenium trichloride hydrate (39.70 mg, 0.19 mmol) and sodium periodate (4.09 g, 19.14 mmol), and the reaction system reacted at 25 °C for 1 h. Extraction with water (50 mL) and ethyl acetate (80 mL) was carried out, and the organics were combined to obtain a crude product. The crude product was purified by column chromatography (MeOH/DCM=10%~20%) and concentrated to obtain C-19-6 (130 mg).

Step 6:

IM-6 (20.0 mg, 0.025 mmol) and C-19-6 (16.0 mg, 0.025 mmol) were added to DMF (1 mL) and stirred to be dissolved, followed by addition of HATU (19.0 mg, 0.050 mmol) and DIPEA (12.9 mg, 0.100 mmol), and the resulting solution reacted at room temperature for 2 h. The reaction solution was directly purified by preparative high performance liquid chromatography and then freeze-dried to obtain C-19 (20.4 mg).

The structural characterization data were as follows:
ESI-MS (m/z):1372.4 $[M+H]^+$.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 4.00 | 30 | 70 | 30 |
| 24.00 | 90 | 10 | 30 |

Example 11 N-((7S,10S,13S)-1-(((1S,9S)-5-chloro-9-ethyl-9-hydroxy-4-methyl-10, 13-dioxo-2,3,9,10,13,15-hexahydro-1H,12H-benzo[de]pyrano[3',4':6,7]indolizino[1,2-b]quin olin-1-yl)amino)-7,1,13-trimethyl-1,6,9,12,15-pentaoxo-3,18,21,24,27,30,33,36,39-nonoxy-5,8,11,14-tetraazatetra-41-yl)-3,5-bis(2-(methylsulfonyl)pyrimidin-5-yl)benzamide (C-21)

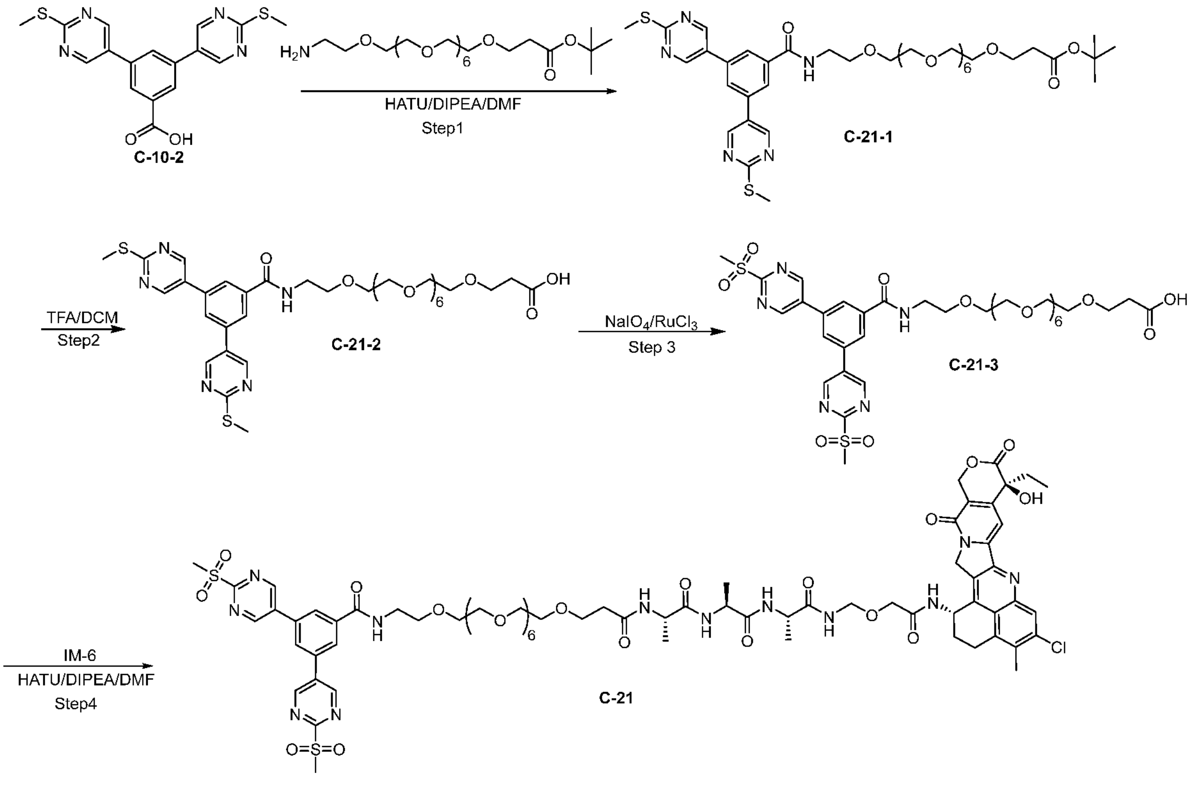


Step 1:

C-10-2 (3.00 g, 8.10 mmol) and tert-butyl 1-amino-3,6,9,12,15,18,21,24-octaoxaheptane-27-carboxylate (4.03 g, 8.10 mmol) were added to DMF (40 mL); HOBt (3.28 g, 24.3 mmol), EDCI (4.66 g, 24.3 mmol) and DIPEA (4.19 g, 32.4 mmol, 5.64 mL) were added in sequence, and the reaction system was stirred at 60°C for 2 h. Water (100 mL) and ethyl acetate (60 mL x 3) were added to the reaction solution for extraction; and the organics were combined and dried with anhydrous sodium sulfate. The resulting solution was filtered and concentrated to obtain C-21-1 (4.20 g, 4.14 mmol), which was directly used for the next step without purification.

Step 2:

C-21-1 (3.60 g, 4.24 mmol) was dissolved in dichloromethane (30 mL), followed by addition of TFA (15.3 g, 134 mmol, 10 mL), and the reaction system was stirred at 25 °C for 6 h. Water (60 mL) and ethyl acetate (40 mL x 3) were added to the reaction solution for extraction; and the organics were combined and dried with anhydrous sodium sulfate. The resulting solution was filtered and concentrated to obtain a crude product. The crude product was purified by preparative high performance liquid chromatography and then freeze-dried to obtain C-21-2 (2.93 g, 3.63 mmol).

The preparation method was as follows:

Chromatographic column: Phenomenex luna C18 (250 mm*70mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 15.00 | 60 | 40 | 30 |

Step 3:

C-21-2 (148 mg, 0.186 mmol) was added to acetonitrile (15 mL) and water (7.5 mL); sodium periodate (398.71 mg, 1.86 mmol) and ruthenium trichloride hydrate (15.47 mg, 74.56 μmol) were added to the reaction system, and the reaction system was stirred at 25°C to react for 30 min. The reaction system was extracted with water and ethyl acetate and

concentrated to obtain C-21-3 (155 mg).

Step 4:

IM-6 (27.91 mg, 34.97 μmol), C-21-3 (30 mg, 34.97 μmol), HATU (26.59 mg, 69.93 μmol) and DIPEA (22.60 mg, 174.84 μmol) were added to DMF (3 mL), and the reaction system reacted at 25 °C for 1 h. The reaction solution was purified by high performance liquid chromatography and then freeze-dried to obtain C-21 (15 mg).

The structural characterization data were as follows:
ESI-MS (m/z):1591.7 [M+H]$^+$.

The preparation method was as follows:

Chromatographic column: Waters XBridge Prep C18OBD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 90 | 10 | 30 |

Example 12 N-((7S,10S,13S)-1-(((1S,9S)-5-chloro-9-ethyl-9-hydroxy-4-methyl-10, 13-dioxo-2,3,9,10,13,15-hexahydro-1H,12H-benzo[de]pyrano[3',4':6,7]indolizino[1,2-b]quin olin-1-yl)amino)-7,1,13-trimethyl-1,6,9,12,15-pentaoxo-3,18,21,24,27,30,33,36,39-nonoxy-5,8,11,14-tetraazatetra-4-yl)-2,6-bis(2-(methylsulfonyl)pyrimidin-5-yl)isonicotinamide (C-23)

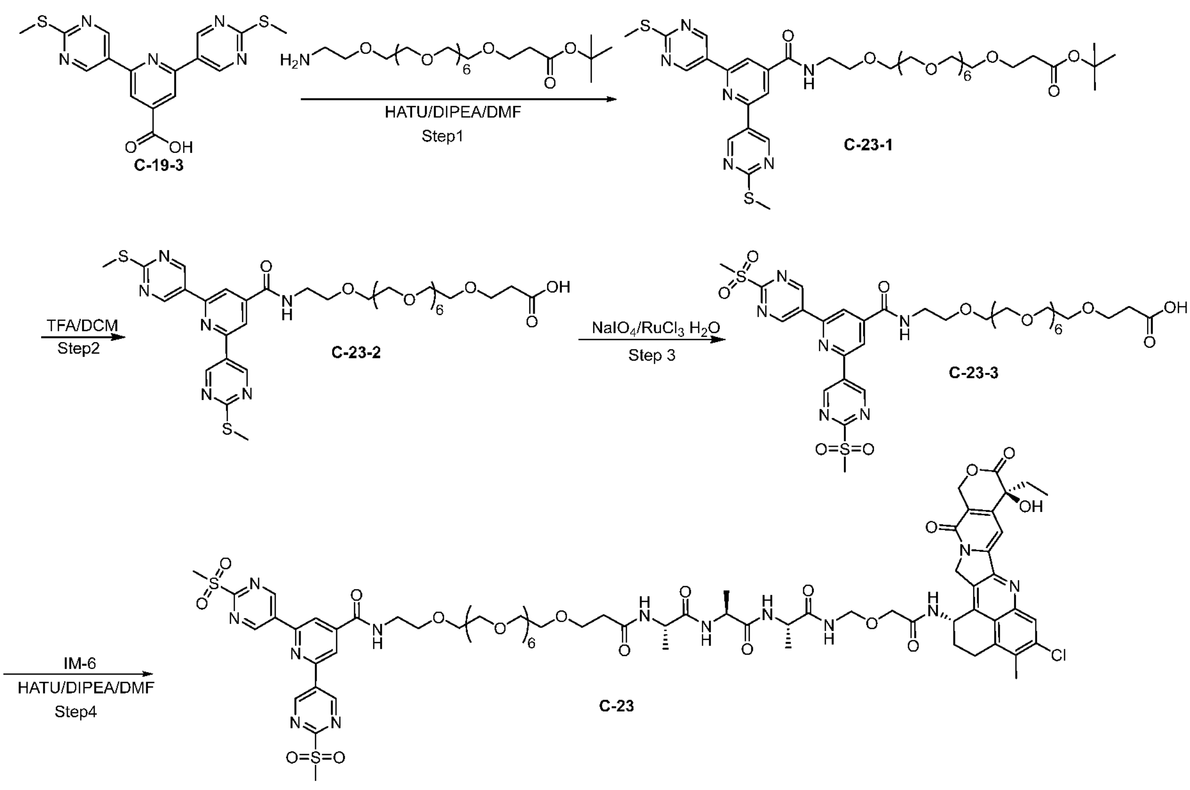


Step 1:

C-19-3 (1.50 g, 4.04 mmol) and tert-butyl 1-amino-3,6,9,12,15,18,21,24-octaoxaheptane-27-carboxylate (2.01 g, 4.04 mmol) were added to DMF (20.0 mL), followed by addition of HOBt (1.64 g, 12.1 mmol), EDCI (2.32 g, 12.1 mmol

and DIEA (2.09 g, 16.2 mmol), and the resulting solution was heated to 60 °C and stirred for 2 h. After the reaction solution was cooled to room temperature, water (10.0 mL) and ethyl acetate (20.0 mL) were added for separation; the aqueous phase was extracted twice with ethyl acetate (25.0 mL x 2); the organic phases were combined and dried with anhydrous sodium sulfate, filtered and concentrated to obtain a crude product C-23-1 (3.00 g), which was directly used for the next step.

Step 2:

C-23-1 (3.00 g, 3.53 mmol) was added to dichloromethane (10.0 mL), followed by addition of TFA (15.4 g, 134 mmol), and the reaction solution was stirred at 25 °C for 12 h. The reaction solution was directly concentrated to obtain a crude product. The crude product was purified by preparative high performance liquid chromatography and then freeze-dried to obtain C-23-2 (1.20 g).

The preparation method was as follows:

Chromatographic column: Welch Ultimate C18 (150 mm*25mm*5 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 34 | 66 | 25 |
| 10.00 | 64 | 36 | 25 |

Step 3:

C-23-2 (500 mg, 0.63 mmol) was added to acetonitrile (10 mL) and water (5 mL), followed by addition of ruthenium trichloride hydrate (13.0 mg, 0.063 mmol) and sodium periodate (1.35 g, 6.29 mmol), and the reaction system reacted at 25 °C for 1 h. Extraction with water (10 ml) and ethyl acetate (40 ml) was carried out, and the organics were concentrated to obtain a crude product. The crude product was purified by column chromatography (MeOH/DCM=10~20%) and concentrated to obtain C-23-3 (350 mg).

Step 4:

IM-6 (20.0 mg, 0.025 mmol) and C-23-3 (21.5 mg, 0.025 mmol) were dissolved in DMF (1 mL), followed by addition of HATU (19.0 mg, 0.050 mmol) and DIPEA (12.9 mg, 0.100 mmol), and the resulting solution reacted at room temperature for 2 h. The reaction solution was directly purified by preparative high performance liquid chromatography and then freeze-dried to obtain C-23(17.0 mg).

The structural characterization data were as follows:
ESI-MS (m/z):1592.6 $[M+H]^+$.

The preparation method was as follows:

Chromatographic column: Waters XBridge Prep C18OBD (5μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 4.00 | 30 | 70 | 28 |
| 24.00 | 90 | 10 | 28 |

Example 13 4-((S)-2-(4-aminobutyl)-35-(4-((6-(2-(methylsulfonyl)pyrimidin-5-yl)he xyl-5-alkynylamido) methyl)-1H-1,2,3-triazol-1-yl)-4,8-dioxo-6,12,15,18,24,27,30,33-nonoxy-3, 9-diazapentaazatriamido)benzyl((1S,9R)-5-chloro-9-ethyl-1-(2-hydroxyacetamido)-4-methyl-10, 13-dioxo-2,3,9,10,13,15-hexahydro-1H,12H-benzo[d]pyrano [3',4':6,7]indolizino[1,2-b]quinolin-9-yl)carbonate (B-03)

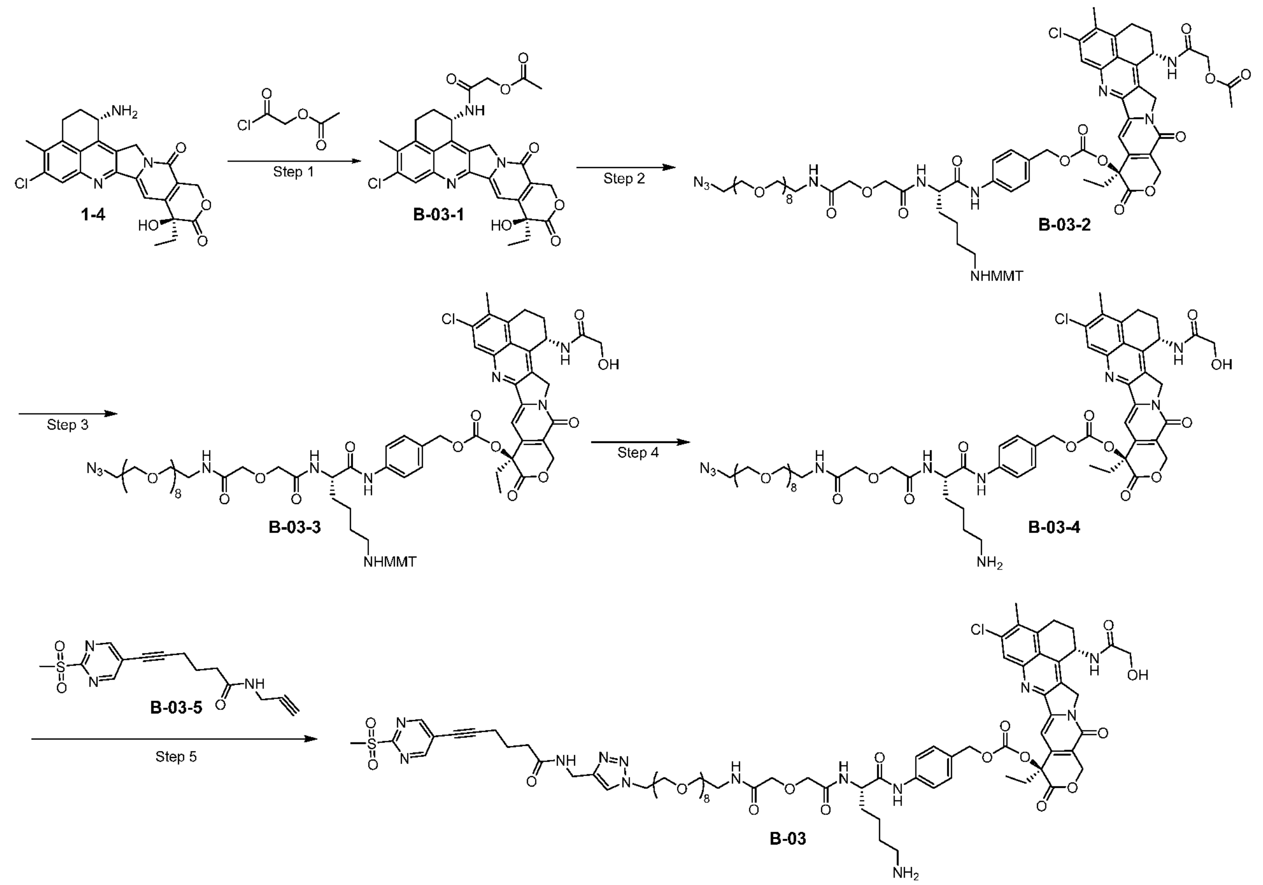


Step 1: Preparation of 2-(((1S,9S)-5-chloro-9-ethyl-9-hydroxy-4-methyl-10,13-dioxo-2,3, 9,10,13,15-hexahydro-1H,12H-benzo[d]pyrano[3',4':6,7]indolizino[1,2-b]quinolin-1-yl)amino)-2-oxoacetate (B-03-1)

(1S,9S)-1-amino-5-chloro-9-ethyl-9-hydroxy-4-methyl-1,2,3,9,12,15-hexahydro-10H,13H -benzo[d]pyrano [3',4':6,7]indolizino[1,2-b]quinolin-10,13-diketone (2 g, 3.65 mmol) was dis solved in DMF (50 mL), then DIPEA (1.18 g, 9.12 mmol, 1.59 mL) was added dropwis e, acetoxyacetyl chloride (548.12 mg, 4.01 mmol, 431.59 μL) was added dropwise under the condition of ice-bath cooling and stirring, and the reaction solution was stirred to rea ct for 1 h. The reaction solution was added to a 0.1 M aqueous solution of dilute hydro chloric acid, and the solid was precipitated and collcted by filtration. The filter cake was dissolved in dichloromethane and methanol, dried with anhydrous sodium sulfate, filt ered and concentrated to obtain a crude product. The title compound (1.7 g, 3.077 mmol) was obtained by silica gel column purification (methanol/dichloromethane = 0% ~ 5%) and re-concentration.

The structural characterization data were as follows:

ESI-MS (m/z):552.2[M+1]$^+$.

Step 2: Preparation of 2-(((1S,9S)-9-(((4-((S)-35-azido-2-(4-(4-methoxyphenyl)diphenyl methyl)amino)butyl)-4,8-dioxo-6,12,15,18,21,24,27,30,33-nonoxy-3,9-diazapentabenzotriamid o)benzyl)oxo)carbonyl)oxo-5-chloro-9-ethyl-4-methyl-10,13-dioxo-2,3,9,10,13,15-hexahydro-1 H,12H-benzo[de]pyrano[3',4':6,7]indazosin[1,2-b]quinolin-1-yl)amino)-2-oxoacetate (B-03-2)

2-(((1S,9S)-5-chloro-9-ethyl-9-hydroxy-4-methyl-10,13-dioxo-2,3,9,10,13,15-hexahydro-1 H,12H-benzo[d] pyrano[3',4':6,7]indolizino[1,2-b]quinolin-1-yl)amino)-2-oxoacetate (500 mg, 0.905mmol) and DMAP (885.33 mg, 7.25 mmol) were dissolved in dry dichloromethane (5 mL). The reaction solution was cooled to 0 °C under the protection of nitrogen, and triphosgene (268.81 mg, 0.905 mmol) in dichloromethane solution (5 mL) was added dr opwise, and reaction was carried out for 0.5 h while stirring and keeping the temperatur e. (S) -2-(32-azido-5-oxo-3,9,12,15,18,21,24,27,30-nonoxy-6-azatrinitroamino)-N-(4-(hydroxy methyl)phenyl)-6-(((4-methoxyphenyl)diphenylmethyl)amino)hexanamide (1.44 g, 1.36 mmo l) in dichloromethane solution was slowly added to the reaction solution, and reactio n was carried out for 4 h when the temperature naturally return to room temperature. W ater was added to quench the reaction, extraction with dichloromethane (100 ml x 3) w as carried out 3 times, and the organic phases were combined, washed with saturated

sal ine, dried and concentrated. Silica gel column purification (MeOH/DCM = 0% ~ 5%) wa s performed to obtain title compound (498 mg, 0.304 mmol).

The structural characterization data were as follows:
ESI-MS (m/z):1352.8[M+1]$^+$.

Step 3: Preparation of 4-((S)-35-azido-2-(4-((4-methoxyphenyl)benzhydryl)amino)butyl) -4,8-dioxo-6,12,15,18,24,27,30,33-nonoxy-3,9-diazapentaazatriamido)benzyl((1S,9S)-5-chloro-9-ethyl-1-(2-hydroxyacetamido)-4-methyl-10,13-dioxo-2,3,9,10,13,15-hexahydro-1H,12H-benzo [de]pyrano[3',4':6,7]indeno[1,2-b]quinolin-9-yl) carbonate (B-03-3)

2-(((1S,9S)-9-(((4-((S)-35-azido-2-(4-(4-methoxyphenyl)diphenylmethyl)amino)butyl)-4,8-dioxo-6,12,15,18,21,24,27,30,33-nonoxy-3,9-diazapentabenzotriamido)benzyl)oxo)carbonyl)ox o-5-chloro-9-ethyl-4-methyl-10,13-dioxo-2,3,9,10,13,15-hexahydro-1H,12H-benzo[de]pyrano[3 ',4':6,7]indazosin[1,2-b]quinolin-1-yl)amino)-2-oxoacetate (200 mg, 0.122mmol) was dissolv ed in THF (3 mL) and MeOH (3 mL). An aqueous solution (1 mL) of sodium carbonat e (25.88 mg, 0.224 mmol) was added dropwise with stirring, and stirring was continued for 1 h after completion of the dropwise addition. Dilute hydrochloric acid was dropped into the reaction solution to neutralize the reaction, and the next step was directly carrie d out after concentration under reduced pressure.

The structural characterization data were as follows:
ESI-MS (m/z):1596.7[M+1]$^+$.

Step 4: Preparation of 4-((S)-2-(4-aminobutyl)-35-azido-4,8-dioxo-6,12,15,18,21,24,27,3 0,33-nonoxy-3,9-diazapentabenzotriamido)benzyl((1S,9S)-5-chloro-9-ethyl-1-(2-hydroxyacetam ido)-4-methyl-10,13-dioxo-2,3,9,10,13,15-hexahydro-1H,12H-benzo[de]pyrano[3',4':6,7]indoliz ino[1,2-b]quinolin-9-yl)carbonate (B-03-4)

4-((S)-35-azido-2-(4-((4-methoxyphenyl)benzhydryl)amino)butyl)-4,8-dioxo-6,12,15,18, 2 4,27,30,33-nonoxy-3,9-diazapentaazatriamido)benzyl((1S,9S)-5-chloro-9-ethyl-1-(2-hydroxyace tamido)-4-methyl-10,13-dioxo-2,3,9,10,13,15-hexahydro-1H,12H-benzo[de]pyrano[3',4':6,7]ind eno[1,2-b]quinolin-9-yl)carbonate (190 mg, 119.04 μmol) was dissolved in dichloromethan e (5 mL), and then trifluoroacetic acid (0.5 mL) was added to the reaction solution to further react for 1 h. A saturated sodium bicarbonate aqueous solution was dropped into the reaction solution for neutralization and then the liquid was separated. The organic ph ase was concentrated to obtain a crude product. The crude product was purified by rever sed-phase column chromatography (acetonitrile/1% formic acid aqueous solution=0% ~50%) and freeze-dried to obtain the title compound (95 mg, 69.35 μmol).

The structural characterization data were as follows:
ESI-MS (m/z):1323.6[M+1]$^+$.

Step 5: Preparation of 4-((S)-2-(4-aminobutyl)-35-(4-((6-(2-(methylsulfonyl)pyrimidin-5-yl)hexyl-5-alkynylamido) methyl)-1H-1,2,3-triazol-1-yl)-4,8-dioxo-6,12,15,18,24,27,30,33-nono xy-3,9-diazapentaazatriamido)benzyl((1S,9R)-5-chloro-9-ethyl-1-(2-hydroxyacetamido)-4-meth yl-10,13-dioxo-2,3,9,10,13,15-hexahydro-1H,12H-benzo[d]pyrano [3',4':6,7]indolizino[1,2-b]qui nolin-9-yl)carbonate (B-03)

4-((S)-2-(4-aminobutyl)-35-azido-4,8-dioxo-6,12,15,18,21,24,27,30,33-nonoxy-3,9-diazape ntabenzotriamido)benzyl((1S,9S)-5-chloro-9-ethyl-1-(2-hydroxyacetamido)-4-methyl-10,13-dio xo-2,3,9,10,13,15-hexahydro-1H,12H-benzo[de]pyrano[3',4':6,7]indolizino[1,2-b]quinolin-9-yl) carbonate (90 mg, 0.066 mmol) and 6-(2-(methylsulfonyl)pyrimidin-5-yl)-N-(prop-2-yne-1-y l) hexan-5-yneamid (24.07 mg, 0.079 mmol) were dissolved in DMSO (2 mL) and water (0.2 mL), followed by addition of cuprous bromide (9.42 mg, 0.066 mmol) to the reacti on solution to continue stirring for 2 h. The reaction solution was directly filtered; the c oncentrated crude product was purified by preparative high performance liquid chromatogr aphy and then freeze-dried to obtain the titled compound (42.2 mg, 24.69 μmol).

The structural characterization data were as follows:
ESI-MS (m/z):1628.7[M+1]$^+$.

The method for preparing high performance liquid chromatography was as follows:

Chromatographic column: SunFire Prep C18 OBD 19 mm×150 mm×5.0μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |

(continued)

| Time [min] | Mobile phase A [%] | Mobile phase B [%] | Flow rate [mL/min] |
|---|---|---|---|
| 18.00 | 90 | 10 | 28 |

II. Antibody Preparation

Human B7-H3-4Ig-His protein was used to immunize fully human mice, and serum titers were monitored by ELISA and FACS. The best mice were selected according to the titer results, spleen cells were fused, screened and subcloned, and different monoclones were tested for binding affinity to human\monkey proteins and cells to obtain the preferred clone 20G11G6/2#. The antibody sequence was modified by PTM site removal, PI reduction, and ADCC removal from the CH, and finally the fully human antibody 2#8890 (VH, SEQ ID NO: 3; VL, SEQ ID NO: 4), CH of human IgG1 with ADCC removal modification (SEQ ID NO: 31) and human κ CL (SEQ ID NO:32), to form an intact human antibody (see Table 1)). The codon optimization and gene synthesis for the antibody was done by Nanjing GenScript, and the gene was constructed into the pTT5 plasmid. The heavy chain and light chain plasmids were transfected into CHO-S cells simultaneously, and the expressed antibody in the supernatant was purified by Protein A to obtain the corresponding antibody protein 2#8890. The amino acid sequences of the heavy and light chains of 2#8890 were as shown in SEQ ID NO:42 and SEQ ID NO:43, respectively.

hIgG1 is an anti-chicken-lysozyme antibody, its VH is fused to the mutant CH of human IgG1 (SEQ ID NO: 31), and its VL was fused to the wild-type human kappa CL (SEQ ID NO: 32). Expression and purification were performed in the same way as above to obtain the antibody hIgG1.

The B7-H3 control antibody DS7300 came from patent CN 103687945A. After codon optimization, the nucleotide sequence of the VH of the antibody was synthesized and cloned into the mutant CH of human IgG1 (SEQ ID NO: 31), and the nucleotide sequence of CL was synthesized into the pTT5 vector containing the wild-type kappa CL (SEQ ID NO: 32). Expression and purification were performed in the same way as above to obtain the antibody DS7300.

Table 1: Amino acid sequences of variable regions and CDRs of 2#8890

| Antibody | VH (SEQ ID NO:) | VL (SEQ ID NO: ) | Defined by | CDR1 of heavy chain (SEQ ID NO:) | CDR2 of heavy chain (SEQ ID NO:) | CDR3 of heavy chain (SEQ ID NO:) | CDR1 of light chain (SEQ ID NO:) | CDR2 of light chain (SEQ ID NO:) | CDR3 of light chain (SEQ ID NO:) |
|---|---|---|---|---|---|---|---|---|---|
| 2# 8890 | 3 | 4 | IMGT | 18 | 19 | 20 | 8 | 9 | 10 |
| | | | Chothia | 21 | 22 | 23 | 14 | 15 | 10 |
| | | | Kabat | 24 | 25 | 23 | 14 | 15 | 10 |
| | | | AbM | 28 | 29 | 23 | 14 | 15 | 10 |

III. Conjugation of a compound containing a cell bioactive molecule and a linker with an antibody

The antibodies 2#8890, DS7300, and hIgG1 involved in the antibody-drug conjugates prepared in the following examples are the corresponding antibodies described in the second part above.

1. Preparation of ADC 1 (DS7300-M-01, DAR 4)

1M $Na_2HPO_4$ solution was added to 38.041 ml of homemade antibody DS7300 (26.287 mg/mL) to adjust pH of the antibody to 7.4; the antibody was then diluted to 3 mg/mL with 20 mM PB; 4 mM $ZnCl_2$ (3.413 mL) and 10 mM TCEP (tris(2-carboxyethyl)phosphine, 4.096 mL, pH 7.4) were added in sequence to the diluted antibody solution under the condition of an ice bath and well mixed, and the resulting solution was placed at 4°C overnight. Then, the solution of M-01 (4.18 mL, 10 mM) dissolved in dimethyl sulfoxide was added in six times molar mass and well mixed for reaction at 4°C for 4 h, followed by addition of cysteine solution (10 mM, 6.826 mL) for reaction for 1.5 h; the reaction solution was then moved to environment of room temperature, followed by addition of EDTA solution (10 mM, 6.826 mL); 30 min later, DHAA solution (10 mM, 6.826 mL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 1 (DS7300-M-01). The DAR determined by mass spectrometry was 3.8.

2. Preparation of ADC 2 (hIgG1-M-01, DAR 4)

1M $Na_2HPO_4$ solution was added to 1.2245 ml of antibody hIgG1 (24.5 mg/mL) to adjust pH of the antibody to 7.3; the antibody was then diluted to 3 mg/mL with 20 mM PB; 4 mM $ZnCl_2$ (52.04 uL) and 10 mM TCEP (tris(2-carboxyethyl) phosphine, 124.89 uL, pH 7.3) were added in sequence to the diluted antibody solution under the condition of an ice bath and well mixed, and the resulting solution was placed at 4°C overnight. Then, the solution of 171 (127.44 uL, 10 mM) dissolved in dimethyl sulfoxide was added in six times molar mass and well mixed for reaction at 4°C for 4 h, followed by addition of cysteine solution (10 mM, 208.15 uL) for reaction for 1.5 h; the reaction solution was then moved to environment of room temperature, followed by addition of EDTA solution (10 mM, 208.15 uL); 30 min later, DHAA solution (10 mM, 208.15 uL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 2 (hIgG1-M-01). The DAR determined by mass spectrometry was 4.46.

3. Preparation of ADC 3 (2#8890-M-01, DAR 4)

1M $Na_2HPO_4$ solution was added to 9.36 ml of antibody 2#8890 (3.205 mg/mL) to adjust pH of the antibody to 7.4; the antibody was then diluted to 3 mg/mL with 20 mM PB; 4 mM $ZnCl_2$ (57.31 uL) and 10 mM TCEP (tris(2-carboxyethyl) phosphine, 124.9 uL, pH 7.4) were added in sequence to the diluted antibody solution under the condition of an ice bath and well mixed, and the resulting solution was placed at 4°C overnight. Then, the solution of M-01 (124.9 uL, 10 mM) dissolved in dimethyl sulfoxide was added in six times molar mass and well mixed for reaction at 4°C for 4 h, followed by addition of cysteine solution (10 mM, 208.2 uL) for reaction for 1.5 h; the reaction solution was then moved to environment of room temperature, followed by addition of EDTA solution (10 mM, 208.2 uL); 30 min later, DHAA solution (10 mM, 208.2 uL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 3 (2#8890-M-01). The DAR determined by mass spectrometry was 3.89.

4. Preparation of ADC 4 (hIgG1-A-05, DAR 8)

0.943ml of antibody hIgG1 (11 mg/mL) was diluted with 47 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 57 uL, pH 7.60) was then added and mixed well and the resulting solution was placed at room temperature for 1.5 h. Then, the solution of A-05 (103 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 4 (hIgG1-A-05). The DAR determined by mass spectrometry was 8.03.

5. Preparation of ADC 5 (2#8890-A-05, DAR 8)

3.052 ml of antibody 2#8890 (9.83 mg/mL) was diluted with 153 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 114.5 uL, pH 7.60) was then added and mixed well and the resulting solution was placed at room temperature for 1.5 h. Then, the solution of A-05 (219.1 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 5) (2#8890-A-05). The DAR determined by mass spectrometry was 7.90.

6. Preparation of ADC 6 (hIgG1-A-07, DAR 8)

0.518 ml of antibody hIgG1 (19.3 mg/mL) was diluted with 25.9 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 38.1 uL, pH 7.60) was then added and mixed well and the resulting solution was placed at room temperature for 1.5 h. Then, the solution of A-07 (69.2 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 6) (hIgG1-A-07). The DAR determined by mass spectrometry was 8.04.

7. Preparation of ADC 7 (2#8890-A-07, DAR 8)

1.017 ml antibody 2#8890 (9.83 mg/mL) was diluted with 50.85 uL 20 mM PB + 0.1M EDTA (pH 7.60), followed by addition of 1M $Na_2HPO_4$ solution to adjust pH of the antibody solution to 7.60; 10 mM TCEP (tris(2-carboxyethyl) phosphine, 38.2 uL, pH 7.60) solution was then added and mixed well and the resulting solution was placed at room temperature for 1.5 h. A-07 (87.6 uL, 10 mM) solution dissolved in dimethyl sulfoxide was added in 12 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody drug conjugate ADC 7 (2#8890-A-07). The DAR determined by mass spectrometry was 7.76.

8. Preparation of ADC 8 (hIgG1-A-14, DAR 8)

1.9126 ml of antibody hIgG1 (18.3 mg/mL) was diluted with 95.6 uL of 20 mM PB + 0.1 M EDTA (pH 7.60), followed by addition of 1M $Na_2HPO_4$ solution to adjust pH of the antibody solution to 7.60; 10 mM TCEP(tris(2-carboxyethyl) phosphine, 66.86 uL, pH 7.60) solution was then added and mixed well and the resulting solution was placed at room temperature for 1.5 h. A-14 (260.53 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody-drug conjugate ADC 8 (hIgG1-A-14). The DAR determined by mass spectrometry was 8.0.

9. Preparation of ADC 9 (2#8890-A-14, DAR 8)

3.6788 ml of antibody 2#8890 (10.873 mg/mL) was diluted with 183.94 uL 20mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 152 uL, pH 7.60) solution was then added and mixed well and the resulting solution was placed at room temperature for 1.5 h. A-14 (292.26 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody drug conjugate ADC 9 (2#8890-A-14). The DAR determined by mass spectrometry was 7.22.

10. Preparation of ADC 10 (hIgG1-B-01, DAR 8)

1.533ml of antibody hIgG1 (19.57 mg/mL) was dilute with 76.65 uL of 20 mM PB + 0.1M EDTA (pH 7.60), followed by addition of 1M $Na_2HPO_4$ solution to adjust pH of the antibody solution to 7.60; 10 mM TCEP (tris(2-carboxyethyl) phosphine, 114.5 uL, pH 7.60) solution was then added and mixed well and the resulting solution was placed at room temperature for 1.5 h. B-01 (212.4 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody-drug conjugate ADC 10 (hIgG1-B-01). The DAR determined by mass spectrometry was 8.03.

11. Preparation of ADC 11 (2#8890-B-01, DAR 8)

3.052 ml of antibody 2#8890 (9.83 mg/mL) was diluted with 152.6 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl)phosphine, 114.5 uL, pH 7.60) was then added and the resulting solution was placed at room temperature for 1.5 h. Then, the solution of B-01 (212.4 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 11) (2#8890-B-01). The DAR determined by mass spectrometry was 7.75.
The ADC samples after conjugation were subjected to LC-MS for molecular weight analysis.
Liquid chromatography conditions:

Liquid chromatography column: Thermo MAbPac RP 3.0*100 mm;
Mobile phase A: 0.1% FA/$H_2O$; Mobile phase B: 0.1% FA/ACN;
Flow rate :0.25 ml/min; Sample room temperature: 8 °C; Column temperature: 60 °C; Sample load: 2 μl;

| Time (min) | 2 | 20 | 22 | 25 | 26 | 30 |
|---|---|---|---|---|---|---|
| Mobile phase A (vol%) | 75 | 20 | 5 | 5 | 75 | 75 |
| Mobile phase B (vol%) | 25 | 80 | 95 | 95 | 25 | 25 |

Mass chromatography conditions:

Mass spectrometer model: AB Sciex Triple TOF 5600+;
GS1 35;GS2 35; CUR 30; TEM 350; ISVF 5500; DP 200; CE 10; Accumulation time 0.5 s; m/z 600-4000; Time bins to sum 40.

12. Preparation of ADC 12 (2#8890-C-07, DAR 4)

0.2274 ml of antibody 2#8890 (10.994 mg/mL) was diluted with 11.4 uL 20mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 9.5 uL, pH 7.60) solution was then added and mixed well and the resulting solution was placed at room temperature for 1.5 h. C-07 (14.6 uL, 10 mM) solution dissolved in dimethyl sulfoxide was slowly added in 8 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody drug conjugate ADC 12 (2#8890-C-07). The DAR determined by mass spectrometry was 4.12.

13. Preparation of ADC 13 (2#8890-C-10, DAR 4)

0.2274 ml of antibody 2#8890 (10.994 mg/mL) was diluted with 11.4 uL 20mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 9.5 uL, pH 7.60) solution was then added and mixed well and the resulting solution was placed at room temperature for 1.5 h. C-10 (14 uL, 10 mM) solution dissolved in dimethyl sulfoxide was slowly added in 8 times molar mass and well mixed and the resulting solution was set still overnight at room temperature. At the end of the reaction, the buffer was replaced with 20 mM histidine buffer solution at pH 6.0 using an NAP-5 gel column (Cytiva) to obtain an antibody-drug conjugate ADC 13 (2#8890-C-10). The DAR determined by mass spectrometry was 4.17.
The ADC samples after conjugation were subjected to LC-MS for molecular weight analysis.
Liquid chromatography conditions:

Liquid chromatography column: ACQUITY UPLC MAbPac BEH SEC;
Mobile phase A:20 mM NH4Ac;
Flow rate: 0.1 ml/min; Sample room temperature: 8 °C; Column temperature: 60 °C; Sample load:2 µl;

| Time (min) | 2 | 20 | 22 | 25 | 26 | 30 |
|---|---|---|---|---|---|---|
| Mobile phase A (vol%) | 75 | 20 | 5 | 5 | 75 | 75 |
| Mobile phase B (vol%) | 25 | 80 | 95 | 95 | 25 | 25 |

Mass chromatography conditions:

Mass spectrometer model: AB Sciex Triple TOF 5600+;
GS1 55; GS2 55; CUR 30; TEM 450; ISVF 5500; DP 75; CE 5; Accumulation time 0.5 s; m/z 900-7000; Time bins to sum 40.

14. Preparation of ADC 14 (2#8890-C-17, DAR 4)

0.292 mL of antibody 2#8890 (8.565 mg/mL) was diluted with 14.6 uL 20 mM PB + 0.1M EDTA (pH 7.60), followed by addition of 1M $Na_2HPO_4$ solution to adjust pH of the antibody solution to 7.60; 20 mM TCEP (tris(2-carboxyethyl) phosphine, 4.77 uL, pH 7.60) solution was then added and mixed well and the resulting solution was placed at room temperature for 1.5 h. C-17 (8.76 uL, 10 mM) solution dissolved in dimethyl sulfoxide was slowly added in 5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody

drug conjugate ADC 14 (2#8890-C-17). The DAR determined by mass spectrometry was 3.86.

15. Preparation of ADC 15 (2#8890-C-19, DAR 4)

0.584 mL antibody 2#8890 (8.565 mg/mL) was diluted with 29.2uL 20mM PB + 0.1M EDTA (pH 7.60), followed by addition of 1M $Na_2HPO_4$ solution to adjust pH of the antibody solution to 7.60; 20 mM TCEP (tris(2-carboxyethyl) phosphine, 9.54 uL, pH 7.60) solution was then added and mixed well and the resulting solution was placed at room temperature for 1.5 h. C-19 (17.9 uL, 10 mM) solution dissolved in dimethyl sulfoxide was slowly added in 5.5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody-drug conjugate ADC 15 (2#8890-C-19). The DAR determined by mass spectrometry was 4.05.

16. Preparation of ADC 16 (2#8890-C-21, DAR 4)

0.584 mL antibody 2#8890 (8.565 mg/mL) was diluted with 29.2uL 20mM PB + 0.1M EDTA (pH 7.60), followed by addition of 1M $Na_2HPO_4$ solution to adjust pH of the antibody solution to 7.60; 20 mM TCEP (tris(2-carboxyethyl) phosphine, 9.54 uL, pH 7.60) solution was then added and mixed well and the resulting solution was placed at room temperature for 1.5 h. C-21 (17.9 uL, 10 mM) solution dissolved in dimethyl sulfoxide was slowly added in 5.5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody-drug conjugate ADC 16 (2#8890-C-21). The DAR determined by mass spectrometry was 3.92.

17. Preparation of ADC 17( 2#8890-B-03, DAR 8)

0.867 ml of antibody 2#8890 (34.6 mg/mL) was diluted with 93.4 μL of 20 mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 112.65 μL, pH 7.60) solution was then added and mixed well and the resulting solution was placed at room temperature for 1.5 h. B-03 (227.58 uL, 10 mM) solution dissolved in dimethyl sulfoxide was added in 11 times molar mass and well mixed and the resulting solution was set still at room temperature for 2 h. At the end of the reaction, the buffer was replaced with 20 mM histidine buffer solution at pH 6.0 using NAP-5 gel column (Cytiva) to obtain an antibody drug conjugate ADC 17 (2#8890-B-03, DAR 8). The DAR determined by mass spectrometry was 7.82.

18. Preparation of ADC 18( 2#8890-B-03, DAR 8)

0.867 ml of antibody 2#8890 (34.6 mg/mL) was diluted with 58.35 μL of 20mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 112.65 μL, pH 7.60) solution was then added and mixed well and the resulting solution was placed at room temperature for 1.5 h. B-03 (211.1 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody drug conjugate ADC 18 (2#8890-B-03, DAR 8). The DAR determined by mass spectrometry was 7.23.

19. Preparation of ADC 19 (19F6-B-02)

The antibody 19F6_Hu35v1 described in the following examples is the 19F6_Hu35v1 antibody described in the international patent application WO2022253035A1, and was prepared using the method described in Example 2 of the patent application.

The sample was prepared by conjugation as follows:

0.46 ml of antibody 19F6_Hu35v1 (11.0 mg/mL) was diluted with 0.1 M disodium edetate solution (pH 7.7), followed by addition of 1M $Na_2HPO_4$ solution to adjust pH of the antibody solution to 7.7; 10 mM TCEP (tris(2-carboxyethyl) phosphine) solution was then added and mixed well and the resulting solution was placed at room temperature for 90 min. Then, 10 times molar mass of B-02 in dimethyl sulfoxide solution was added into the above solution system and well mixed and the resulting solution was set still at room temperature for 2 h. At the end of the reaction, the buffer solution was replaced with 10 mM histidine buffer solution at pH 6.0 using an NAP-5 gel column (Cytiva), and then sucrose and Tween 20 were added and mixed well to obtain an antibody-drug conjugate ADC 19 (19 F6-B-02). The DAR determined by mass spectrometry was 7.92.

20. ADC 20 (Trastuzumab-A-05,DAR 8)

2.469 mL of antibody Trastuzumab (16.2 mg/mL) was diluted with 123 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 151.57 uL, pH 7.60) solution was then added and mixed well and the resulting solution was placed at room temperature for 1.5 h. Then, 10 times molar mass of A-05 (290.09 uL, 10 mM) solution in dimethyl sulfoxide was slowly added and mixed well, and the resulting solution was set still at room temperature for 2 h. At the end of the reaction, the buffer solution was replaced with 20 mM histidine buffer solution (pH 6.0) using an NAP-5 gel column (Cytiva) to obtain an antibody drug conjugate (i.e. Trastuzumab-A-05). The DAR determined by mass spectrometry was 8.04.

21. ADC 21 (Trastuzumab-A-14, DAR 8)

2.564 mL antibody Trastuzumab (15.6 mg/mL) was diluted with 128.2 ul 20mM Pb + 0.1 M EDTA (pH 7.60), followed by addition of 1M $Na_2HPO_4$ to adjust pH of the antibody solution to 7.60; 10 mM TCEP (tris(2-carboxyethyl) phosphine, 151.57 uL, pH 7.60) was then added and mixed well and the resulting solution was placed at room temperature for 1.5 h. Then, 10 times molar mass of A-14 (290.1 uL, 10 mM) solution in dimethyl sulfoxide was slowly added and mixed well, and the resulting solution was set still at room temperature for 2 h. At the end of the reaction, the buffer solution was replaced with 20 mM histidine buffer solution at pH 6.0 using an NAP-5 gel column (Cytiva) to obtain an antibody drug conjugate (Trastuzumab-A-14). The DAR determined by mass spectrometry was 8.02.

22. ADC 22 (Trastuzumab-A-24,DAR 8)

0.617 mL antibody Trastuzumab (16.2 mg/mL) was diluted with 30.86 ul 20mM PB+0. 1M EDTA (pH 7.60), followed by addition of 1M $Na_2HPO_4$ to adjust pH of the antibody solution to 7.60; 10 mM TCEP (tris(2-carboxyethyl) phosphine, 37.89 uL, pH 7.60) was then added and mixed well and the resulting solution was placed at room temperature for 1.5 h. 14 times molar mass of A-24 (98.42 uL, 10 mM) solution in dimethyl sulfoxide was added and mixed well, and the resulting solution was set still at room temperature for 2 h. At the end of the reaction, the buffer solution was replaced with 20 mM histidine buffer solution at pH 6.0 using an NAP-5 gel column (Cytiva) to obtain an antibody drug conjugate (i.e. Trastuzumab-A-24). The DAR determined by mass spectrometry was 7.37.

23. ADC23(Trastuzumab-A-32,DAR 8)

0.423 mL antibody Trastuzumab (4.73 mg/mL) was diluted with 21.17 ul 20mM PB+0. 1M EDTA (pH 7.60), followed by addition of 1M $Na_2HPO_4$ Solution to adjust pH of the antibody solution to 7.60; 10 mM TCEP (tris(2-carboxyethyl) phosphine, 7.58 uL, pH 7.60) solution was then added and mixed well and the resulting solution was placed at room temperature for 1.5 h. 10 times molar mass of A-32 (13.92 uL, 10 mM) solution in dimethyl sulfoxide was added and mixed well and the resulting solution was set still at room temperature for 2 h. At the end of the reaction, the buffer solution was replaced with 20 mM histidine buffer solution at pH 6.0 using an NAP-5 gel column (Cytiva) to obtain an antibody drug conjugate (i.e. Trastuzumab-A-32). The DAR determined by mass spectrometry was 6.77.

24. ADC24 (Trastuzumab-C-21,DAR 4)

0.617 mL antibody Trastuzumab (16.2 mg/mL) was diluted with 30.86 ul 20mM PB+0. 1M EDTA (pH 7.60), followed by addition of 1M $Na_2HPO_4$ to adjust pH of the antibody solution to 7.60; 19.83 mM TCEP (tris(2-carboxyethyl) phosphine, 19.1 uL, pH 7.60) and the resulting solution was placed at room temperature 1.5 h. 5.5 times molar mass of C-21 (38.28 uL, 10 mM) solution in dimethyl sulfoxide was added in sequence and mixed well, and the resulting solution was set still at room temperature for 18 h. At the end of the reaction, the buffer solution was replaced with 20 mM histidine buffer solution at pH 6.0 using NAP-5 gel column (Cytiva) to obtain an antibody drug conjugate (Trastuzumab-C-21). The DAR determined by mass spectrometry was 4.41, in which DAR4 accounted for 60.06%.

IV. Activity Detection of ADCs

1. Detection of dynamic affinity of anti-human B7-H3 antibody conjugate

The dynamic affinity of anti-human B7-H3 antibody conjugate, human B7-H3-4Ig-his, human B7-H3-2Ig-his, rat B7-H3-his and monkey B7-H3-his proteins was determined by ForteBio(Pall life sciences). The method was described specifically as follows: the conjugate to be tested was diluted with PBST (0.02% Tween-20) to 5 μg/ml, and the human B7-H3-4Ig-his, human B7-H3-2Ig-his, rat B7-H3-his and monkey B7-H3-his proteins were gradient diluted to 200 nM, 100 nM,

50 nm, 25 nM, 12.50 nM, 6.25 nM, 3.125 nM, and 0 nM; then, the conjugate to be tested was captured in PBST (0.02% Tween-20) solution using Protein A Sensor (Pall life) separately for 60 s, and then bound to the four proteins for 60 s respectively, and then dissociated for 180 s. The obtained results were opened in the software Data Analysis 11.0, and the 1:1 mode was selected for global fitting to analyze the results and obtain the affinity constant. The results are shown in Table 2. The results showed that the conjugates prepared from the drug-linker compounds of the present invention bound to monkey B7-H3, but not to rat B7-H3.

Table 2: Result of detection of dynamic affinity of anti-human B7-H3 antibody conjugates

| ADC | Human B7-H3-4Ig-his KD (M) | Human B7-H3-2Ig-his KD (M) | Rat B7-H3-his KD (M) | Monkey B7-H3-his KD (M) |
|---|---|---|---|---|
| ADC 5 | 5.88E-10 | 2.34E-08 | Not bond | 9.89E-10 |
| ADC 9 | 4.12E-10 | 2.30E-08 | Not bond | 7.10E-10 |
| ADC 17 | 1.04E-09 | 4.20E-08 | Not bond | 2.57E-09 |

2. Detection of cell affinity of anti-human-B7-H3 antibody conjugates

The affinity of the anti-human-B7-H3 fully human antibody conjugates to human colon cancer cell HT29 (National Collection of Authenticated Cell Cultures), human gastric cancer cell NCI-N87 (ATCC), human mammary squamous cancer cell HCC1806 (ATCC) and human non-small cell lung cancer cell HCC827 (ATCC) was detected by a flow cytometer (Beckman, model: Cytoflex); the affinity of the anti-human-B7-H3 fully human antibody conjugates to CHOS-human B7-H3-4Ig and CHOS-human B7-H3-2Ig was detected. Species crossover of the anti-human-B7-H3 fully human antibody conjugates was detected: affinity to CHOS-rat B7-H3 and CHOS-monkey B7-H3. The adherent cells were digested with Trypsin-EDTA (0.25%) (Thermo) solution and then counted and adjusted to the cell density of $4.0\times10^6$ cells/ml, washed twice with 1% BSA, and re-suspended in 1% BSA solution; the cell suspension was added to 96-well V-bottomed plates with 50 μl/well ($2\times10^5$ cells/well). The antibody conjugates were diluted with 1% BSA, starting at a final concentration of 10 μg/ml, 3 times gradient, with a total of 11 concentration points. The hIgG1 antibody conjugates were used as controls (final concentration 10 ug/ml); 50 μl of the diluted antibody was added to the V-bottomed plates containing cells, and incubated at 4°C for 60 min. The cells were washed twice with 1% BSA, and then 50 μl of diluted secondary antibody was added to each well, well mixed, and incubated at 4°C for 30 min. The cells were washed twice with 1% BSA and then resuspended in 200 μl of 1% BSA and detected by FACS. Data processing: The Median PE values were exported, and then imported into the software Graph Prism 6 to calculate $EC_{50}$.

According to the results of test for affinity of the anti-human-B7-H3 antibody conjugates to tumor cells HT29, NCI-N87, HCC1806 and HCC827, it can be seen that the $EC_{50}$ of ADC17 to tumor cells HT29 and HCC827 is 3.213 ng/ml and 8.054 ng/ml, respectively; the affinity of other antibody conjugates is shown in Table 3; the affinity of the anti-human-B7-H3 fully human antibody conjugates to CHOS-human B7-H3-4Ig and CHOS-human B7-H3-2Ig cells is shown in Table 4; and the affinity of the anti-human B7-H3 antibody conjugates to CHOS-rat B7-H3 and CHOS-monkey B7-H3 cells is shown in Table 5. The results show that the conjugates prepared from the drug-linker compounds of the present invention are capable of binding to monkey B7-H3-overexpressed cells, but not to rat B7-H3-overexpressed cells.

Table 3: Affinity of ADCs to tumor cells

| ADC | $EC_{50}$ (ng/ml) | | | |
|---|---|---|---|---|
| | HT29 | NCI-N87 | HCC1806 | HCC827 |
| ADC 5 | 2.335 | 2.329 | 3.129 | 7.579 |
| ADC 9 | 5.656 | 5.953 | 7.264 | 13.05 |

Table 4: Affinity of ADCs to CHOS-human B7-H3-4Ig and CHOS-human B7-H3-2Ig cells

| ADC | $EC_{50}$ (ng/ml) | |
|---|---|---|
| | CHOS-B7-H3-4Ig | CHOS-B7-H3-2Ig |
| ADC 5 | 41.48 | 26.89 |
| ADC 9 | 45.96 | 41.34 |

Table 5: Affinity of ADCs to CHOS-rat B7-H3 and CHOS-monkey B7-H3 cells

| ADC | $EC_{50}$ (ng/ml) | |
|---|---|---|
| | CHOS-rat B7-H3 | CHOS-monkey B7-H3 |
| ADC 5 | Not bond | 31.85 |
| ADC 9 | Not bond | 40.94 |

3. Detection of endocytosis of anti-human B7-H3 antibody conjugate

The endocytosis of the anti-human B7-H3 antibody conjugate to human gastric cancer cell NCI-N87 (ATCC), human mammary squamous carcinoma cell HCC1806 (ATCC) and human non-small cell lung cancer cell HCC827(ATCC) was assayed on a flow cytometer (Thermo, model: Attune NxT). Adherent cells were digested with Trypsin-EDTA (0.25%) (Thermo) solution and counted. The cell density was adjusted to $1\times10^5$ cells/ml with a complete medium, and the cell suspension was added to 96-well plates with 100 μl/well (the number of cells was $1\times10^4$ per well). The 96-well plates were placed in a 37 °C, $CO_2$ constant-temperature incubator for incubation for 24 h. The 96-well plates were taken out, the medium was sucked away, and 50 μl of fresh complete medium was added to each well. The conjugate to be tested were diluted with a complete medium to obtain a total of 6 concentration points; 300 μg/ml pHrodo reagent (Thermo, Cat#Z25612) was diluted with complete medium to 12 μg/ml (the final concentration of pHrodo was 3 μg/ml). The gradient diluted antibody to be tested was well mixed with the diluted pHrodo reagent at a ratio of 1: 1 (30 μl: 30 μl) and incubated in dark at room temperature for 30 min. 50 μl of the mixture of the antibody to be tested and the pHrodo reagent was added to 96-well plates and incubated at 37 °C, 5% $CO_2$ for 24 h. The 96-well plates were then taken out, the medium was sucked away; the plates were washed once with aseptic PBS; the Trypsin-EDTA (0.25%) was added with 100 μl/well to digest the cells, and 100 μl of complete medium was then added for neutralization. The cells in the wells were blown and dispersed and then assayed by FACS. Data processing: Median YL-1H values were exported and then imported into the software GraphPad Prism 6 to calculate $EC_{50}$. As shown in Table 6, the antibody-drug conjugates (such as ADC 5 and ADC 9) prepared from the drug-linker compounds of the present invention have good endocytosis.

Table 6: Endocytosis of ADCs

| ADC | $EC_{50}$ (ng/ml) | | |
|---|---|---|---|
| | NCI-N87 | HCC1806 | HCC827 |
| ADC 5 | 7.984 | 10.91 | 9.835 |
| ADC 9 | 8.649 | 18.94 | 10.65 |

4. Detection of in vitro cytotoxicity of anti-human-B7-H3 antibody conjugates

The adherent cells A375, Calu6-B7-H3 and U87MG-B7-H3 were digested with Trypsin-EDTA (0.25%) (Thermo) solution and counted. The cell density was adjusted to $1\times10^4$, $5\times10^4$ and $1\times10^4$ cells/ml respectively with a complete medium, and the cell suspensions were respectively added to 96-well plates with 100 μl/well (the number of cells was 1000, 5000 and 1000 per well). The 96-well plates were placed in a 37°C, $CO_2$ constant-temperature incubator for incubation for 24 h. The ADCs to be tested were diluted with complete medium, starting at a final concentration of 3333.3 nM, 4 times gradient, with a total of 12 concentration points; 100 μl of each diluted ADC was added to a 96-well plate and incubated at 37 °C, 5% $CO_2$ for 4 to 7 days. The 96-well plates were taken out, 20 μl of CCK8 reagent was added to each well, followed by incubation at 37°C for 2-3 h. The $OD_{450nm}$ signal value was given by a microplate reader, and then imported into the software Graph Pad Prism 6 to calculate $IC_{50}$. The results are shown in Table 7.

Table 7 In vitro cytotoxicity of ADCs

| ADC | $IC_{50}$ (nM) | | |
|---|---|---|---|
| | A375 | Calu6-B7-H3 | U87MG-B7-H3 |
| ADC 5 | 0.4753 | 0.2529 | 0.08650 |
| ADC 9 | 2.638 | 0.3282 | 0.09748 |
| ADC 2 | 28.52 | 148.7 | 30.15 |

(continued)

| ADC | $IC_{50}$ (nM) | | |
|---|---|---|---|
| | A375 | Calu6-B7-H3 | U87MG-B7-H3 |
| ADC 8 | 11.25 | 52.51 | 14.48 |

The results show that the antibody drug conjugates prepared from the drug-linker compounds of the present invention have a clear cytotoxicity to tumor cells.

5. In vivo efficacy test of different antibody-drug conjugates of 2#8890 in HCC1806 model

Human breast squamous carcinoma cell line HCC1806 (ATCC) was cultured in RPMI 1640 medium supplemented with 10% fetal calf serum at 37 °C in a 5% $CO_2$ incubator. When the cells were in the exponential growth phase, the culture medium was taken for mycoplasma detection, and the cells were collected and counted. $2\times10^6$ HCC1806 cells suspended in 0.1 ml PBS were subcutaneously inoculated at the right shoulder of each mouse. When the average tumor volume grew to 100-200 $mm^3$, mice with too small or too large tumor volumes were eliminated, and the remaining mice were randomly divided into 6 groups according to tumor volume and body weight, with 6 mice in each group. The drugs were administered by tail vein injection once (DAR4 drug-treated group: 10 mg/kg, DAR8 drug-treated group: 5 mg/kg). After administration, the tumor volume and body weight were measured twice a week, and results are shown in Table 8.

Table 8 Efficacy of different conjugates of 2#8890 on HCC1806 tumor-bearing mouse model

| Drug | Dose (mg/kg) | $V_{P0}$ ($mm^3$, mean$\pm$SEM) | $V_{P52}$ ($mm^3$, mean$\pm$SEM) | TGI (%) | *P*-value |
|---|---|---|---|---|---|
| Normal saline | / | 150.56$\pm$12.13 | 3538.70$\pm$1032. 32 | / | / |
| ADC 11 | 5 | 151.62$\pm$11.10 | 819.27$\pm$346.44 | 80.29* | 0.032 |
| ADC 5 | 5 | 151.51$\pm$13.21 | 6.10$\pm$3.98 | 195.97** | 0.007 |
| Note: *P<0.05, **P<0.01, ***P<0.001 indicate significant differences compared with the vehicle group. | | | | | |

The results show that the mice in the drug-treated groups were well tolerated, with stable body weight. 95 days after a single dose, the tumors of mice in the ADC 5 group completely disappeared. It shows that the antibody-drug conjugates prepared from the drug-linker compounds of the present invention have obvious tumor inhibition effect.

6. Efficacy testing of different doses of antibody-drug conjugates in HCC1806 model

Human breast squamous carcinoma cell line HCC1806 (ATCC) was cultured in RPMI 1640 medium supplemented with 10% fetal calf serum at 37 °C in a 5% $CO_2$ incubator. When the cells were in the exponential growth phase, the culture medium was taken for mycoplasma detection, and the cells were collected and counted. $2\times10^6$ HCC1806 cells suspended in 0.1 ml PBS were subcutaneously inoculated at the right shoulder of each mouse. When the average tumor volume grew to 100-200 $mm^3$, mice with too small or too large tumor volumes were eliminated, and the remaining mice were randomly divided into 12 groups according to tumor volume and body weight, with 6 mice in each group. The drugs were administered by tail vein injection once. After administration, the tumor volume and body weight were measured twice a week, and results are shown in Table 9.

Table 9 Efficacy of different doses of antibody-drug conjugates on HCC1806 tumor-bearing mouse model

| Drug | Dose (mg/kg) | VP0 ($mm^3$, mean$\pm$SEM) | VP27 ($mm^3$, mean$\pm$SEM) | TGI (%) | *P*-value |
|---|---|---|---|---|---|
| Normal saline | / | 172.26$\pm$16.70 | 2984.81$\pm$647.24 | / | / |
| ADC 5 | 5 | 172.53$\pm$13.65 | 44.57$\pm$7.16 | 174.17* * | 0.001 |
| ADC 9 | 5 | 172.94$\pm$16.05 | 75.12$\pm$27.55 | 156.56* * | 0.001 |
| Note: *P<0.05, **P<0.01, ***P<0.001 indicate significant differences compared with the vehicle group. | | | | | |

The results show that both ADC 5 and ADC 9 groups had good efficacy, and the mice had stable body weight and

show good tolerance, indicating that the antibody-drug conjugates prepared from the drug-linker compounds of the present invention have obvious tumor inhibition effect and good safety.

7. Efficacy detection of different antibody-drug conjugates in the NCI-N87 model

Human gastric cancer cells NCI-N87 (ATCC) were cultured in RPMI 1640 medium supplemented with 10% fetal calf serum at 37 °C in a 5% $CO_2$ incubator. When the cells were in the exponential growth phase, the culture medium was taken for mycoplasma detection, and the cells were collected and counted. $5\times10^6$ NCI-N87 cells suspended in 0.1 ml PBS matrix gel were subcutaneously inoculated at the right shoulder of each mouse. When the average tumor volume grew to 100-200 $mm^3$, mice with too small or too large tumor volumes were eliminated, and the remaining mice were randomly divided into 8 groups according to tumor volume and body weight, with 6 mice in each group. The drugs were administered by tail vein injection twice, by once a week (QW). After administration, the tumor volume and body weight were measured twice a week, and results are shown in Table 10.

Table 10 Efficacy of different antibody-drug conjugates on NCI-N87 tumor-bearing mouse model

| Drug | Dose (mg/kg) | VP0 (mm3, mean±SEM) | VP42 (mm3, mean±SEM) | TGI (%) | P-value |
|---|---|---|---|---|---|
| Normal saline | / | 119.85±11.01 | 1351.59±211.29 | / | / |
| ADC 3 | 10 | 120.27±9.29 | 875.71±114.43 | 38.67 | 0.0758 |
| ADC 11 | 5 | 119.98±9.38 | 688.71±105.48 | 53.83* | 0.0186 |
| ADC 5 | 5 | 118.93±10.37 | 532.12±102.51 | 66.45** | 0.0058 |
| ADC 9 | 5 | 118.90±7.18 | 474.09±121.07 | 71.16** | 0.0048 |
| Note: *P<0.05, **P<0.01, ***P<0.001 indicate significant differences compared with the vehicle group. | | | | | |

The results show that the mice in the drug-treated groups were well tolerated, with stable body weight. It shows that the antibody-drug conjugates prepared from the drug-linker compounds of the present invention have obvious tumor inhibition effect and good safety.

8. Detection of binding affinity of anti-human-B7-H3 antibody conjugates to Fc receptors

ForteBio (Pall life sciences) was used to detect the dynamic affinity of 2#8890 ADCs to human Fc receptor proteins CD16a, CD32a, CD32b, C1q, and FcRn. The specific detection method was as follows: The biotinylated proteins to be tested were captured separately in PBST solution using SA Sensor (Pall Life Sciences). The antibodies and conjugates to be tested were diluted with PBST to a starting concentration of 5000 nM and diluted two times to 7 concentration points. The binding and dissociation were performed, and the results were opened in Data Analysis 11.0 software. The global fitting was performed in 1:1 mode to analyze the results and obtain the binding rate, dissociation rate and affinity constant. The results are shown in Table 11.

Table 11 Binding affinity of antibodies and conjugates to Fc receptors

| ADC | Fc receptor protein | KD(M) |
|---|---|---|
| ADC 11 | FcRn | 1.07E-08 |
| ADC 5 | CD32a | Not bond |
| ADC 5 | FcRn | 1.45E-08 |
| ADC 9 | CD32a | Not bond |
| ADC 9 | FcRn | 1.5E-08 |

The results show that the conjugates prepared from the drug-linker compounds of the present invention did not bind to Fc receptors CD16a, CD32a, CD32b, and C1q proteins and could reduce nonspecific killing mediated by Fc receptors and improve drug safety. Moreover, the conjugates retained binding affinity to FcRn proteins and did not affect the half-life of the drug.

9. Pharmacokinetic study of total antibody (Tab), ADCs, and Payload in serum of cynomolgus monkeys after multiple intravenous injections of anti-human-B7-H3 ADCs

ELISA and LC-MS/MS methods were used to quantitatively detect ADCs (ADC 5, ADC 9), total antibody (TAb), and Payload in cynomolgus monkey serum. The standard curve quantitative range of ADC (ADC 5, ADC 9) and total antibody (TAb) methods was 11.72-3000.00 ng/mL, and the linear range of Payload method was 0.1-40 ng/mL. B7-H3 protein as the capture protein were used in both methods for ADCs (ADC5, ADC9) and total antibody (TAb) and added to 96-well ELISA plates. Then, total antibody (Tab) used Goat Anti-Human IgG-HRP as the test antibody; ADCs (ADC 5, ADC 9) used Anti-Toxin mouse antibody and Goat Anti-Mouse IgG as the second antibody and test antibody respectively. Color was developed by the action of enzyme and substrate, and reads were given by the SpectraMax i3x (Molecular Devices) microplate reader. The 4-P parameter method was used to fit the standard curve and calculate the concentration of each sample. The concentration of ADCs (ADC 5, ADC 9) and total antibody (TAb) was positively correlated with the shade of color. LC-MS/MS was performed by Shimadzu LC 30-AD liquid phase system coupled with SCIEX QTRAP5500+ (SCIEX) mass spectrometer, using (+)ESI ionization mode and selecting multiple reaction monitoring mode (MRM) analysis. The chromatographic column was XbridgeC1850*4.6mm, 5 μm, the ion pair of the test compound 1-10 was 510.2/435.2, and acetonitrile was used for protein precipitation in sample pretreatment. Results: after multiple intravenous injections of ADC 5 and ADC 9 in cynomolgus monkeys, the test results showed that the ADC molecules prepared from the drug-linker compounds of the present invention (such as ADC 5 and ADC 9) exhibited good pharmacokinetic properties, were relatively stable in the systemic circulation, and released less free toxins.

Table 12 Pharmacokinetic parameters of TAb in serum after the last intravenous injection of ADC at 50 mg/kg

| Sample for test | $T_{1/2}$ H | $T_{max}$ h | $C_{max}$ **μg/mL** | $AUC_{0-t}$ h*μg/mL | $AUC_{0-\infty}$ h*μg/mL | Vz mL/kg | CL mL/h/kg | $MRT_{last}$ h |
|---|---|---|---|---|---|---|---|---|
| ADC 5 | 126.16 | 0.08 | 727.25 | 46406.21 | 79399.78 | 117.81 | 0.68 | 64.31 |
| ADC 9 | 161.44 | 0.50 | 1179.45 | 97998.68 | 188256.01 | 61.87 | 0.27 | 68.33 |

Table 13 Pharmacokinetic parameters of ADCs in serum after the last intravenous injection of ADC at 50 mg/kg

| Sample for test | $T_{1/2}$ h | $T_{max}$ h | $C_{max}$ **μg/mL** | $AUC_{0-t}$ h*μg/mL | $AUC_{0-\infty}$ h*μg/mL | Vz mL/kg | CL mL/h/kg | $MRT_{last}$ h |
|---|---|---|---|---|---|---|---|---|
| ADC 5 | 113.80 | 0.29 | 731.80 | 50982.95 | 81318.16 | 102.58 | 0.66 | 62.92 |
| ADC 9 | 145.63 | 0.29 | 1242.45 | 95874.14 | 174007.67 | 60.21 | 0.29 | 67.38 |

Table 14 Pharmacokinetic parameters of Payload in serum after the last intravenous injection of ADCs at 50 mg/kg

| Sample for test | $T_{1/2}$ h | $T_{max}$ h | $C_{max}$ ng/mL | $AUC_{0-t}$ h*ng/mL | $AUC_{0-\infty}$ h*ng/mL | $CL_{F\ obs}$ mL/h/kg | $MRT_{last}$ h |
|---|---|---|---|---|---|---|---|
| ADC 5 | 110 | 0.083 | 3.03 | 99 | 157 | 347008 | 57 |
| ADC 9 | 90 | 0.083 | 8.20 | 96 | 119 | 422692 | 36 |

10. Repeated dose toxicity test

The repeated dose toxicity test included repeated toxicity test of ADC 5 and ADC 9 administered intravenously 4 times to cynomolgus monkeys.

This test had total of 3 groups, 1/group/sex, and the animals were subjected to intravenous injection of 30 mg/kg of ADC 5, ADC 9 and saline (volume: 10 mL/kg), once a week, for a total of 2 times; then the dose was increased to 50 mg/kg of ADC 5, ADC 9 and saline, once a week, for a total of 2 times. During the test, no death or dying related to the test product was observed. During the administration period, animals in the ADC 5-treated group showed loss of appetite, hair loss, skin pigmentation and weight loss. WBC, NEUT, LYM, and MONO were reduced in female monkeys. RBC, HGB, and HCT were reduced in male monkeys, and FGB was increased, with a recovery trend during the recovery period. During the administration period, animals in the ADC 9-treated group showed loss of appetite. In female monkeys, WBC, NEUT, LYM and MONO decreased and FBG increased. In male monkeys, FBG increased and RBC, HGB and HCT decreased. During the recovery period, local pigmentation and slight hair loss were also observed, and other changes showed a recovery trend. Under the conditions of this test, cynomolgus monkeys were intravenously injected with 30 mg/kg of ADC 5 and ADC 9 once a week for two consecutive weeks; then the dose was increased to 50 mg/kg of ADC 5 and ADC 9 once a week for 2

consecutive weeks. All animals were able to tolerate the drug. The highest no serious toxicity dose (HNSTD) was 50 mg/kg.

11. Inhibition effect of ADCs on tumor growth in a mouse subcutaneous transplanted tumor model

The formulations containing the ADCs of the present invention were administered to the subcutaneously transplanted human breast squamous cell carcinoma cell HCC1806 mouse CDX model by tail vein injection, and the changes in tumor volume and body weight of animals were measured twice a week to calculate the tumor inhibition efficacy of the ADCs of the present invention on tumor-bearing mice.

Experimental animals: Balb/cNude mice (Chengdu GemPharmatech)
Cell line: human breast squamous cell carcinoma cell HCC1806 (ATCC)

Experimental method:
HCC1806 cells were incubated in RPMI 1640 medium supplemented with 10% fetal bovine serum at 37 °C, 5% $CO_2$. HCC1806 cells in the exponential growth phase were collected, re-suspended in PBS to a suitable concentration, and inoculated subcutaneously in female Balb/c-nude mice to establish a breast squamous cell carcinoma model. When the average tumor volume was about 200 $mm^3$, the mice were randomly divided into groups according to the tumor size and given drugs respectively. The groups and their doses were as follows: vehicle control group (i.e., negative control, Vehicle group): 0.9% NaCl injection; ADC 5: 3 mg/kg; ADC 9: 3 mg/kg; ADC 11: 3.16 mg/kg; ADC 17: 3.32 mg/kg. Each group was injected by tail vein (i.v.), and the drug was given on Day 0, for a total of 1 dose. After administration, the body weight of mice was measured twice a week and the long and short diameters of the tumors were measured with vernier calipers, and the tumor volume was calculated by: $V=0.5\ a\times b^2$, where a and b represent the long diameter and short diameter of the tumor, respectively, and the death of animals was observed and recorded every day.
The tumor growth inhibition rate TGI (%) was calculated by:

$$V_{T\ end}>V_{T0}, TGI\ (\%) = [1-(V_{T\ end}-V_{T0})/(V_{C\ end}-V_{C0})]*100\% \text{ or } V_{T\ end}\leq V_{T0}, TGI\ (\%) = [1-(V_{T\ end}-V_{T0})/\ V_{T0}]*100\%$$

where $V_{T\ end}$: mean tumor volume of the drug-treated group at the end of experiment
$V_{T0}$: mean tumor volume of the drug-treated group at the beginning of drug administration
$V_{C\ end}$: mean tumor volume of the negative control group at the end of experiment
$V_{C0}$: mean tumor volume of the negative control group at the beginning of drug administration

The relative tumor proliferation rate T/C (%) was calculated by:

$$T/C = (V_{T\ end}/\ V_{T0})/(V_{C\ end}/\ V_{C0}).$$

The ADCs of the present invention have a significant tumor growth inhibition effect on the HCC1806 transplanted tumor model. On day 14, compared with the Vehicle group, the tumor growth inhibition rates (TGI) of ADC 5, ADC 9, ADC 11 and ADC 17 of the present invention were 96.68%, 98.50%, 82.61% and 86.69% respectively, which was significantly different from that of the control group. During the treatment period, there were no animal deaths or significant weight loss in each drug-treated group, and no obvious drug toxic reactions were observed. The mice tolerated the ADCs of the present invention well. Results were shown in Table 15.

Table 15 Efficacy of different antibody-drug conjugates on HCC1806 tumor-bearing mouse model

| Group | Dose (mg/kg) | Day 14 | | | |
|---|---|---|---|---|---|
| | | Tumor volume ($mm^3$) ($\bar{x}\pm$SEM) | TGI (%) | T/C (%) | *P*-value (vs.Vehicle) |
| Vehicle | - | 1274.42±80.85 | - | - | - |
| ADC 5 | 3 | 218.24±57.69 | 96.68 | 16.99 | 0.0000 |
| ADC 9 | 3 | 201.74±45.01 | 98.50 | 15.42 | 0.0000 |
| ADC 11 | 3.16 | 370.35±41.88 | 82.61 | 29.12 | 0.0000 |

(continued)

| Group | Dose (mg/kg) | Day 14 | | | |
|---|---|---|---|---|---|
| | | Tumor volume ($mm^3$) ($\bar{x}\pm$SEM) | TGI (%) | T/C (%) | *P*-value (vs.Vehicle) |
| ADC 17 | 3.32 | 329.35±72.16 | 86.69 | 25.40 | 0.0000 |
| Note: TGI is tumor growth inhibition rate, T/C is relative tumor proliferation rate. | | | | | |

12. Inhibitory effect of ADCs on in vitro cell activity

(1) Cell plating: First, tumor cells NCI-H1975 and HT-29 were cultured in a corresponding culture medium, the cells were digested with trypsin and after centrifugation, the cells were re-suspended and counted. The cell suspension was adjusted to an appropriate concentration for plating. The source of tumor cells is shown in Table 16.

Table 16: Source of tumor cells

| Cell name | Tumor type | Source |
|---|---|---|
| NCI-H1975 | Human lung adenocarcinoma cell | Nanjing Co-bioer Bioscience |
| HT-29 | Human colon cancer cell | National Collection of Authenticated Cell Cultures |

After the cells adhered to the wall, the culture medium in the cells was removed, and the diluted ADC 19 was added to the above plate wells and incubated for 96 h.

In vitro cell activity detection: After the incubation, 50 μL of Cell Counting-Lite™ 2.0 reagent (Vazyme) was added to each well, and shaken and mixed in the dark. After reaction for 10 min, the detection was carried out, and reads were given by a microplate reader (manufacturer: BMG, model: PHERAStar-FS). By adding Cell Counting-Lite™ background RLU was obtained from the culture medium wells without cells, and control RLU was obtained from the culture wells with cells but without the compound. Cell inhibition rate = 1-(sample RLU-background RLU)/(control RLU-background RLU)×100%, and the $IC_{50}$ of ADC 19 was calculated according to a four-parameter model fitting curve.

The $IC_{50}$ of ADC 19 against NCI-H1975 cell line is 5.00 μg/mL; the $IC_{50}$ of ADC 19 against HT-29 cell line is 4.56 μg/mL.

The results show that the antibody drug conjugates (such as ADC 19) prepared from the drug-linker of the present invention have obvious cytotoxicity to tumor cells.

13. Efficacy detection of anti-human-Her2 antibody-drug conjugate in NCI-N87 model

NCI-N87 cells were incubated in RPMI1640 medium supplemented with 10% fetal bovine serum at 37°C, 5% $CO_2$. NCI-N87 cells in the exponential growth phase were collected, re-suspended in PBS to a suitable concentration, and inoculated subcutaneously in female Balb/c-nu mice to establish a gastric cancer model. When the average tumor volume was about 160 $mm^3$, the mice were randomly grouped according to the tumor size: vehicle control group (i.e., negative control, Vehicle group), disclosed Trastuzumab-A-05 (1 mg/kg) group, disclosed Trastuzumab-A-14 (1 mg/kg) group, and disclosed Trastuzumab-A-24 (1 mg/kg) group. Each group was injected by tail vein (i.v.) and administered on Day 0, Day 7, and Day 14, for a total of 3 times. After administration, the body weight of mice was measured twice a week and the long and short diameters of the tumors were measured with vernier calipers, and the tumor volume was calculated by: V=0.5 a×b2, where a and b represent the long diameter and short diameter of the tumor, respectively, and the death of animals was observed and recorded every day.

The ADCs of the present invention have a significant tumor growth inhibition effect on the NCI-N87 gastric cancer transplanted tumor model. Compared with the vehicle control group, the disclosed Trastuzumab-A-05 (1 mg/kg) group, disclosed Trastuzumab-A-14 (1 mg/kg) group and disclosed Trastuzumab-A-24 (1 mg/kg) group have the tumor growth inhibition rates (TGI) of 81.62%, 107.38% and 61.43%, respectively. On Day 30, there were no animal deaths and significant weight loss in each treatment group, and no obvious drug toxic reactions were observed. During the treatment period, the mice had good tolerance to the ADCs prepared from the drug-linker compounds of the present invention. The results are shown in Table 17.

Table 17 Human gastric cancer cell NCI-N87 CDX model

| Group | Dose (mg/kg) | Day 26 | | | |
|---|---|---|---|---|---|
| | | Tumor volume ($mm^3$) ($\bar{x}\pm$SEM) | TGI (%) | T/C (%) | P-value (vs.Ve hicle) |
| Vehicle control | - | 506.05±102.02 | - | - | - |
| Trastuzumab-A-05 | 1 | 194.42±53.15 | 81.62 | 37.27 | <0.05 |
| Trastuzumab-A-14 | 1 | 111.44±37.23 | 107.38 | 21.91 | <0.01 |
| Trastuzumab-A-24 | 1 | 270.12±29.44 | 61.43 | 52.76 | <0.05 |
| Note: TGI is tumor growth inhibition rate, T/C is relative tumor proliferation rate. | | | | | |

Although specific embodiments of the invention have been described in detail, those skilled in the art should understand that various modifications and substitutions may be made to the details in accordance with all the published teachings, and all modifications and changes are also within the scope of the invention. The scope of the invention is defined by the appended claims and any equivalents thereof.

## Claims

**1.** A drug-linker compound having a structure represented by formula G-M-[L-E-D]$_x$, wherein

G is a functional group or leaving group capable of reacting with a specific amino acid or glycosyl;
M, which is a joint linked to G, is

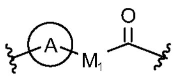,

ring A is a 5- to 6-membered aliphatic heterocycle or a 5- to 20-membered aromatic ring system, the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond and $C_{1-20}$ alkylene, $C_{2-20}$ alkenylene, $C_{2-20}$ alkynylene or amino; the $C_{1-20}$ alkylene, $C_{2-20}$ alkenylene, $C_{2-20}$ alkynylene or amino is optionally substituted by one or more suitable substituents;
L is a linker between the joint M and E, and L is selected from structures composed of one or more of the following: $C_{1-6}$ alkylene, -N(R')-, carbonyl, -O-, natural amino acids or non-natural amino acids and analogs thereof, short peptides consisting of amino acids,

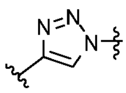, 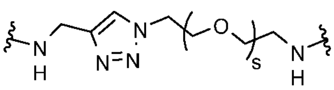,

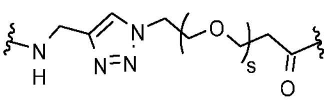, 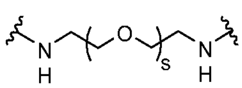, 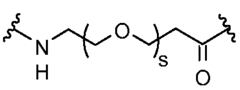, 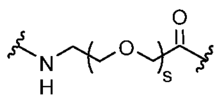,

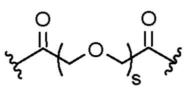, 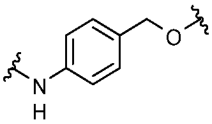, $-(CH_2CH_2O)_s-$ and 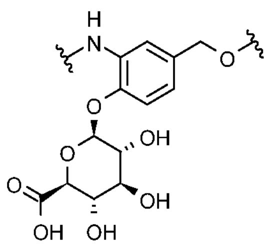;

wherein R' represents H, $C_{1-6}$ alkyl or a polyethylene glycol fragment containing 1-10 EO units; s is an integer from

1 to 20;
E is a structural fragment connecting L and D, wherein E is a single bond, - $NHCH_2$- or a structure selected from the group consisting of

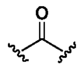, 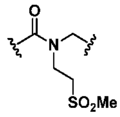,

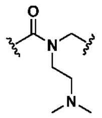 and 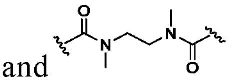;

D is a cytotoxic drug fragment; and/or
x is selected from 1 to 10.

**2.** The drug-linker compound according to claim 1, wherein the G is selected from the group consisting of halogen, halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, nitro, azido, cyano, alkenyl, alkynyl and an alkynyl-containing structural fragment, and the halo $C_{1-6}$ alkyl, $C_{1-6}$ sulfonyl, halo $C_{1-6}$ sulfonyl, halosulfonyl, $C_{1-6}$ sulfonate, halo $C_{1-6}$ sulfonate, $C_{1-6}$ sulfinate, $C_{1-6}$ sulfoxide, alkenyl, alkynyl and an alkynyl-containing structural fragment are optionally substituted by one or more suitable substituents.

**3.** The drug-linker compound according to claim 1 or 2, M is

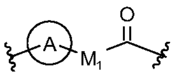,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, and the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, $C_{2-20}$ alkynylene or amino, and the $C_{1-20}$ alkylene, $C_{2-20}$ alkenylene, $C_{2-20}$ alkynylene or amino is optionally substituted by one or more suitable substituents.

**4.** The drug-linker compound according to any one of claims 1-3, M is

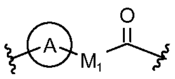,

wherein ring A is selected from the group consisting of

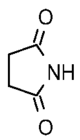, 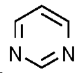,

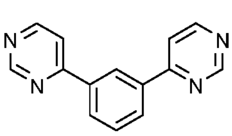, 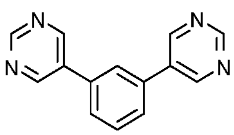, 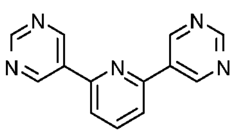, 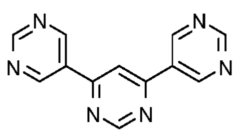 and

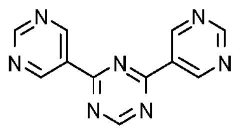
;

$M_1$ is selected from the group consisting of a single bond, and $C_{1-6}$ alkylene, $C_{2-6}$ alkenylene, $C_{2-6}$ alkynylene or amino, and the $C_{1-6}$ alkylene, $C_{2-6}$ alkenylene, $C_{2-6}$ alkynylene or amino is optionally substituted by one or more suitable substituents.

**5.** The drug-linker compound according to any one of claims 1-4, the M is selected from the group consisting of

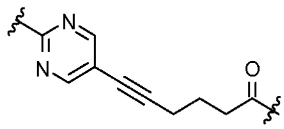 and 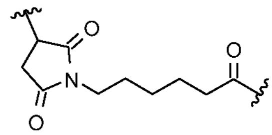 .

**6.** The drug-linker compound according to claim 5, the M is

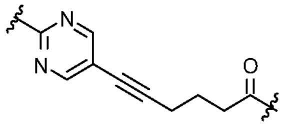 .

**7.** The drug-linker compound according to any one of claims 1-4, the M is selected from the group consisting of

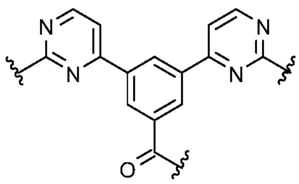 , 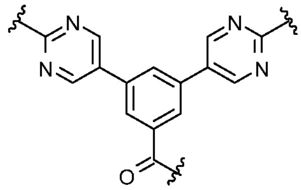 ,

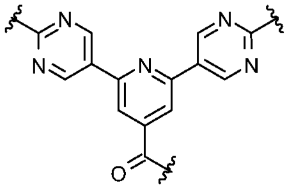 , 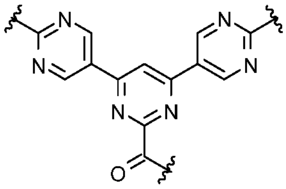 , 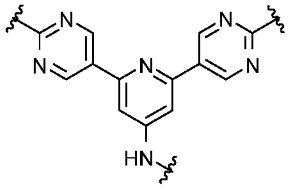

and

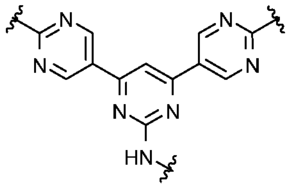 .

**8.** The drug-linker compound according to any one of claims 1-4, the M is selected from the group consisting of

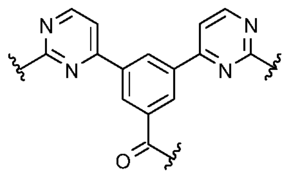
,
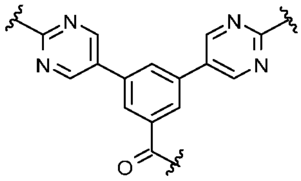

and

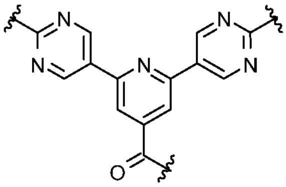
.

**9.** The drug-linker compound according to any one of claims 1-8, the L is selected from structures composed of one or more of the following: $C_{1-6}$ alkylene, -N(R')-, carbonyl, -O-, Ala, Arg, Asn, Asp, Cit, Cys, Gln, Glu, Gly, His, Ile, Leu, Lys, Met, Phe, Pro, Ser, Thr, Trp, Tyr, Val, Lys($COCH_2CH_2(OCH_2CH_2)_rOCH_3$)), Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly),

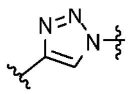
,
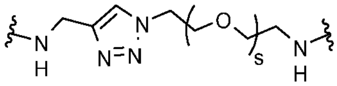
,

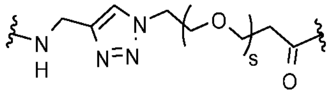
,
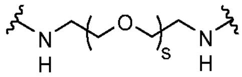
,
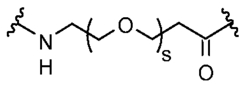
,
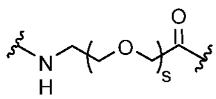

,
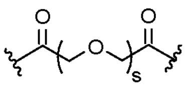
,
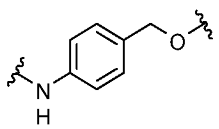
,
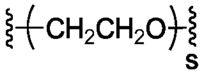
and
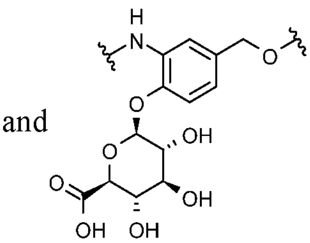
;

wherein R' represents H, $C_{1-6}$ alkyl or a polyethylene glycol fragment containing 1-10 EO units; s is an integer from 1 to 20.

**10.** The drug-linker compound according to any one of claims 1-9, the L is selected from structures composed of one or more of the following: $C_{1-6}$ alkylene, carbonyl, -NH-, Ala-Ala, Ala-Lys, Ala-Pro, Gly-Glu, Gly-Gly, Phe-Lys,Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly,

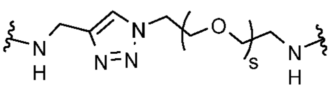
,

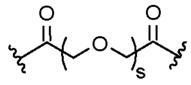 , 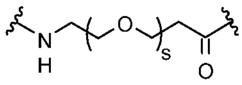 , 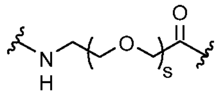 and 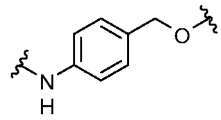 ;

wherein s is an integer from 1 to 20.

**11.** The drug-linker compound according to any one of claims 1-10, the L is selected from structures composed of one or more of the following:

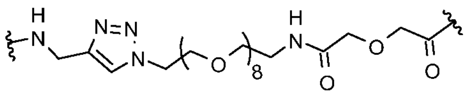 , 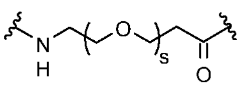 , 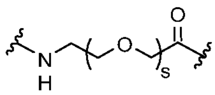 ,

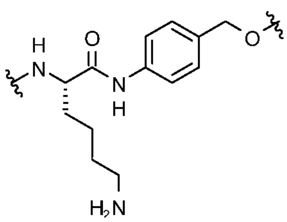 , 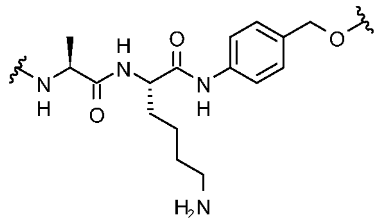 , 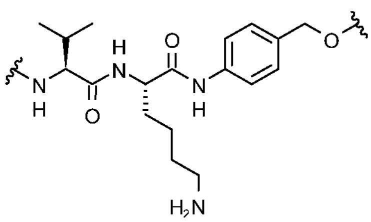 ,

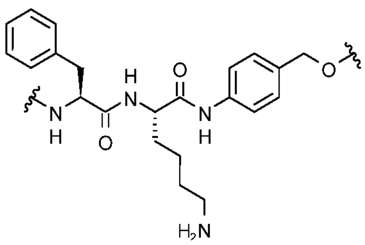 . 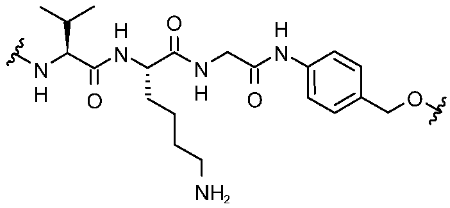 .

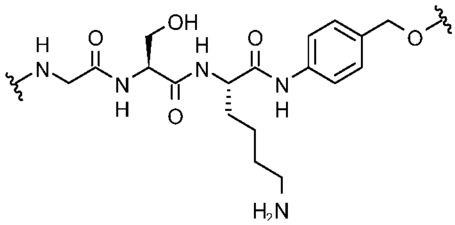 , 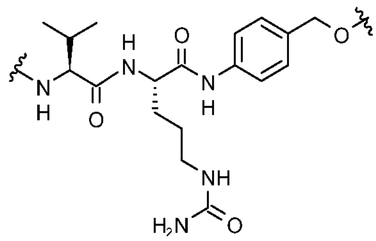 ,

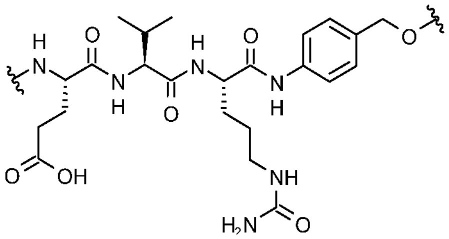 , 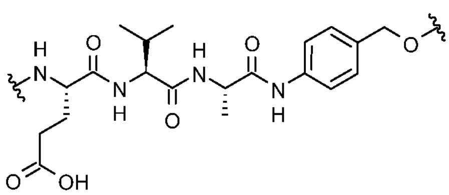 ,

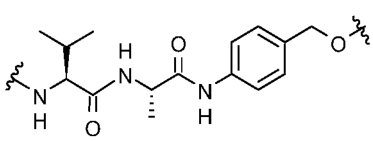 , 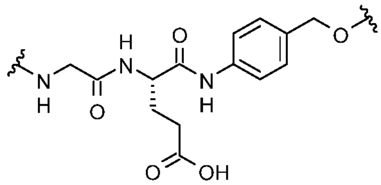 ,

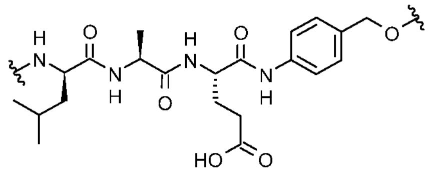, 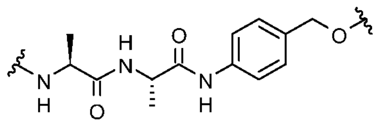,

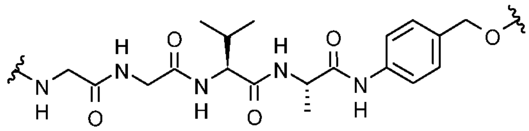, 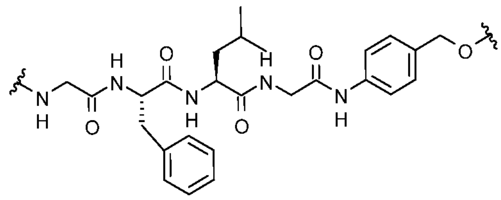,

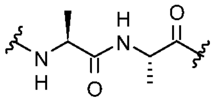, 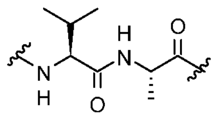, 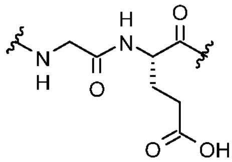, 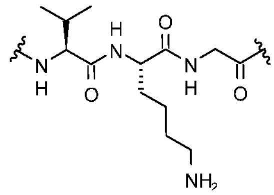,

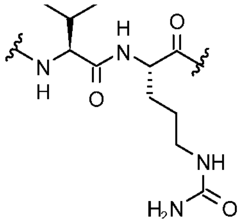, 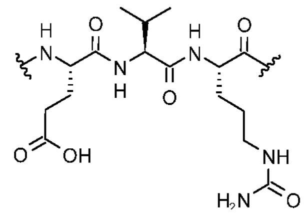, 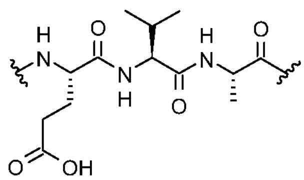,

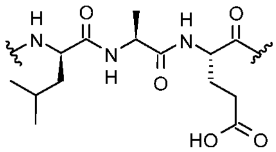, 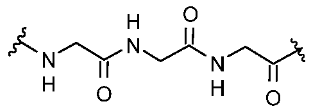, 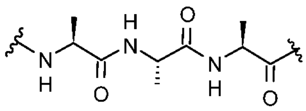,

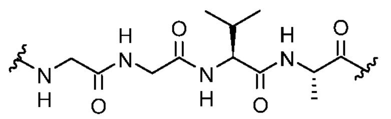, 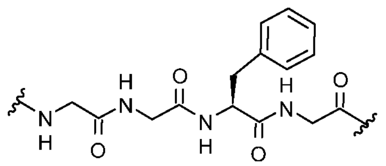

and

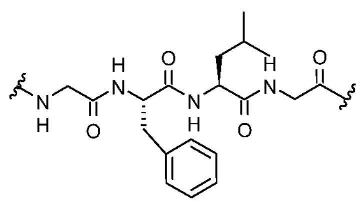.

**12.** The drug-linker compound according to any one of claims 1-11, the L is a structure selected from the group consisting of:

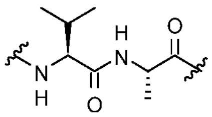,

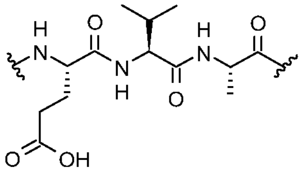, 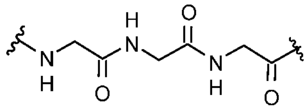, 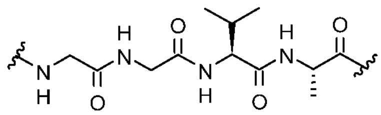,

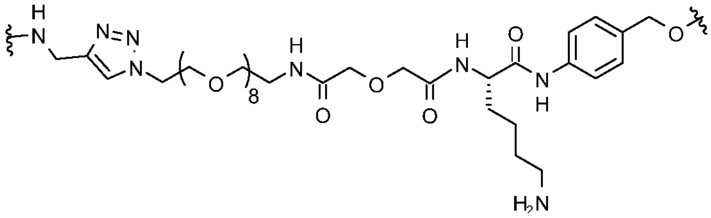,

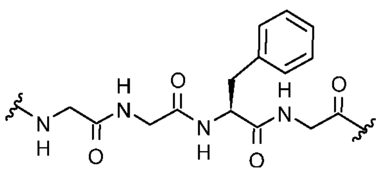, 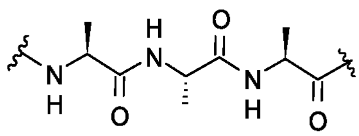,

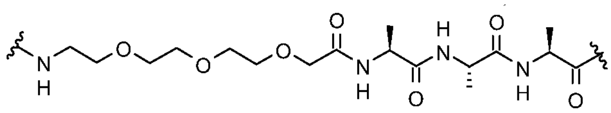

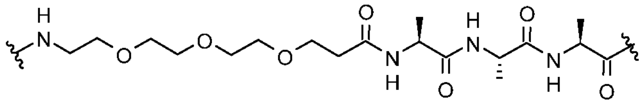,

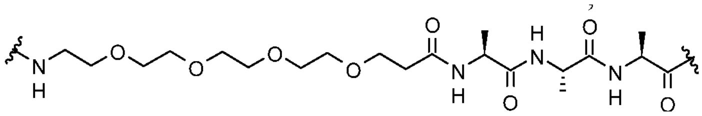,

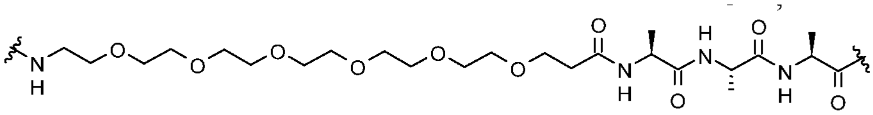 and

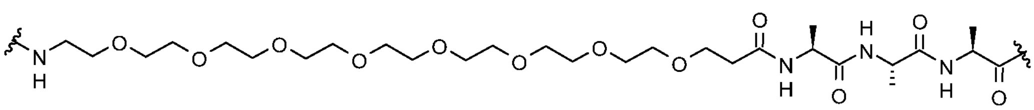.

**13.** The drug-linker compound according to any one of claims 1-12, the L is a structure selected from the group consisting of:

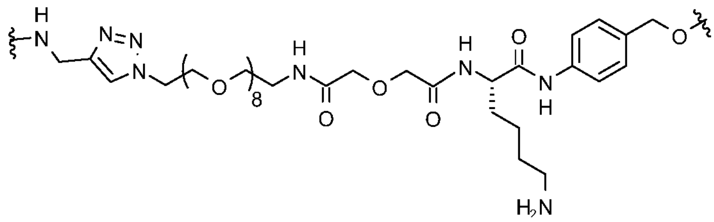

,

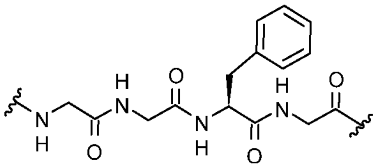

and

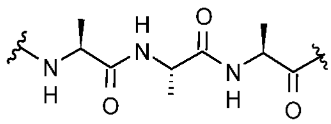

.

**14.** The drug-linker compound according to any one of claims 1-13, the L is a structure selected from the group consisting of:

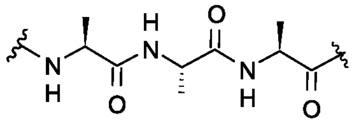

.

**15.** The drug-linker compound according to any one of claims 1-12, the L is a structure selected from the group consisting of:

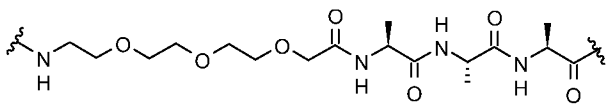

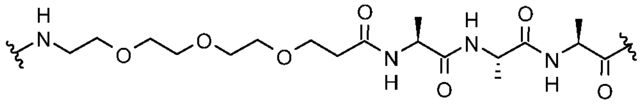

and

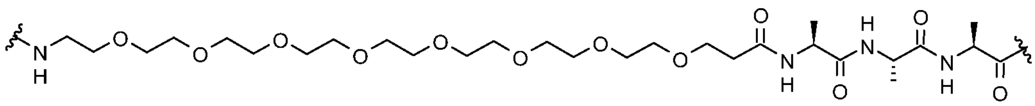

.

**16.** The drug-linker compound according to any one of claims 1-15 the E is a single bond, $-NHCH_2-$,

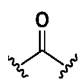

or

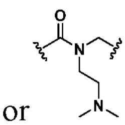

.

**17.** The drug-linker compound according to claim 16, the E is $-NHCH_2-$.

**18.** The drug-linker compound according to any one of claims 1-17,

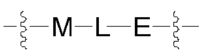

is a structure selected from the group consisting of:

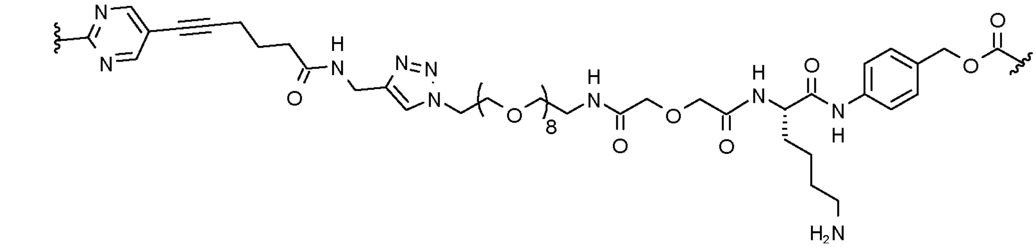

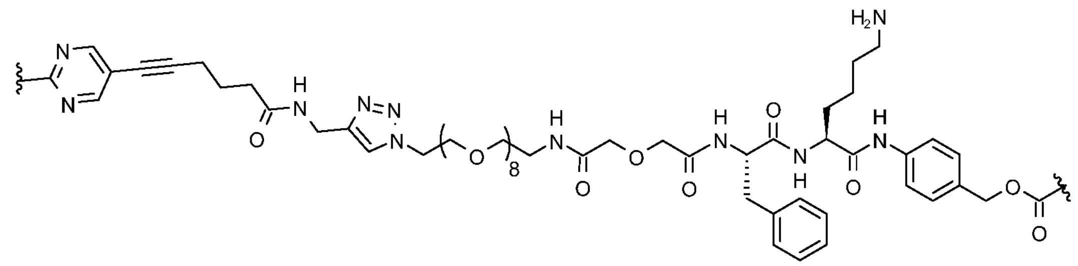

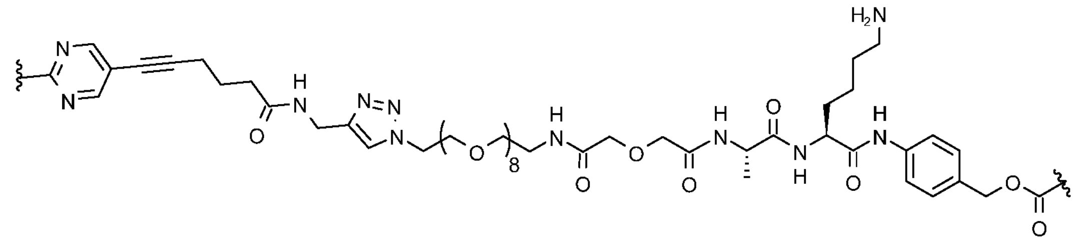

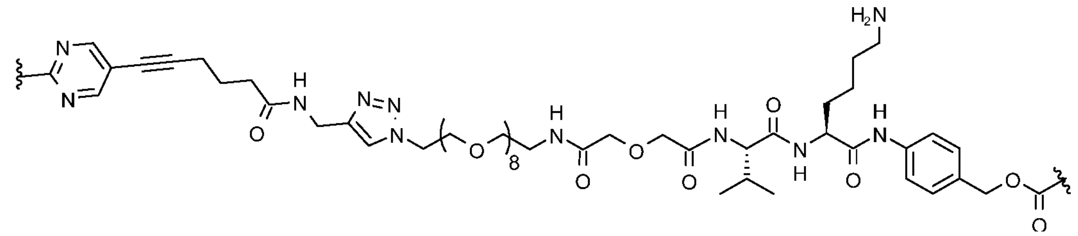

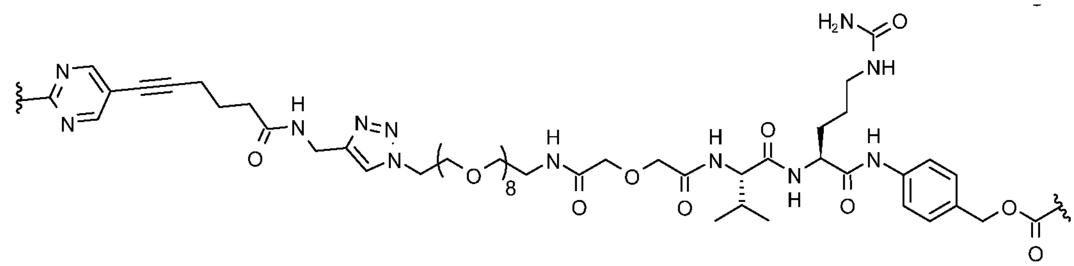

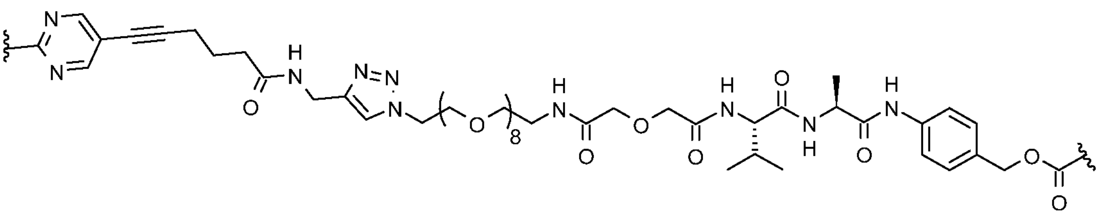

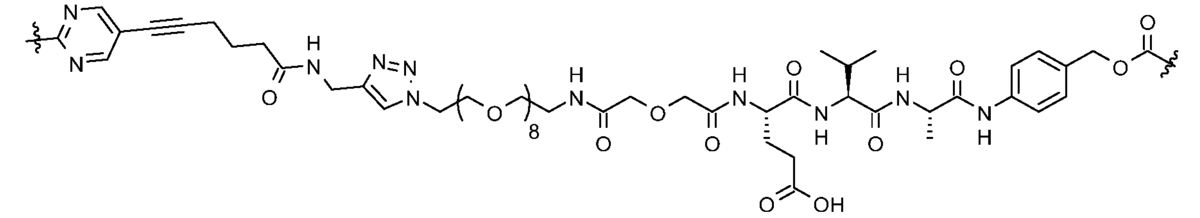

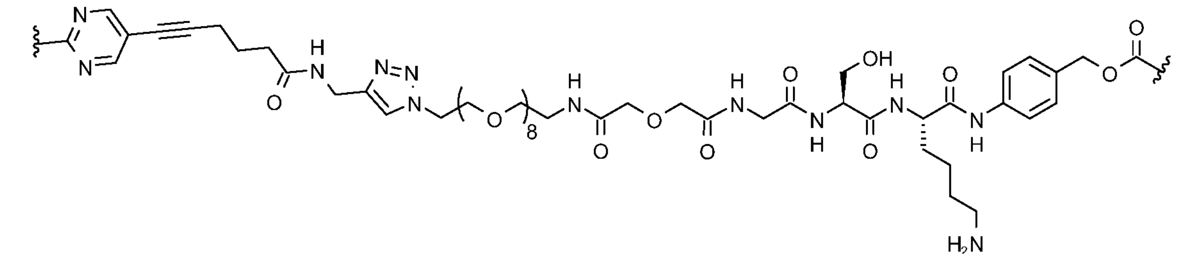

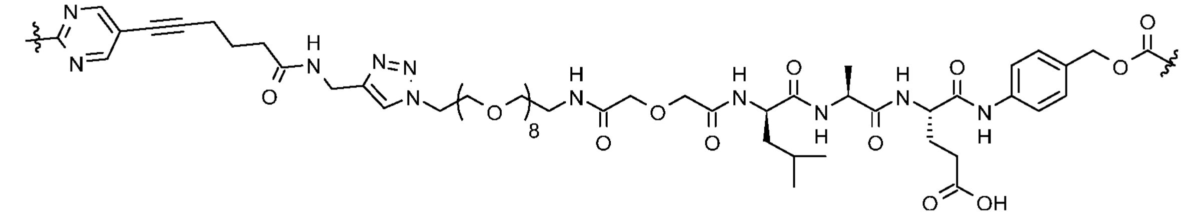

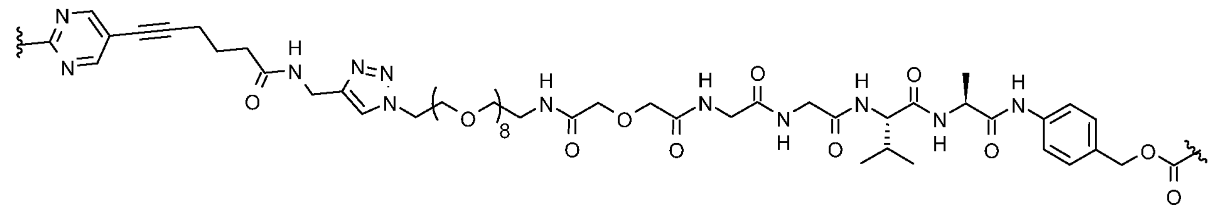

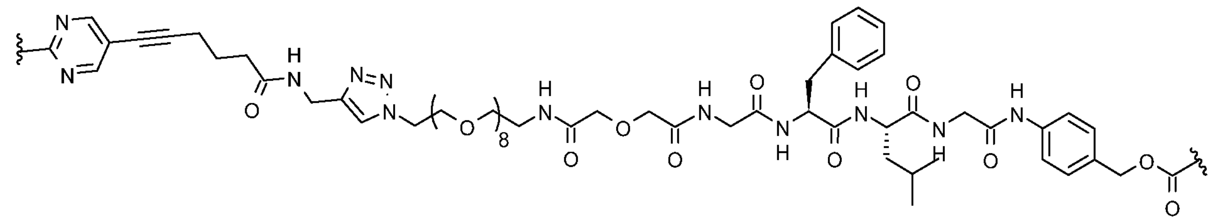

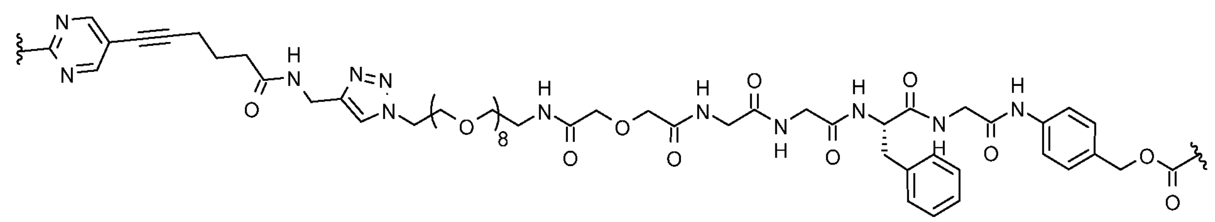

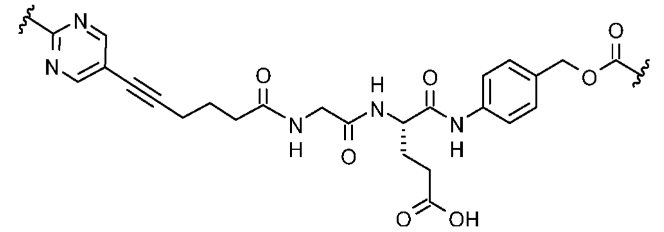

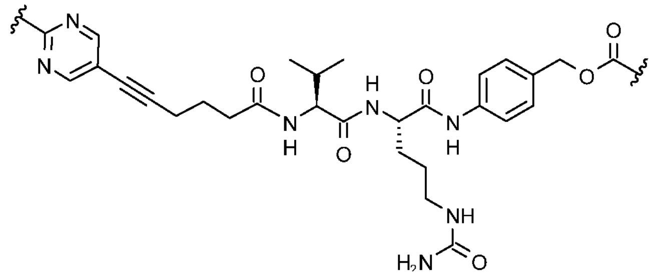

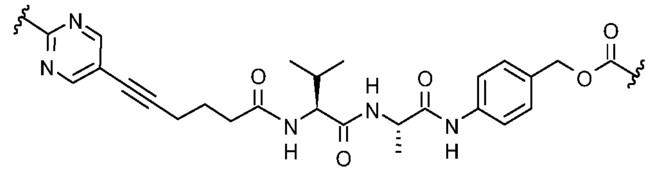

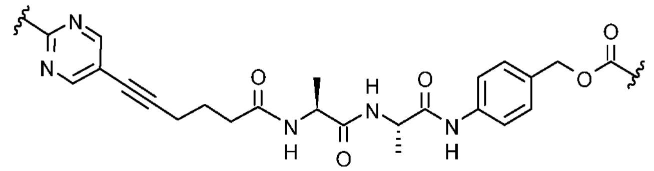

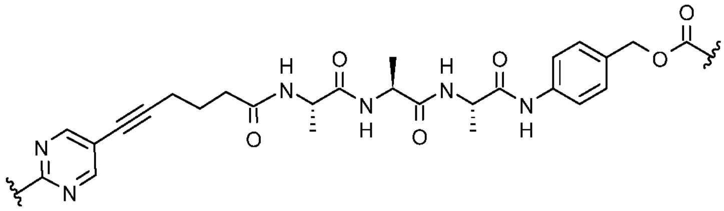

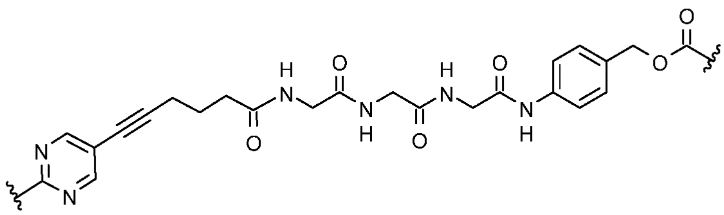

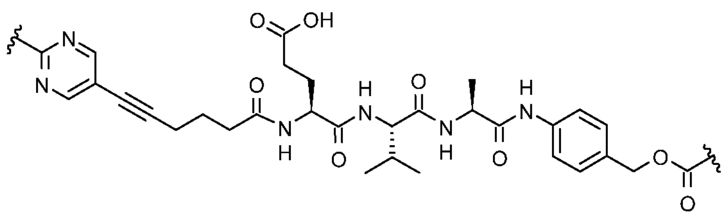

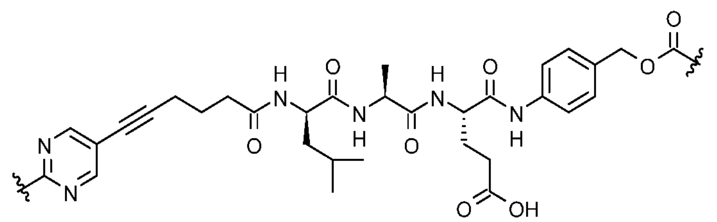

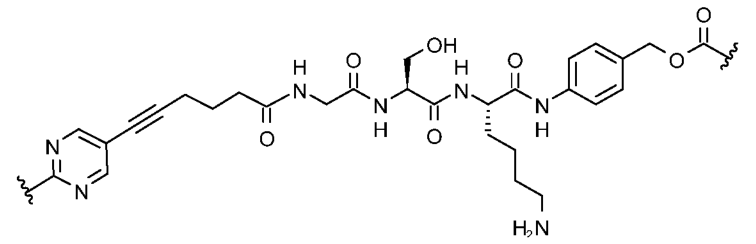

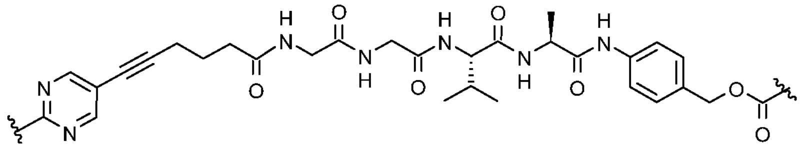

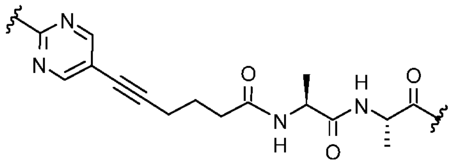

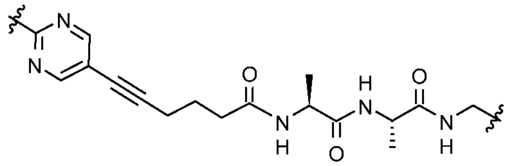

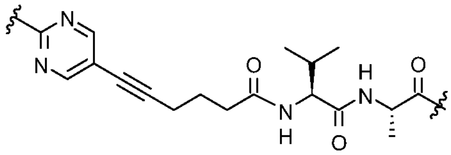

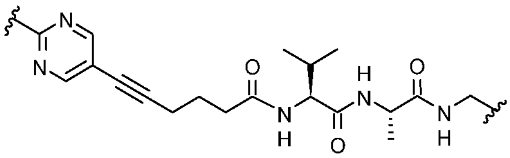

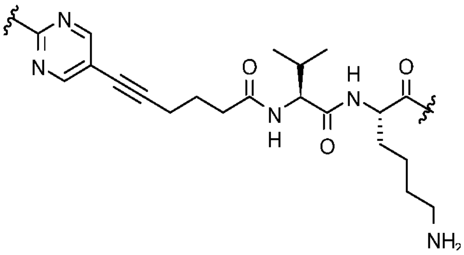

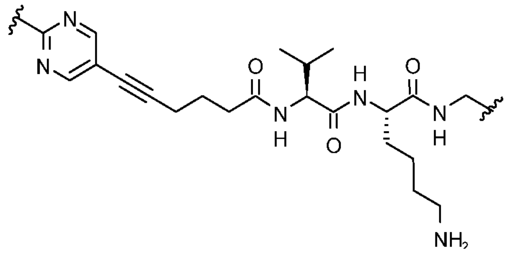

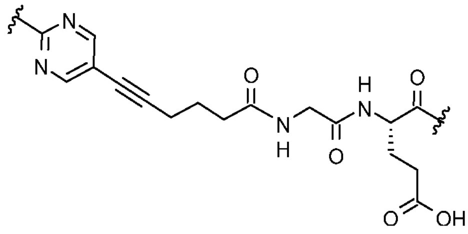

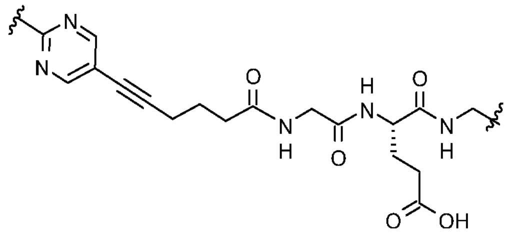

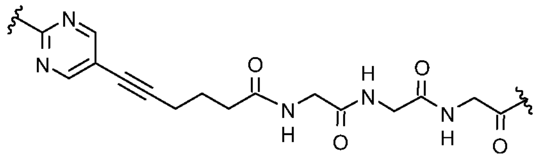

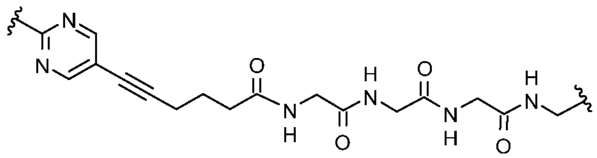

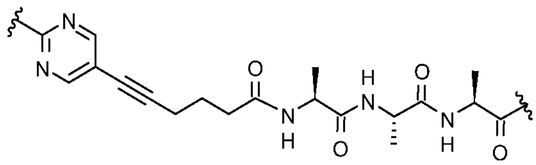

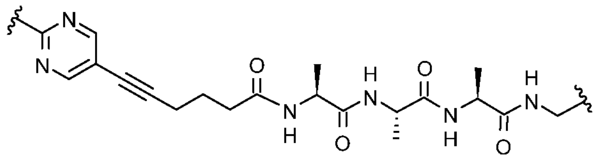

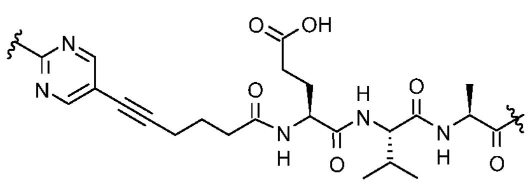

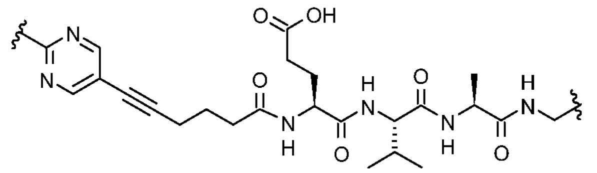

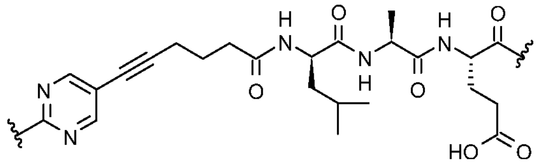

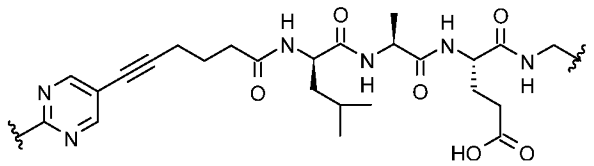

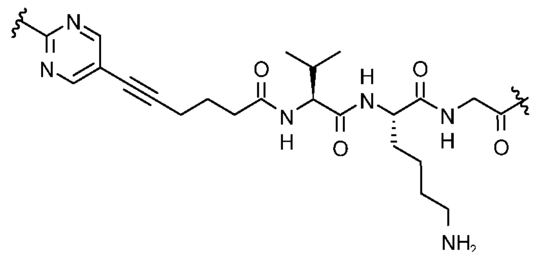

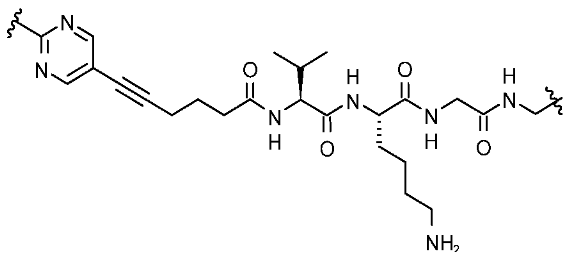

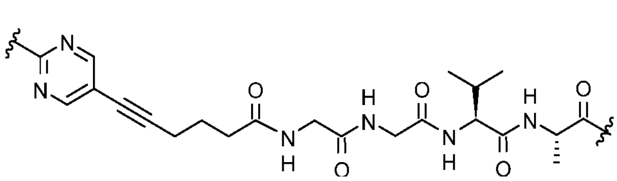

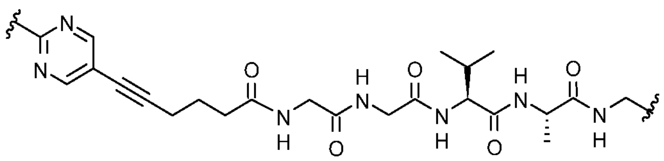

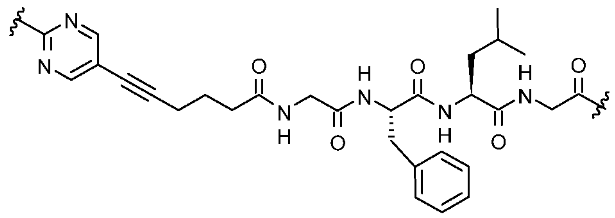

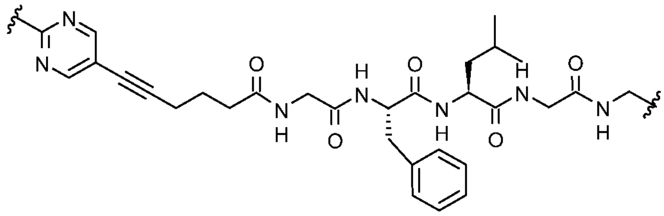

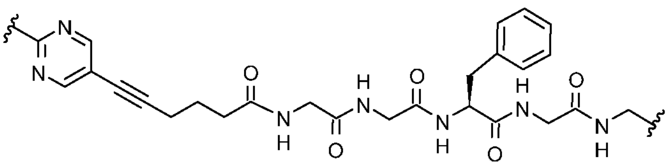

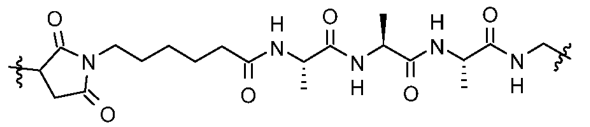

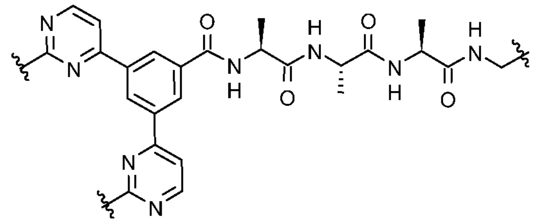

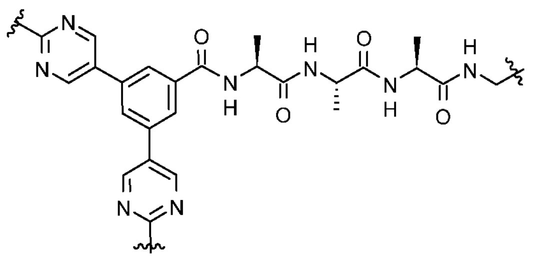

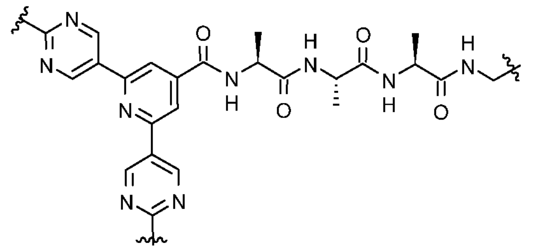

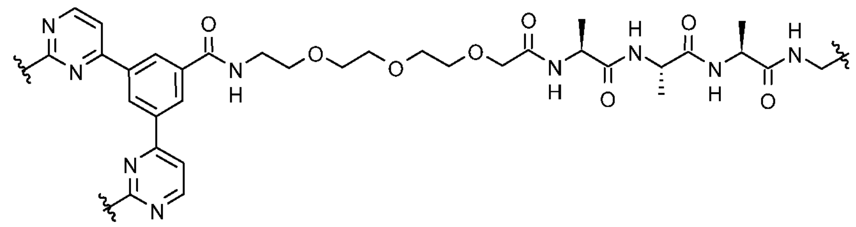

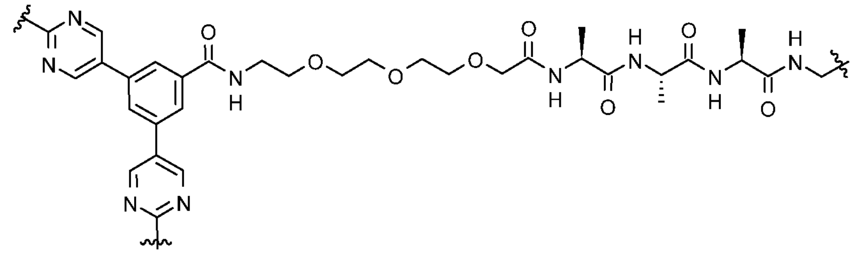

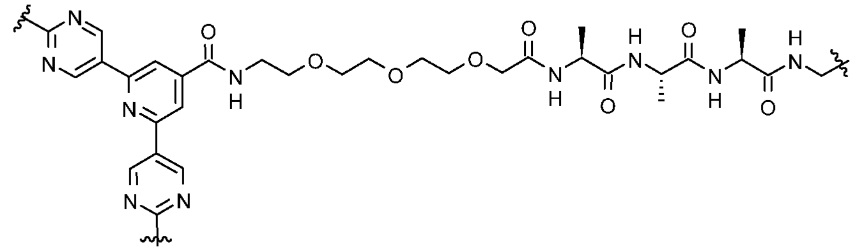

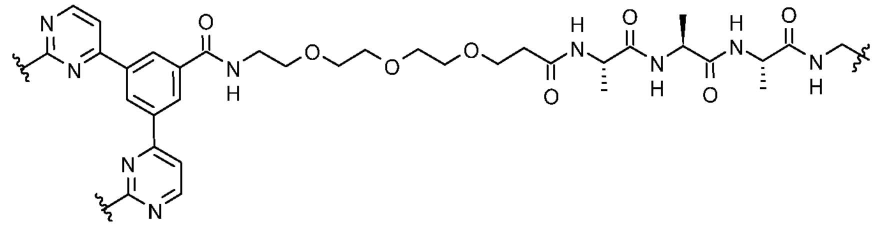

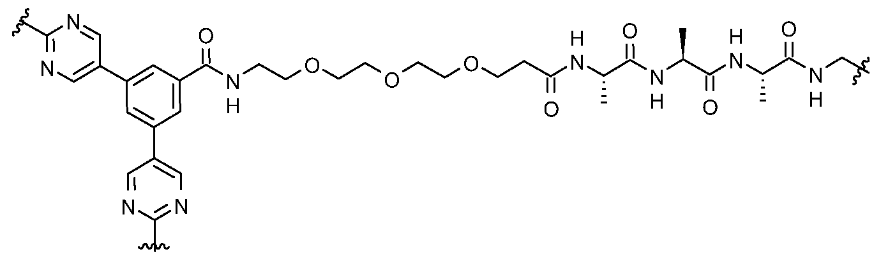

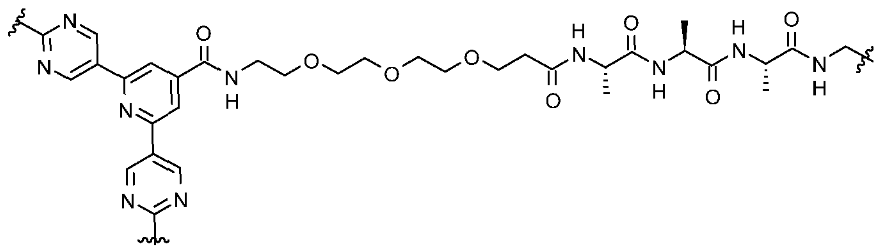

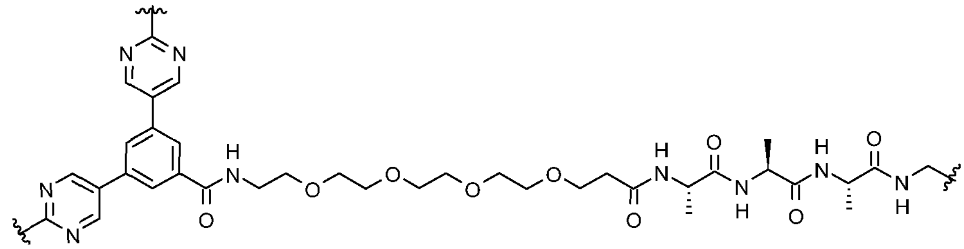

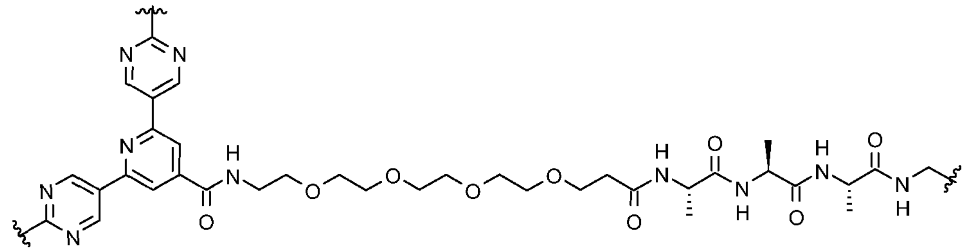

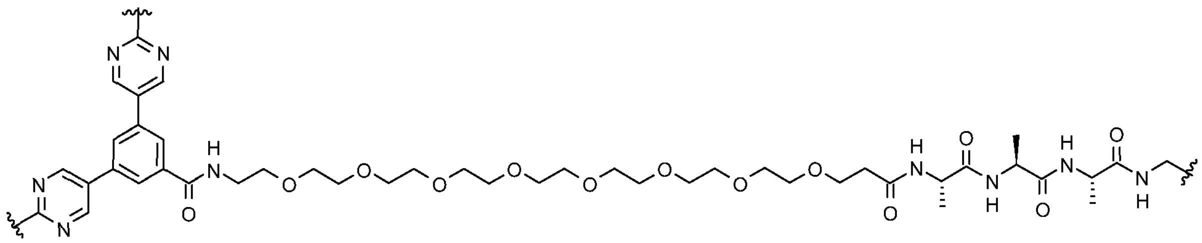

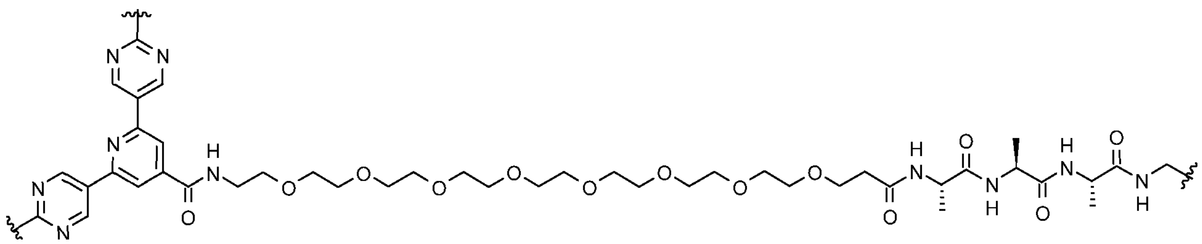

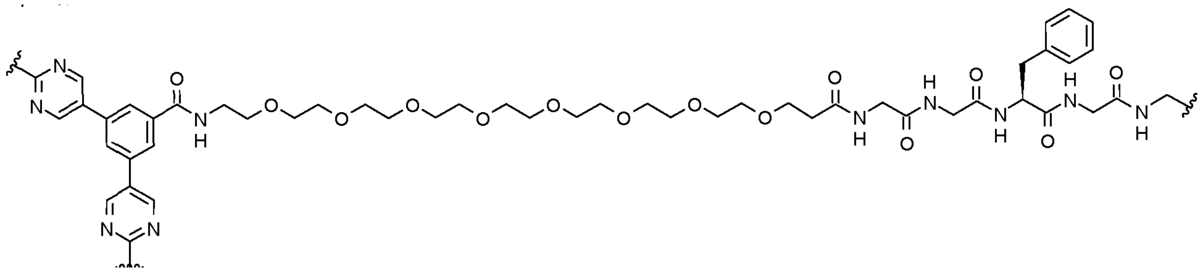

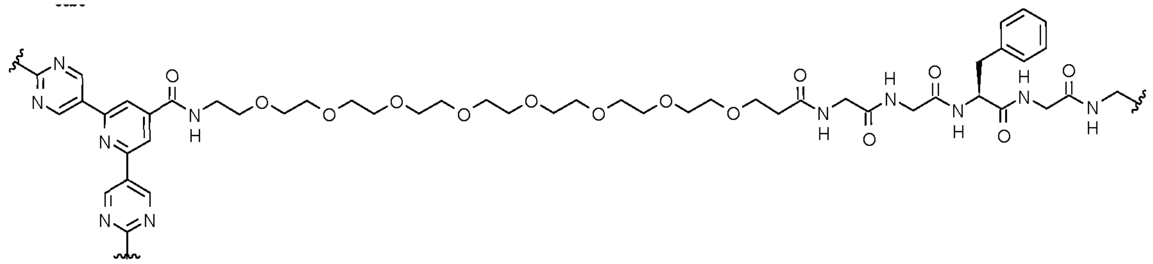

**19.** The drug-linker compound according to any one of claims 1-18,

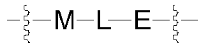

is a structure selected from the group consisting of:

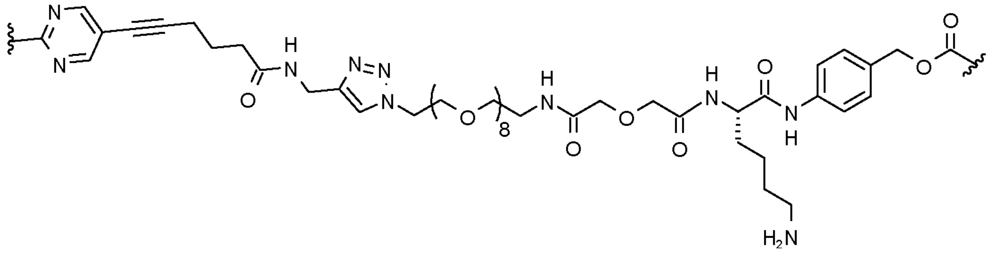

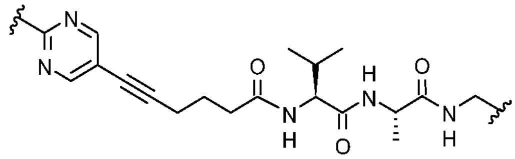

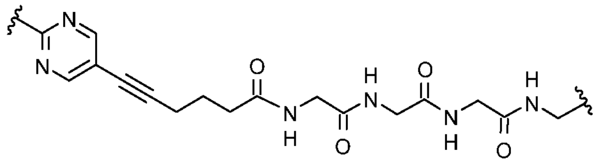

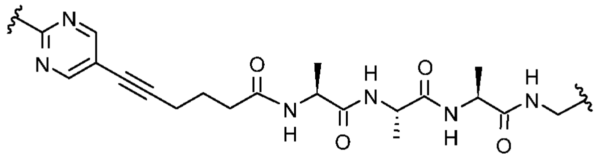

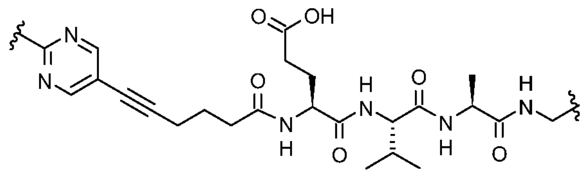

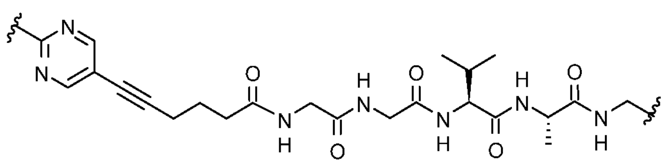

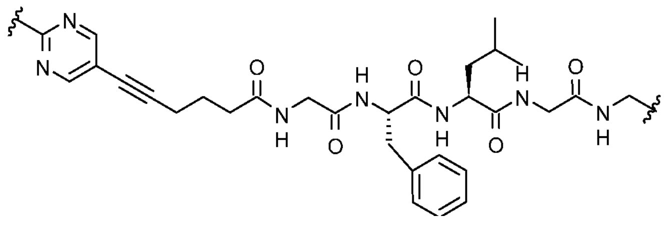

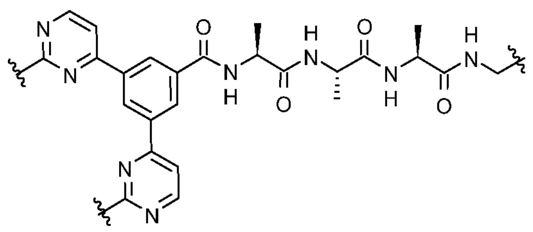

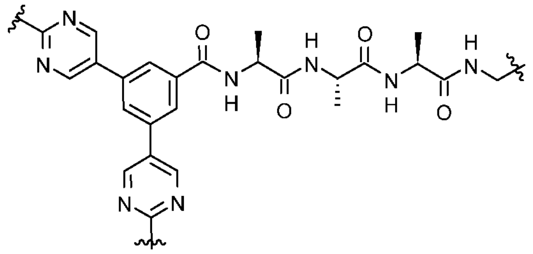

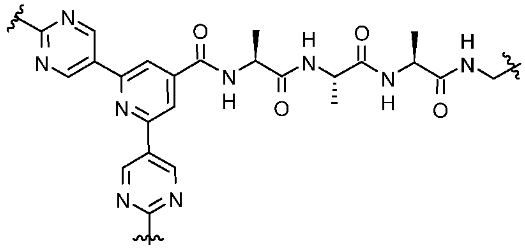

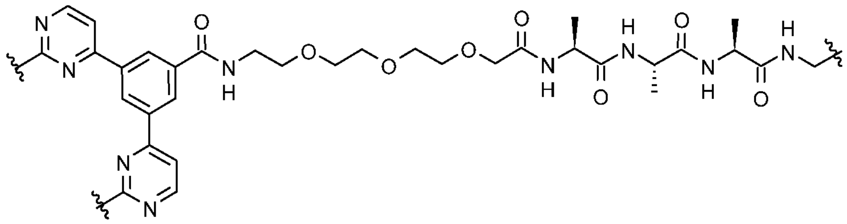

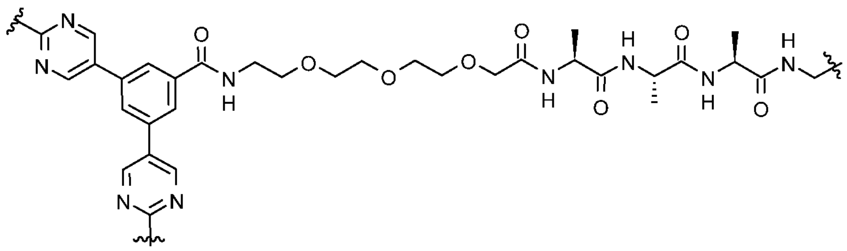

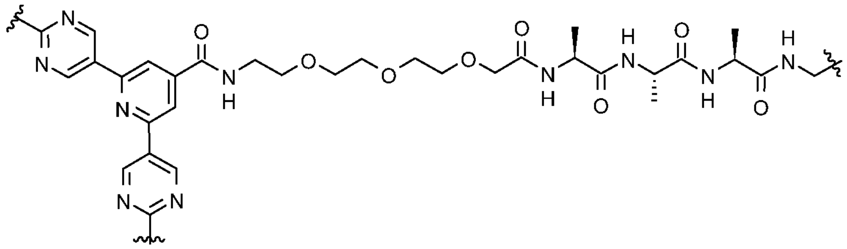

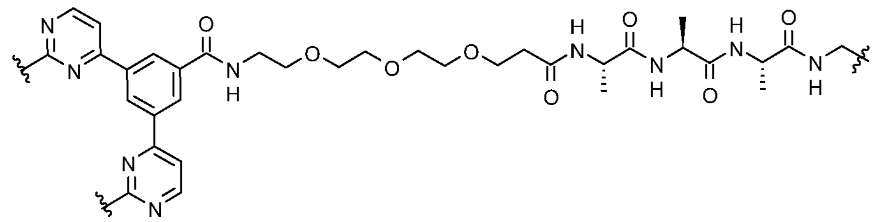

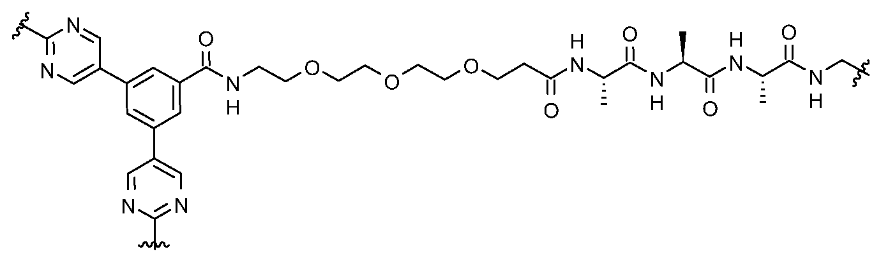

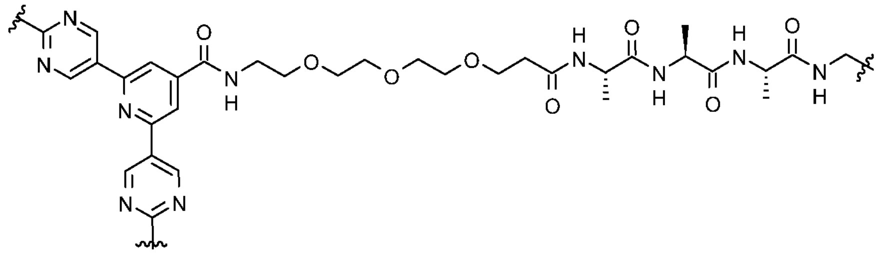

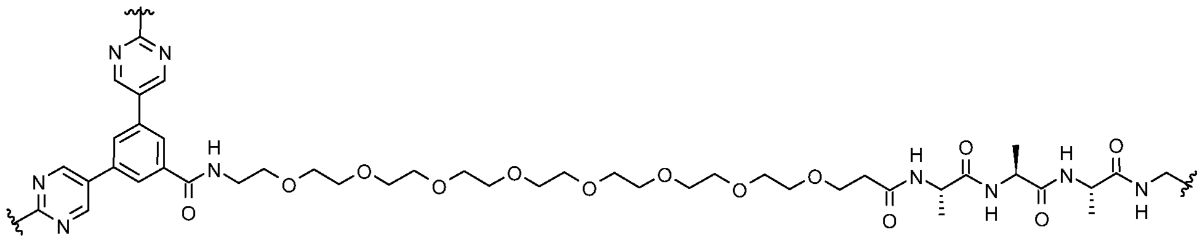

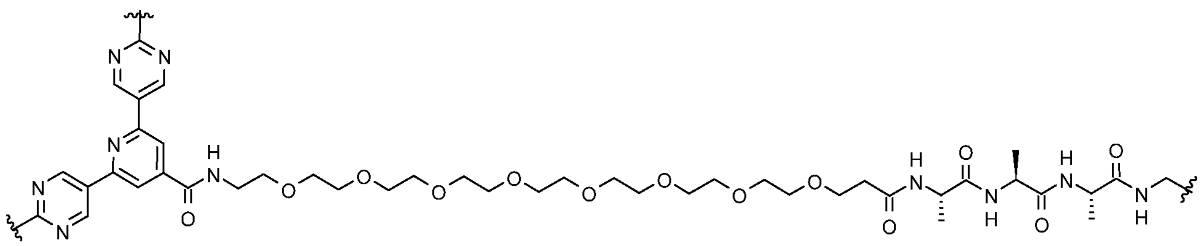

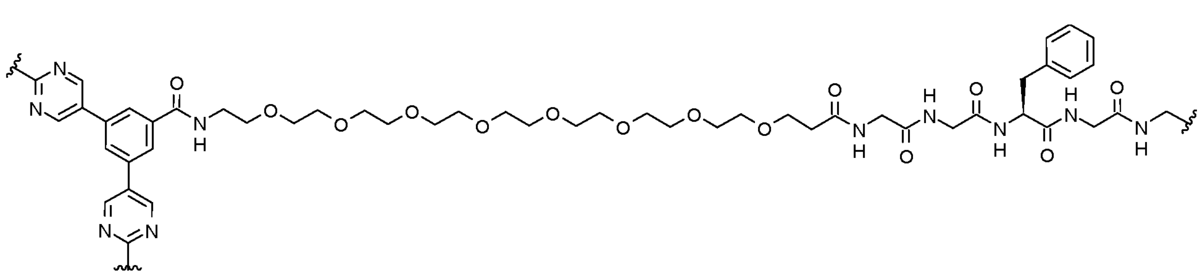

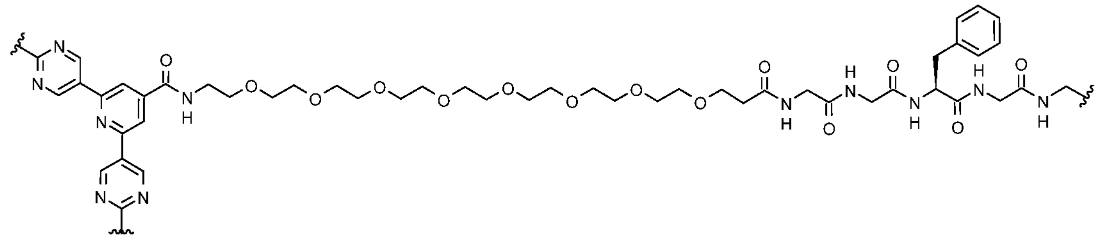

.

**20.** The drug-linker compound according to any one of claims 1-19, the cytotoxic drug is selected from the group consisting of a tubulin inhibitor, a DNA intercalator, a DNA topoisomerase inhibitor and an RNA polymerase inhibitor.

**21.** The drug-linker compound according to any one of claims 1-20, the tubulin inhibitor is an auristatin compound or a maytansine compound; the DNA intercalator is pyrrolobenzodiazepine (PBD); the DNA topoisomerase inhibitor is a topoisomerase I inhibitor (such as camptothecin, hydroxycamptothecin, 9-amino-camptothecin, SN-38, irinotecan, topotecan, belotecan, or rubitecan) or a topoisomerase II inhibitor (such as doxorubicin, PNU-159682, docamicin, daunorubicin, mitoxantrone, podophyllotoxin or etoposide); the RNA polymerase inhibitor is $\alpha$-amanitin or a pharmaceutically acceptable salt, ester or an analogue thereof.

**22.** The drug-linker compound according to any one of claims 1-21, the cytotoxic drug is selected from the compounds of formula I and formula II or pharmaceutically acceptable salts, esters, stereoisomers, tautomers or prodrugs of the compounds of formula I and formula II:

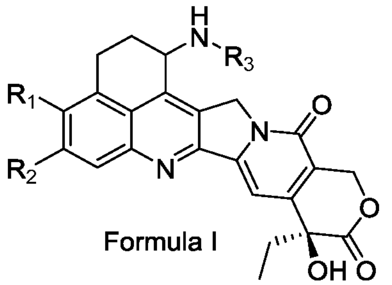

Formula I

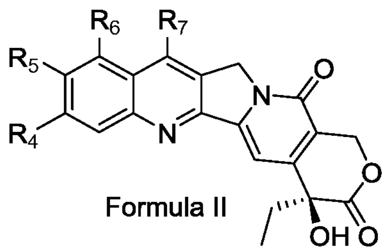

Formula II

wherein $R_1$ and $R_2$ are each independently selected from the group consisting of $C_{1-6}$ alkyl and halogen;
$R_3$ is selected from the group consisting of H and $-CO-CH_2OH$;
$R_4$ and $R_5$ are each independently selected from the group consisting of H, halogen and hydroxyl; or $R_4$ and $R_5$ are linked to carbon atoms connected thereto to form a 5-to 6-membered oxo-heterocycle;
$R_6$ is selected from the group consisting of H or $-C_{1-4}$ alkylene-$NR_aR_b$;
$R_7$ is selected from the group consisting of $C_{1-6}$ alkyl and $-C_{1-4}$ alkylene-$NR_aR_b$;
wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence.

**23.** The drug-linker compound according to any one of claims 1-22, the cytotoxic drug is selected from the following compounds or pharmaceutically acceptable salts, esters, stereoisomers, tautomers or prodrugs of the compounds:

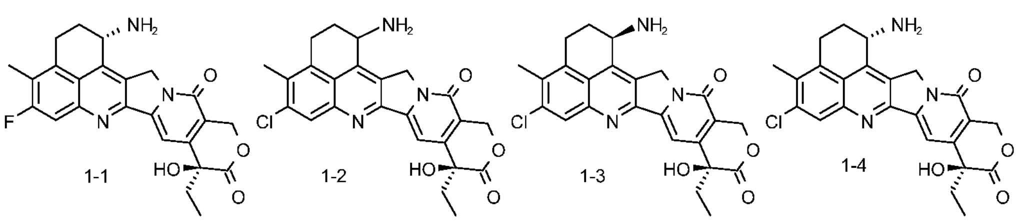

1-1 1-2 1-3 1-4

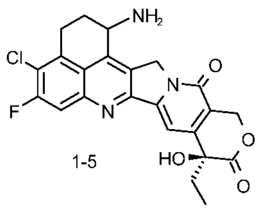
1-5

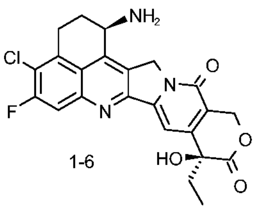
1-6

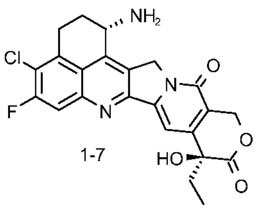
1-7

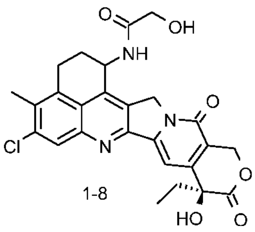
1-8

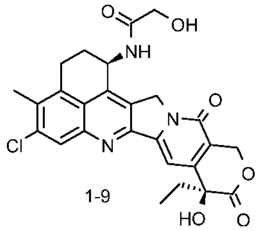
1-9

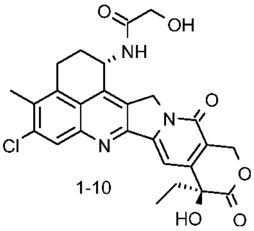
1-10

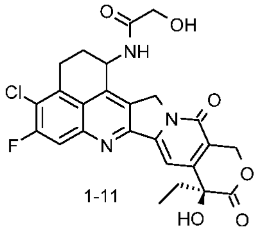
1-11

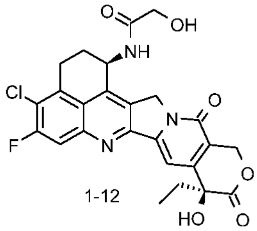
1-12

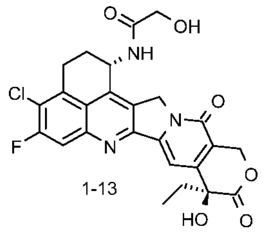
1-13

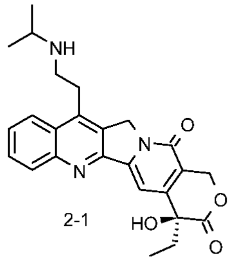
2-1

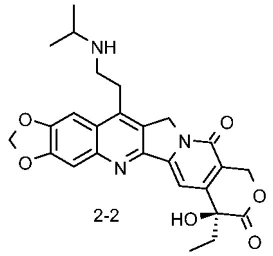
2-2

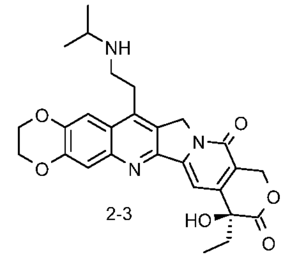
2-3

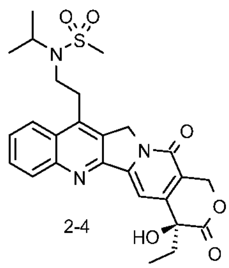
2-4

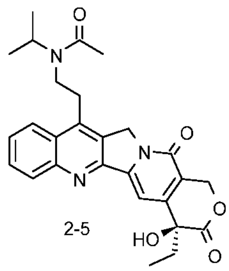
2-5

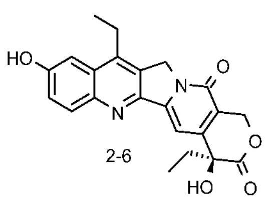
2-6

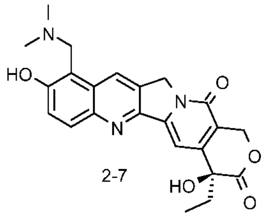
2-7

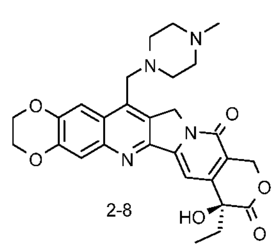
2-8

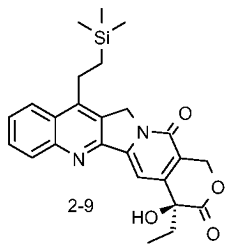
2-9

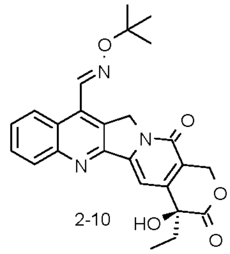
2-10

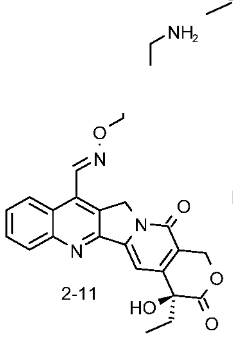
2-11

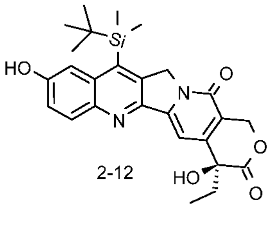
2-12

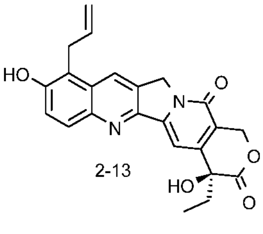
2-13

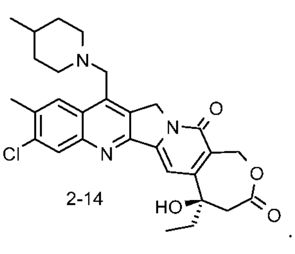
2-14

**24.** The drug-linker compound according to any one of claims 1-23, the cytotoxic drug is selected from the following

compounds or pharmaceutically acceptable salts, esters, stereoisomers, tautomers or prodrugs of the compounds:

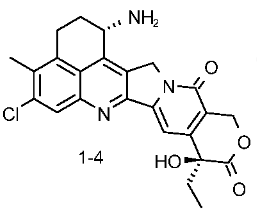
1-4

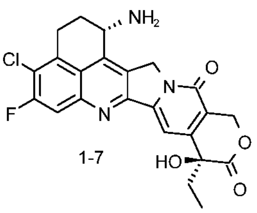
1-7

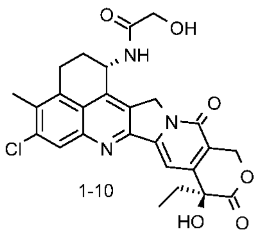
1-10

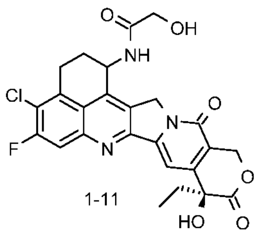
1-11

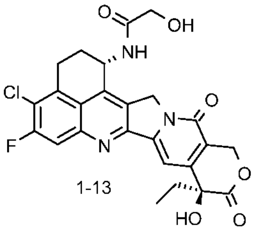
1-13

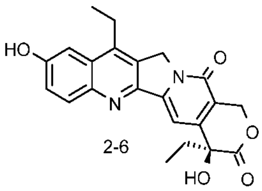
2-6
.

**25.** The drug-linker compound according to any one of claims 1-24, D is a structure selected from the group consisting of:

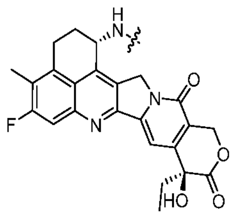

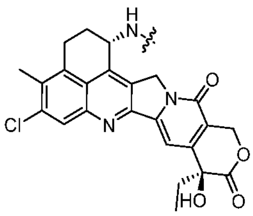

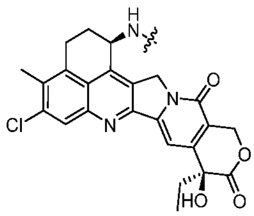

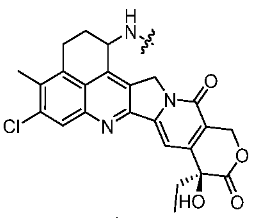

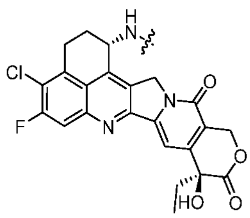

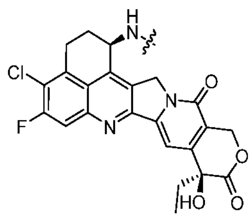

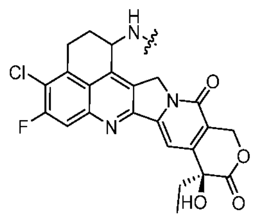

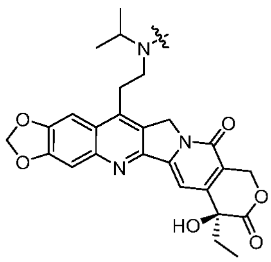

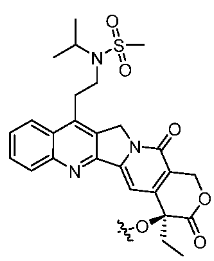

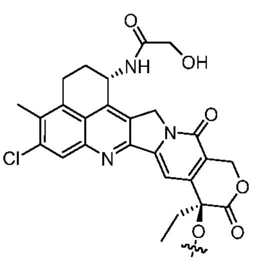

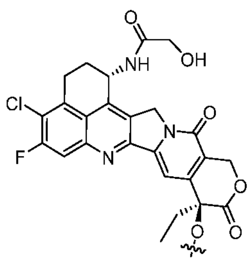

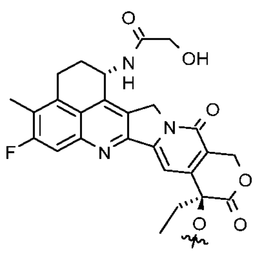

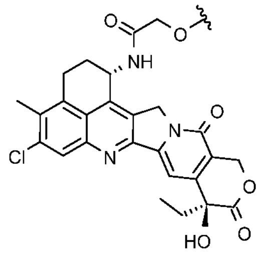 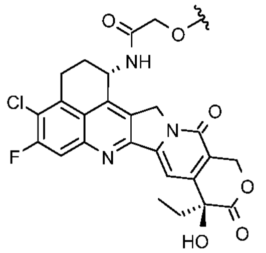 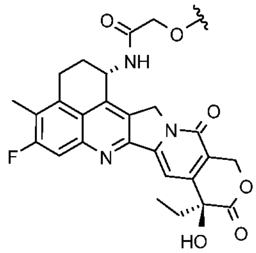 .

**26.** The drug-linker compound according to any one of claims 1-25, wherein the drug-linker compound is selected from A-01 to A-34, B-01 to B-07, and C-01 to C-28 shown below:

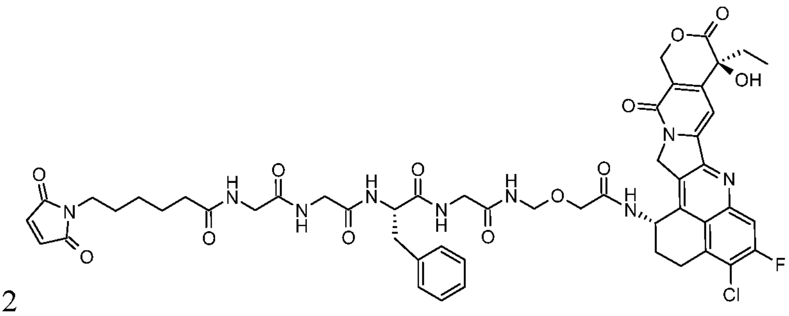

A-02

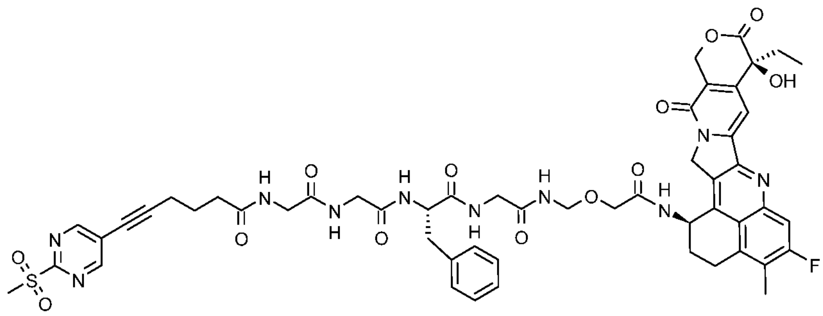

A-04

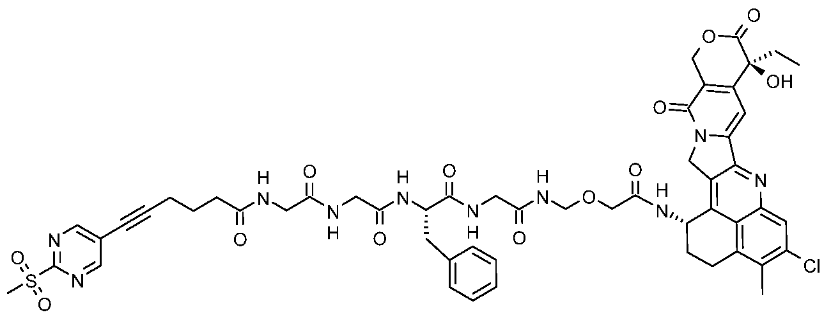

A-05

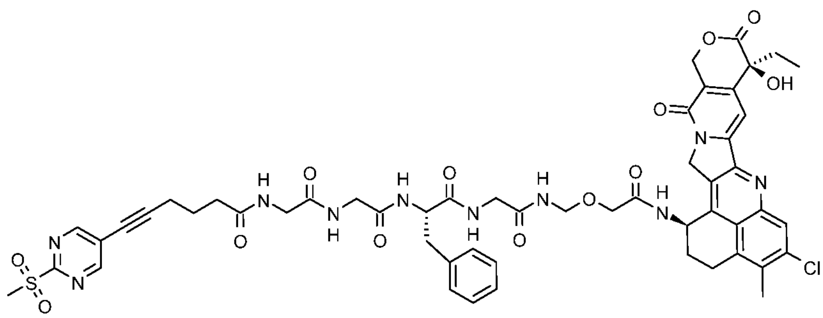

A-06

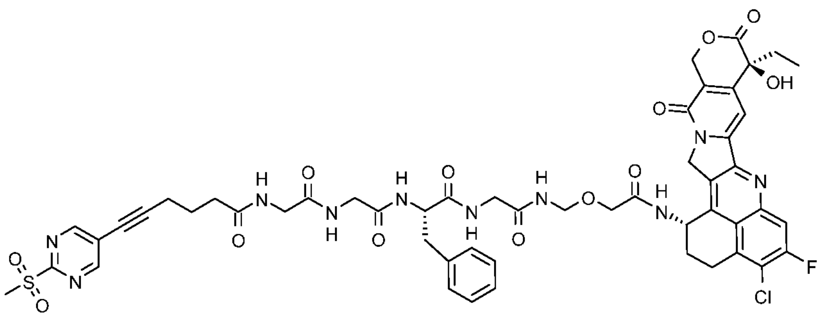

A-07

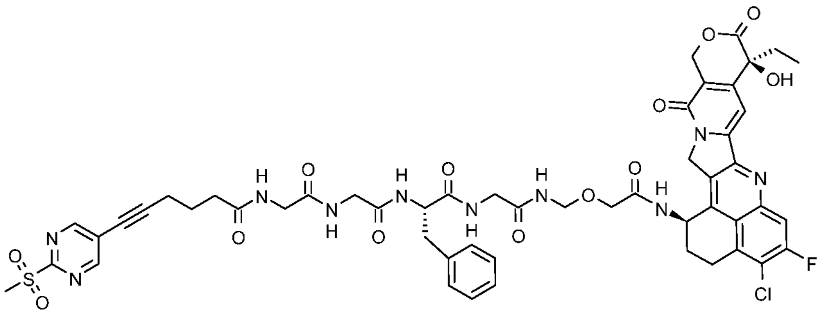

A-08

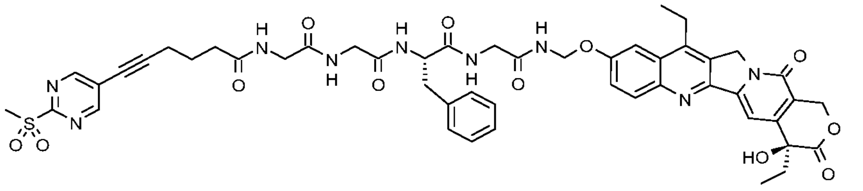

A-09

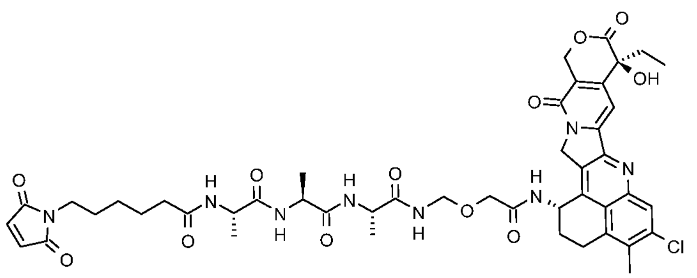

A-10

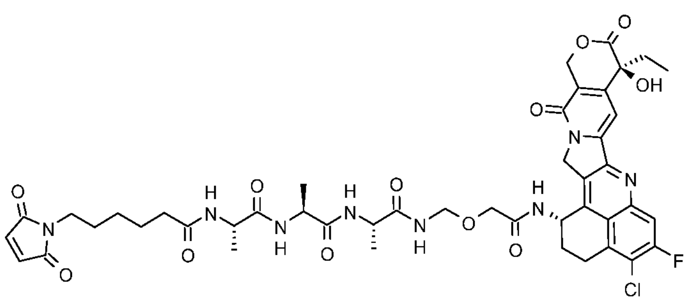

A-11

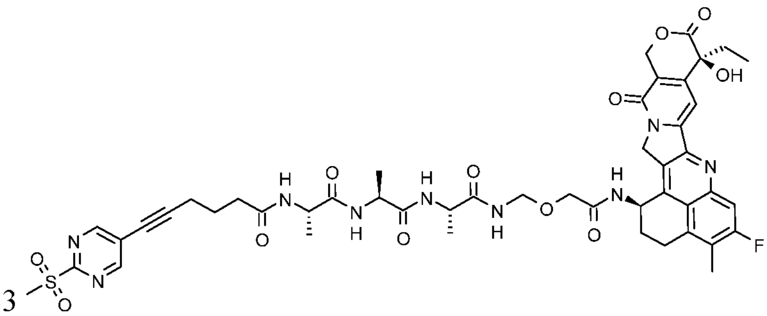

A-13

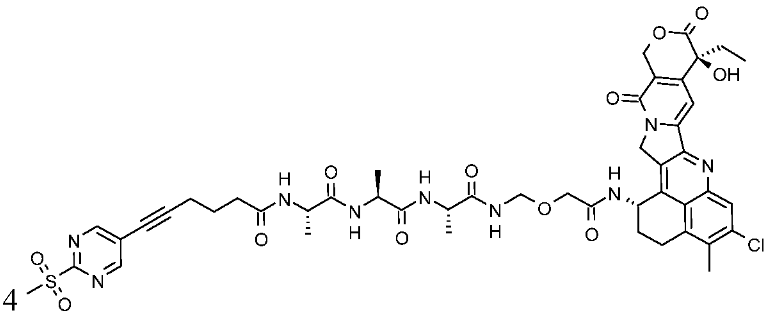

A-14

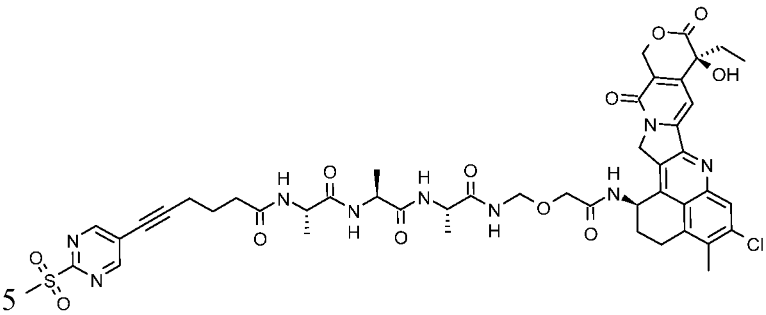

A-15

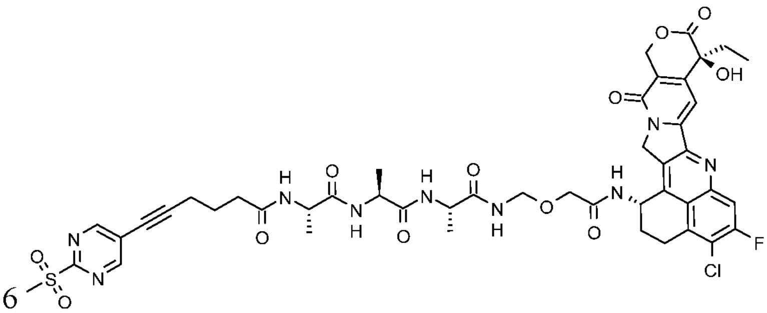

A-16

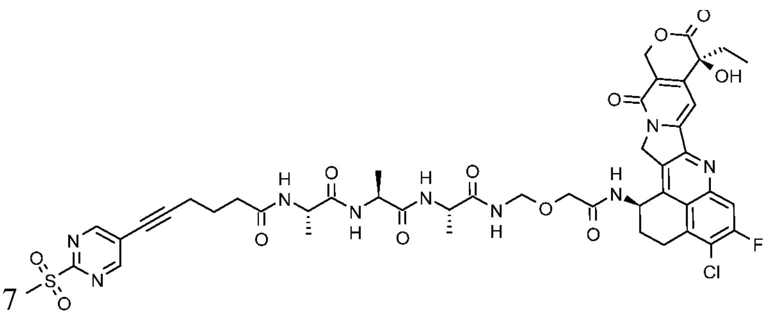

A-17

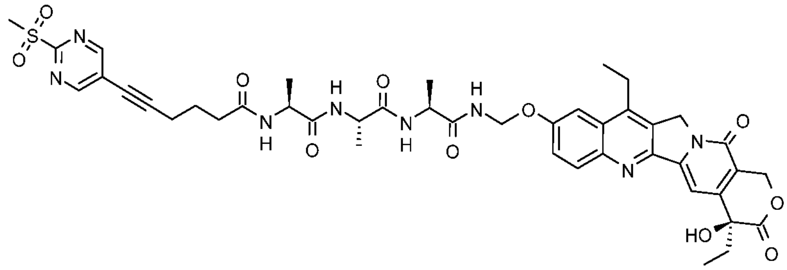

A-18

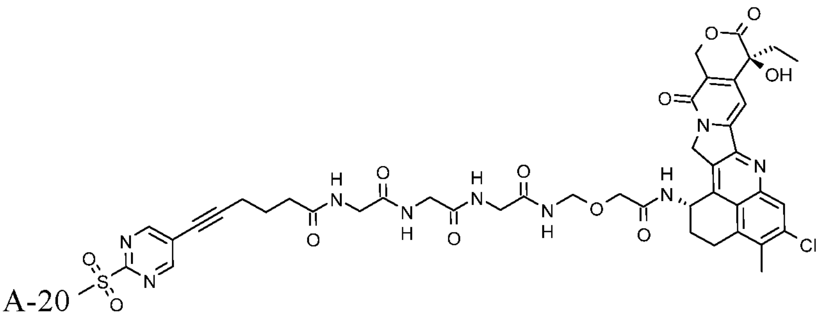

A-20

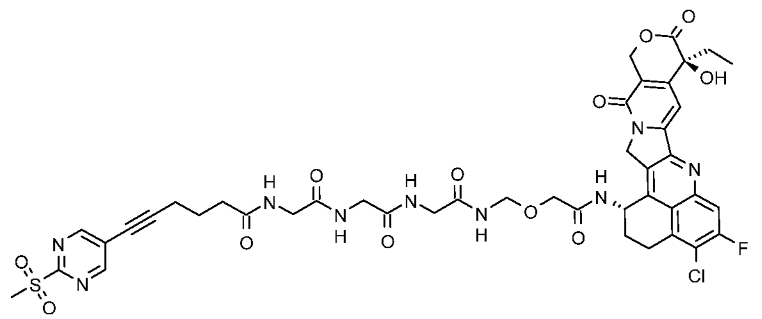

A-21

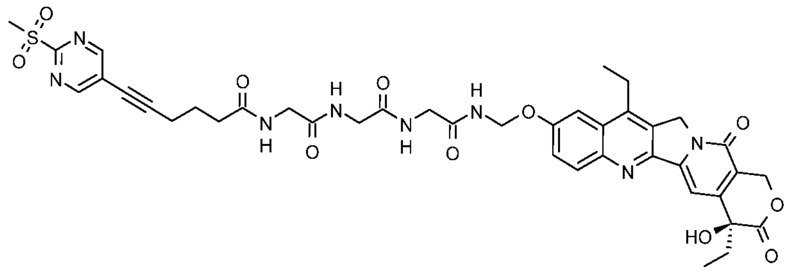

A-22

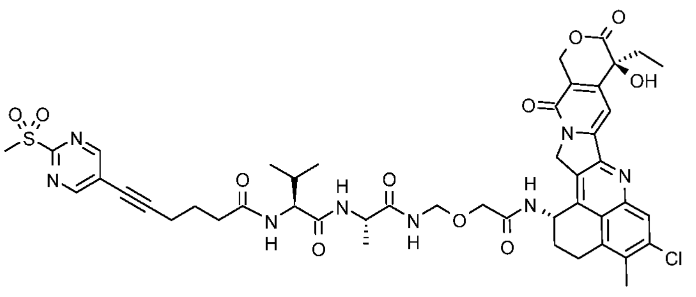

A-24

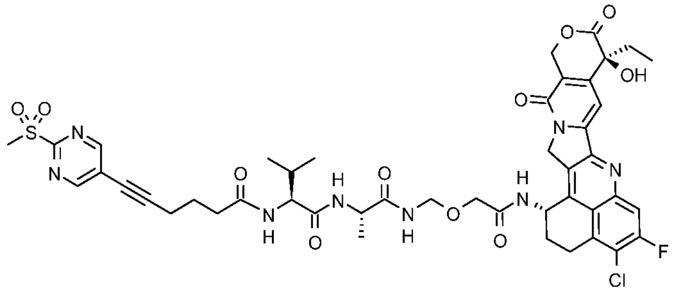

A-25

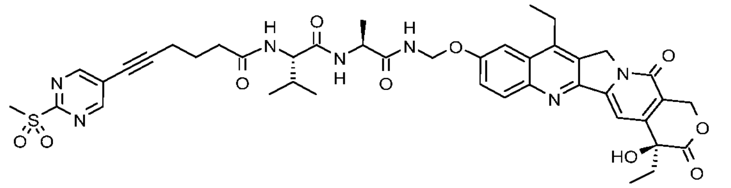

A-26

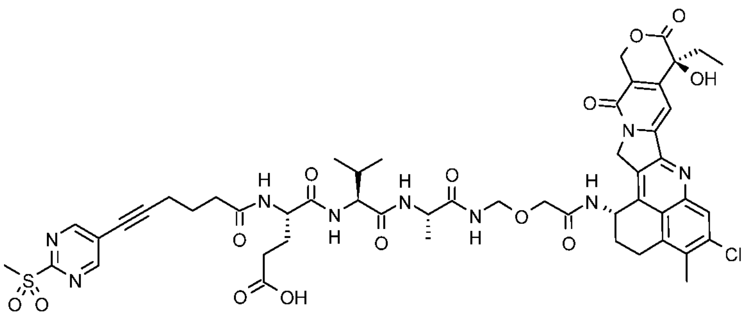

A-28

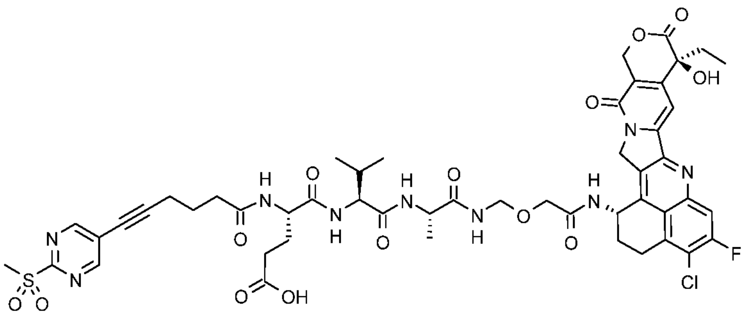

A-29

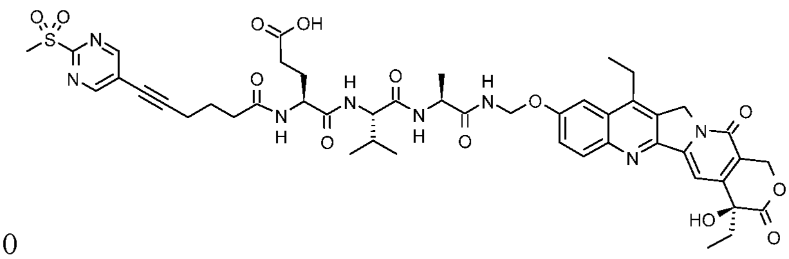

A-30

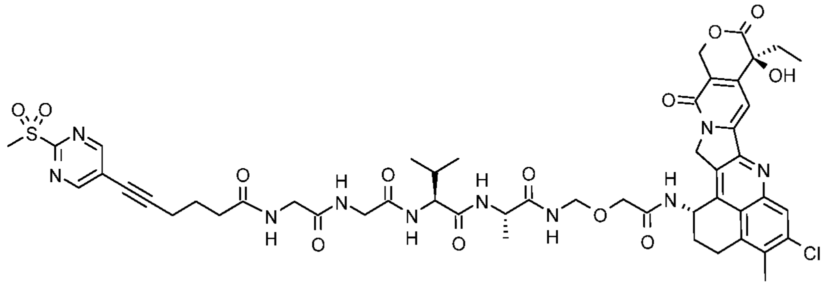

A-32

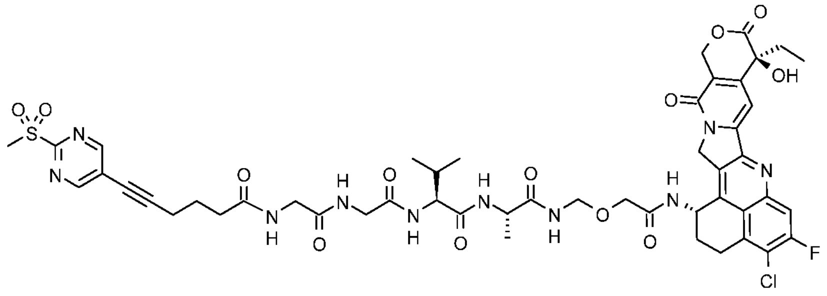

A-33

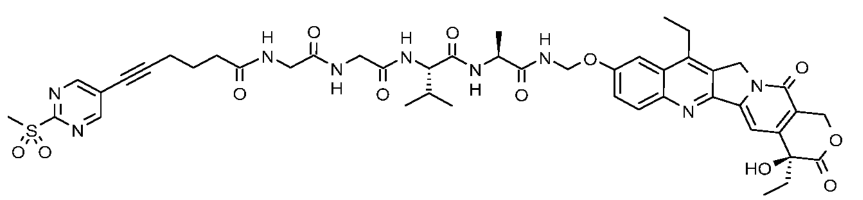

A-34

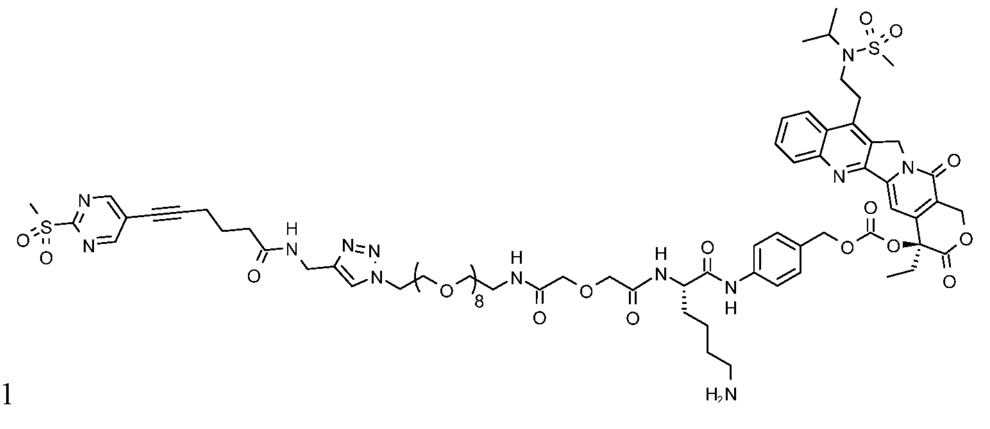

B-01

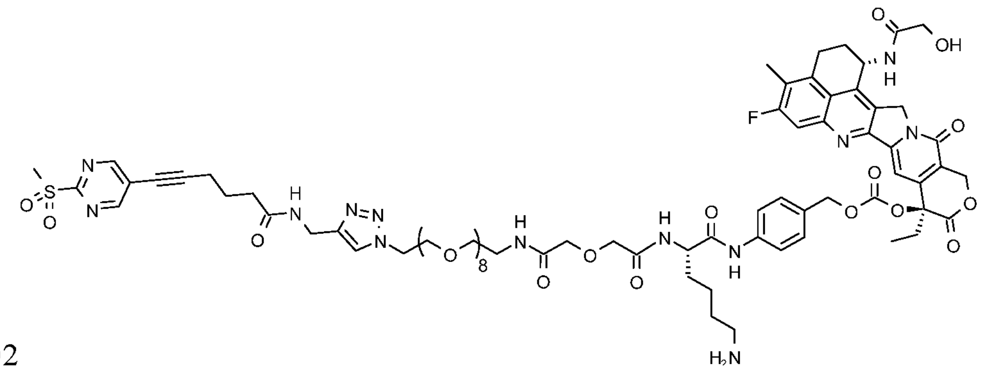

B-02

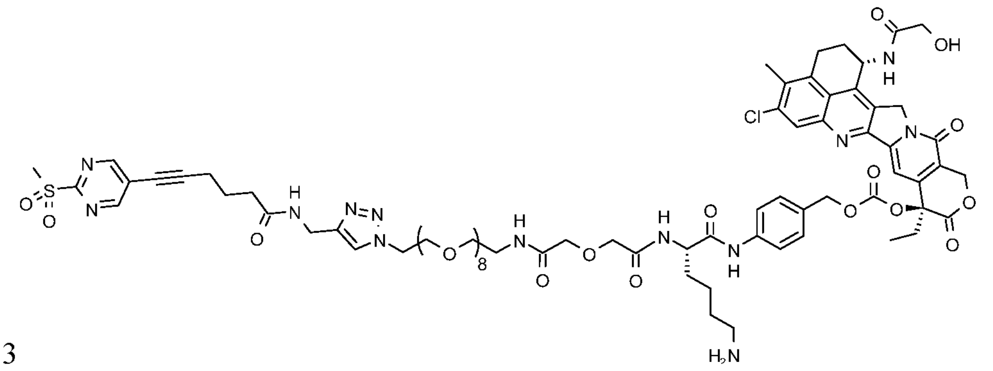

B-03

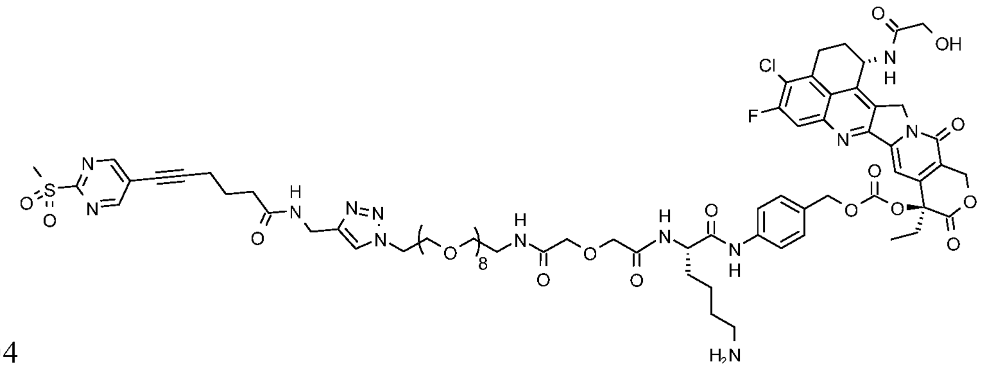

B-04

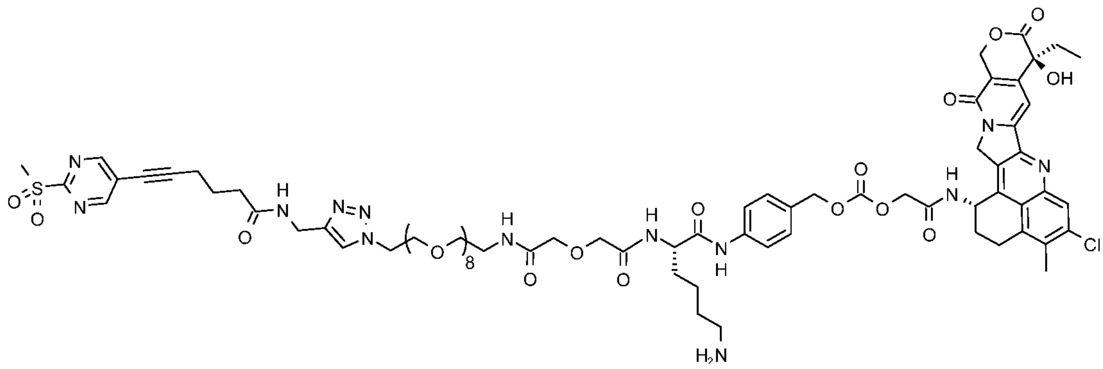

B-06

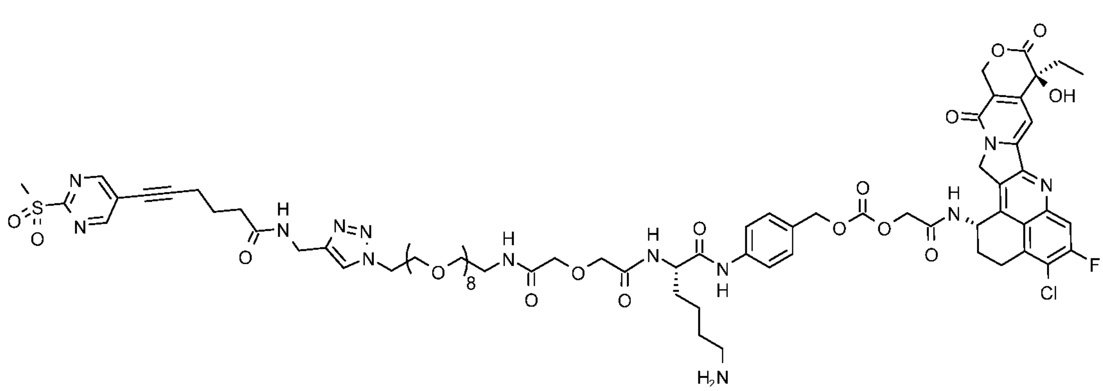

B-07

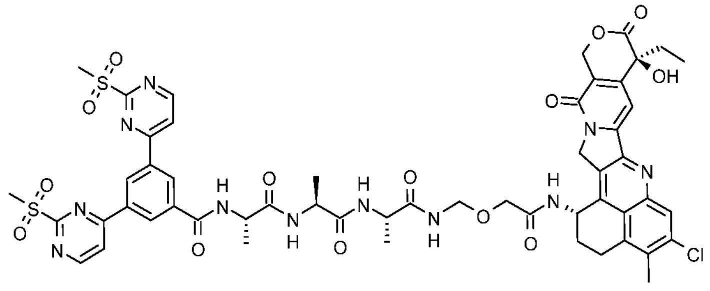

C-02

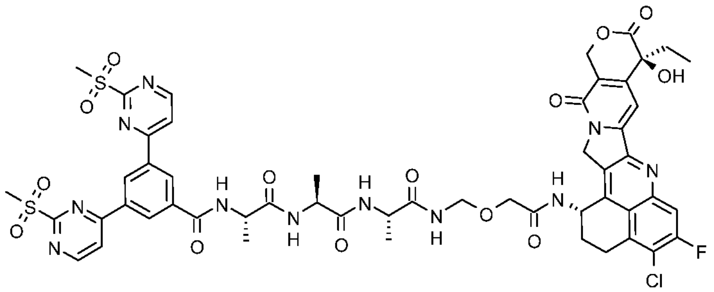

C-03

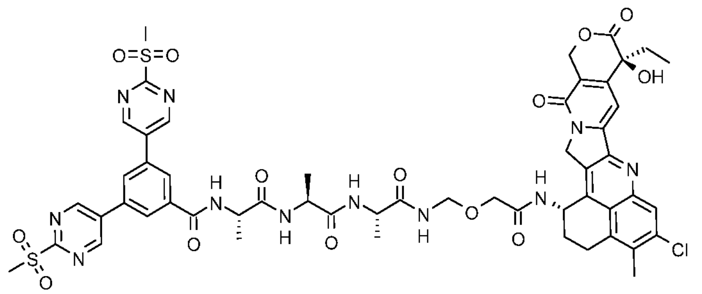

C-05

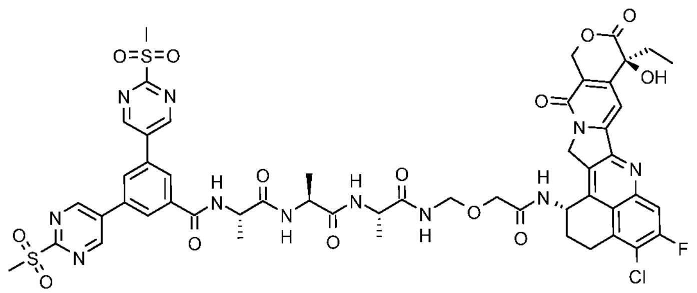

C-06

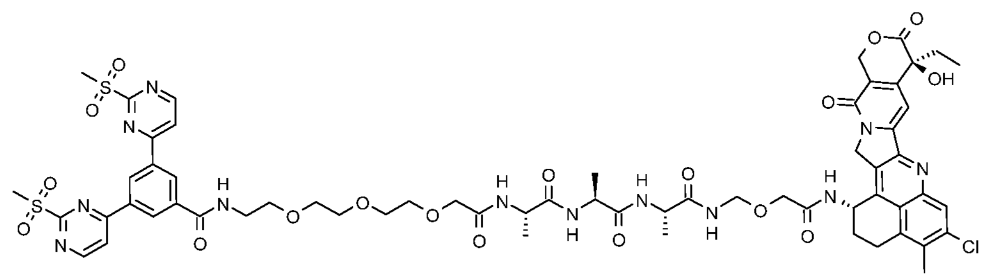

C-08

C-09

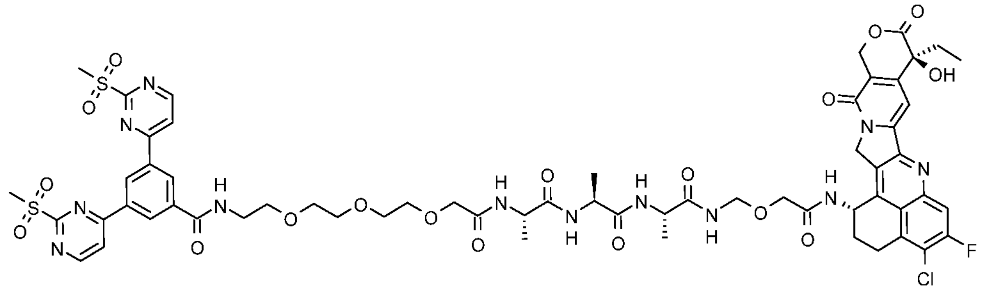

C-11

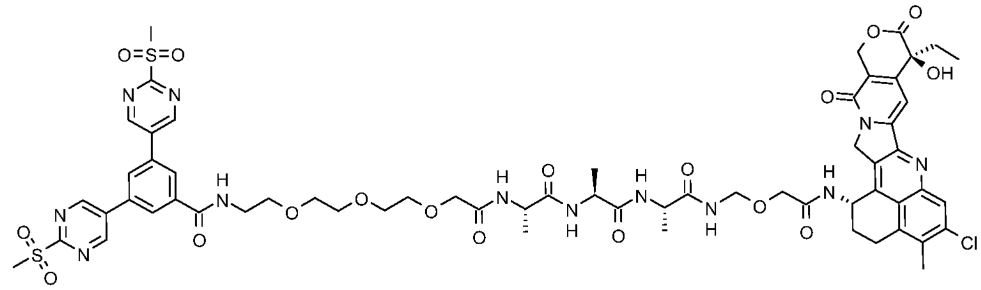

C-12

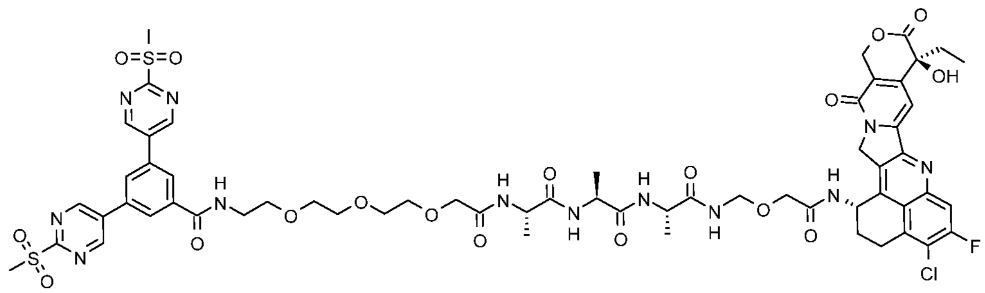

C-14

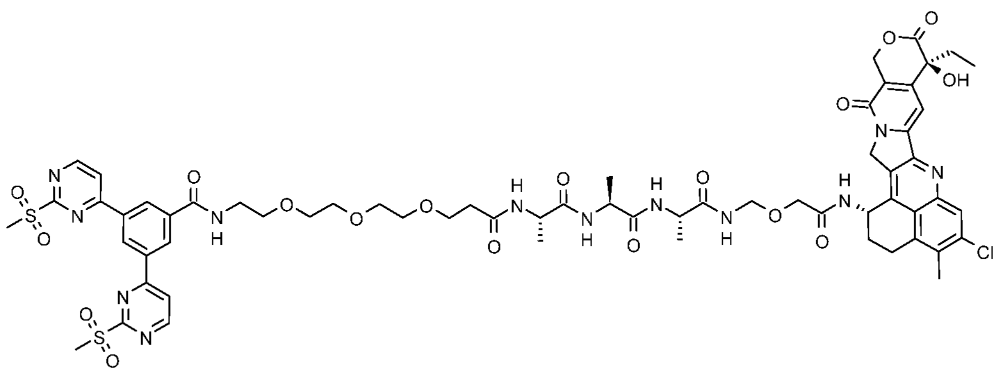

C-15

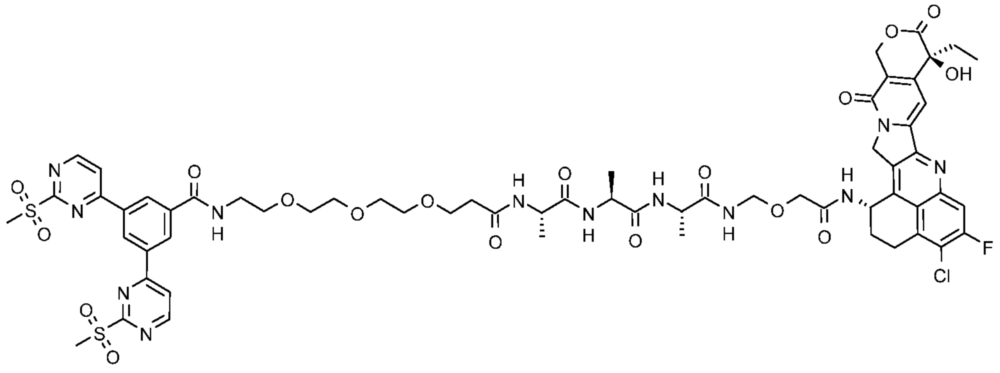

C-17

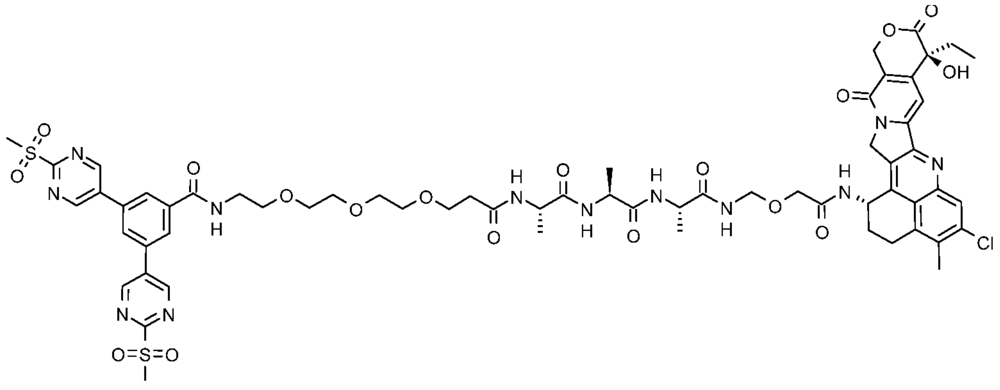

C-18

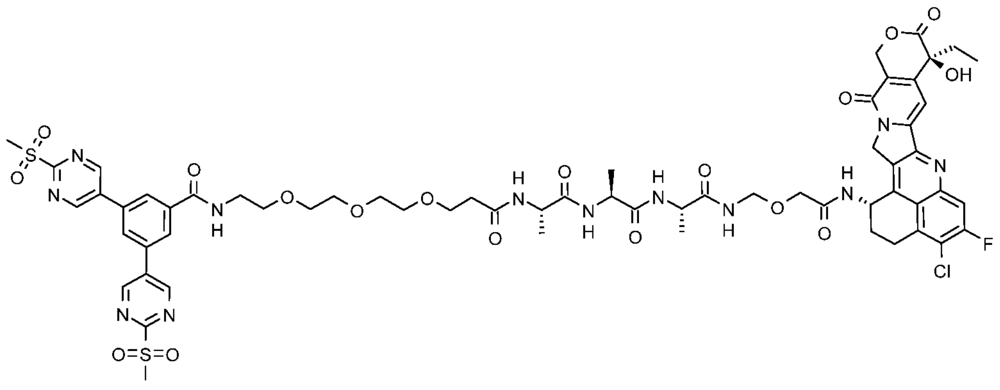

C-19

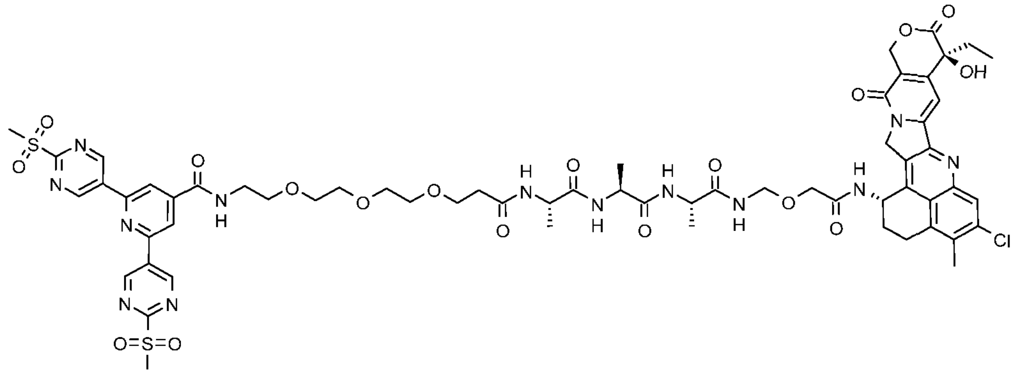

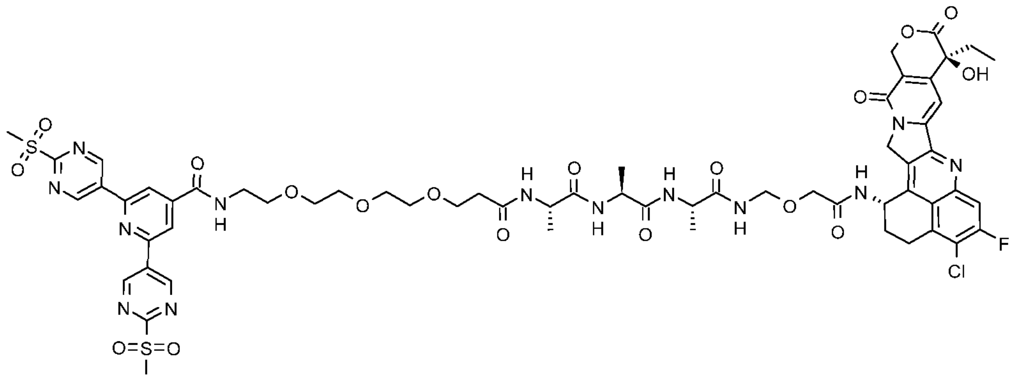

C-20

C-21

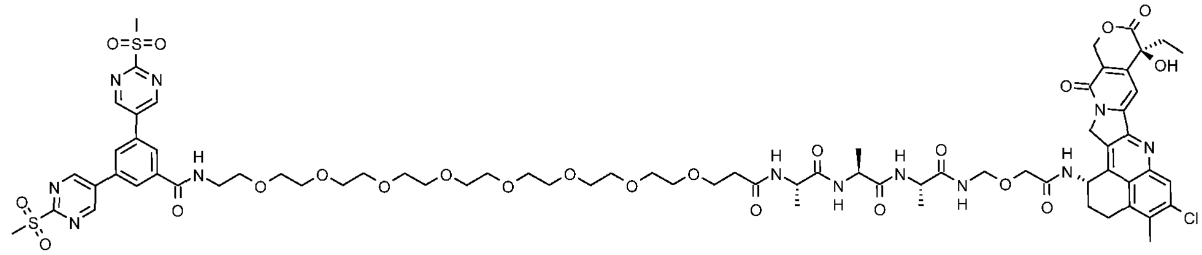

C-22

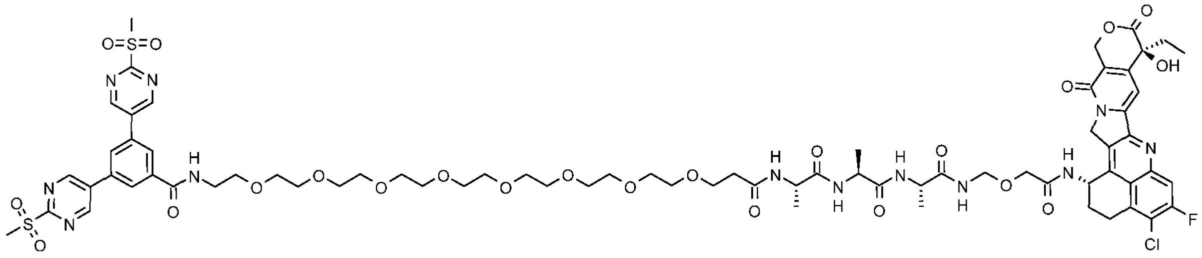

C-23

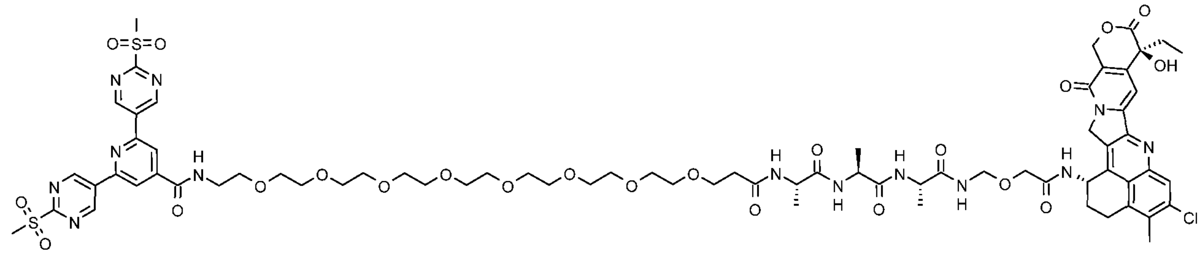

C-24

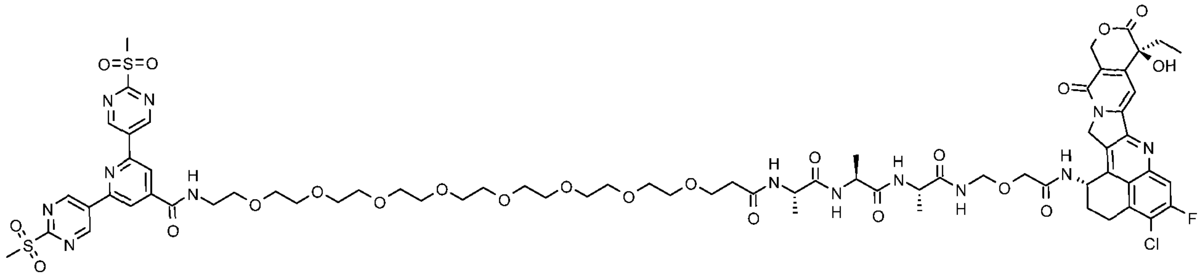

C-25

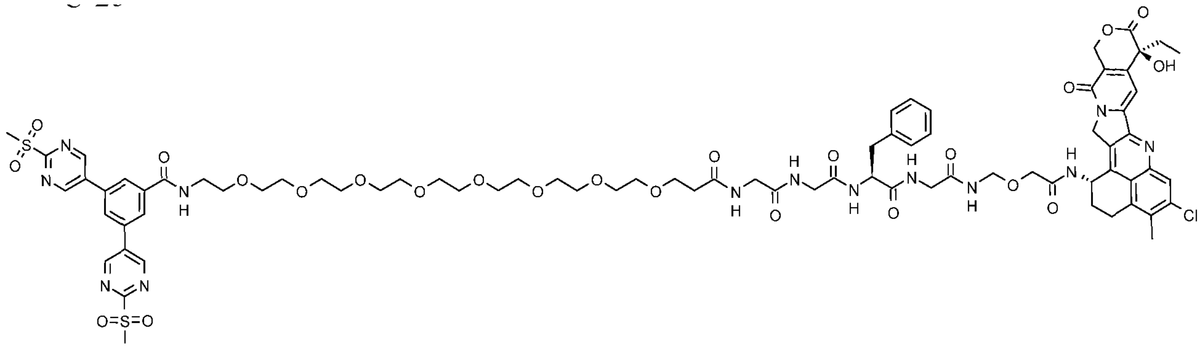

C-26

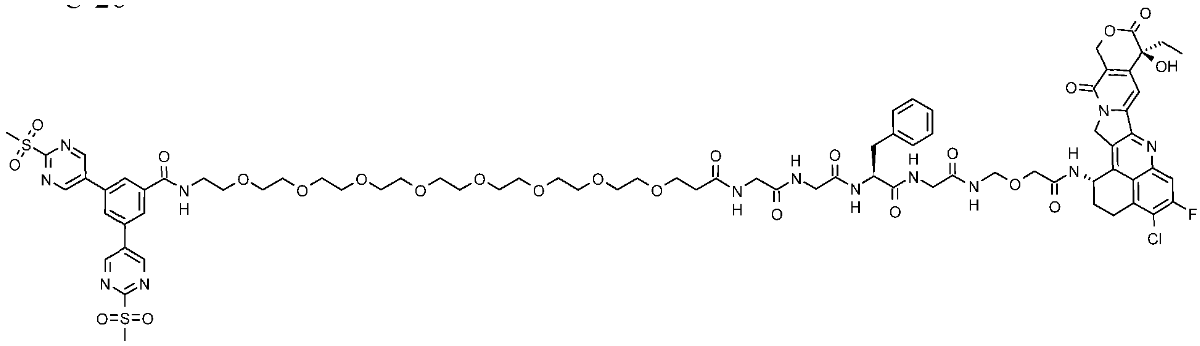

C-27

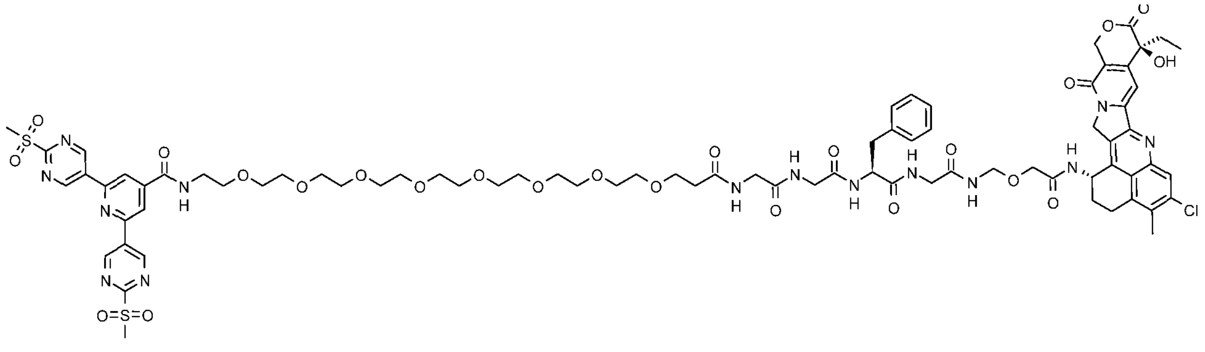

C-28

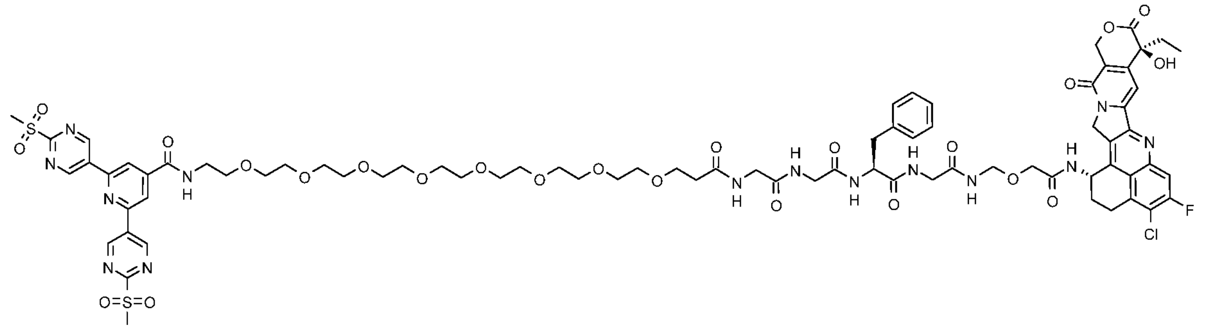

**27.** The drug-linker compound according to any one of claims 1-26, wherein the drug-linker compound may be optionally substituted by one or more suitable substituents.

**28.** The drug-linker compound according to claim 27 having the following structure:

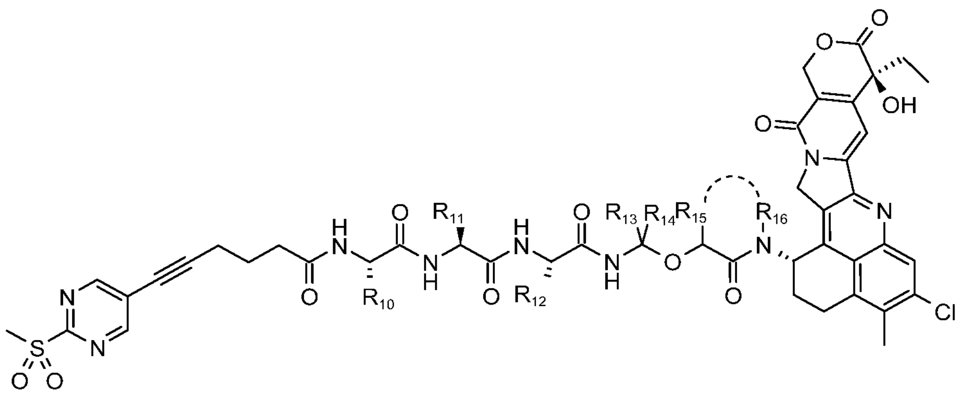

$R_{10}$, $R_{11}$ and $R_{12}$ are each independently selected from the group consisting of H, $C_{1-6}$ alkyl, $C_{3-6}$ cycloalkyl, 5- to 12-membered heterocyclyl, $C_{6-10}$ aryl, 5- to 12-membered heteroaryl, -$C_{1-6}$ alkyl-$C_{6-10}$ aryl and -$C_{1-6}$ alkyl-5- to 12-membered heteroaryl; the alkyl, cycloalkyl, heterocyclyl, aryl and heteroaryl are optionally substituted by one or more substituents selected from the group consisting of hydroxyl, CN, halogen, $C_{1-6}$ alkyl, halo $C_{1-6}$ alkyl, $C_{1-6}$ alkoxy, $C_{6-10}$ aryl, and 5- to 12-membered heteroaryl;

$R_{13}$ and $R_{14}$ are each independently selected from the group consisting of H, $C_{1-6}$ alkyl, $C_{3-6}$ cycloalkyl and 4- to 6-membered heterocyclyl; the alkyl, cycloalkyl and heterocyclyl are optionally substituted by one or more substituents selected from the group consisting of hydroxy, CN, halogen, $C_{1-6}$ alkyl, halo $C_{1-6}$ alkyl, $C_{1-6}$ alkoxy, $C_{6-10}$ aryl and 5- to 12-membered heteroaryl;

$R_{15}$ is selected from the group consisting of H, $C_{1-6}$ alkyl, $C_{3-6}$ cycloalkyl, halo $C_{1-6}$ alkyl, $C_{1-6}$ alkyl-O-$C_{1-6}$ alkyl, $C_{2-6}$ alkenyl, $C_{2-6}$ alkynyl and 3- to 6-membered heterocycloalkyl; $R_{16}$ is H; or, $R_{15}$ and $R_{16}$ and the atoms connected thereto jointly form a 4- to 7-membered ring; the 4- to 7-membered ring is optionally substituted by one or more substituents selected from the group consisting of hydroxyl, CN, halogen, $C_{1-6}$ alkyl, halo $C_{1-6}$ alkyl, $C_{1-6}$

alkoxy, $C_{6-10}$ aryl, and 5- to 12-membered heteroaryl.

**29.** The drug-linker compound according to claim 28, $R_{10}$, $R_{11}$ and $R_{12}$ are each independently selected from the group consisting of H, $C_{1-6}$ alkyl, $C_{3-6}$ cycloalkyl, $C_{6-10}$ aryl, benzyl, hydroxy-substituted benzyl, and indolyl-$C_{1-6}$ alkyl;

$R_{13}$ and $R_{14}$ are each independently selected from the group consisting of H, $C_{1-6}$ alkyl, $C_{3-6}$ cycloalkyl, and 4- to 6-membered heterocyclyl; and

$R_{15}$ is selected from the group consisting of H, $C_{1-6}$ alkyl, $C_{3-6}$ cycloalkyl, halo $C_{1-6}$ alkyl, $C_{1-6}$ alkyl-O-$C_{1-6}$ alkyl, $C_{2-6}$ alkenyl, $C_{2-6}$ alkynyl and 3- to 6-membered heterocycloalkyl; $R_{16}$ is H; or, $R_{15}$ and $R_{16}$ and the atoms connected thereto jointly form a 4- to 7-membered ring.

**30.** Use of the drug-linker compound according to any one of claims 1-29 in preparation of a conjugate (such as an antibody drug conjugate).

**31.** A compound or pharmaceutically acceptable salt thereof having a structure selected from the following:

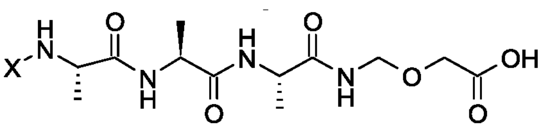

,

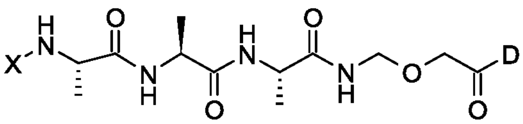

,

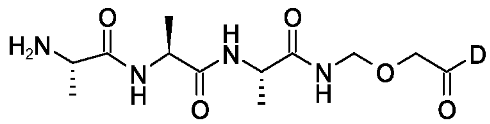

,

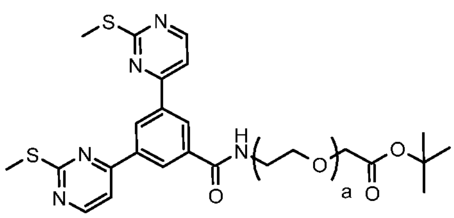

,

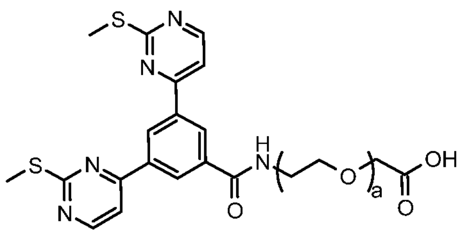

,

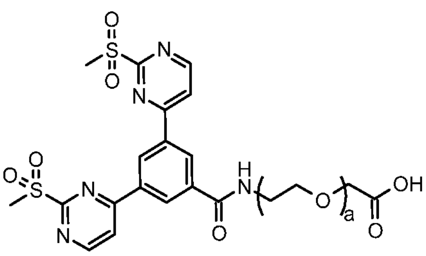

,

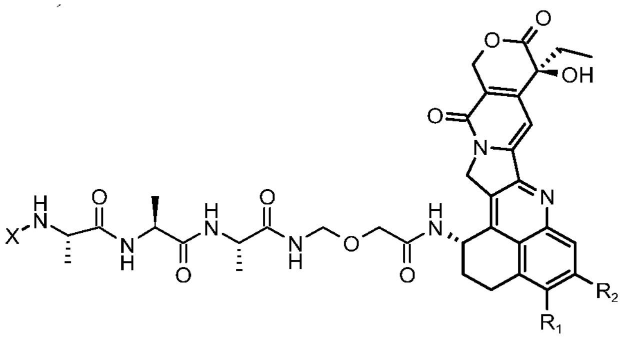

.

, ,

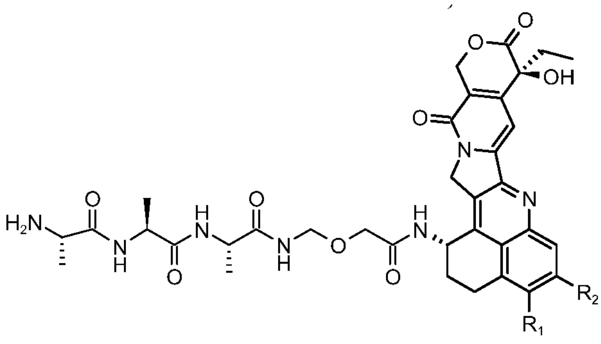
,
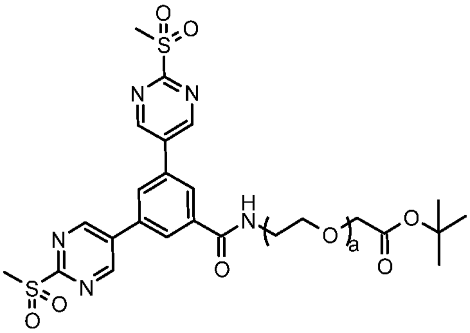
,

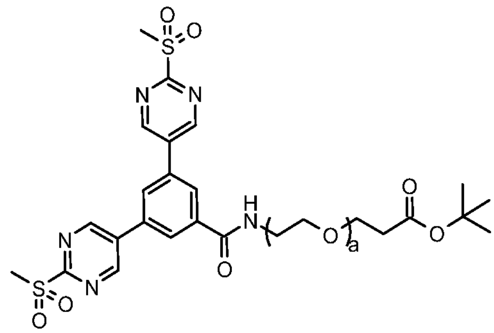
,
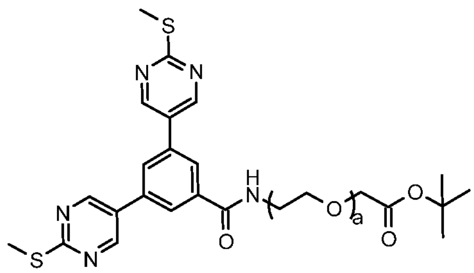
,

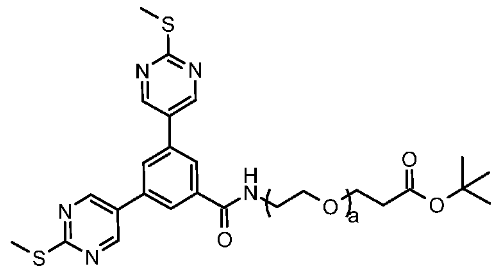
,
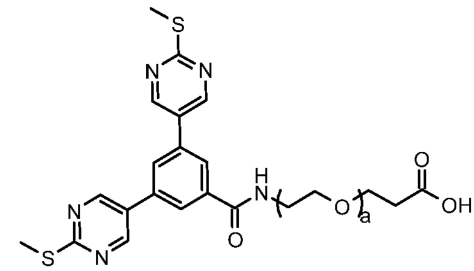
,

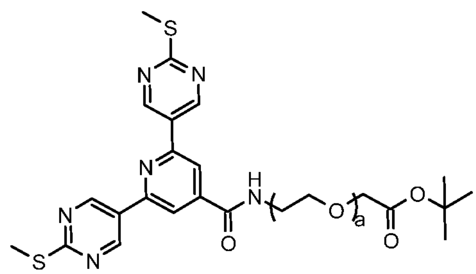
,
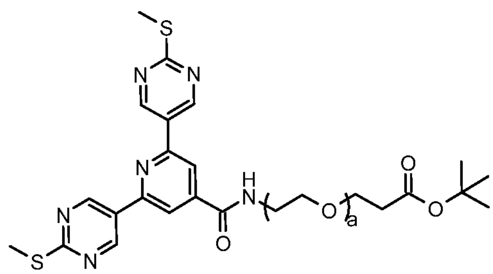
,

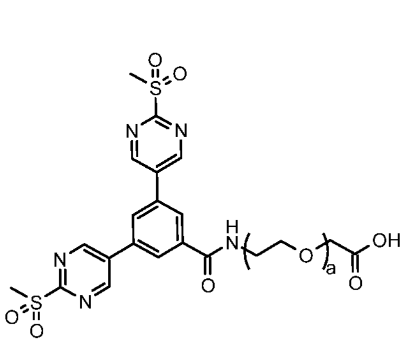
,
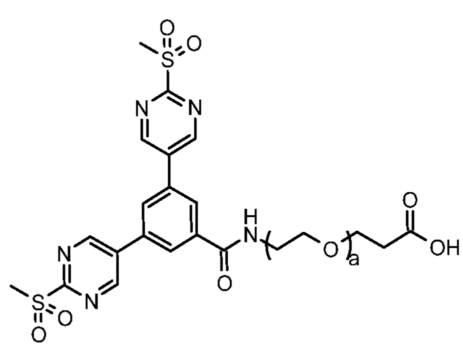
,

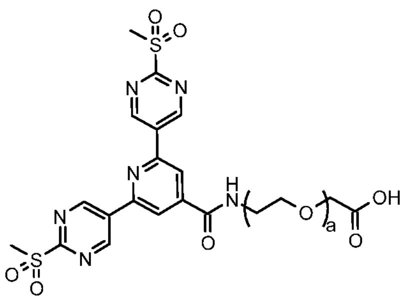,

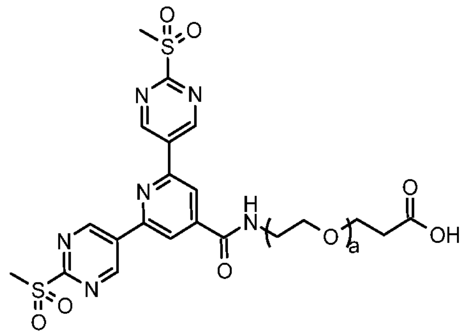,

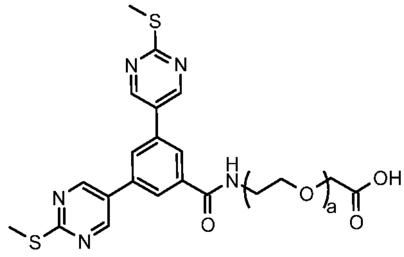,

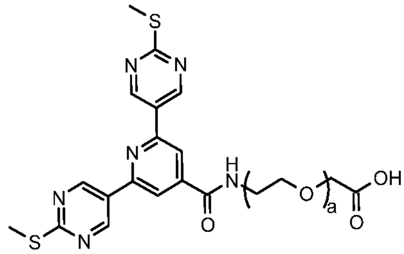,

and

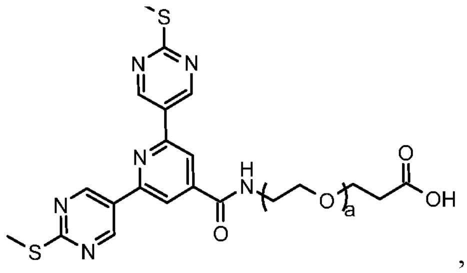,

wherein X is selected from the group consisting of benzyloxycarbonyl, te rt-butyloxycarbonyl, fluorenylmethoxycarbonyl, allyloxycarbonyl, trimethylsilyleth oxycarbonyl, methoxycarbonyl, ethoxycarbonyl, phthaloyl, p-toluenesulfonyl, trifl uoroacetyl, nitrobenzenesulfonyl, benzoyl, pivaloyl, triphenylmethyl, 4-methoxyph enyldiphenylmethyl, dimethoxytrityl, 2,4-dimethoxybenzyl, p-methoxybenzyl and benzyl; a is an integer from 1 to 10, preferably an integer from 3 to 8; $R_1$, $R_2$ and D are as defined in any one of claims 1 or 20-25.

**32.** A compound, having a structure selected from the following:

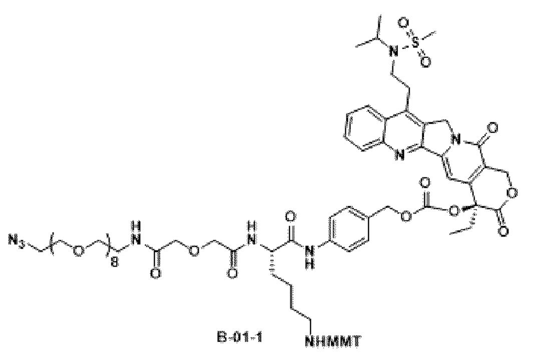

B-01-1

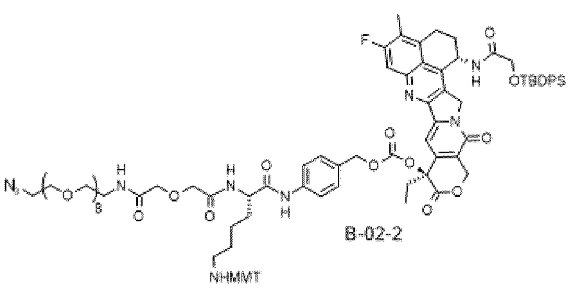

B-02-2

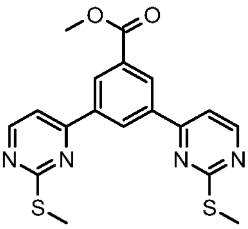

,

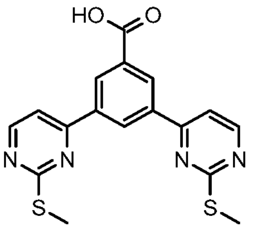

,

,

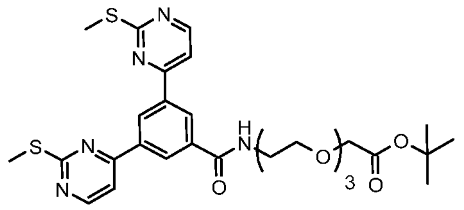

,

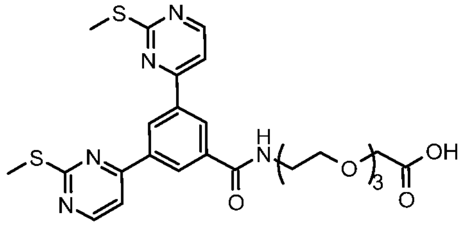

,

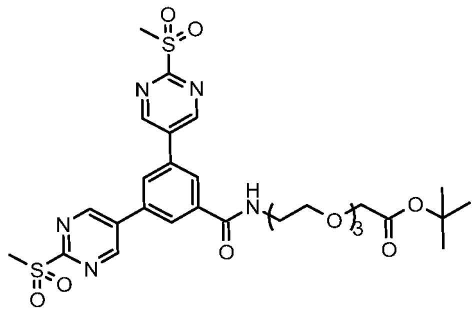

,

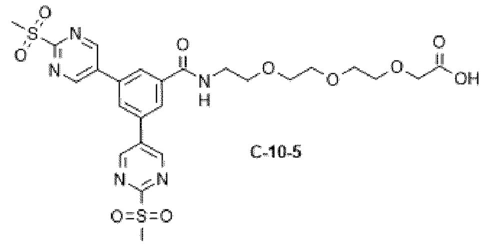

C-10-5

,

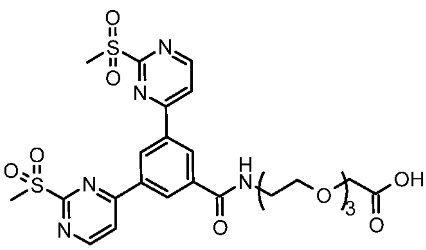

,

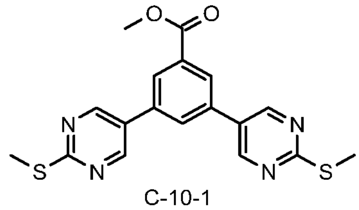

C-10-1

,

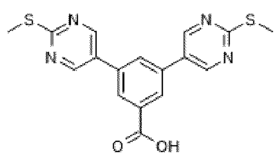

C-10-2

,

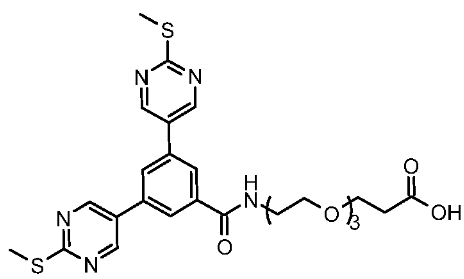

,

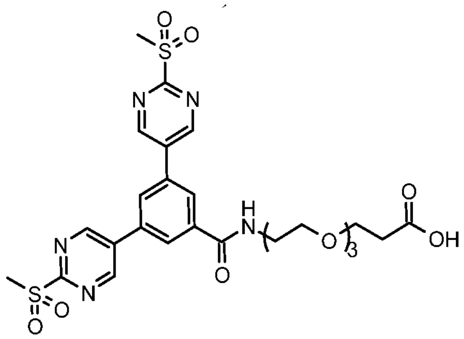

,

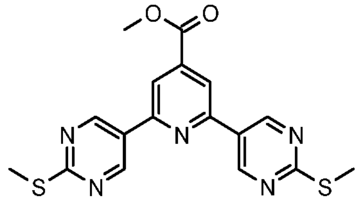,

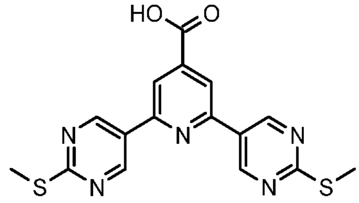,

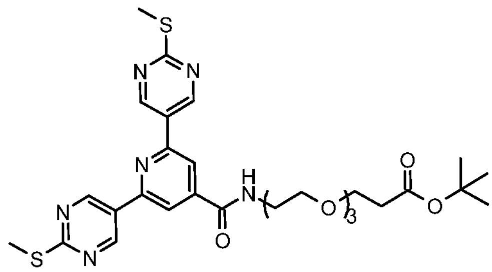,

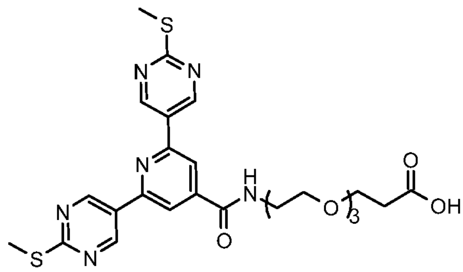,

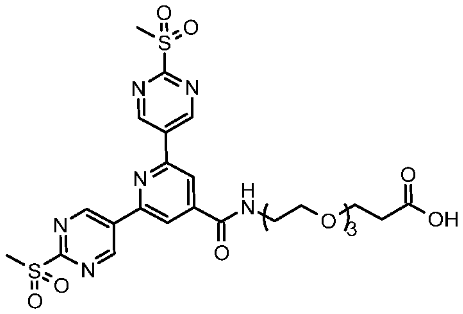,

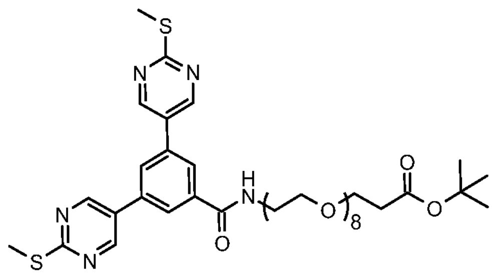,

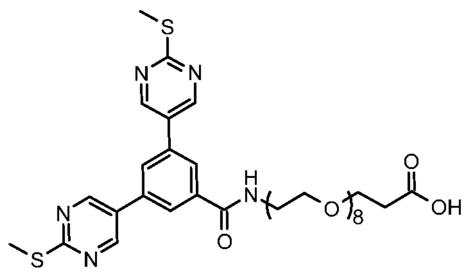,

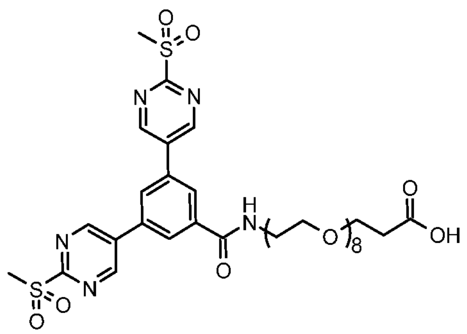,

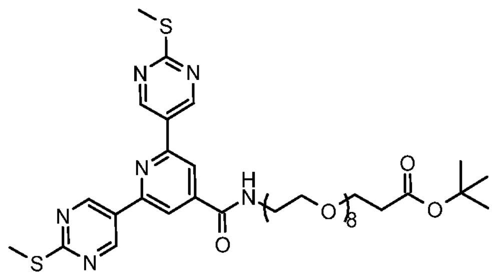,

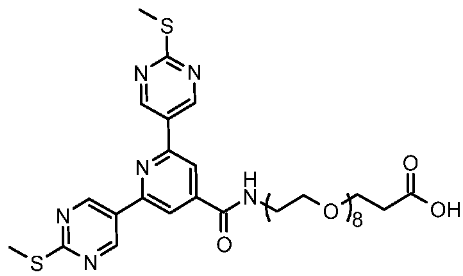,

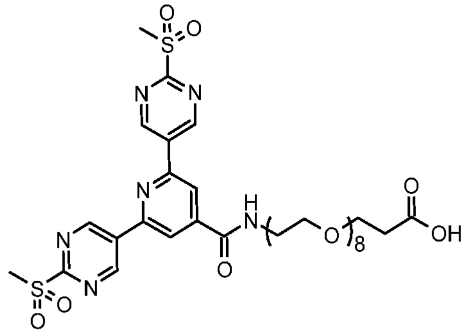

,

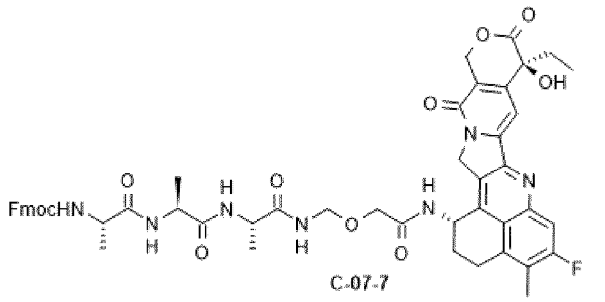

C-07-7

,

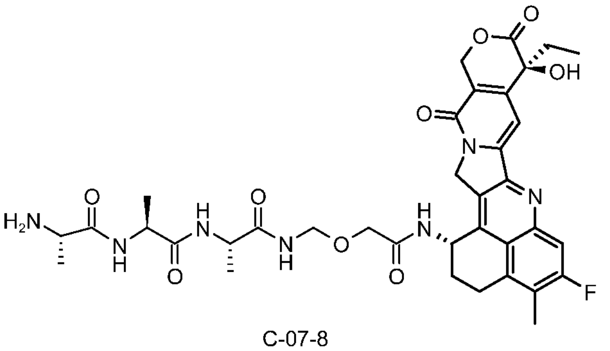

C-07-8

,

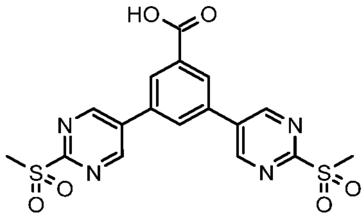

,

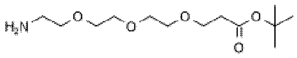

,

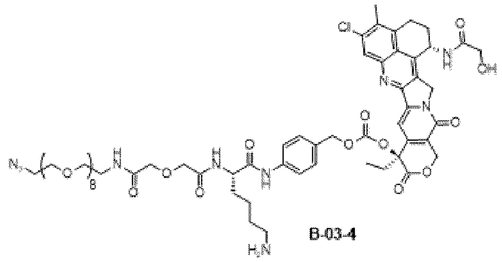

B-03-4

,

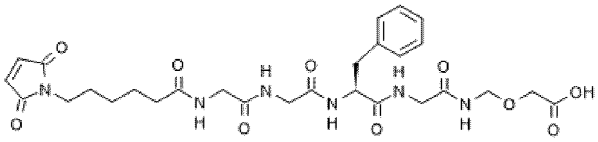

IM-1

,

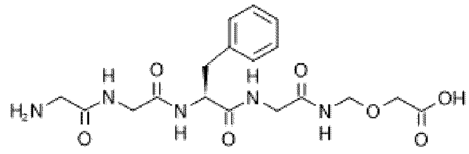

IM-3

,

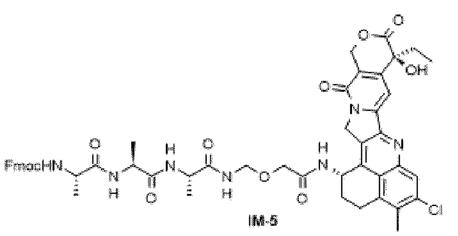

IM-5

,

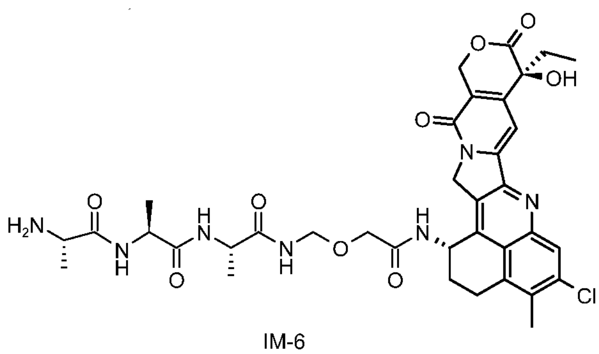

IM-6

,

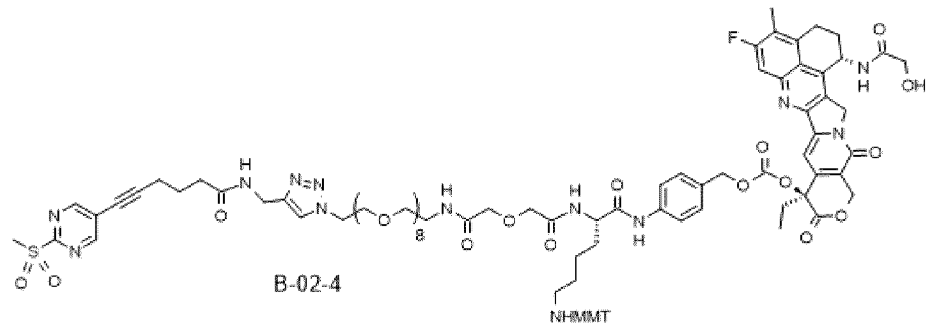

B-02-4

and .

**33.** Use of the compound according to claim 32 in preparation of the drug-linker compound according to any one of claims 1-29.

**34.** A preparation method of a compound, wherein the method comprises a step of deprotecting IM-5-a to obtain a compound of formula IM-6-a.

IM-5-a: 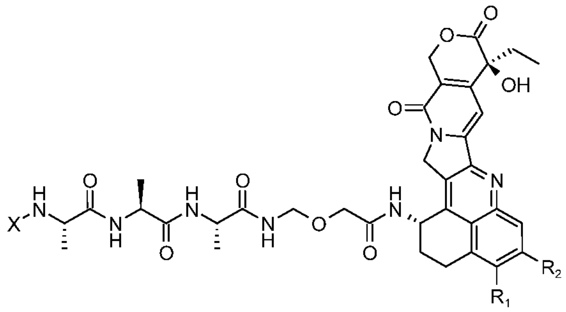

IM-6-a: 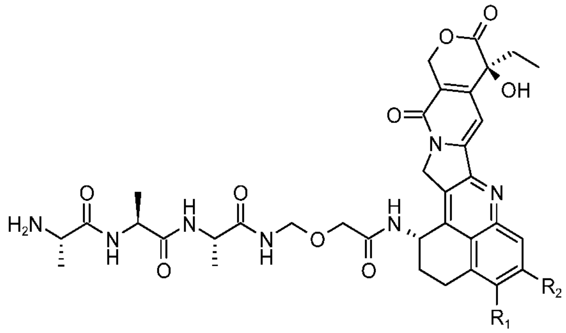

wherein X, $R_1$ and $R_2$ are defined as in claim 31 or 32.

**35.** The preparation method according to claim 34, the deprotection reaction is carried out under one or more of the following conditions:

(1) N,N-dimethylformamide is used as a solvent;
(2) an alkylamine compound is added into reaction system;
(3) the reaction is carried out at room temperature;
(4) the reaction is carried out for 1-5 h.

**36.** The preparation method according to claim 34, the method further comprises a step of reacting IM-6-a with IM-2 to obtain compound A-14-a:

A-14-a: 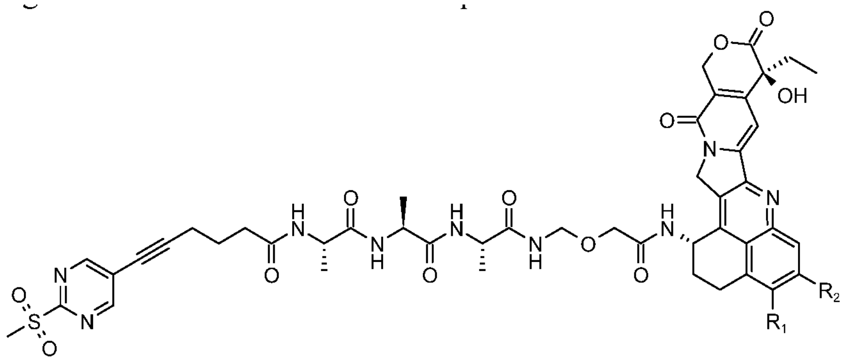 ;

wherein $R_1$ and $R_2$ are as defined in claim 31 or 32.

**37.** The preparation method according to claim 36, wherein the reaction is carried out under one or both of the following conditions:

(1) a solvent for the reaction is selected from the group consisting of N,N-dimethylformamide and N,N-dimethylacetamide;
(2) the reaction further includes a step of adding N,N-diisopropylethylamine.

**38.** A preparation method of a compound, wherein the compound having a structure selected from the following:

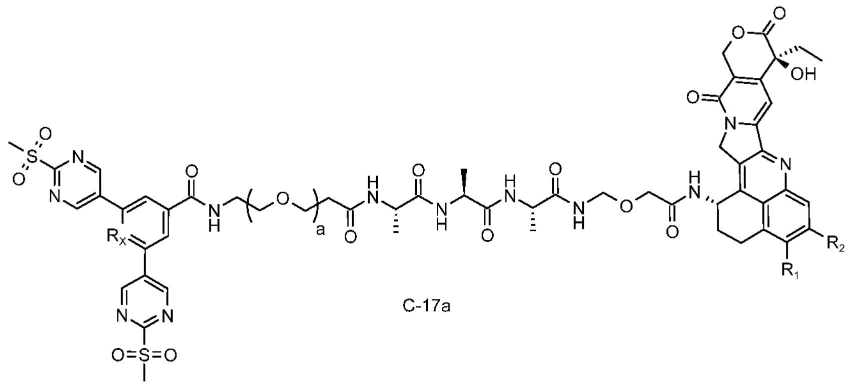

C-17a

,

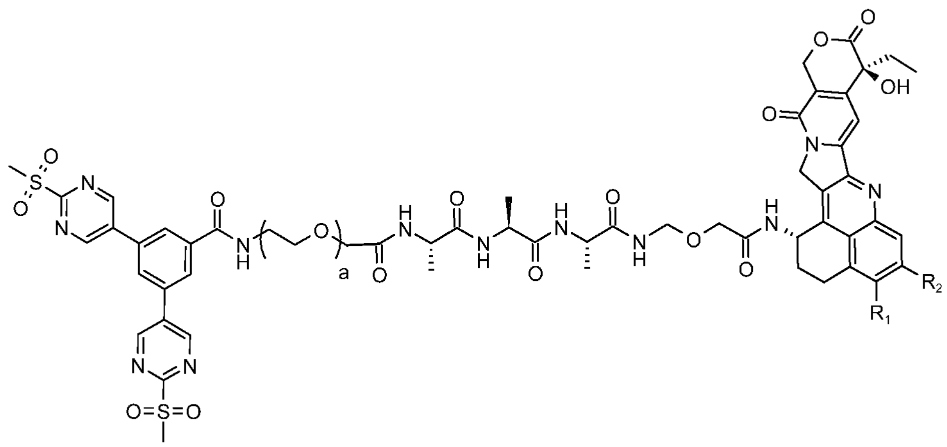

,

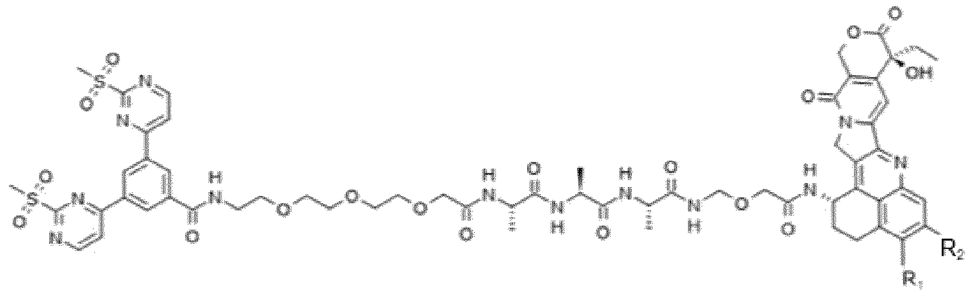

,

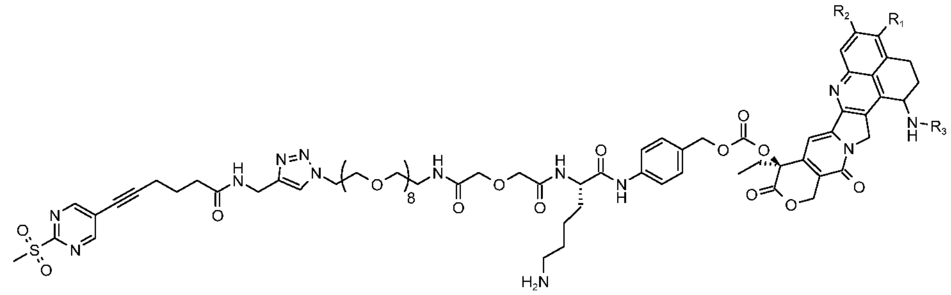

,

or

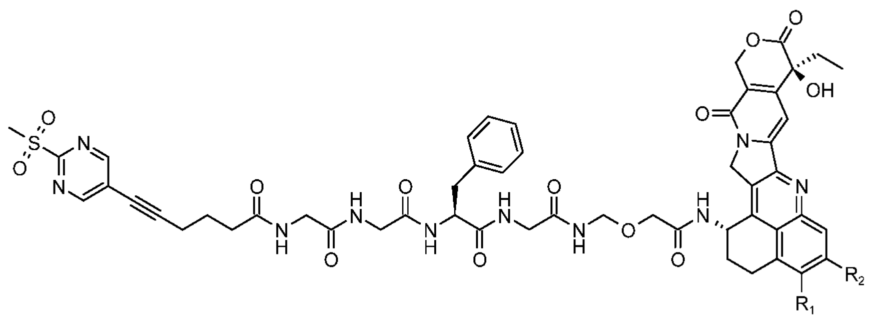

;

wherein $R_1$ and $R_2$ are as defined in claim 31 or 32; a is an integer from 1 to 10; preferably, a is 3 or 8.

## INTERNATIONAL SEARCH REPORT

International application No.
**PCT/CN2023/134038**

**A. CLASSIFICATION OF SUBJECT MATTER**

C07D403/14(2006.01)i；C07D239/24(2006.01)i；A61K47/68(2017.01)i；A61K35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CJFD, CNTXT, DWPI, ENTXTC, WPABSC, CNABS, VEN, CNKI, PUBMED, STN: 抗体, 偶联, 缀合, ADC, 结构检索（权利要求15, 18-19, 26, 31-32, 34, 36, 38）, antibody, conjugat+

**C. DOCUMENTS CONSIDERED TO BE RELEVANT**

| Category* | Citation of document, with indication, where appropriate, of the relevant passages | Relevant to claim No. |
|---|---|---|
| X | WO 2022102695 A1 (DAIICHI SANKYO CO., LTD.) 19 May 2022 (2022-05-19) abstract, and description, paragraphs [0086]-[0087] | 1, 3-5, 9-13, 20-23, 25, 27, 30 |
| A | WO 2022102695 A1 (DAIICHI SANKYO CO., LTD.) 19 May 2022 (2022-05-19) abstract, and description, paragraphs [0086]-[0087] | 2, 6-8, 14-19, 24, 26, 28-29, 31-38 |
| X | WO 2022166762 A1 (SICHUAN KELUN-BIOTECH BIOPHARMACEUTICAL CO., LTD.) 11 August 2022 (2022-08-11) claims 7-9, and description, embodiment 9 | 1-18, 20-30, 32-33 |
| Y | WO 2022166762 A1 (SICHUAN KELUN-BIOTECH BIOPHARMACEUTICAL CO., LTD.) 11 August 2022 (2022-08-11) claims 7-9, and description, embodiment 9 | 36-37 |
| A | WO 2022166762 A1 (SICHUAN KELUN-BIOTECH BIOPHARMACEUTICAL CO., LTD.) 11 August 2022 (2022-08-11) claims 7-9, and description, embodiment 9 | 19, 31, 34-35, 38 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 February 2024** | **01 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2023/134038**

**C. DOCUMENTS CONSIDERED TO BE RELEVANT**

| Category* | Citation of document, with indication, where appropriate, of the relevant passages | Relevant to claim No. |
|---|---|---|
| X | WO 2022253035 A1 (SICHUAN KELUN-BIOTECH BIOPHARMACEUTICAL CO., LTD.; KLUS PHARMA INC.) 08 December 2022 (2022-12-08) abstract, claims 16-17, and description, embodiments 11, 14, 17, and 18 | 1-6, 9-30, 32-33, 38 |
| Y | WO 2022253035 A1 (SICHUAN KELUN-BIOTECH BIOPHARMACEUTICAL CO., LTD.; KLUS PHARMA INC.) 08 December 2022 (2022-12-08) abstract, claims 16-17, and description, embodiments 11, 14, 17, and 18 | 36-37 |
| A | WO 2022253035 A1 (SICHUAN KELUN-BIOTECH BIOPHARMACEUTICAL CO., LTD.; KLUS PHARMA INC.) 08 December 2022 (2022-12-08) abstract, claims 16-17, and description, embodiments 11, 14, 17, and 18 | 5-8, 31, 34-35 |
| X | CN 115160403 A (CHEMVON BIOTECHNOLOGY CO., LTD.) 11 October 2022 (2022-10-11) abstract, claims 1-6, and description, paragraphs [0075]-[0077] | 1, 3-5, 9-17, 20-21, 27, 30, 32-33 |
| Y | CN 115160403 A (CHEMVON BIOTECHNOLOGY CO., LTD.) 11 October 2022 (2022-10-11) abstract, claims 1-6, and description, paragraphs [0075]-[0077] | 36-37 |
| A | CN 115160403 A (CHEMVON BIOTECHNOLOGY CO., LTD.) 11 October 2022 (2022-10-11) abstract, claims 1-6, and description, paragraphs [0075]-[0077] | 2, 4-8, 15-19, 22-26, 28-29, 31, 34-38 |
| X | CN 114929284 A (XYGEN, INC.) 19 August 2022 (2022-08-19) claims 36-62, and description, paragraph [0621] | 1, 3-5, 9-17, 20-21, 27, 30 |
| A | CN 114929284 A (XYGEN, INC.) 19 August 2022 (2022-08-19) claims 36-62, and description, paragraph [0621] | 2, 6-8, 18-19, 22-26, 28-29, 31-38 |
| X | WO 2022170971 A1 (MEDILINK THERAPEUTICS (SUZHOU) CO., LTD.) 18 August 2022 (2022-08-18) claims 25-32 | 1-6, 9-12, 16-18, 20-22, 30, 33 |
| A | WO 2022170971 A1 (MEDILINK THERAPEUTICS (SUZHOU) CO., LTD.) 18 August 2022 (2022-08-18) claims 25-32 | 7-8, 13-15, 19, 23-29, 31-32, 34-38 |
| PX | WO 2023216956 A1 (SICHUAN KELUN-BIOTECH BIOPHARMACEUTICAL CO., LTD.) 16 November 2023 (2023-11-16) claims 1-7, and description, embodiment 10 | 1-6, 9-14, 16-21, 27-31 |
| PA | WO 2023216956 A1 (SICHUAN KELUN-BIOTECH BIOPHARMACEUTICAL CO., LTD.) 16 November 2023 (2023-11-16) claims 1-7, and description, embodiment 10 | 7-8, 15, 22-26, 32-38 |
| PX | WO 2023207710 A1 (SICHUAN KELUN-BIOTECH BIOPHARMACEUTICAL CO., LTD.) 02 November 2023 (2023-11-02) description, pages 139-141, and intermediate preparation example 35 | 34-35 |
| PY | WO 2023207710 A1 (SICHUAN KELUN-BIOTECH BIOPHARMACEUTICAL CO., LTD.) 02 November 2023 (2023-11-02) description, pages 139-141, Intermediate Preparation Example 35 | 36-37 |
| PX | WO 2022253033 A1 (SICHUAN KELUN-BIOTECH BIOPHARMACEUTICAL CO., LTD.) 08 December 2022 (2022-12-08) claims 7 and 8 | 1-4, 7-11, 16-25, 27, 30-33 |
| PA | WO 2022253033 A1 (SICHUAN KELUN-BIOTECH BIOPHARMACEUTICAL CO., LTD.) 08 December 2022 (2022-12-08) claims 7 and 8 | 5-6, 12-15, 26, 28-29, 34-38 |

Form PCT/ISA/210 (second sheet) (July 2022)

**INTERNATIONAL SEARCH REPORT**

International application No.
**PCT/CN2023/134038**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/134038**

| Patent document cited in search report | | | Publication date (day/month/year) | Patent family member(s) | | | Publication date (day/month/year) |
|---|---|---|---|---|---|---|---|
| WO | 2022102695 | A1 | 19 May 2022 | KR | 20230107239 | A | 14 July 2023 |
| | | | | JPWO | 2022102695 | A1 | 19 May 2022 |
| | | | | TW | 202233249 | A | 01 September 2022 |
| | | | | US | 2023398230 | A1 | 14 December 2023 |
| | | | | CA | 3198382 | A1 | 19 May 2022 |
| | | | | EP | 4245322 | A1 | 20 September 2023 |
| | | | | CN | 116615250 | A | 18 August 2023 |
| WO | 2022166762 | A1 | 11 August 2022 | KR | 20230142710 | A | 11 October 2023 |
| | | | | CA | 3209426 | A1 | 11 August 2022 |
| | | | | AU | 2022216696 | A1 | 17 August 2023 |
| | | | | EP | 4289851 | A1 | 13 December 2023 |
| | | | | CN | 116829561 | A | 29 September 2023 |
| WO | 2022253035 | A1 | 08 December 2022 | AU | 2022287001 | A1 | 26 October 2023 |
| | | | | CA | 3218527 | A1 | 08 December 2022 |
| | | | | CN | 117500527 | A | 02 February 2024 |
| CN | 115160403 | A | 11 October 2022 | | None | | |
| CN | 114929284 | A | 19 August 2022 | KR | 20220079606 | A | 13 June 2022 |
| | | | | JP | 2022550851 | A | 05 December 2022 |
| | | | | MX | 2022003930 | A | 04 July 2022 |
| | | | | EP | 4037717 | A1 | 10 August 2022 |
| | | | | IL | 291686 | A | 01 May 2022 |
| | | | | CA | 3152316 | A1 | 08 April 2021 |
| | | | | AU | 2020356955 | A1 | 14 April 2022 |
| | | | | WO | 2021067861 | A1 | 08 April 2021 |
| WO | 2022170971 | A1 | 18 August 2022 | CA | 3206117 | A1 | 18 August 2022 |
| | | | | AU | 2022220512 | A1 | 13 July 2023 |
| | | | | KR | 20230145038 | A | 17 October 2023 |
| | | | | IL | 304804 | A | 01 September 2023 |
| | | | | EP | 4257154 | A1 | 11 October 2023 |
| | | | | TW | 202241521 | A | 01 November 2022 |
| | | | | CN | 115279417 | A | 01 November 2022 |
| WO | 2023216956 | A1 | 16 November 2023 | | None | | |
| WO | 2023207710 | A1 | 02 November 2023 | | None | | |
| WO | 2022253033 | A1 | 08 December 2022 | CN | 117255790 | A | 19 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111936169 A
- CN 111295389 B
- CN 103687945 A
- WO 2022253035 A1


### Non-patent literature cited in the description


- Remington's Pharmaceutical Sciences. 1980
- **KABAT et al.** Sequences of Proteins of Immunological Interest. Public Health Service, National Institutes of Health, 1991
- **CHOTHIA** ; **LESK**. *J. Mol. Biol.*, 1987, vol. 196, 901-917
- **CHOTHIA et al.** *Nature*, 1989, vol. 342, 878-883
- **LEFRANC et al.** *Dev. Comparat. Immunol.*, 2003, vol. 27, 55-77
- **MARTIN ACR** ; **CHEETHAM JC** ; **REES AR**. Modelling antibody hypervariable loops: A combined algorithm.. *Proc Natl Acad Sci USA*, 1989, vol. 86, 9268-9272
- Fundamental Immunology. Raven Press, 1989
- **HOLLIGER et al.** *Nat Biotechnol*, 2005, vol. 23, 1126-1136
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of Monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **HOLT, L. et al.** *Trends in Biotechnology*, 2003, vol. 21 (11), 484-490
- **NEEDLEMAN et al.** *J. Mol. Biol.*, 1970, vol. 48, 443-453
- **E. MEYERS** ; **W. MILLER**. *Comput. Appl Biosci.*, 1988, vol. 4, 11-17
- **NEEDLEMAN** ; **WUNSCH**. *J Mol Biol.*, 1970, vol. 48, 444-453
- **MALMQVIST M**. *Nature*, 1993, vol. 361, 186-187
- **DAVIES et al.** *Annual Rev Biochem*, 1990, vol. 59, 439-473
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991
- Remington's Pharmaceutical Sciences.. Mack Publishing Company, 1995
- **KABAT et al.** Sequences of Proteins of Immunological Interest. Public Health Service, National Institutions of Health, 1991